# EUROPEAN PATENT APPLICATION

(11) **EP 2 639 316 A1**
(43) Date of publication of application: **18.09.2013**
(21) Application number: 13157659.7
(22) Date of filing: 12.05.2008
(51) Int. Cl.: C12Q 1/68

(54) **Biomarkers for melanoma**

(30) Priority: 11.05.2007 US 928813 P; 19.09.2007 US 994416 P; 01.11.2007 US 1457 P
(62) Divisional of application: 08769451.9
(71) Applicant: The Johns Hopkins University, Baltimore, MD 21218 (US)
(72) Inventor: Alani, Rhoda Myra, Baltimore, MD 21208 (US); Byungwoo, Ryu, Columbia, MD 21045 (US); Stine, Megan J., Columbia, MD 21045 (US)
(74) Representative: Hiebl, Inge Elisabeth

(57) **Abstract**

The present invention relates to methods of determining melanoma status in a subject. The invention also relates to kits for determining melanoma status in a subject. The invention further relates to methods of identifying biomarkers and correlating biomarker expression to melanoma status or stage in a subject.

SEQUENCE LISTING

<110> THE JOHNS HOPKINS UNIVERSITY

<120> BIOMARKERS FOR MELANOMA

<130> 20894EP2

<140>
<141> 2008-05-12

<150> 61/001,457
<151> 2007-11-01

<150> 60/994,416
<151> 2007-09-19

<150> 60/928,813
<151> 2007-05-11

<150> 60/846,703
<151> 2006-09-22

<160> 49

<170> PatentIn version 3.5

<210> 1
<211> 20
<212> DNA
<213> Artificial Sequence

<220>
<221> source
<223> /note="Description of Artificial Sequence: Synthetic
      primer"

<400> 1
tggctctctc ccagtagcat                                                   20


<210> 2
<211> 20
<212> DNA
<213> Artificial Sequence

<220>
<221> source
<223> /note="Description of Artificial Sequence: Synthetic
      primer"

<400> 2
tagcactggc ttgtccacag                                                   20


<210> 3
<211> 20
<212> DNA
<213> Artificial Sequence

<220>
<221> source
<223> /note="Description of Artificial Sequence: Synthetic

primer"

<400> 3
cagagagttc atggcgaaca                                                    20


<210> 4
<211> 20
<212> DNA
<213> Artificial Sequence

<220>
<221> source
<223> /note="Description of Artificial Sequence: Synthetic
      primer"

<400> 4
ctcccaaagt gctgggatta                                                    20


<210> 5
<211> 20
<212> DNA
<213> Artificial Sequence

<220>
<221> source
<223> /note="Description of Artificial Sequence: Synthetic
      primer"

<400> 5
tattggtgtg ccctttgtga                                                    20


<210> 6
<211> 20
<212> DNA
<213> Artificial Sequence

<220>
<221> source
<223> /note="Description of Artificial Sequence: Synthetic
      primer"

<400> 6
cagggatatt gggattgtgg                                                    20


<210> 7
<211> 20
<212> DNA
<213> Artificial Sequence

<220>
<221> source
<223> /note="Description of Artificial Sequence: Synthetic
      primer"

<400> 7
   acgacaccct cttggtgttc                                                    20


<210> 8

<211> 20
<212> DNA
<213> Artificial Sequence

<220>
<221> source
<223> /note="Description of Artificial Sequence: Synthetic
      primer"

<400> 8
gtcaaactgc ccacattcct                                                   20


<210> 9
<211> 20
<212> DNA
<213> Artificial Sequence

<220>
<221> source
<223> /note="Description of Artificial Sequence: Synthetic
      primer"

<400> 9
tgacttacga caggctcgtg                                                   20


<210> 10
<211> 20
<212> DNA
<213> Artificial Sequence

<220>
<221> source
<223> /note="Description of Artificial Sequence: Synthetic
      primer"

<400> 10
aaggagtgaa cagggtgtgg                                                   20


<210> 11
<211> 20
<212> DNA
<213> Artificial Sequence

<220>
<221> source
<223> /note="Description of Artificial Sequence: Synthetic
      primer"

<400> 11
caaattgaag cagccagaca                                                   20


<210> 12
<211> 20
<212> DNA
<213> Artificial Sequence

<220>
<221> source
<223> /note="Description of Artificial Sequence: Synthetic

primer"

<400> 12
cagggctttg gagatctgag                                                   20


<210> 13
<211> 20
<212> DNA
<213> Artificial Sequence

<220>
<221> source
<223> /note="Description of Artificial Sequence: Synthetic
      primer"

<400> 13
gaagccatat cgagggatga                                                   20


<210> 14
<211> 20
<212> DNA
<213> Artificial Sequence

<220>
<221> source
<223> /note="Description of Artificial Sequence: Synthetic
      primer"

<400> 14
tgacaagttg tgggcatgtt                                                   20


<210> 15
<211> 20
<212> DNA
<213> Artificial Sequence

<220>
<221> source
<223> /note="Description of Artificial Sequence: Synthetic
      primer"

<400> 15
tgtttgagca tcgcttagga                                                   20


<210> 16
<211> 20
<212> DNA
<213> Artificial Sequence

<220>
<221> source
<223> /note="Description of Artificial Sequence: Synthetic
      primer"

<400> 16
gatctcattg gccatttgct                                                   20


<210> 17

<211> 20
<212> DNA
<213> Artificial Sequence

<220>
<221> source
<223> /note="Description of Artificial Sequence: Synthetic
      primer"

<400> 17
ttgcgcctaa tgtgtttgag                                                   20


<210> 18
<211> 20
<212> DNA
<213> Artificial Sequence

<220>
<221> source
<223> /note="Description of Artificial Sequence: Synthetic
      primer"

<400> 18
atacatttcc ctgccgtcac                                                   20


<210> 19
<211> 20
<212> DNA
<213> Artificial Sequence

<220>
<221> source
<223> /note="Description of Artificial Sequence: Synthetic
      primer"

<400> 19
agggttgcca gatgcaatac                                                   20


<210> 20
<211> 20
<212> DNA
<213> Artificial Sequence

<220>
<221> source
<223> /note="Description of Artificial Sequence: Synthetic
      primer"

<400> 20
agcagactag ggttgccaga                                                   20


<210> 21
<211> 20
<212> DNA
<213> Artificial Sequence

<220>
<221> source
<223> /note="Description of Artificial Sequence: Synthetic

primer"

<400> 21
gctacagcat gcagagcaag                                                    20


<210> 22
<211> 20
<212> DNA
<213> Artificial Sequence

<220>
<221> source
<223> /note="Description of Artificial Sequence: Synthetic
      primer"

<400> 22
aacatgtggt gagcattcca                                                    20


<210> 23
<211> 20
<212> DNA
<213> Artificial Sequence

<220>
<221> source
<223> /note="Description of Artificial Sequence: Synthetic
      primer"

<400> 23
gatcatcagc aatgcctcct                                                    20


<210> 24
<211> 20
<212> DNA
<213> Artificial Sequence

<220>
<221> source
<223> /note="Description of Artificial Sequence: Synthetic
      primer"

<400> 24
ttcagctcag ggatgacctt                                                    20


<210> 25
<211> 19
<212> DNA
<213> Artificial Sequence

<220>
<221> source
<223> /note="Description of Artificial Sequence: Synthetic
      primer"

<400> 25
ggatcccagt gtggtggta                                                     19


<210> 26

<211> 19
<212> DNA
<213> Artificial Sequence

<220>
<221> source
<223> /note="Description of Artificial Sequence: Synthetic
      primer"

<400> 26
ccactgtgct ggcgaattc                                                    19


<210> 27
<211> 18
<212> DNA
<213> Artificial Sequence

<220>
<221> source
<223> /note="Description of Artificial Sequence: Synthetic
      probe"

<400> 27
ccctaaccct aaccctaa                                                     18


<210> 28
<211> 19
<212> DNA
<213> Artificial Sequence

<220>
<221> source
<223> /note="Description of Artificial Sequence: Synthetic
      oligonucleotide"

<400> 28
gagcaagatg tggacttag                                                    19


<210> 29
<211> 19
<212> DNA
<213> Artificial Sequence

<220>
<221> source
<223> /note="Description of Artificial Sequence: Synthetic
      oligonucleotide"

<400> 29
gattcaggtg tagaacgag                                                    19


<210> 30
<211> 20
<212> DNA
<213> Artificial Sequence

<220>
<221> source
<223> /note="Description of Artificial Sequence: Synthetic

primer"

<400> 30
tgcttgaccc tcagacagct                                                   20


<210> 31
<211> 20
<212> DNA
<213> Artificial Sequence

<220>
<221> source
<223> /note="Description of Artificial Sequence: Synthetic
      primer"

<400> 31
gatgggaggc aagttgaaaa                                                   20


<210> 32
<211> 20
<212> DNA
<213> Artificial Sequence

<220>
<221> source
<223> /note="Description of Artificial Sequence: Synthetic
      primer"

<400> 32
gatcatcagc aatgcctcct                                                   20


<210> 33
<211> 20
<212> DNA
<213> Artificial Sequence

<220>
<221> source
<223> /note="Description of Artificial Sequence: Synthetic
      primer"

<400> 33
ttcagctcag ggatgacctt                                                   20


<210> 34
<211> 10
<212> DNA
<213> Artificial Sequence

<220>
<221> source
<223> /note="Description of Artificial Sequence: Synthetic
      oligonucleotide"

<400> 34
gggccttccc                                                              10


<210> 35

<211> 10
<212> DNA
<213> Artificial Sequence

<220>
<221> source
<223> /note="Description of Artificial Sequence: Synthetic
      oligonucleotide"

<400> 35
agaaatttcc                                                            10


<210> 36
<211> 10
<212> DNA
<213> Artificial Sequence

<220>
<221> source
<223> /note="Description of Artificial Sequence: Synthetic
      oligonucleotide"

<400> 36
gtggatttcc                                                            10


<210> 37
<211> 10
<212> DNA
<213> Artificial Sequence

<220>
<221> source
<223> /note="Description of Artificial Sequence: Synthetic
      oligonucleotide"

<400> 37
gaacattccc                                                            10


<210> 38
<211> 10
<212> DNA
<213> Artificial Sequence

<220>
<221> source
<223> /note="Description of Artificial Sequence: Synthetic
      oligonucleotide"

<400> 38
gggatttttc                                                            10


<210> 39
<211> 10
<212> DNA
<213> Artificial Sequence

<220>
<221> source
<223> /note="Description of Artificial Sequence: Synthetic

oligonucleotide"

<400> 39
ggggctttcc                                                              10


<210> 40
<211> 10
<212> DNA
<213> Artificial Sequence

<220>
<221> source
<223> /note="Description of Artificial Sequence: Synthetic
      oligonucleotide"

<400> 40
gggaaattcc                                                              10


<210> 41
<211> 10
<212> DNA
<213> Artificial Sequence

<220>
<221> source
<223> /note="Description of Artificial Sequence: Synthetic
      oligonucleotide"

<400> 41
ggcaattttc                                                              10


<210> 42
<211> 10
<212> DNA
<213> Artificial Sequence

<220>
<221> source
<223> /note="Description of Artificial Sequence: Synthetic
      oligonucleotide"

<400> 42
gaaaattgcc                                                              10


<210> 43
<211> 11
<212> DNA
<213> Artificial Sequence

<220>
<221> source
<223> /note="Description of Artificial Sequence: Synthetic
      oligonucleotide"

<400> 43
ggaaagtctc c                                                            11


<210> 44

<211> 4
<212> PRT
<213> Artificial Sequence

<220>
<221> source
<223> /note="Description of Artificial Sequence: Synthetic
      peptide"

<400> 44
Tyr Arg Pro Trp
1


<210> 45
<211> 4
<212> PRT
<213> Artificial Sequence

<220>
<221> source
<223> /note="Description of Artificial Sequence: Synthetic
      peptide"

<400> 45
Asp Glu Ala Asp
1


<210> 46
<211> 4
<212> PRT
<213> Artificial Sequence

<220>
<221> source
<223> /note="Description of Artificial Sequence: Synthetic
      peptide"

<400> 46
Asp Glu Ala His
1


<210> 47
<211> 4
<212> PRT
<213> Artificial Sequence

<220>
<221> source
<223> /note="Description of Artificial Sequence: Synthetic
      peptide"

<400> 47
Cys Cys Cys His
1


<210> 48
<211> 16
<212> PRT
<213> Artificial Sequence

<220>
<221> source
<223> /note="Description of Artificial Sequence: Synthetic
      peptide"

<400> 48
Cys Cys Cys His Cys Cys Cys Cys Cys Cys Cys His Cys Cys Cys Cys
1               5                   10                  15


<210> 49
<211> 4
<212> PRT
<213> Artificial Sequence

<220>
<221> source
<223> /note="Description of Artificial Sequence: Synthetic
      peptide"

<400> 49
Pro Trp Trp Pro
1

## Description

### RELATED APPLICATIONS

This application claims priority from U.S. Provisional Application No. 60/928,813, filed May 11, 2007, U.S. Provisional Application No. 60/846,703, filed September 22, 2006, U.S. Provisional Application No. 60/994,416 filed September 19, 2007, and U.S. Provisional Application No. 61/001,457, filed November 1, 2007, all of which are incorporated herein by reference in their entireties.

### BACKGROUND

The incidence of melanoma is increasing at one of the highest rates for any form of cancer in the United States (Jemal A, Siegel R, Ward E, Murray T, XuJ, et al. (2006) Cancer statistics, 2006. CA CancerJ Clin 56: 106-130), with a current lifetime risk of 1 in 58. In the United States in 2008, over 60,000 patients are expected to be diagnosed with melanoma with more than 8,000 deaths. There are currently no effective systemic therapies for late stage disease, and the average lifetime expectancy for patients with advanced melanoma is 6 - 9 months. Moreover, diagnosis of melanoma can be difficult as there is histological overlap between benign and malignant lesions which can lead to both over and under diagnosis. At present, there are no systemic agents available that significantly extend the lifespan of patients with advanced disease, and the key to improved survival in all affected individuals remains early diagnosis and treatment. While early stage disease may result in occasional deaths, there are no available tests to predict which early stage tumors have a high likelihood of progression and therefore a worse prognosis. Thus, an urgent need exists for the identification of molecular signatures of melanoma progression which can be used to develop accurate prognostic markers and effective targeted therapies.

Multiple studies have shown that there is a high rate of discordance when pathologic specimens of melanocyfic lesions are reviewed by multiple pathologists. These changes in diagnosis can have implications in clinical management in up to 40% of patients who may require further surgical procedures, adjuvant therapy or who may not have needed aggressive surgery. This underlines the importance of defining additional tests that may assist in making a histological diagnosis. In addition, identification of independent predictors of melanoma outcome would allow for identification of patients most at-risk for developing invasive disease and therefore most in need of aggressive early treatment.

Current microarray technologies and the sequencing of the human genome have significantly enhanced the potential for investigations in all fields and particularly in the area of melanoma research. High-thraughput gene expression profiling technologies offer an opportunity to uncover critical molecular events in the development and progression of human melanoma and can be used to design improved prognostic testing and effective treatment strategies. Previous transcriptome analyses in other malignancies have provided valuable information for the assessment of patient group classifications such as subgroups of patients that are likely to respond to a particular therapy (Sondak, V-K. Adjuvant therapy for melanoma. Cancer J7 Suppl 1, S24-7. (2001)). Expression profiling of metastatic melanomas was able to identify previously unrecognized subtypes of disease and predict phenotypic characteristics which may be of importance to melanoma progression (Bittner, M. et al. Molecular classification of cutaneous malignant melanoma by gene expression profiling. Nature 406, 536-40 (2000)). Further studies using serial analysis of gene expression (SAGE) and cDNA arrays have yielded the identification of additional novel molecules and pathways which may be involved in melanoma development (Su, Y.A. et al. Identification of tumor-suppressor genes using human melanoma cell lines UACC903, UACC903(+6), and SRS3 by comparison of expression profiles. Mol Carcinog 28, 119-27 (2000); Satyamoorthy, K. et al. Melanoma cell lines from different stages of progression and their biological and molecular analyses. Melanoma Res 7 Suppl 2, S35-42 (1997); Polsky, D., et al. The transcriptional repressor of p16/Ink4a, Idl, is upregulated in early melanomas. Cancer Res 61, 6008-11 (2001); Furuse, S. et al. Serum concentrations of the CXC chemokines interleukin 8 and growth regulated oncogene-alpha are elevated in patients with systemic sclerosis. J Rheumatol 30, 1524-8 (2003)). Such studies have been limited in utility due to the lack of concordance from one study to the next suggesting tumor heterogeneity. To date, melanoma research has been hampered by the lack of accessible tissue specimens for study and tools for broad-scope genetic analysis. This shortcoming of tissue availability has largely restricted gene expression profiling studies in melanoma to the use of small numbers of established tumor cell lines and cases of metastatic disease.

Thus, there is a need in the art for a more thorough understanding of the molecular defects associated with this malignancy and a need for accurate and early diagnosis of melanoma. In present clinical practice, for example, screening for melanoma is based on clinical examination. Current methods for detection, diagnosis, prognosis, and treatment of melanoma, fails to satisfactorily reduce the morbidity associated with the disease. There is thus a need in the art for further reduction of mortality rates, and early melanoma detection in minimally invasive, cost efficient formats.

### SUMMARY

The present invention provides, for the first time, novel biomarkers that are differentially present in samples of melanoma subjects and in the samples of control subjects, and that can be correlated to certain stages or steps of melanoma progression. The measurement of these markers alone or in combination, in subject samples provides information that a diagnostician can not only use to determine a probable diagnosis of melanoma or a negative diagnosis (e.g., normal or disease-free), but the measurement of these markers can be used to determine the course of disease progression, to determine the risk of recurrence of disease, the propensity of an individual to develop melanoma or to have recurrent melanoma, or to provide information about the stage of melanoma present. The present invention also provides sensitive and quick methods and kits that are useful for determining the melanoma status or stage by measuring these novel markers. In particular, the novel biomarkers can be of use in diagnostic assays, for example a blood test that will be available to screen for disease onset/progression in subjects with a known history of melanoma and those at risk for melanoma, such as those subjects with multiple nevi, patients with a family history of melanoma, patients with a dysplastic nevi, and patients with a history of significant sun exposure.

In a first aspect, the invention provides a biomarker for melanoma status comprising one or more of a marker identified in any one of Tables 1 - 8, or combinations thereof, wherein the biomarker is correlated with melanoma progression.

In a first embodiment, the melanoma is one or more of in situ, radial growth phase, vertical growth phase, and metastatic melanoma.

In another embodiment, the biomarker is further used to determine the malignant potential of a melanocytic tumor of undetermined malignant potential, or a tumor of undetermined classification.

In a related embodiment, melanoma progression is further defined as a stage selected from the group consisting of: stage I, stage II, stage III and stage IV.

In another related embodiment, the marker corresponds to a gene involved in a biological process selected from the group consisting of: cell adhesion, regulation of cell-cell adhesion, cell differentiation, negative regulation of cell differentiation, DNA replication initiation, regulation of apoptosis, angiogenesis, regulation of cell migration, cell proliferation, extracellular matrix organization and intracellular junction organization or maintenance.

In certain embodiments, the melanoma is recurrent

In another aspect, the invention features a biomarker for melanoma status comprising one or more of a marker identified in Table 2 or Table 8, or combinations thereof, wherein the biomarker is correlated with a stage melanoma progression.

In one embodiment, the biomarker is associated with aggressive melanoma.

In another aspect, the invention features a biomarker for melanoma status comprising one or more of a marker identified in Table 3, or combinations thereof, wherein the biomarker is correlated with a stage of melanoma progression.

In one embodiment, the biomarker is associated with less- aggressive melanoma.

In still another aspect, the invention features a biomarker for melanoma status comprising one or more of a marker identified in Table 4, or combinations thereof, wherein the biomarker is correlated with metastatic melanoma.

In one embodiment, the marker or combinations thereof are down-regulated. In a further embodiment, the downregulation is conelated with metastatic melanoma.

In another aspect, the invention features a biomarker for melanoma status comprising one or more of a marker identified in Table 5, or combinations thereof, wherein the biomarker is correlated with invasive or metastatic melanoma.

In one particular embodiment, the markers or combinations thereof are overexpressed, and the overexpression is correlated with invasive or metastatic melanoma. In another embodiment, the overexpression is correlated with invasive or metastatic melanoma.

In another aspect the invention features a biomarker for melanoma status comprising one or more of a marker identified in Table 6, or combinations thereof, wherein the biomarker is correlated with a stage of melanoma progression.

In one embodiment, the markers or combinations thereof are overexpressed. In another embodiment, the overexpression is correlated with invasive or metastatic melanoma. In a particular embodiment, the marker corresponds to neuropilin (NRP-2).

In another aspect, the invention features a biomarker for melanoma status comprising one or more of a marker identified in Table 7 or Table 8, or combinations thereof, wherein the biomarker is upregulated in primary melanocytes.

In a particular embodiment, the markers or combinations thereof are overexpressed in primary melanocytes. In a further embodiment, the overexpression is correlated with melanocyte proliferation. In another further embodiment, the marker is selected from the group consisting of: matrix metalloproteinase-1 (MMP-1), SerpinB2, amphiregulin, CXCL5, IL-8, RAP la and epiregulin.

In another aspect, the invention features a method of qualifying melanoma status in a subject comprising (a) measuring at least one biomarker in a sample from the subject, wherein the biomarker is selected from one or more of the biomarkers identified in any one of Tables 1 - 8, or combinations thereof; and (b) correlating the measurement with melanoma status, thereby qualifying melanoma status in a subject.

In another aspect, the invention features a method of predicting the recurrence of melanoma in a subject comprising (a) measuring at least one biomarker in a sample from the subject, wherein the biomarker is selected from one or more of the biomarkers identified in any one of Tables 1 - 8, or combinations thereof; and (b) correlating the measurement with the risk melanoma recurrence, thereby predicting the recurrence of melanoma in a subject.

In one embodiment, the detection of NRP-2 is predictive of disease recurrence, In a further embodiment, the detection of MMP-1 is predictive of disease recurrence.

In another aspect, the invention features a method of identifying a risk of developing melanoma in a subject comprising (a) measuring at least one biomarker in a sample from the subject, wherein the biomarker is selected from one or more of the biomarkers identified in any one of Tables 1 - 8, or combinations thereof; and (b) correlating the measurement with a risk of developing melanoma, thereby identifying a risk of developing melanoma in a subject.

In another aspect, the invention features a method of detecting or diagnosing melanoma in a subject comprising (a) measuring at least one biomarker in a sample from the subject, wherein the biomarker is selected from one or more of the biomarkers identified in any one of Tables 1 - 8, or combinations thereof; and (b) correlating the measurement with the presence of melanoma, thereby detecting or diagnosing melanoma in a subject.

In still another aspect, the invention features a method of determining the prognosis of a subject suffering from melanoma comprising: (a) measuring at least one biomarker in a sample from the subject, wherein the biomarker is selected from one or more of the markers identified in any one of Tables 1 - 8, or combinations thereof; and (b) correlating the measurement with prognosis, thereby determining the prognosis of a subject suffering from melanoma.

In one embodiment, the prognosis determines course of treatment.

In another embodiment of any one of the above aspects, the biomarker is selected from one or more markers identified in Table 2 or Table 8, or combinations thereof.

In another embodiment of any one of the above aspects, the biomarker is selected from one of more markers identified in Table 4, or combinations thereof.

In another embodiment of any one of the above aspects, the biomarker is selected from one or more markers identified in Table 5, or combinations thereof.

In still another embodiment of any one of the above aspects, the biomarker is selected from one or more markers identified in Table 6, or combinations thereof.

In still another embodiment of any one of the above aspects, the biomarker is selected from one or more markers identified in Table 7, 8 or 9.

In a particular embodiment of any one of the above aspects, In another embodiment of any one of the above aspects, the biomarker is NRP-2.

In a particular embodiment of any one of the above aspects, the biomarker is MMP-1.

In another embodiment of any one of the above aspects, the melanoma is one or more in situ, radial growth phase, vertical growth phase, or metastatic melanoma.

In further embodiment of any one of the above aspects, the method further comprises (c) managing subject treatment based on the status. In a related embodiment, managing subject treatment is selected from ordering further diagnostic tests, administering at least one therapeutic agent, surgery, surgery followed or preceded by administering at least one therapeutic agent, biotherapy, and taking no further action. In another related embodiment, the therapeutic agent is selected from one or more of fotemustine, dacarbazine, interferon, cisplatin, tamoxifen, interleukin-2, interferon alpha, vinblastin, carmubris, avastin, BRAF-kinase inhibitor, CTLA-4 antibody, angiogenesis inhibitors, targeted immunotherapy, or vaccines.

In another embodiment of any one of the above aspects, the method further comprises (d) measuring the at least one biomarker after subject management.

In still another embodiment of any one of the above aspects, the melanoma status is selected from one or more of the presence, absence or amount of one or more of the markers identified in any one of Tables 1 - 8, or combinations thereof. In a related embodiment, the method further comprises assessing the status of the melanoma. In a further related embodiment, the melanoma status is assessed by one or more of visual examination, tissue sample examination, subject's symptoms, or blood evaluation.

In another embodiment of any one of the above aspects, the subject has previously been diagnosed with melanoma.

In another embodiment of any one of the above aspects, the subject has melanoma with a BRAF mutation.

In yet another embodiment of any one of the above aspects, the subject has previously been treated for melanoma.

In another embodiment of any one of the above aspects, the measurement is performed after surgery or therapy to treat melanoma. In a related embodiment of any one of the above aspects, the measurement is used to predict the recurrence of melanoma. In another related embodiment of any one of the above aspects, the measurement is used to classify a subject as a low or high risk for melanoma recurrence.

In another embodiment of any one of the above aspects, the detection used to determine a course of treatment for a subject.

In another embodiment of any one of the above aspects, the marker is detected by one or more of mass spectrometry, PCR, or microarray analysis. In a related embodiment of any one of the above aspects, the marker is detected by SELDI.

In another embodiment of any one of the above aspects, the sample from the subject is one or more of blood, blood plasma, serum, urine, cells, organs, seminal fluids, bone marrow, saliva, stool, a cellular extract, a tissue sample, a tissue biopsy, or cerebrospinal fluid.

In another embodiment of any one of the above aspects, the biomarkers are protein markers and are measured by immunoassay.

In another embodiment of any one of the above aspects, at least two biomarkers are measured. In a related embodiment, at least three biomarkers are measured.

In another embodiment of any one of the above aspects, wherein measuring is selected from detecting the presence or absence of the biomarkers(s), quantifying the amount of marker(s), and qualifying the type of biomarker.

In another embodiment of any one of the above aspects, the biomarkers are detected by immunoassay.

In another aspect, the invention features a method for identifying a candidate compound for treating melanoma comprising: a) contacting one or more of the biomarkers identified in any one of Tables 1 - 8, or combinations thereof with a test compound; and b) determining whether the test compound interacts with the biomarker, wherein a compound that interacts with the biomarker is identified as a candidate compound for treating melanoma.

In another aspect, the invention features a method of treating melanoma comprising administering to a subject suffering from or at risk of developing melanoma a therapeutically effective amount of a compound capable of modulating the expression or activity of one or more of the biomarkers selected from the group consisting of: one or more of the biomarkers identified in any one of Tables 1 - 8.

In yet another aspect, the invention features a method of treating a condition in a subject comprising administering to a subject a therapeutically effective amount of a compound which modulates the expression or activity of one or more of the biomarkers selected from the group consisting of: one or more of the biomarkers identified in any one of Tables 1 - 8.

In another aspect, the invention features a method of any one of claims 63 - 65, wherein the compound is selected from the group consisting of enzyme inhibitors, cytotoxic drugs, cytokins, chemokines, antibodies, a DNA molecule, an RNA molecule, a small molecule, a peptide, and a peptidomimetic.

In one embodiment, the compound modulates the expression of activity of NRP-2.

In another embodiment, the compound is an antibody. In a related embodiment, the antibody is selected from the group consisting of: monoclonal, polyclonal, humanized, and chimeric antibodies.

In a particular embodiment, the antibodies are radiolabelled.

In certain embodiments, radiolabelled antibodies to any of the biomarkers are used as an imaging tool for melanoma.

In certain aspects, the invention features a method of imaging melanoma in a subject comprising administering to a subject a radiolabelled antibodythat detects one or more of the biomarkers selected from the group consisting of: one or more of the biomarkers identified in any one of Tables 1 - 8.

In a further embodiment, the compound is an inhibitory RNA molecule. In a related embodiment, the inhibitory RNA molecule is one or more siRNAs. In a further embodiment, the siRNA is about 18 - 21 nucleotides in length.

In another embodiment, the at least one biomarker is measured by immunoassay.

In still another embodiment, the correlation is performed by a software classification algorithm.

In another aspect, the invention features a method for identifying a melanoma treatment, comprising: a) contacting a cell with a test compound, b) measuring at least one biomarker, wherein the biomarker is selected from one or more of the markers identified in any one of Tables 1- 8, and c) correlating the measurement with a determination of efficacy.

In one embodiment, the cell is one or more of is one or more of a radial-growth phase line, an early vertical-growth phase line, a late vertical growth phase line, or a metastatic melanoma line.

In another aspect, the invention features a method of determining the melanoma status of a subject, comprising: (a) obtaining a biomarker profile from a sample taken from the subject; and (b) comparing the subject's biomarker profile to a reference biomarker profile obtained from a reference population, wherein the comparison is capable of classifying the subject as belonging to or not belonging to the reference population; wherein the subject's biomarker profile and the reference biomarker profile comprise one or more markers selected from any ane of the markers identified in any one of Tables 1 - 8.

In one embodiment, the method further comprises repeating the method at least once, wherein the subject's biomarker profile is obtained from a separate sample taken each time the method is repeated. In a related embodiment, the samples from the subject are taken about 24 hours apart.

In another embodiment, the comparison of the biomarker profiles can determine melanoma status in the subject with an accuracy of at least about 60% to about 99%. In another embodiment, the reference biomarker profile is obtained from a population comprising a single subject, at least two subjects, and at least 20 subjects.

In another aspect, the invention features a method for the identification of a therapeutic target for melanoma comprising comparing an expression profile of a melanoma cell with an expression profile of one a reference cell, wherein the comparison is capable of classifying proteins or transcripts in the profile as being associated with invasion.

In one embodiment, the melanoma cell is one or more of a blood cell from a melanoma subject, a tissue sample from a melanoma subject or a melanoma cell line. In another embodiment, the melanoma cell line is one or more of a radial-growth phase line, an early vertical-growth phase line, or a metastatic melanoma line.

In a related embodiment, the method further comprises identifying a candidate compound that interacts with the identified therapeutic target.

In a further related embodiment, the method features identifying the candidate compound comprises contacting the identified target with a test compound and determining whether the test compound interacts with the identified target, wherein a compound that interacts with the biomarker is identified as a candidate compound for treating melanoma.

In another related embodiment, the invention features a purified biomolecule selected from any one of the biomarkers identified in any one of Tables 1- 8.

In another aspect, the invention features a kit for aiding the diagnosis of rnelanoma, comprising: an adsorbent, wherein the adsorbent retains one or more biomarkers selected from one or more of the markers identified in Tables 1 - 8, and written instructions for use of the kit for detection of melanoma.

In another embodiment, the kit includes instructions provide for contacting a test sample with the adsorbent and detecting one or more biomarkers retained by the adsorbent. In a related embodiment, the adsorbent is an antibody, single or double stranded oligonucleotide, amino acid, protein, peptide or fragments thereof.

In another embodiment, the one or more protein biomarkers is detected using mass spectrometry, immunoassays, or PCR.

In another aspect, the invention features a biomarker for cancer status comprising one or more of the biomarkers identified in Tables 1 - 8, or combinations thereof, , wherein the biomarker is correlated with cancer status.

In another embodiment, the biomarker is selected from any one of the biomarkers identified in Table 7, 8 or 9. In a related embodiment, the biomarker is MMP-1.

In another embodiment, the cancer is a solid tumor. In a further embodiment, the cancer is a hematological malignancy.

In another aspect, the invention features a method of qualifying cancer status in a subject comprising (a) measuring at least one biomarker in a sample from the subject, wherein the biomarker is selected from one or more of the markers identified in any one of Tables 1 - 8; and (b) correlating the measurement with cancer status, thereby qualifying cancer status in a subject.

In one embodiment, the biomarker is selected from one or more of the markers identified in Tables 7, 8 or 9. In another embodiment, the biomarker is MMP-1.

In a particular embodiment, the cancer is a solid tumor.

In a particular embodiment, the cancer is a hematological malignancy.

In another aspect, the invention features a method for detecting a biomarker in a sample, wherein the marker is selected from one or more of the markers identified in one or more of Tables 1 - 8, or combinations thereof comprising a) contacting one or more of the biomarkers identified in any one of Tables 1 - 8, or combinations thereof with an antibody; and b) determining whether the antibody interacts with the biomarker, wherein an antibody that interacts with the biomarker detects the biomarker.

In this way, it may be useful to detect circulating antibodies to marker, for example a marker as identifies in any one of Tables 1 - 8, for example NRP-2, in patients' serum as a means of detecting early melanoma recurrence rather than looking for the protein directly either as either soluble circulating receptor or tumor-associated receptor. The patient-generated antibody would be the marker of disease, and with more tumor-secreted or tumor-associated antigen present, circulating antibody titers would rise.

In one embodiment, the detection of a biomarker is predictive of disease recurrence. In another embodiment, the sample is blood or serum.

Moreover, the present invention comprises the following embodiments:
1. A biomarker for melanoma, status comprising one or more of a marker identified in any one of Tables 1 - 8, or combinations thereof, wherein the biomarker is correlated with melanoma progression.
2. The biomarker of item 1, wherein the melanoma, is one or more of in situ, radial growth phase, vertical growth phase, metastatic melanoma.
3. The biomarker of item 1, wherein the biomarker is further used to determine the malignant potential of a melanocytic tumor of undetermined malignant potential, or a tumor of undetermined classification.
4. The biomarker of item 1, wherein melanoma progression is further defined as a stage selected from the group consisting of: stage I, stage II, stage III and stage IV.
5. The biomarker of item 1, wherein the marker corresponds to a gene involved in a biological process selected from the group consisting of cell adhesion, regulation of cell-cell adhesion, cell differentiation, negative regulation of cell differentiation, DNA replication initiation, regulation of apoptosis, angiogenesis, regulation of cell migration, cell proliferation, extracellular matrix organization and intracellular junction organization or maintenance.
6. The biomarker of item 1, wherein the melanoma is recurrent.
7. A biomarker for melanoma status comprising one or more of a marker identified in Table 2 or Table 8, or combinations thereof, wherein the biomarker is correlated with a stage melanoma progression.
8. The biomarker of item 7, wherein the biomarker is associated with aggressive melanoma.
9. A biomarker for melanoma status comprising one or more of a marker identified in Table 3, or combinations thereof, wherein the biomarker is correlated with a stage of melanoma progression.
10. The biomarker of item 9, wherein the biomarker is associated with less- aggressive melanoma.
11. A biomarker for melanoma status comprising one or more of a marker identified in Table 4, or combinations thereof, wherein the biomarker is correlated with metastatic melanoma.
12. The biomarker for melanoma status of item 11, wherein the marker or combinations thereof are down-regulated.
13. The biomarker of item 12, wherein the downregulation is correlated with metastatic melanoma.
14. A biomarker for melanoma status comprising one or more of a marker identified in Table 5, or combinations thereof, wherein the biomarker is correlated with invasive or metastatic melanoma.
15. The biomarker for melanoma status of item 14, wherein the markers or combinations thereof are overexpressed.
16. The biomarker for melanoma status of item 15, wherein the overexpression is correlated with invasive or metastatic melanoma.
17. A biomarker for melanoma status comprising one or more of a marker identified in Table 6, or combinations thereof, wherein the biomarker is correlated with a stage of melanoma progression.
18. The biomarker for melanoma status of item 17, wherein the markers or combinations thereof are overexpressed.
19. The biomarker for melanoma status of item 18, wherein the overexpression is correlated with invasive or metastatic melanoma.
20. The biomarker of item 18, wherein the marker corresponds to neuropilin (NILP-2).
21. A biomarker for melanoma status comprising one or more of a marker identified in Table 7 or Table 8, or combinations thereof, wherein the biomarker is upregulated in primary melanocytes.
22. The biomarker for melanoma status of item 21, wherein the markers or combinations thereof are overexpressed in primary melanocytes.
23. The biomarker for melanoma status of item 22, wherein the overexpression is correlated with melanocyte proliferation.
24. The biomarker of item 22, wherein the marker is selected from the group consisting of: matrix metalloproteinase-1 (MMP-1), SerpinB2, amphiregulin, CXCL5, IL-8, RAP1a and epiregulin.
25. A method of qualifying melanoma status in a subject comprising:
   (a) measuring at least one biomarker in a sample from the subject, wherein the biomarker is selected from one or more of the biomarkers identified in any one of Tables 1 - 8, or combinations thereof; and
   (b) correlating the measurement with melanoma status,
      thereby qualifying melanoma status in a subject.
26. A method of predicting the recurrence of melanoma in a subject comprising
   (a) measuring at least one biomarker in a sample from the subject, wherein the biomarker is selected from one or more of the biomarkers identified in any one of Tables 1-8, or combinations thereof; and
   (b) correlating the measurement with the risk melanoma recurrence,
      thereby predicting the recurrence of melanoma in a subject.
27. The method of item 26, wherein the detection of NRP-2 is predictive of disease recurrence.
28. The method of item 26, wherein the detection of MMP-1 is predictive of disease recurrence.
29. A method of identifying a risk of developing melanoma in a subject comprising:
   (a) measuring at least one biomarker in a sample from the subject, wherein the biomarker is selected from one or more of the biomarker identified in any one of Tables 1 - 8, or combinations thereof; and
   (b) correlating the measurement with a risk of developing melanoma,
      thereby identifying a risk of developing melanoma in a subject.
30. A method of detecting or diagnosing melanoma in a subject comprising:
   (a) measuring at least one biomarker in a sample from the subject, wherein the biomarker is selected from one or more of the biomarkers identified in any one of Tables 1 - 8, or combinations thereof; and
   (b) correlating the measurement with the presence of melanoma,
      thereby detecting or diagnosing melanoma in a subject.
31. A method of determining the prognosis of a subject suffering from melanoma comprising:
   (a) measuring at least one biomarker in a sample from the subject, wherein the biomarker is selected from one or more of the markers identified in any one of Tables 1 - 8, or combinations thereof; and
   (b) correlating the measurement with prognosis,
      thereby determining the prognosis of a subject suffering from melanoma.
32. The method of item 31, wherein the prognosis determines course of treatment.
33. The method of any one of items 25 - 32, wherein the biomarker is selected from one or more markers identified in Table 2 or Table 8, or combinations thereof.
34. The method of any one of items 25 - 32, wherein the biomarker is selected from one of more markers identified in Table 4, or combinations thereof.
35. The method of any one of items 25 - 32, wherein the biomarker is selected from one or more markers identified in Table 5, or combinations thereof.
36. The method of any one of items 25 - 32, wherein the biomarker is selected from one or more markers identified in Table 6, or combinations thereof.
37. The method of any one of items 25 - 32, wherein the biomarker is selected from one or more markers identified in Table 7, 8 or 9.
38. The method of any one of items 25 - 32, wherein the biomarker is NRP-2.
39. The method of any one of items 25 - 32, wherein the biomarker is MMP-1.
40. The method of any one of items 25 - 32, wherein the melanoma is one or more in situ, radial growth phase, vertical growth phase, or metastatic melanoma.
41. The method of any one of items 25 - 32, further comprising:
   (c) managing subject treatment based on the status.
42. The method of item. 41, wherein managing subject treatment is selected from ordering further diagnostic tests, administering at least one therapeutic agent, surgery, surgery followed or preceded by administering at least one therapeutic agent, biotherapy, and taking no further action.
43. The method of item 42, wherein the therapeutic agent is selected from one or more of fotemustine, dacarbazine, interferon, cisplatin, tamoxifen, interleukin-2, interferon alpha, vinblastin, carmubris, avastin, BRAF-kinase inhibitor, CTLA-4 antibody, angiogenesis inhibitors, targeted immunotherapy, or vaccines.
44. The method of any one of items 25 - 32, further comprising:
   (d) measuring the at least one biomarker after subject management.
45. The method of any one of items 25 - 32, wherein the melanoma status is selected from one or more of the presence, absence or amount of one or more of the markers identified in any one of Tables 1 - 8, or combinations thereof.
46. The method of item 45, further comprising assessing the status of the melanoma.
47. The method of item 46, wherein the melanoma status is assessed by one or more of visual examination, tissue sample examination, subject's symptoms, or blood evaluation.
48. The method of any one of items 25 - 32, wherein the subject has previously been diagnosed with melanoma.
49. The method of any one of items 25 - 32, wherein the subject has melanoma with a BRAF mutation.
50. The method of any one of items 25 - 32, wherein the subject has previously been treated for melanoma.
51. The method of any one of items 25 - 32, wherein the measurement is performed after surgery or therapy to treat melanoma.
52. The method of any one of items 25 - 32, wherein the measurement is used to predict the recurrence of melanoma.
53. The method of any one of items 25 - 32, wherein the measurement is used to classify a subject as a low or high risk for melanoma recurrence.
54. The method of any one of items 25 - 32, wherein the detection used to determine a course of treatment for a subject
55. The method of any one of items 25 - 32, wherein the marker is detected by one or more of mass spectrometry, PCR, or microarray analysis.
56. The method of any one of items 25 - 32, wherein the marker is detected by SELDI.
57. The method of any one of items 25 - 32, wherein the sample from the subject is one or more of blood, blood plasma, serum, urine, cells, organs, seminal fluids, bone marrow, saliva, stool, a cellular extract, a tissue sample, a tissue biopsy, or cerebrospinal fluid.
58. The method of any one of items 25 - 32, wherein biomarkers are protein markers and are measured by immunoassay.
59. The method of any one of items 25 - 32, wherein at least two biomarkers are measured.
60. The method of any one of items 25 - 32, wherein at least three biomarkers are measured.
61. The method of any one of items 25 - 32, wherein measuring is selected from detecting the presence or absence of the biomarkers(s), quantifying the amount of marker(s), and qualifying the type of biomarker.
62. The method of item 61, wherein the biomarkers are detected by immunoassay.
63. A method for identifying a candidate compound for treating melanoma comprising:
   a) contacting one or more of the biomarker identified in any one of Tables 1 - 8, or combinations thereof with a test compound; and
   b) determining whether the test compound interacts with the biomarker,
      wherein a compound that interacts with the biomarker is identified as a candidate compound for treating melanoma.
64. A method of treating melanoma comprising administering to a subject suffering from or at risk of developing melanoma a therapeutically effective amount of a compound capable of modulating the expression or activity of one or more of the biomarker selected from the group consisting of: one or more of the biomarkers identified in any one of Tables 1 - 8.
65. A method of treating a condition in a subject comprising administering to a subject a therapeutically effective amount of a compound which modulates the expression or activity of one or more of the biomarkers selected from the group consisting of: one or more of the biomarkers identified in any one of Tables 1 - 8.
66. The method of any one of items 63 - 65, wherein the compound is selected from the group consisting of enzyme inhibitors, cytotoxic drugs, cytokins, chemokines, antibodies, a DNA molecule, an RNA molecule, a small molecule, a peptide, and a peptidomimetic.
67. The method of item 66, wherein the compound modulates the expression of activity of NRP-2.
68. The method of item 67, wherein the compound is an antibody.
69. The method of item 68, wherein the antibody is selected from the group consisting of: monoclonal, polyclonal, humanized, and chimeric antibodies.
70. Them ethod of item 68, wherein the antibodies are radiolabelled.
71. The method of item 67, wherein the compound in an inhibitory RNA molecule.
72. The method of item 67, wherein the inhibitory RNA molecule is one or more siRNA.
73. The method of any of items 25 - 65, wherein the at least one biomarker is measured by immunoassay.
74. The method of any of items 25 - 65, wherein the correlation is performed by a software classification algorithm.
75. A method for identifying a melanoma treatment, comprising:
   a) contacting a cell with a test compound,
   b) measuring at least one biomarker, wherein the biomarker is selected from one or more of the markers identified in any one of Tables 1 - 8, and
   c) correlating the measurement with a determination of efficacy.
76. The method of item 75, wherein the cell is one or more of is one or more of a radial-growth phase line, an early vertical-growth phase line, a late vertical growth phase line, or a metastatic melanoma line.
77. A method of determining the melanoma status of a subject, comprising:
   (a) obtaining a biomarker profile from a sample taken from the subject; and
   (b) comparing the subject's biomarker profile to a reference biomarker profile obtained from a reference population, wherein the comparison is capable of classifying the subject as belonging to or not belonging to the reference population; wherein the subject's biomarker profile and the reference biomarker profile comprise one or more markers selected from any one of the markers identified in any one of Tables 1- 8.
78. The method of item 77, further comprising repeating the method at least once, wherein the subject's biomarker profile is obtained from a separate sample taken each time the method is repeated.
79. The method of item 78, wherein samples from the subject are taken about 24 hours apart.
80. The method of item 77, wherein the comparison of the biomarker profiles can determine melanoma status in the subject with an accuracy of at least about 60% to about 99%.
81. The method of item 77, wherein the reference biomarker profile is obtained from a population comprising a single subject, at least two subjects, and at least 20 subjects.
82. A method for the identification of a therapeutic target for melanoma comprising:
   comparing an expression profile of a melanoma cell with an expression profile of one a reference cell, wherein the comparison is capable of classifying proteins or transcripts in the profile as being associated with invasion.
83. The method of item 82, wherein the melanoma cell is one or more of a blood cell from a melanoma subject, a tissue sample from a melanoma subject or a melanoma cell line.
84. The method of item 82, wherein the melanoma cell line is one or more of a radial-growth phase line, an early vertical-growth phase line, or a metastatic melanoma line.
85. The method of item 82, further comprising identifying a candidate compound that interacts with the identified therapeutic target.
86. The method of item 85, wherein identifying the candidate compound comprises contacting the identified target with a test compound and determining whether the test compound interacts with the identified target, wherein a compound that interacts with the biomarker is identified as a candidate compound for treating melanoma.
87. A purified biomolecule selected from any one of the biomarkers identified in any one of Tables 1 - 8.
88. A kit for aiding the diagnosis of melanoma, comprising:
   an adsorbent, wherein the adsorbent retains one or more biomarkers selected from one or more of the markers identified in Tables 1 - 8, and written instructions for use of the kit for detection of melanoma.
89. The kit of item 88, wherein the instructions provide for contacting a test sample with the adsorbent and detecting one or more biomarkers retained by the adsorbent.
90. The kit of item 88, wherein the adsorbent is an antibody, single or double stranded oligonucleotide, amino acid, protein, peptide or fragments thereof.
91. The kit of item 88, wherein one or more protein biomarkers is detected using mass spectrometry, immunoassays, or PCR.
92. A biomarker for cancer status comprising one or more of the biomarkers identified in Tables 1 - 8, or combinations thereof, , wherein the biomarker is correlated with cancer status.
93. The biomarker of item 92, wherein the biomarker is selected from any one of the biomarkers identified in Table 7, 8 or 9.
94. The biomarker of item 92, wherein the biomarker is MMP-1.
95. The biomarker of item 92, wherein the cancer is a solid tumor.
96. The biomarker of item 92, wherein the cancer is a hematological malignancy.
97. A method of qualifying cancer status in a subject comprising:
   (a) measuring at least one biomarker in a sample from the subject, wherein the biomarker is selected from one or more of the markers identified in any one of Tables 1 - 8; and
   (b) correlating the measurement with cancer status,
      thereby qualifying cancer status in a subject.
98. The method of item 97, wherein the biomarker is selected from one or more of the markers identified in Tables 7, 8 or 9.
99. The method of item 97, wherein the biomarker is MMP-1.
100. The method of item 97, wherein the cancer is a solid tumor.
101. The method of item 97, wherein the cancer is a hematological malignancy.
102. A method for detecting a biomarker in a sample, wherein the marker is selected from one or more of the markers identified in one or more of Tables 1 - 8, or combinations thereof comprising:
   a) contacting one or more of the biomarkers identified in any one of Tables 1 - 8, or combinations thereof with an antibody, and
   b) determining whether the antibody interacts with the biomarker,
      wherein an antibody that interacts with the biomarker detects the biomarker.
103. The method of item 102, wherein the detection of a biomarker is predictive of disease recurrence.
104. The method of item 102, wherein the sample is blood or serum.
105. A method of imaging melanoma in a subject comprising administering to a subject a radiolabelled antibody that detects one or more of the biomarkers selected from the group consisting of: one or more of the biomarkers identified in any one of Tables 1 - 8.

### BRIEF DESCRIPTION OF THE DRAWINGS

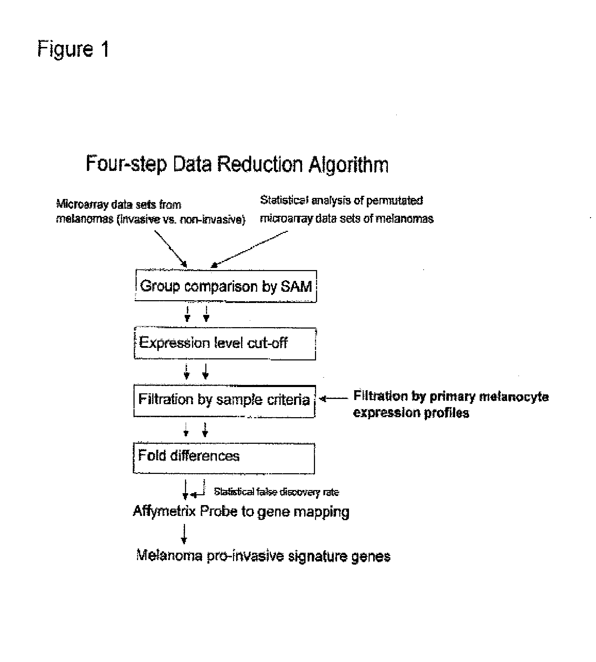

Figure **1** is a schematic that shows a data reduction algorithm used to define a final set of invasion specific genes in melanoma.

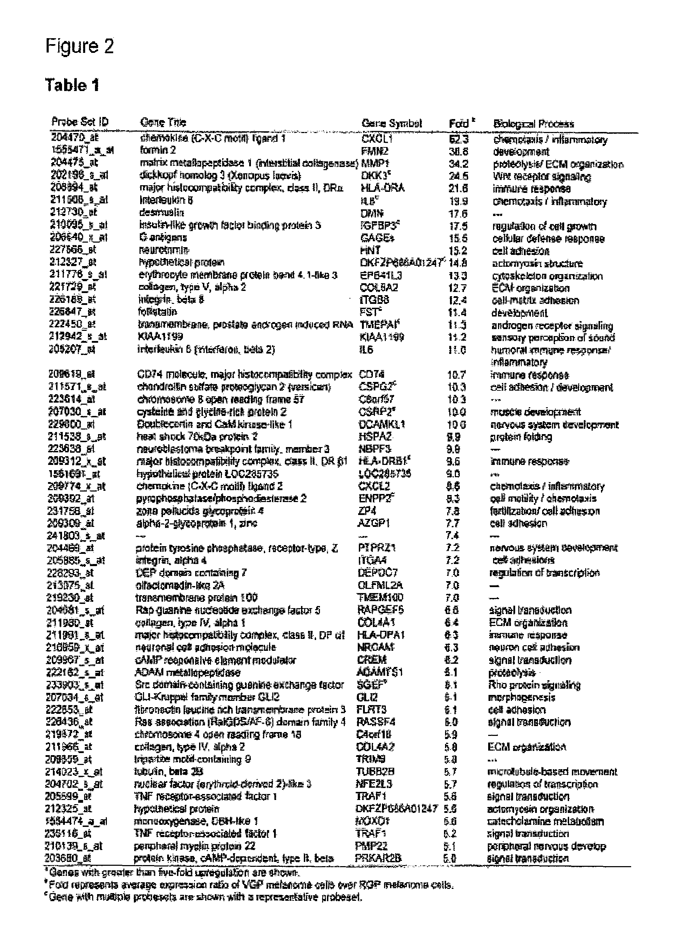

Figure 2 is Table (Table 1) that shows a complete list of melanoma invasion-specific genes that are upregulated in VGP compared to RGP melanoma cells with functional annotations. Genes that have a greater than five-fold difference are shown.

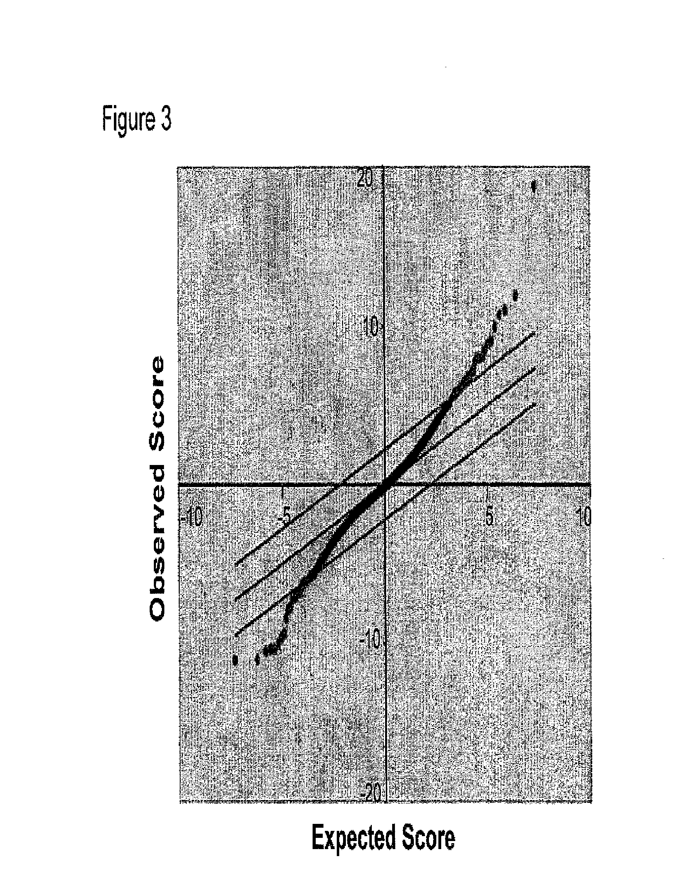

Figure 3 shows SAM analysis of more aggressive versus less aggressive melanomas.

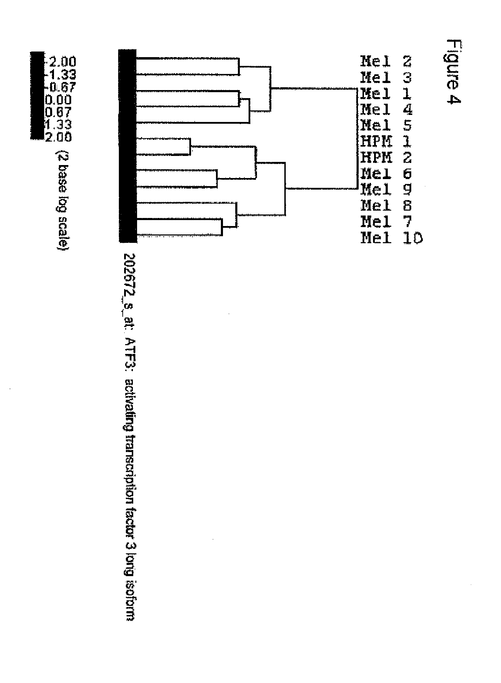

Figure 4 shows unsupervised hierarchical clustering of melanoma tumor cell lines and primary human melanocyte reveals clustered gene profiles for aggressive melanoma cell lines and primary human melanocytes (aggressive melanoma cluster) versus non-aggressive melanoma cell lines (Mel-1 through Mel-5).

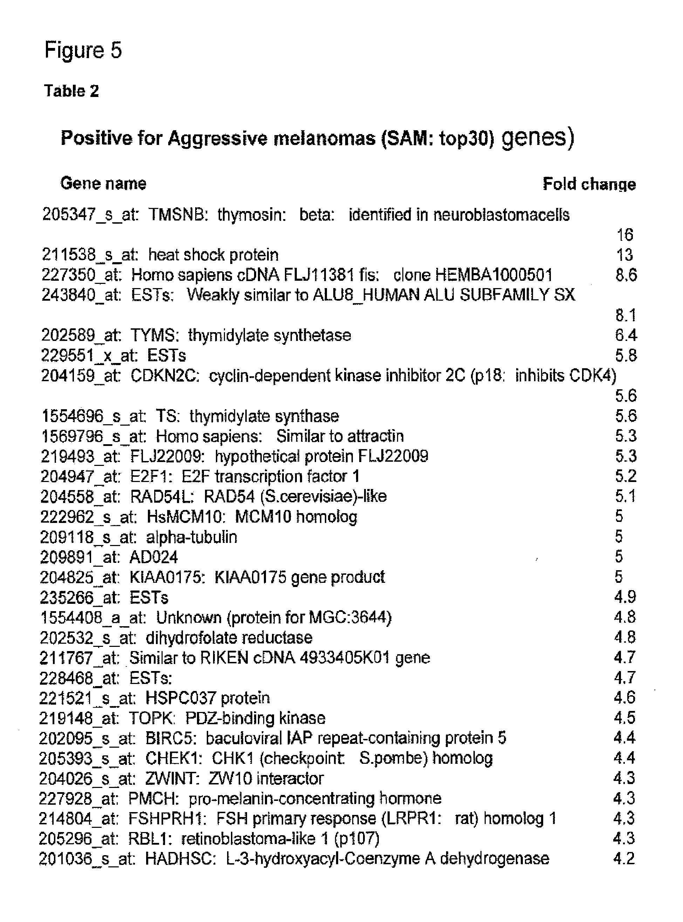

**Figure 5** is a Table (Table 2) that shows a list of genes associated with aggressive melanomas. The Table shows the results of SAM analysis between Group 1 and Group 2 melanoma cells. The results include the SAM Output showing the list of Affymetrix probesets and associated gene symbols that are differentially expressed in the two groups of melanoma cell lines.

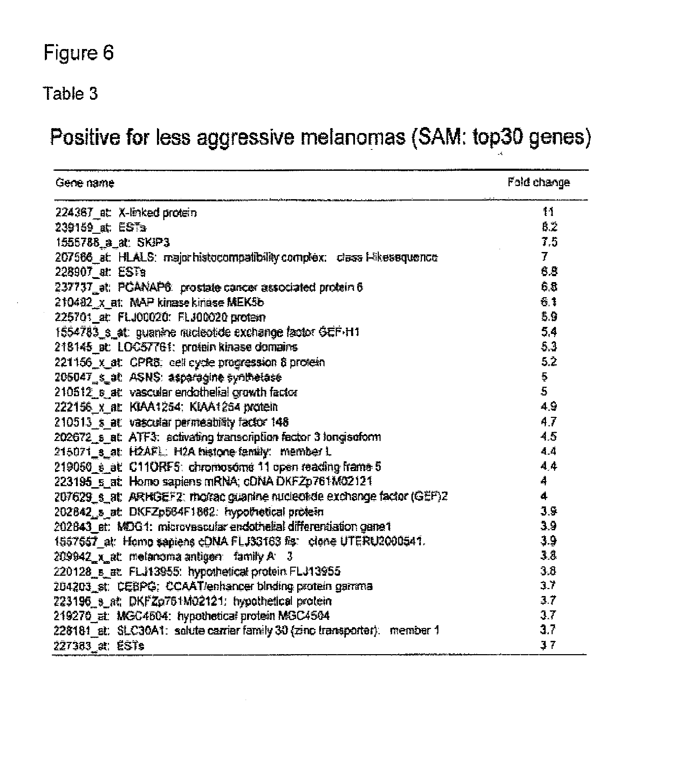

**Figure 6** is a Table (Table 3) that shows a list of genes associated with less- aggressive melanomas. The Table shows the results of SAM analysis between Group 1 and Group 2 melanoma cells. The results include the SAM Output showing the list of Affymetrix probesets and associated gene symbols that are differentially expressed in the two groups of melanoma cell lines.

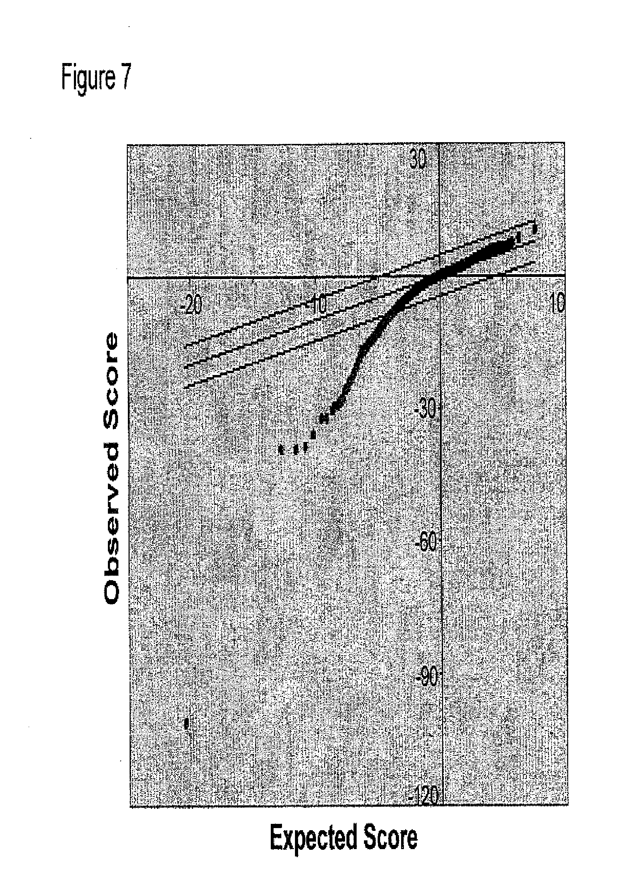

**Figure 7** shows SAM analysis of primary human melanocyte gene expression profiles versus metastatic tumor cell line profiles. Note only statically meaningful gene changes are loss of expression in tumor cell lines versus primary human melanocytes.

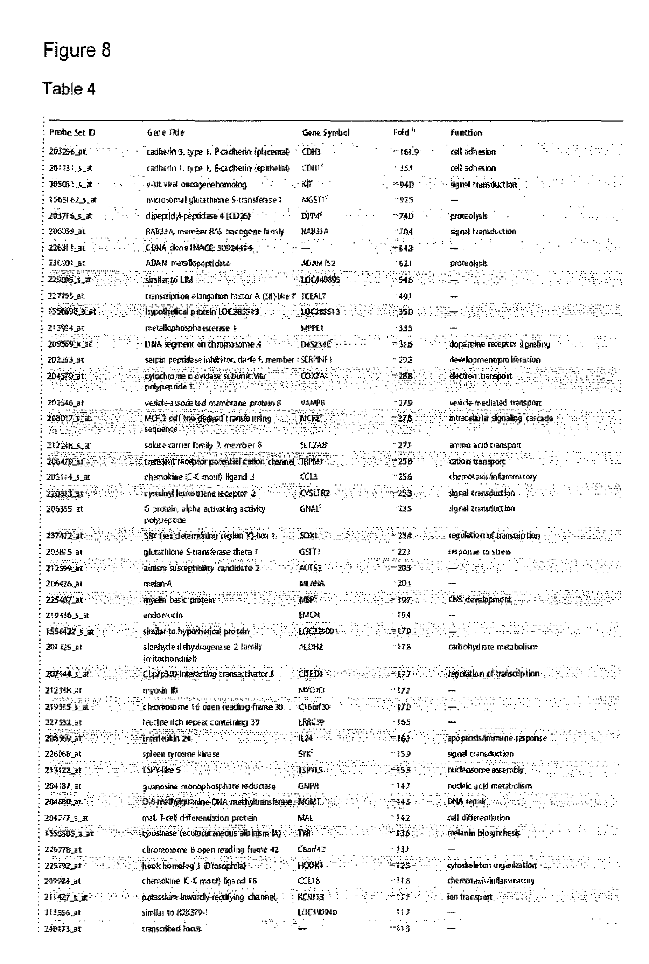

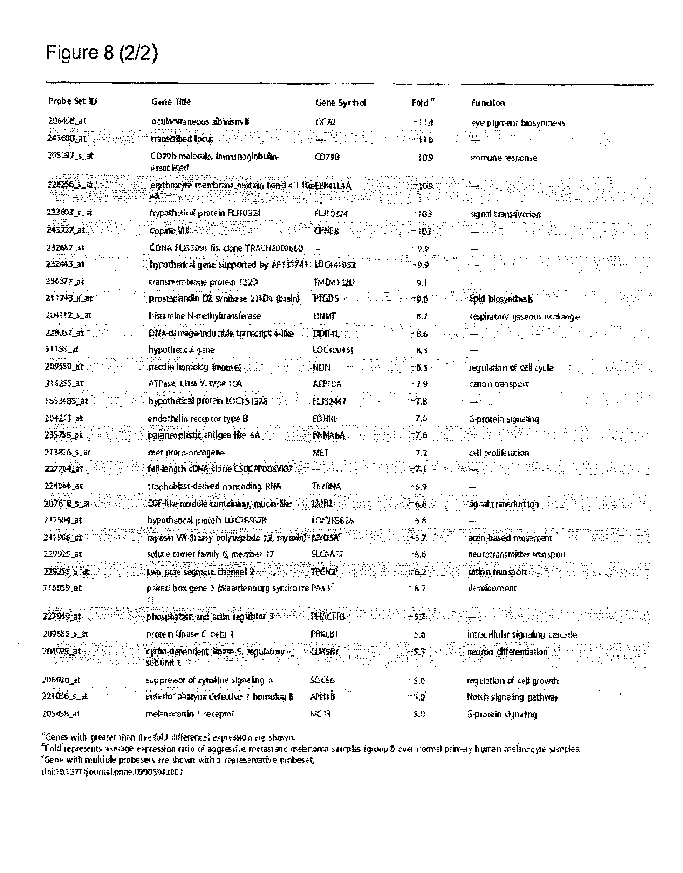

**Figure 8** is a Table (Table 4) that shows differential expression of genes that are downregulated in aggressive melanoma cells (Group2) compared to primary human melanocytes. Genes with greater than five-fold differential expression are shown.

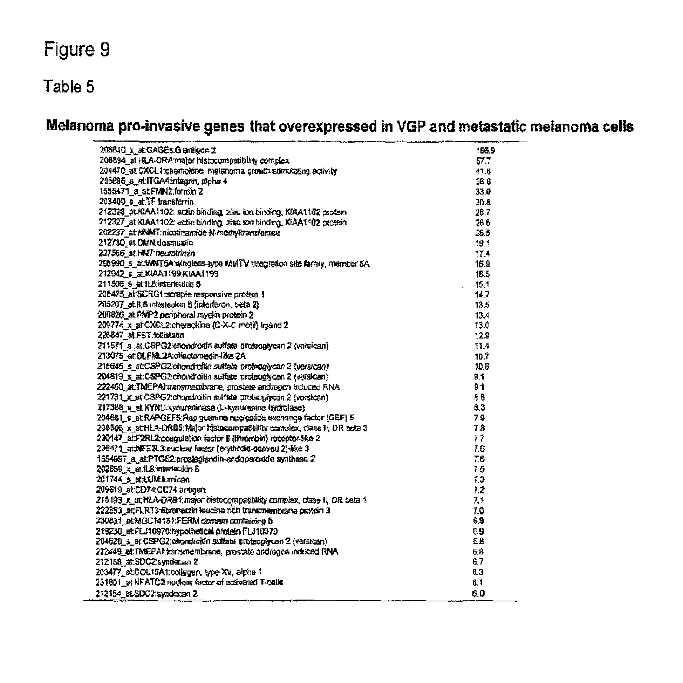

**Figure 9** is a Table (Table 5) that shows a list of pro-invasive genes associated with melanoma invasion and metastasis.

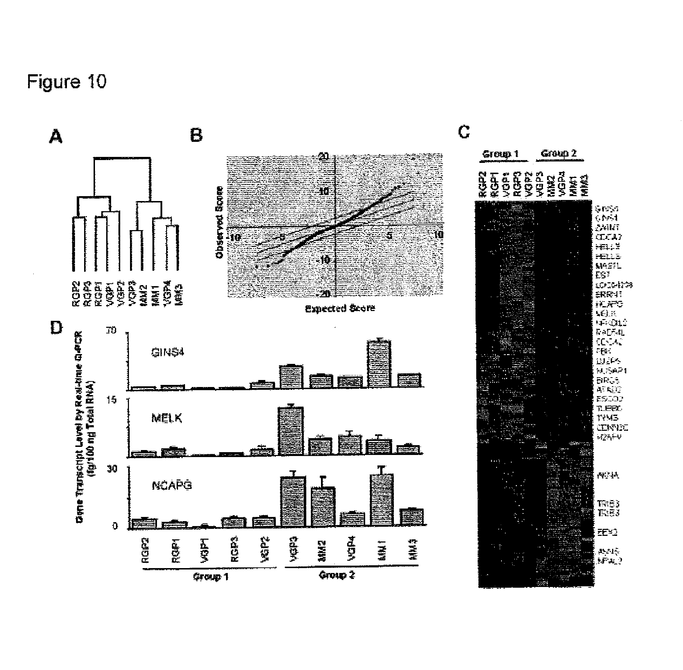

**Figure 10** **(A - D)** shows that the evaluation of gene expression profiles from melanoma cells lines of varying stages of progression identifies a signature for aggressive melanomas. Panel A shows that unsupervised hierarchical clustering of melanoma cells indicates the existence of two distinct groups of melanoma cells based on global gene expression patterns (Group 1: RGP2, RGP3, RGP1, VGP1, and VGP2; Group 2: VGP3, MM2, MM1, VGP4, and MM3). Panel B is a SAM plot sheet illustrating a signature for differentially expressed genes in aggressive melanomas. Gene expression profiles from the two groups of melanomas were compared (Group1 vs. Group 2) and a differentially expressed gene signature was identified by SAM. Red and green dots represent gene probesets upregulated and downregulated respectively in Group 2. In Panel C, the melanoma gene signature was visualized using Java TreeView. Genes that were over four-fold differentially expressed are indicated on the right side of the image. Panel D shows validation of select differentially expressed genes by real-time RT-PCR. Three genes upregulated in aggressive melanomas (Group 2) were selected for analysis and their differential expression was verified. 3.0 mg of total RNA was subjected to cDNA synthesis reaction as described in the materials and methods. 1.0 ml of the final cDNA samples (100 ml) were used for real-time Q-PCR reaction. For the measurement of gene transcript level, standard curves were generated for each gene using known amount of PCR amplified product from the corresponding genes. Error bars are SD of three independent experiments.

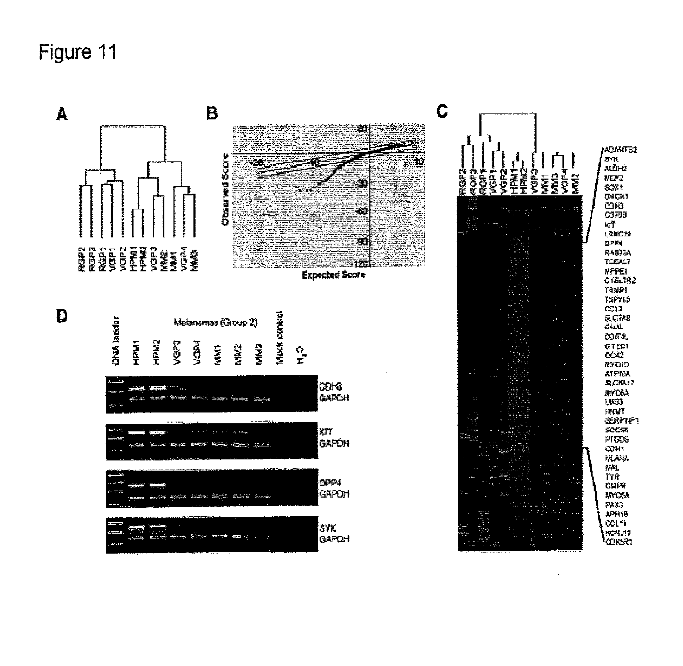

**Figure 11** **(A - D)** shows the evaluation of differential gene expression from aggressive melanomas (Group 2) vs. primary human melanocytes, and identifies a signature characterized by loss of differentiation-associated genes. Panel A shows that a Java TreeView analysis of melanoma cell lines and primary human melanocyte clusters two pools of human primary melanocytes (HPM1 and HPM2) with the Group 2 melanomas. Panel B is a SAM plot sheet illustrating a signature of down-regulated genes in group 2 melanomas compared to HPMs. Gene expression profiles of two pools of human primary melanocytes (HPM1 and HPM2) were compared to those of aggressive melanomas (Group 2) and a differentially expressed gene signature was identified by SAM. In Panel C, the melanoma gene signature was visualized using Java TreeView. Genes over five-fold downregulated are indicated on the right Panel D shows validation of differential expression for selected genes by semi-quantitative duplex RT-PCR. Four genes (CDH3, KIT, DPP4, SYK) downregulated in the aggressive melanoma cells (Group 2) were selected for analysis and their differential expression was verified.

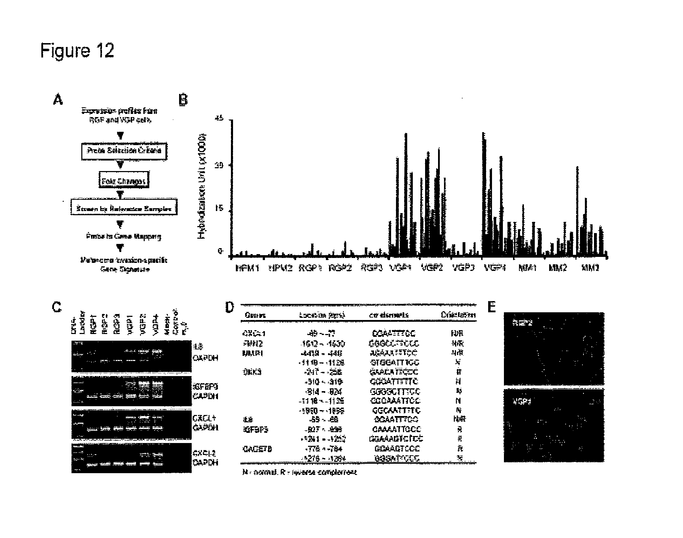

**Figure 12** **(A - E)** shows identification of an invasion-specific gene signature for melanoma. Panel A is a schematic outlining the three-step data reduction algorithm used for identification of a melanoma invasion-specific signature. (see detailed description in Data Extraction and Statistical Analysis section of Methods). Panel B is a graph showing the relative expression levels of melanoma invasion-specific signature genes in all cells analyzed including human primary melanocytes (HPM1, HPM2). Panel C shows validation of differential expression for selected genes by semi-quantitative duplex RT-PCR. Four genes (IL-8, IGFBP3, CXCL1, CXCL2) that are upregulated in invasive melanomas were selected and their differential expression was verified. Panel D is a Table showing results of promoter analysis of selected genes from the melanoma invasion-specific signature identifies putative NF-KB binding cis elements. Panel E shows immunofluorescence staining ofNF-KB in invasive (WM902B) vs. non-invasive (WM1552C) melanoma cells demonstrates constitutive activation and nuclear trafficking of NF-KB in invasive melanomas.

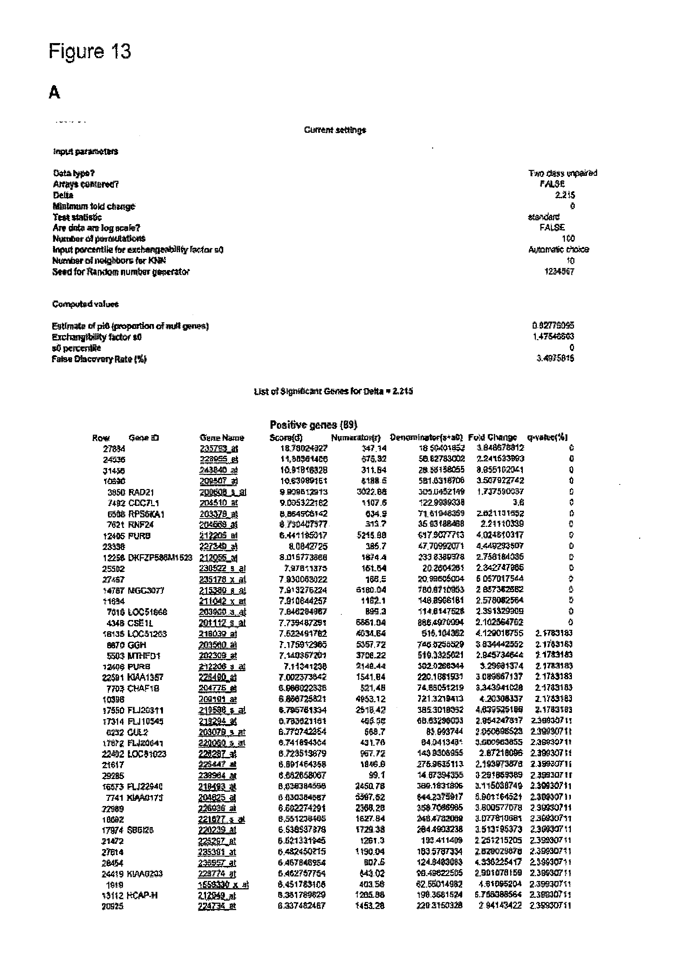

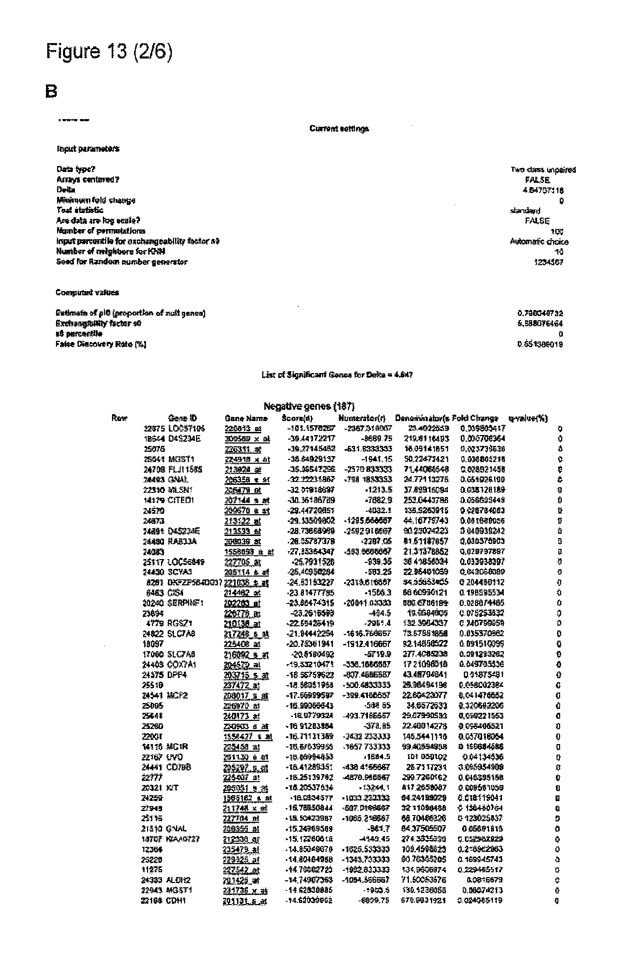

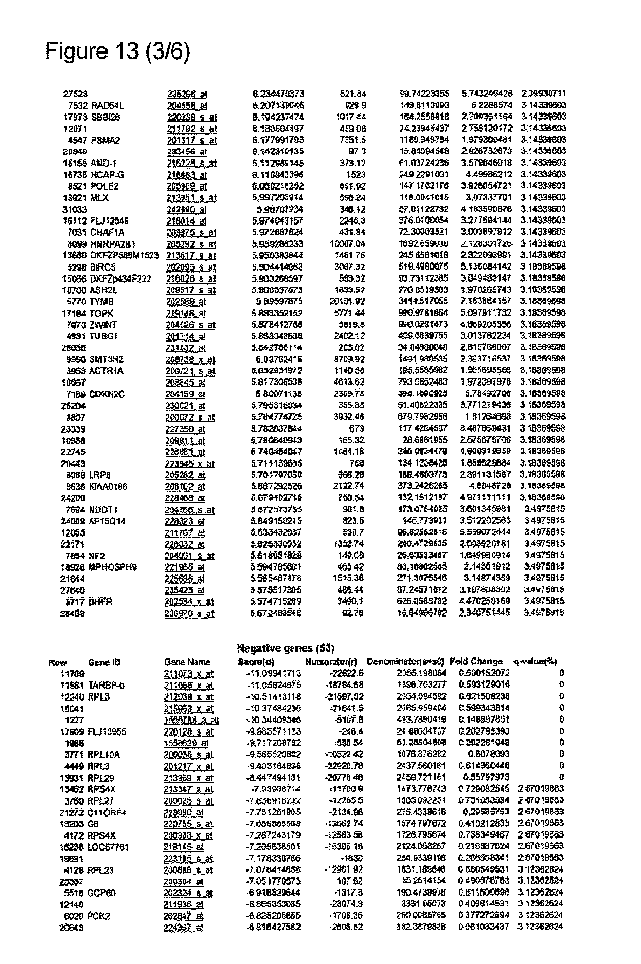

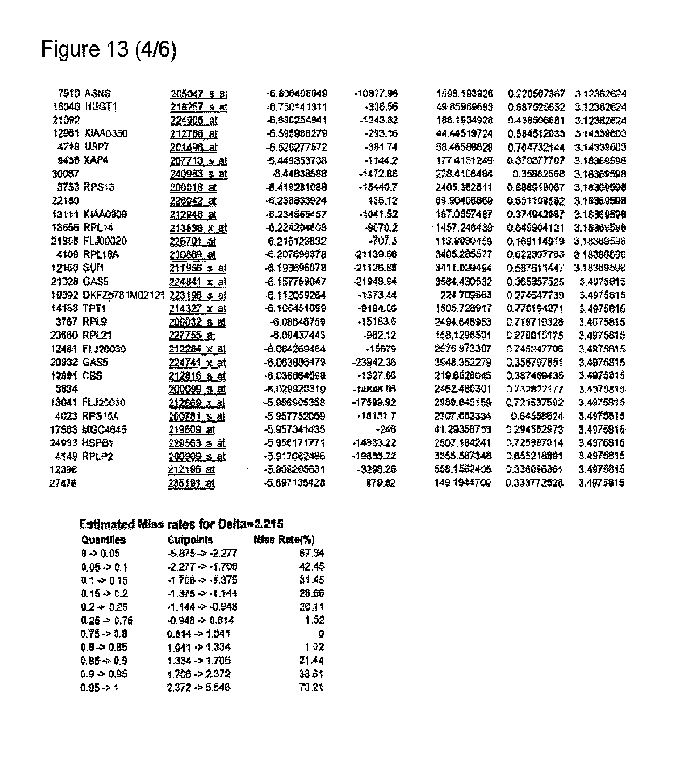

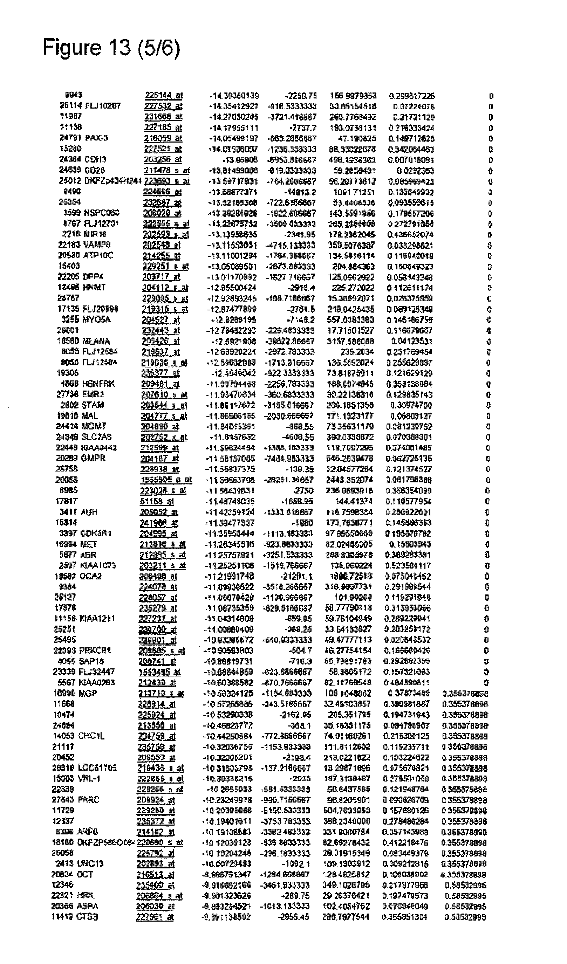

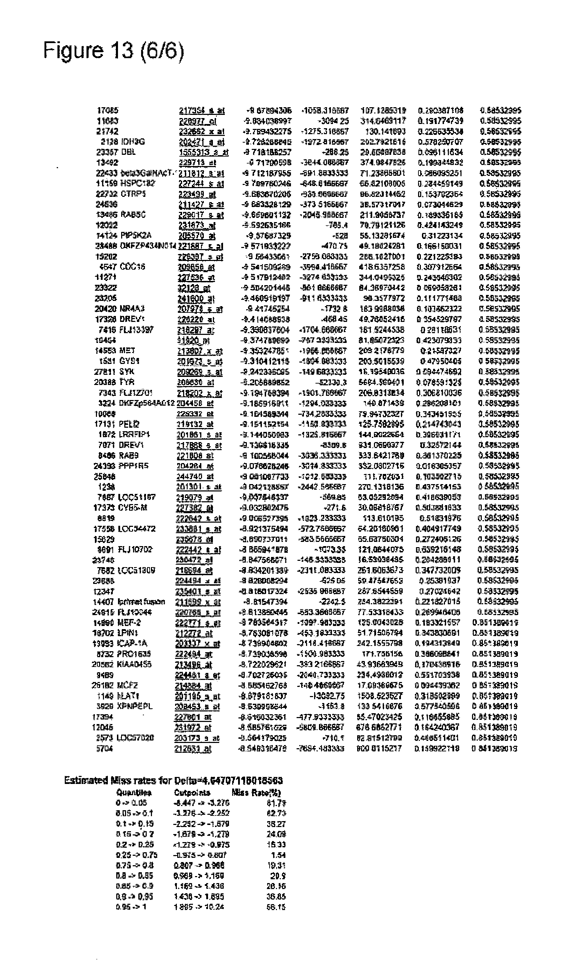

**Figure 13** **(A and B)** Panel A shows results of SAM analysis between Group 1 and Group 2 melanoma cells. The results include the SAM Output showing the list of Affymetrix probesets and representing gene symbols that differentially expressed in the two groups of melanoma cell lines. Panel B shows the results of SAM analysis between PHMs and Group 2 melanoma cells. The results include the SAM Output showing the list of Affymetrix probesets and representing gene symbols that down-regulated in Group 2 melanoma cell lines..

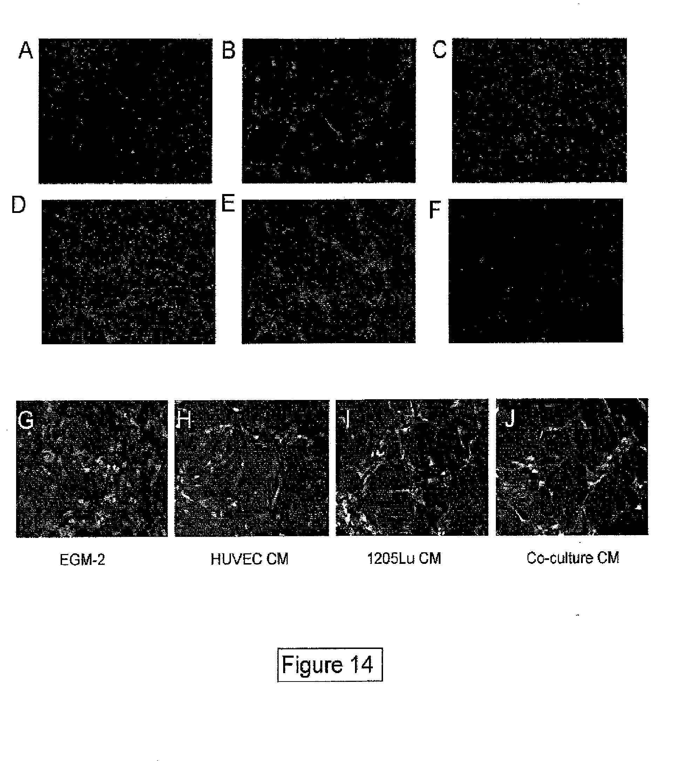

**Figure 14** **(A - J)** is a series of panels showing melanoma-endothelial cell crosstalk in a model for melanoma metastasis. Panel A shows RFP-HUVEC and GFP-1205Lu metastatic melanoma cell migration at the cellular communication interphase. Panel B shows HUVEC (red) network formed observed following migration into area occupied by GFP-1205Lu metastatic melanoma cells. Panels C - E shoe RFP-HUVEC network formation observed following 6 (C), 24 (D), or 48 (E) hours of direct co-culture. Panel F shows RFP-HUVEC cells in single-cell culture for 48 hours. Panels G - J show HUVEC patterning is induced by Secreted Factors in Conditioned Medium (CM) from Cultures Containing Metastatic Melanoma cells. RFP-HUVEC after 48 hr in EGM-2 or CM from RFP-HUVEC, GFP-1205Lu, or co-cultured RFP-HUVEC and GFP-1205Lu, respectively.

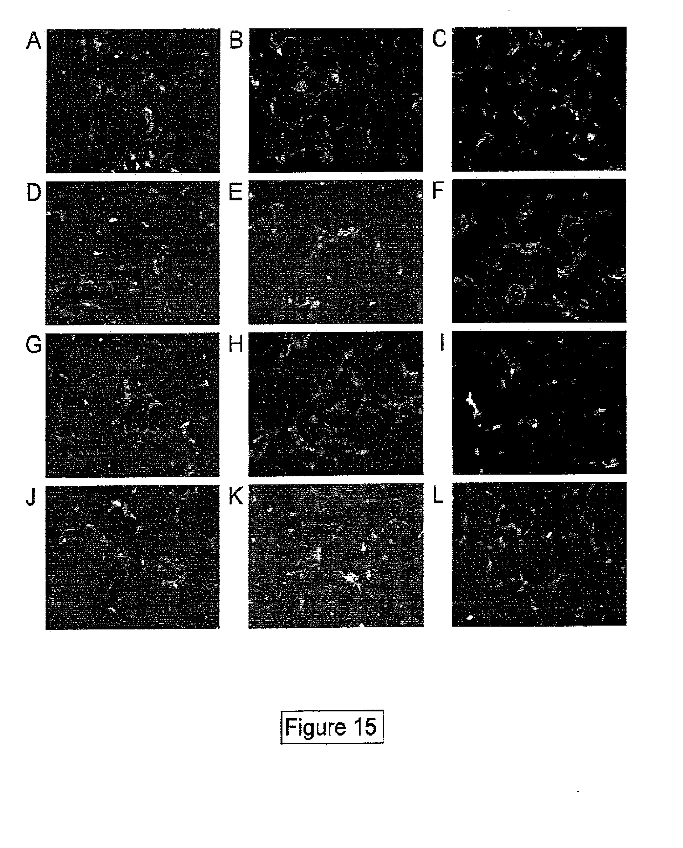

**Figure 15** **(A - L)** is a series of panels showing melanoma crosstalk with endothelial cells is increased in melanomas from advanced stages of progression. The panels show evaluation of melanocytes for their ability to induce HUVEC patterning in the co-culture system. Panel A shows Primary human melanocytes. Panels B - D show radial growth phase melanomas (WM35, SBc12, and WM1552C, respectively). Panels E - I show vertical growth phase melanomas (WM1341D, WM902B, WM278, WM983A, and WM793, respectively). Panels J - L show metastatic melanomas (WM852, WM983B, and 1205Lu, respectively). The photographs are taken at 10X magnification.

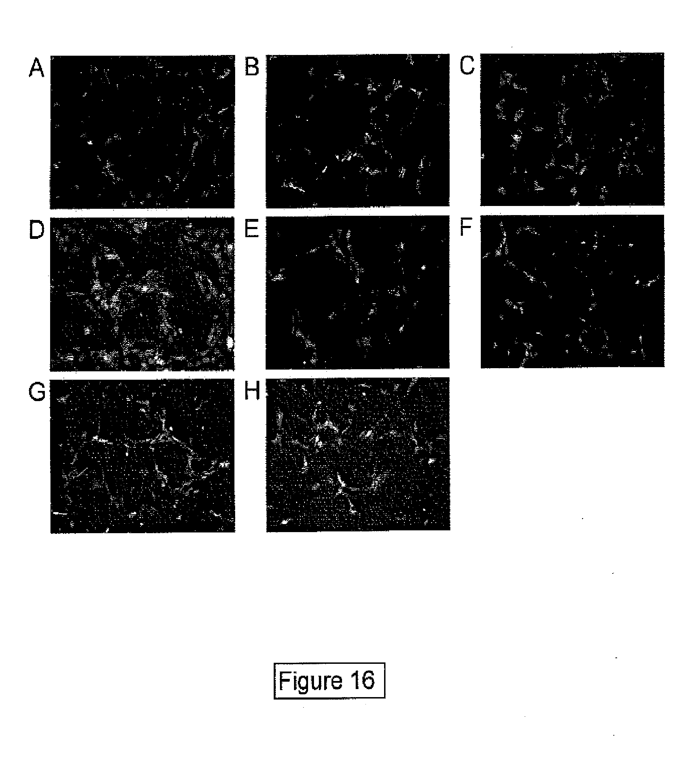

**Figure 16** **(A - H)** is a series of panels showing tumor cells of varying lineage demonstrate variable crosstalk with endothelial cells. RFP-HUVEC are co-cultured with cell lines representing various tumor types. Patterning of RFP-HUVECc co-cultured with the following was evaluated: Panel A ES-2, ovarian cancer; Panel B H460, non-small cell lung carcinoma; Panel C HCT-116, colon cancer; Panel D Panc3.014, pancreatic cancer; Panel E PC3, prostate cancer;Panel F Hs578T, breast cancer; Panel G U87MG, glioblastoma;

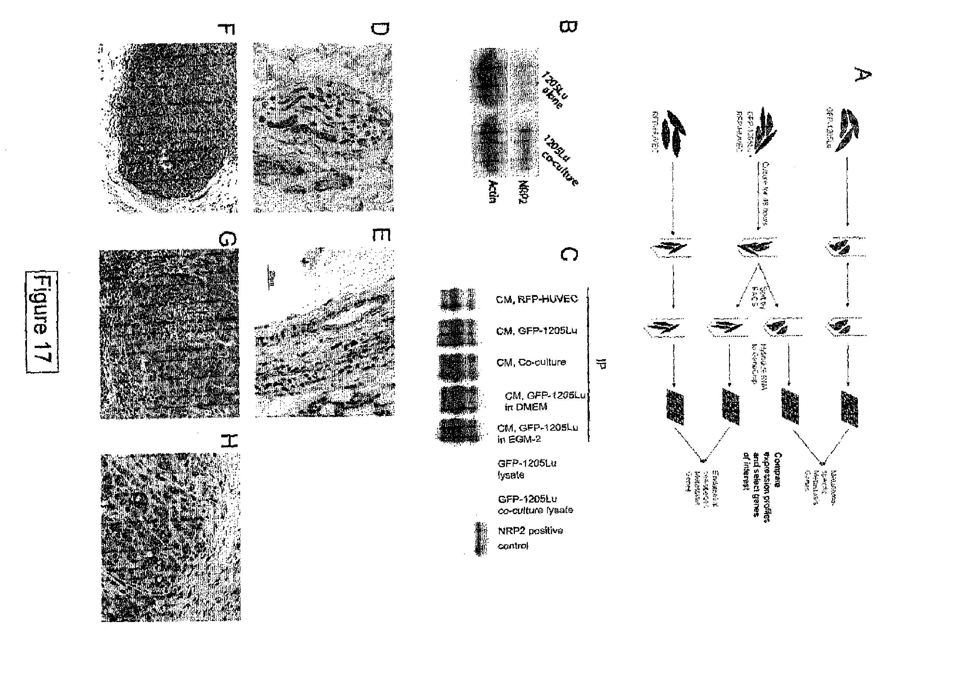

**Figure 17** **(A - U)** is a series of panels that shows global gene expression profiling of melanoma-endothelial cell crosstalk pathways identifies Neuropilin-2 as a mediator cellular communication. Panel A is a schematic representation of screen to identify melanoma-endothelial cell crosstalk genes. Populations of RFP-HUVECs and GFP-1205Lu metastatic melanoma cells were plated in a co-culture system and incubated for 48 hours. Cells were sorted by FACS and RNAs isolated and hybridized to a pan-genomic human GeneChip. Expression profiles altered by co-culture evaluated in order to identify genes associated with melanoma-endothelial cell communication. Panel B is a Western blot of NRP2 expression in GFP-1205Lu cells grown in mono-culture or following co-culture with RFP-HUVECs. Panel C is an IP-Western analysis of NRP2 expression in conditioned medium from RFP-HUVECs, GFP-1205Lu melanoma cells or HLTVEC-1205Lu co-cultures. Panels D - H show immunohistochemical staining for NRP2 showing expression in nerve (C), blood vessels (D), and melanoma metastases (E-G).

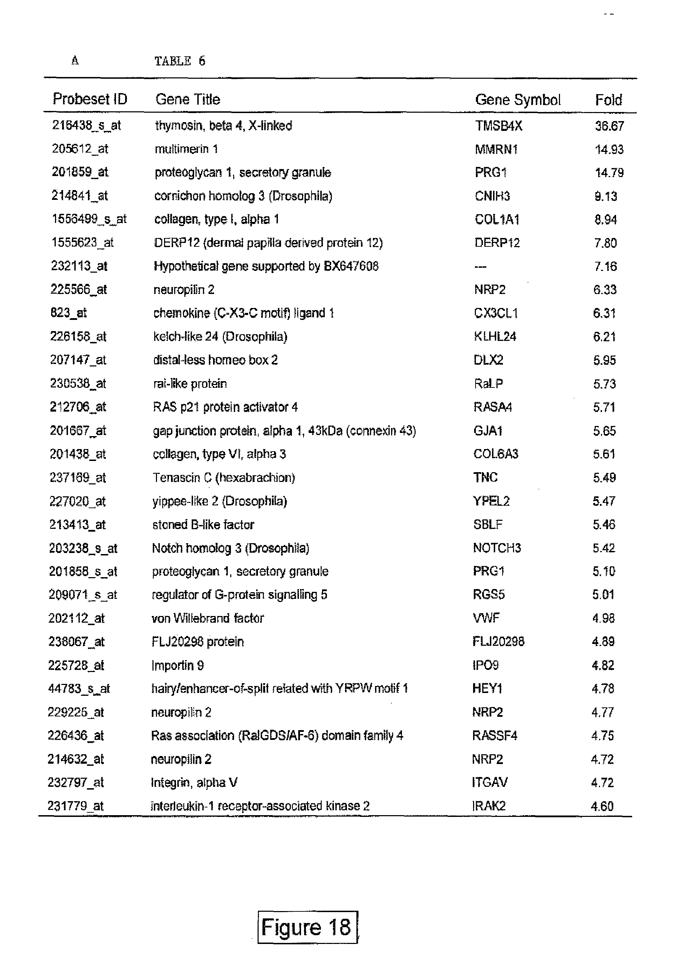

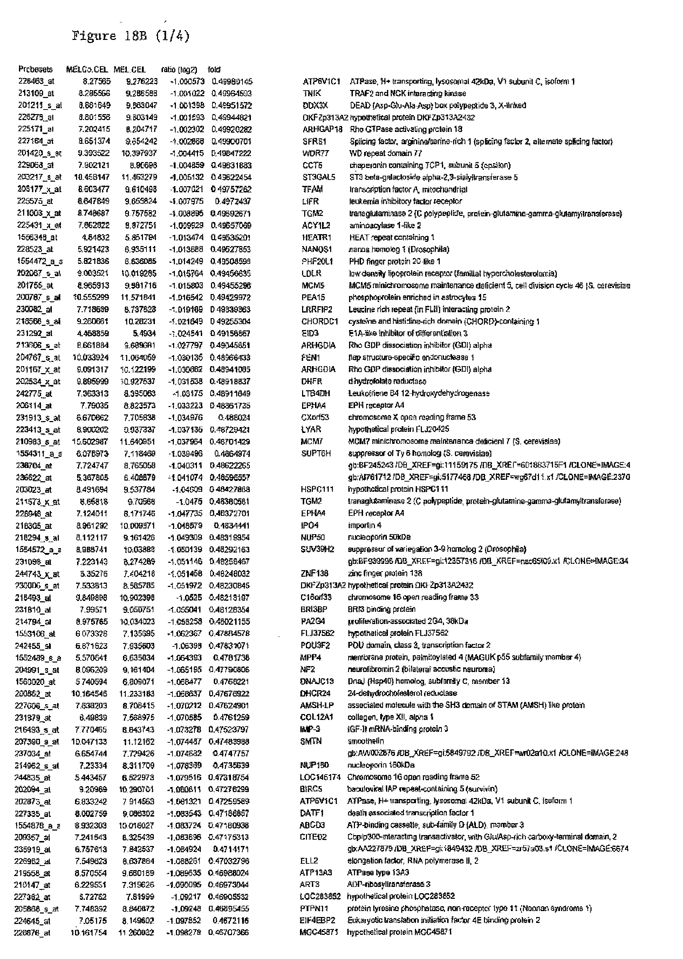

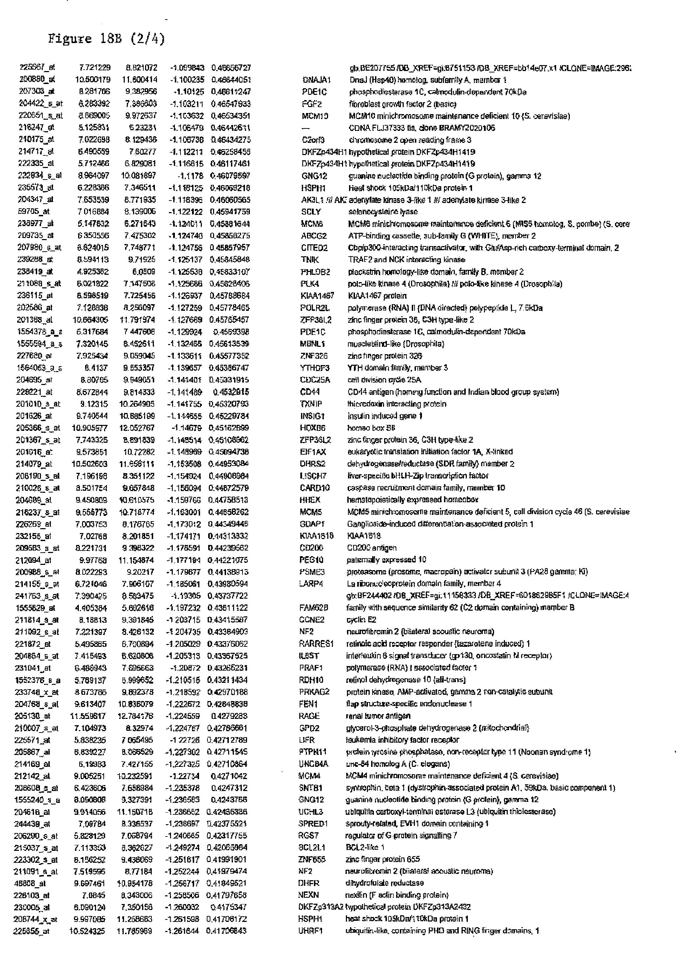

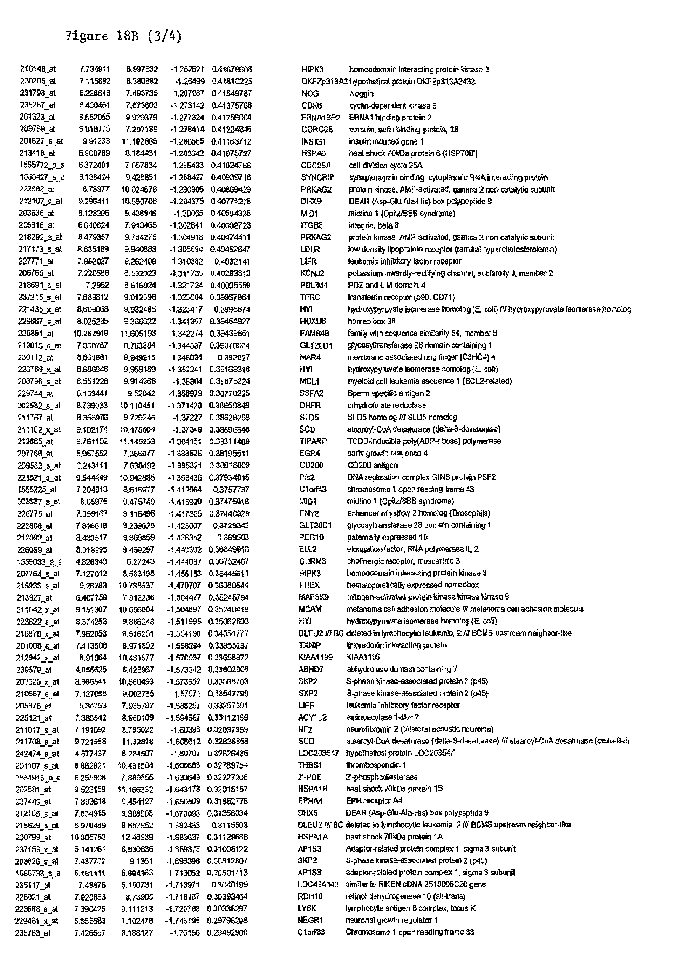

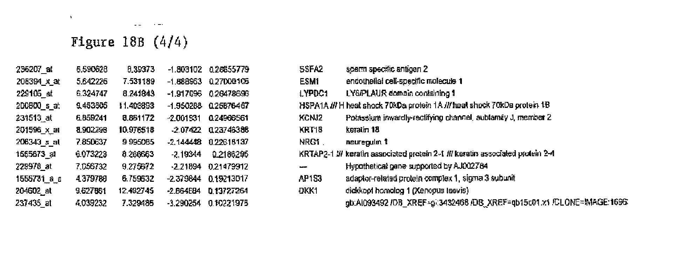

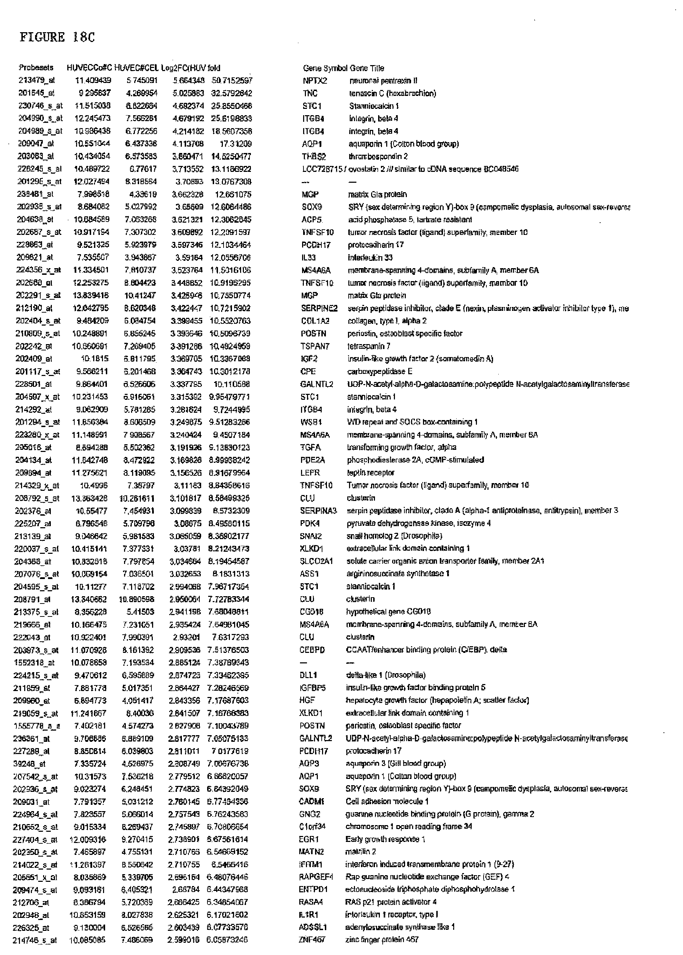

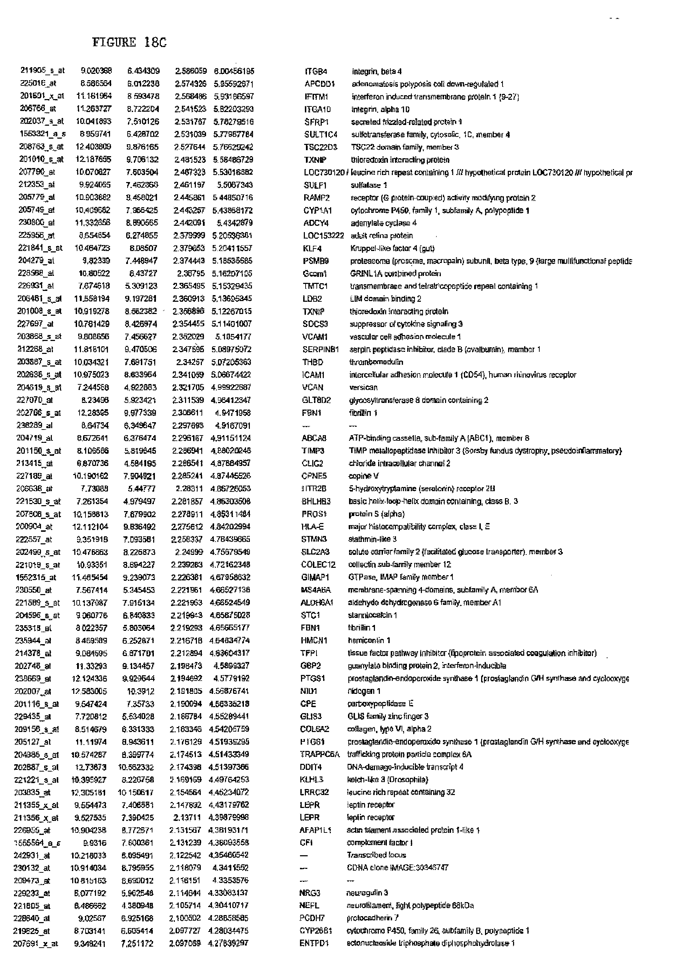

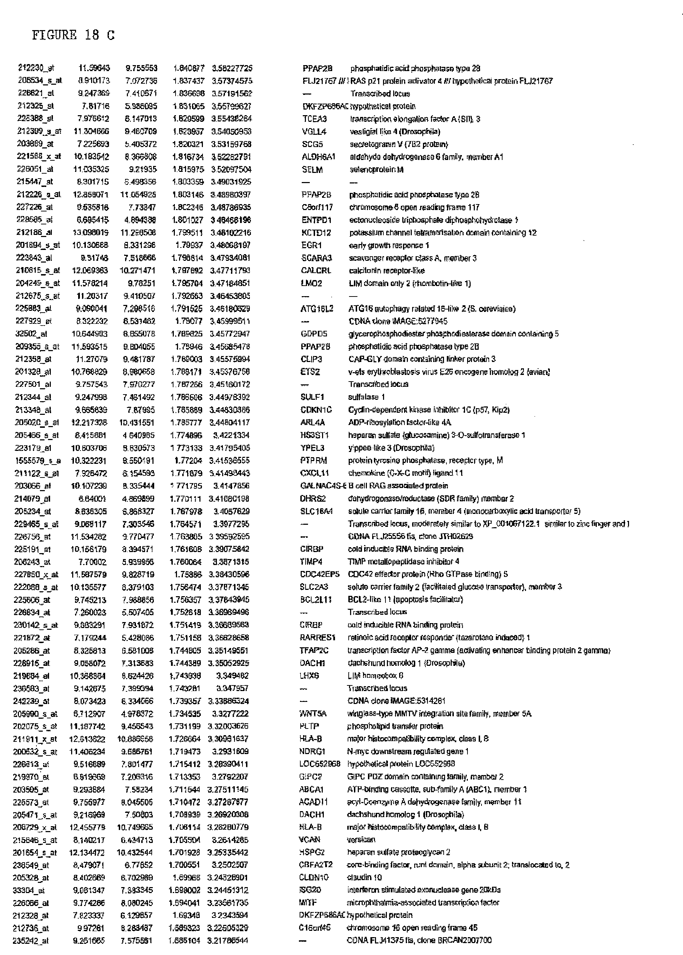

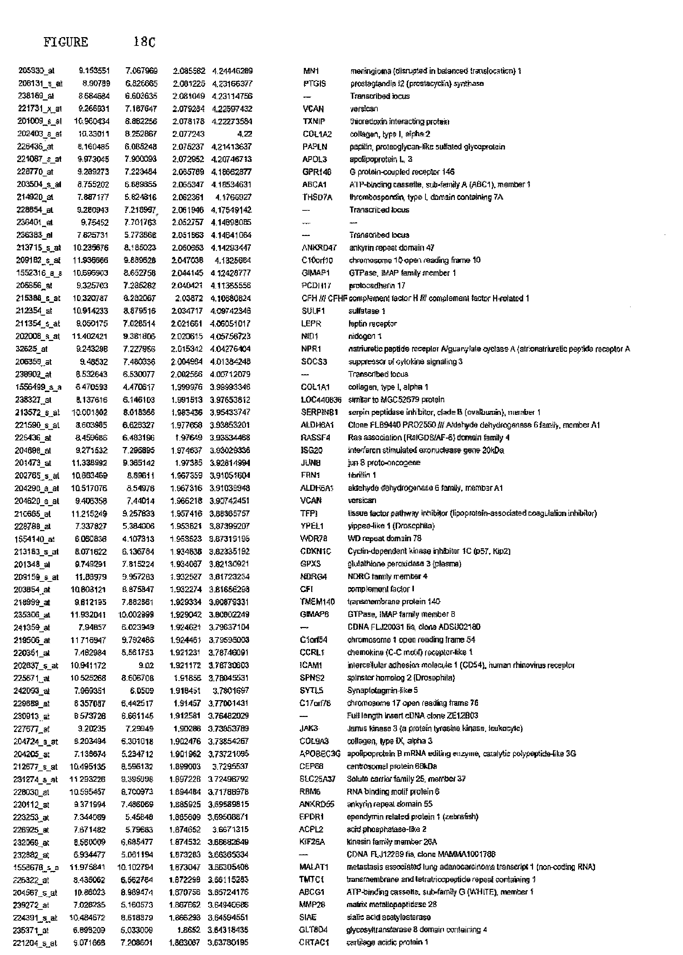

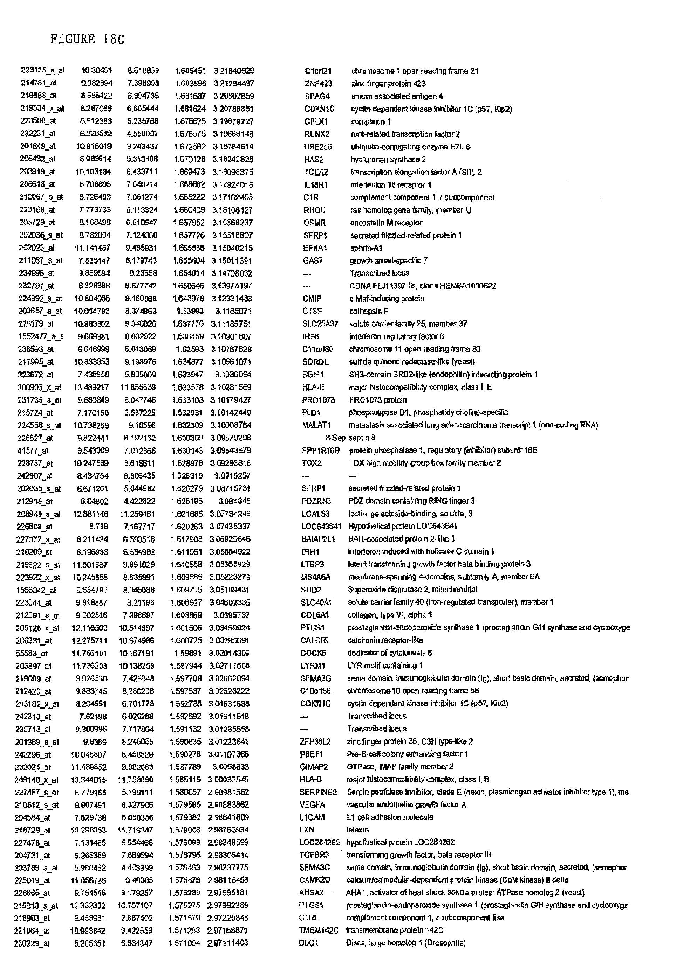

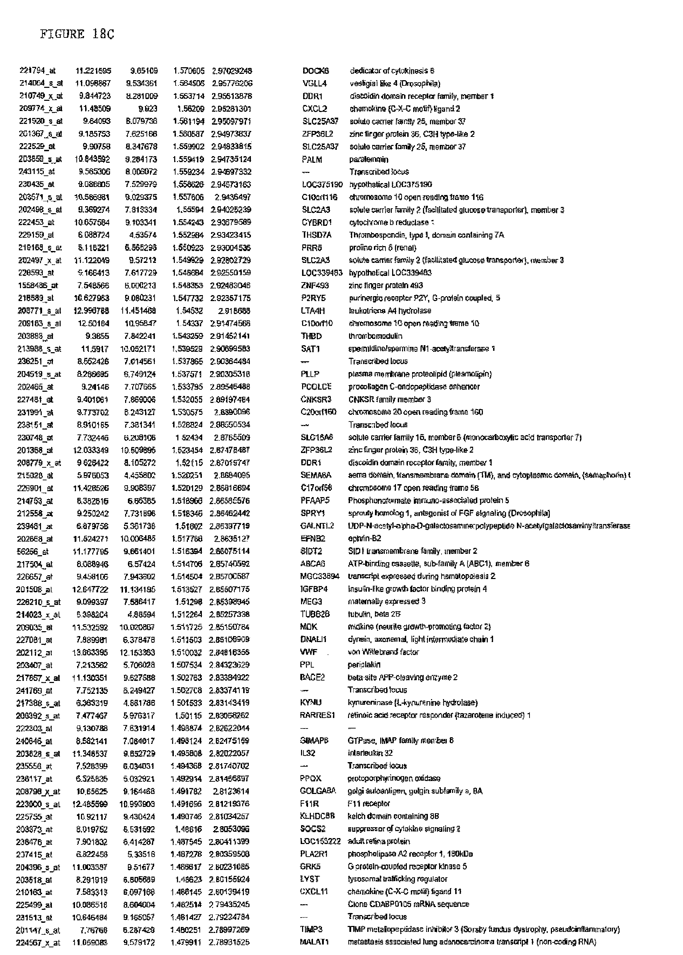

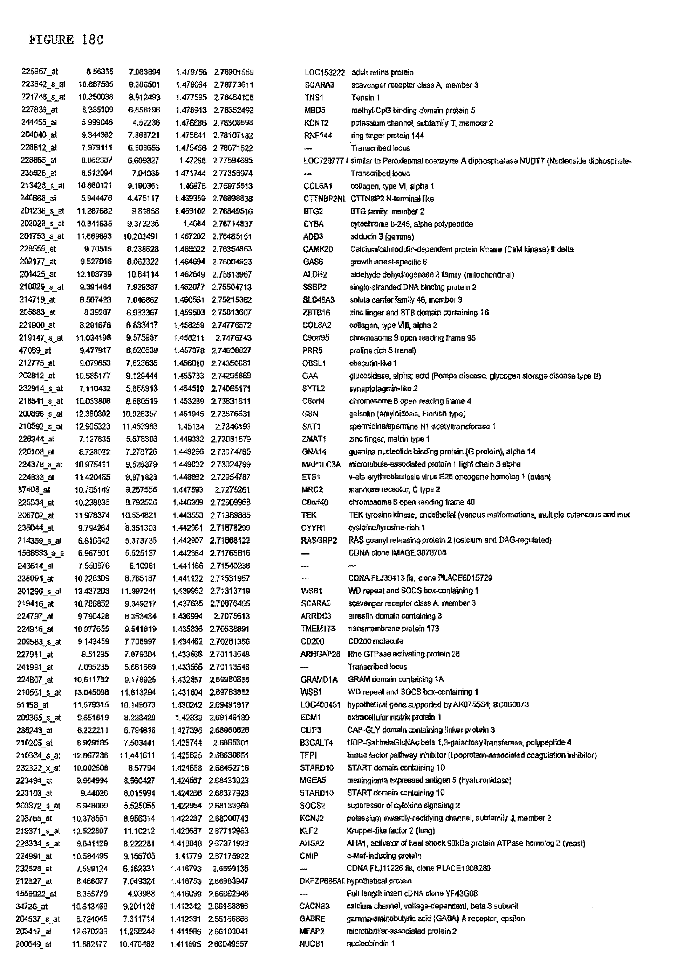

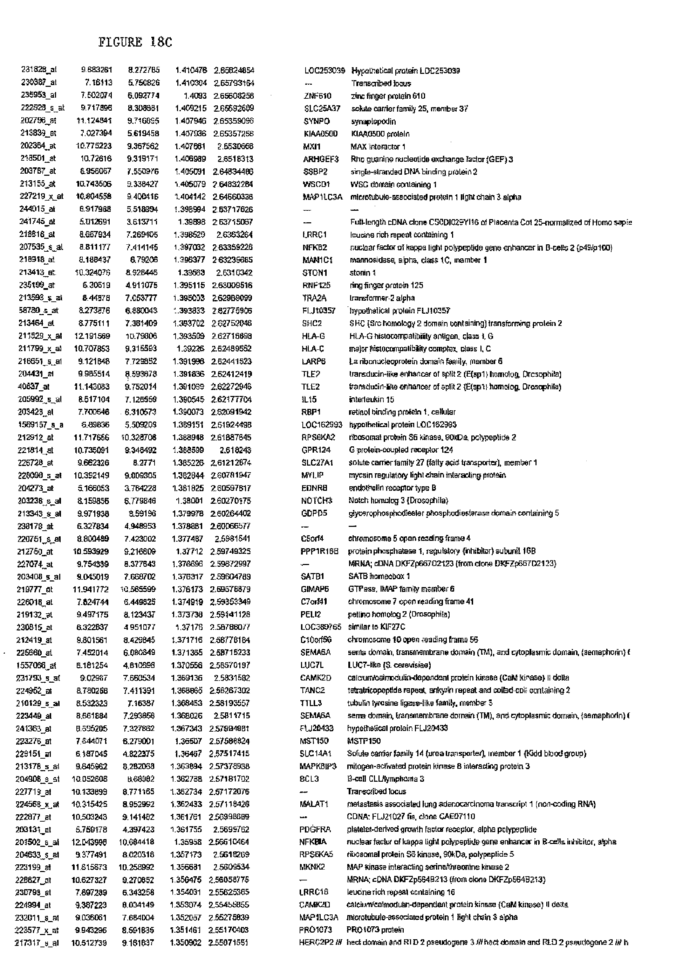

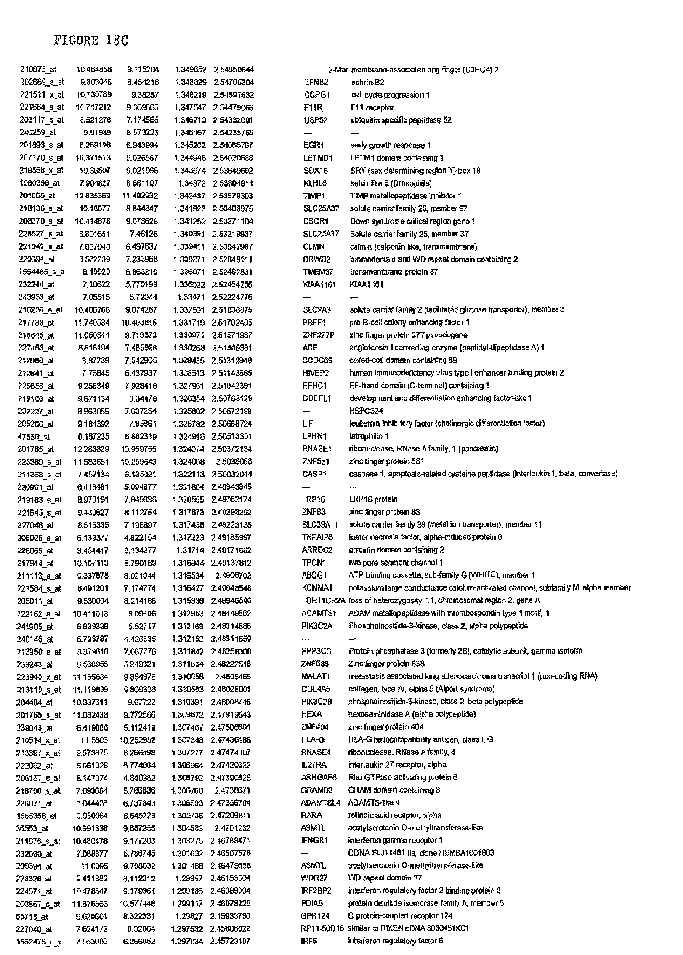

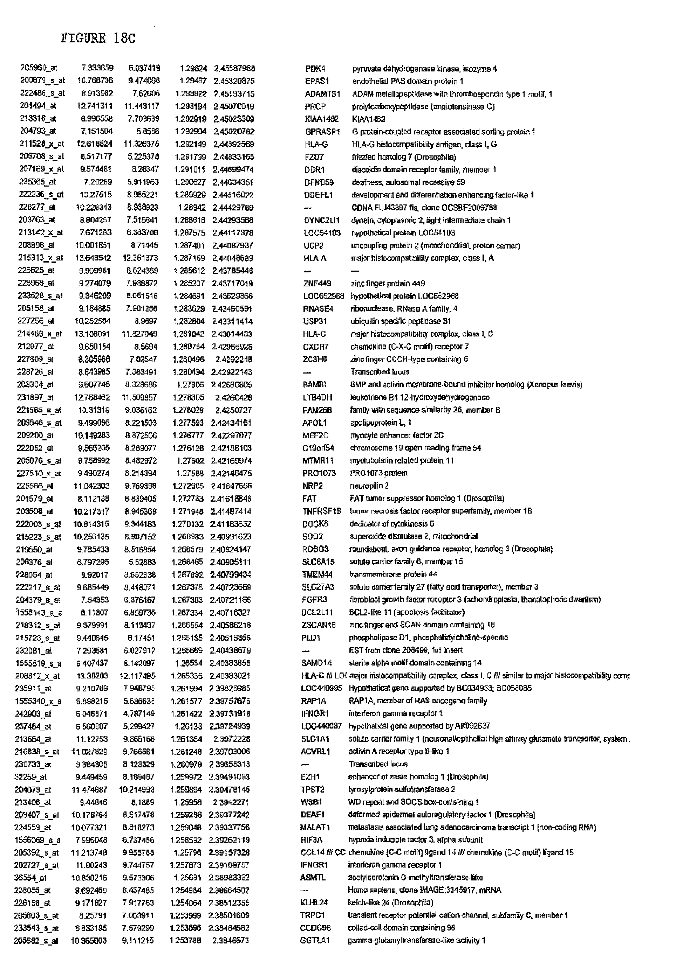

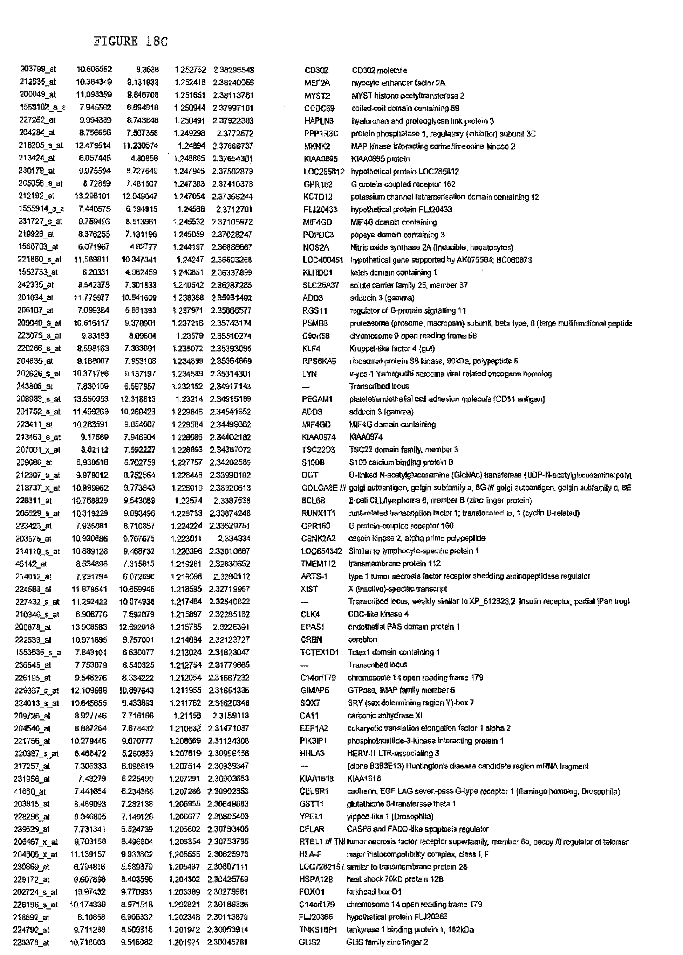

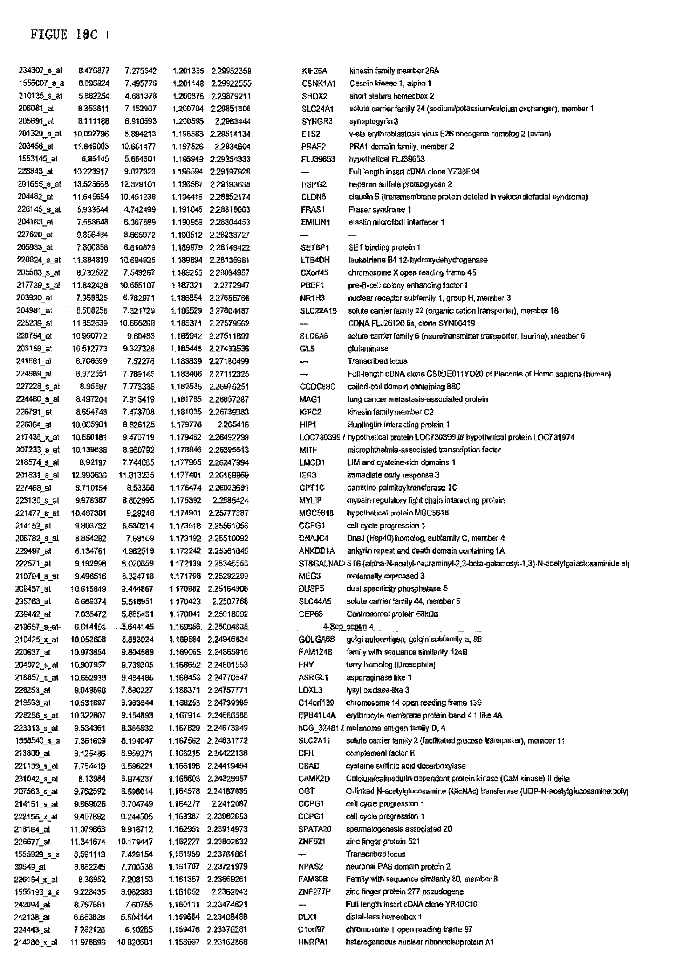

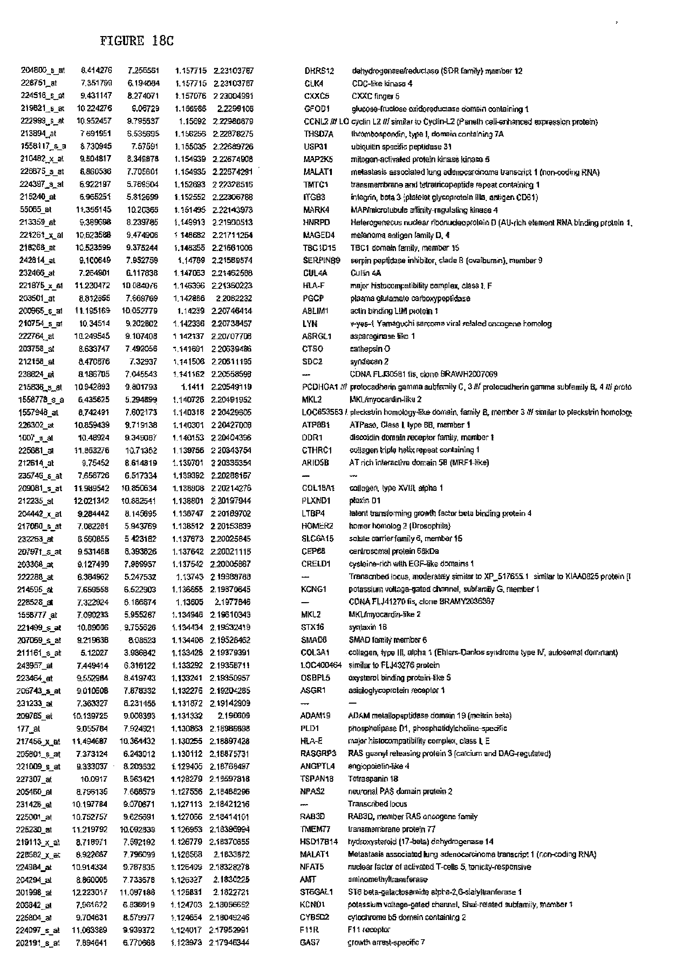

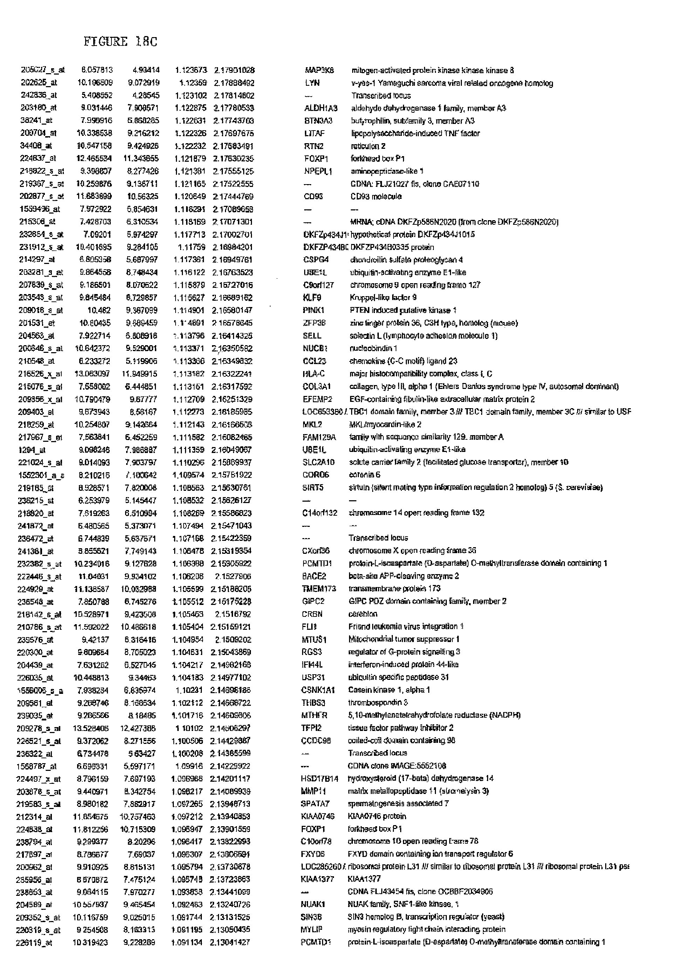

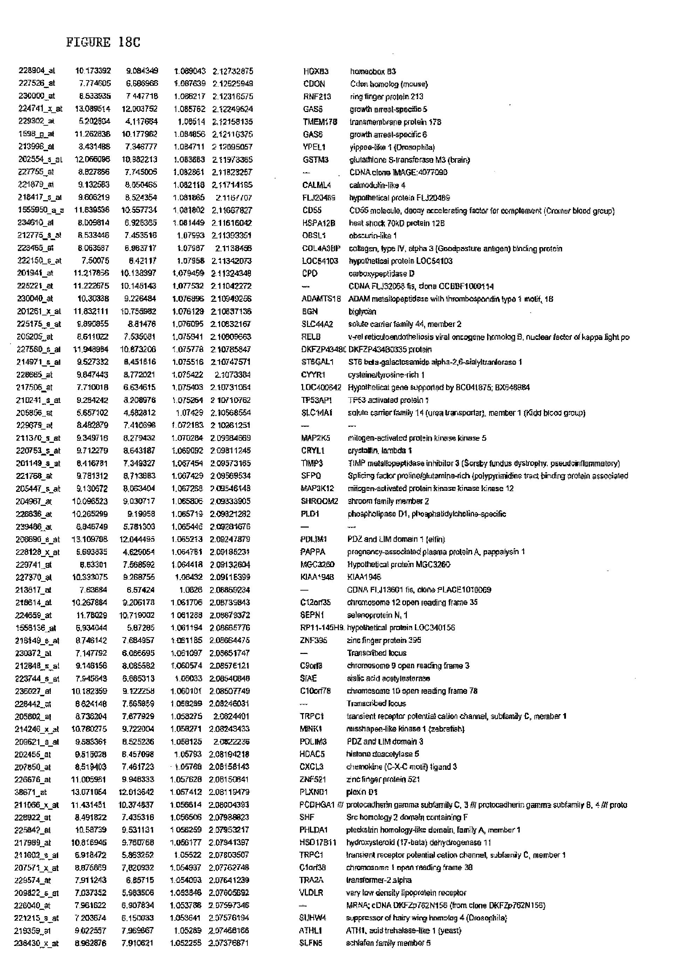

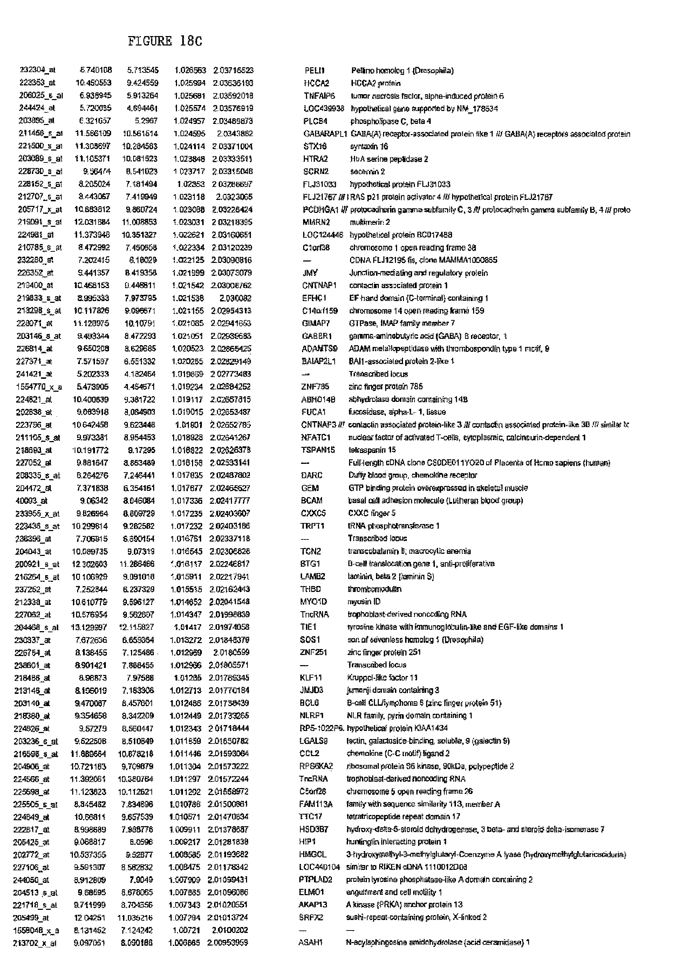

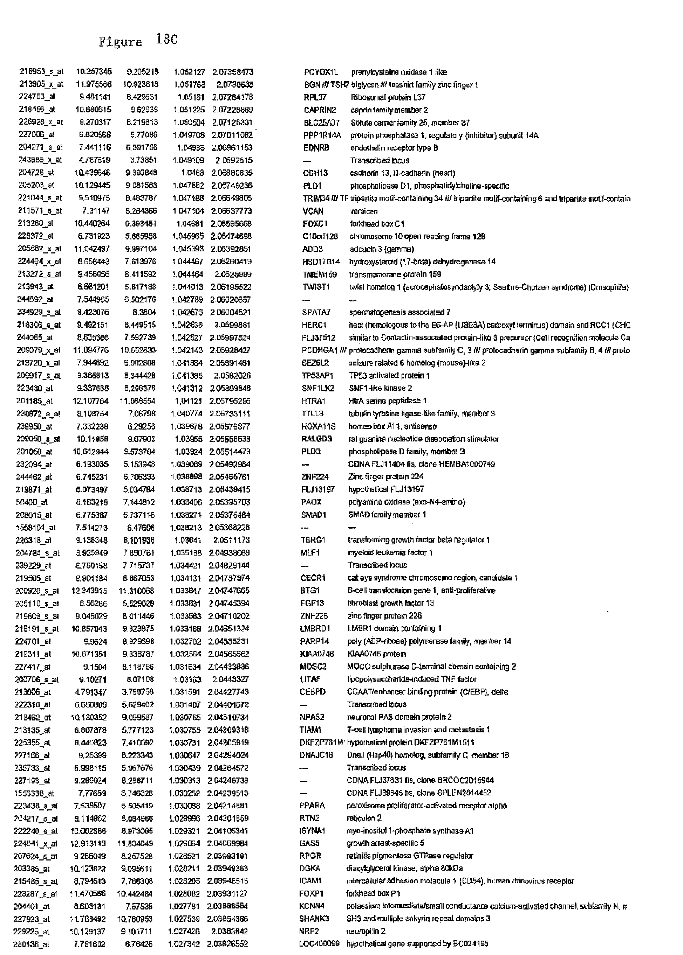

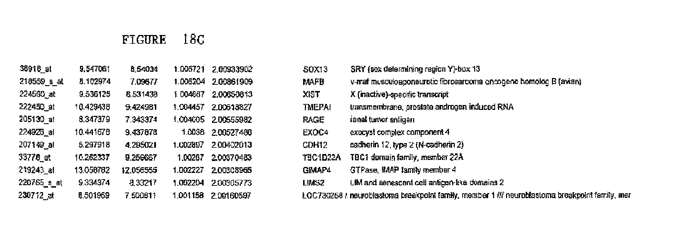

**Figure 18** **(A - C).** Panel A is a Table (Table 6) showing gene expression profiling data for top 30 genes upregulated in melanoma cells following co-culture with HUVECs. A11 data were normalized to melanoma cell expression profiles for single cell cultures. A complete list of all gene expression profiling data is provided in the Tables shown in 18B and 18C for melanoma cells (Panel B) and HUVECs (Panel C). Additionally, raw microarray data from these experiments has been submitted to GEO (Gene Expression Omnibus) under the series record GSE8699.

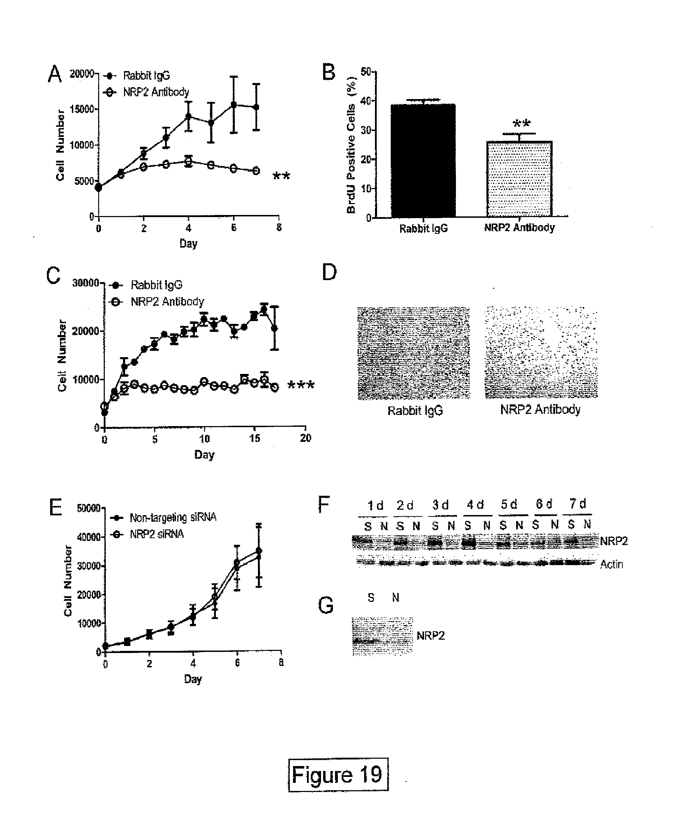

**Figure 19** **(A - G)** shows neutralizing antibody to NRP2 blocks proliferation of metastatic melanoma cells. Panel A is a graph showing the results of a proliferation assay of GFP-1205Lu metastatic melanoma cells in the presence of 10ug/ml normal rabbit IgG or rabbit polyclonal NRP2 antibody. Panel B is a graph showing quantification ofBrdU incorporation in GFP-1205Lu cells following 48 hours of treatment with NRP2 neutralizing antibody. Panel C shows proliferation curves of GFP-1205Lu cells following removal of antibody at day 8. Panel D shows the phenotype of GFP-1205Lu cells following 48 hours of treatment with NRP2 neutralizing antibody or normal rabbit IgG. Panel E is a graph showing the results of a proliferation assay of GFP-1205Lu cells transfected with NRP2 siRNA. Panel F shows the results of Western blot of GFP-1205Lu cells showing loss of NRP2 expression following transfection with NRP2-specific (N) siRNA compared to the non-targeting (-) siRNA control over a 7-day timecourse. Panel G shows the results of Western blot of conditioned medium from GFP-1205Lu cells transfected with non-targeting (-) or NRP2-specific (N) siRNA. *p < 0.05, **p < 0.01, ***p < 0.0001. Error bars represent standard deviation.

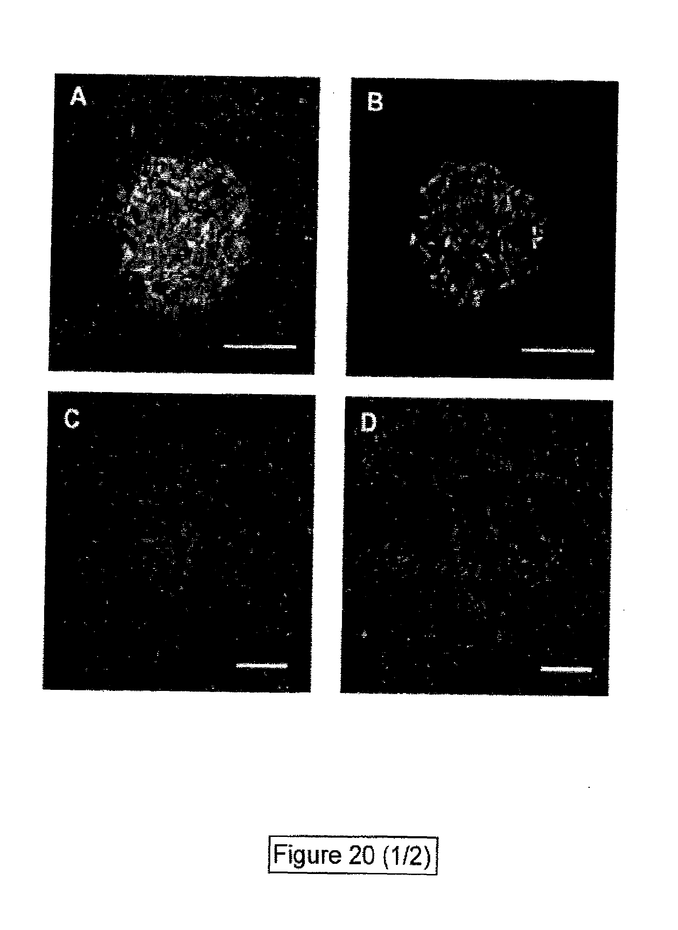

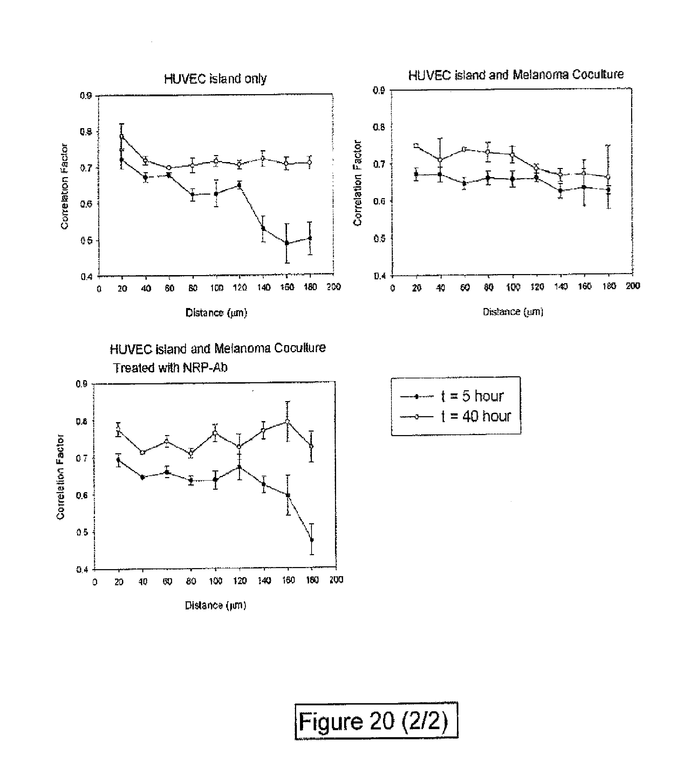

**Figure 20** **(A - E)** shows melanoma cells use NRP-2 to promote distinct cellular patterning by HUVECs. Shown is an analysis of collective cell movements from HUVEC colonies of defined geometry, cell number and size. Panel A shows a typical circular HUVECs colony surrounded by a monolayer of 1205Lu melanoma cells. Panel B shows a typical control HUVEC colony with the same characteristics as in B, but in the absence of melanoma cells. Panel C shows the evolution of a typical circular HUVECs colony organization 5 hrs after initiation of co-culture. Panel D shows the state for the same colony as in C at 40 h after initiation of co-culture. Panel E shows a quantitative analysis of collective cell movement using the velocity correlation function defined in the Methods. The metric is calculated for various distances for three types of HUVEC culture indicated. Three independent co-culture experiments were performed for each condition. The error bars represent the standard error of the mean.

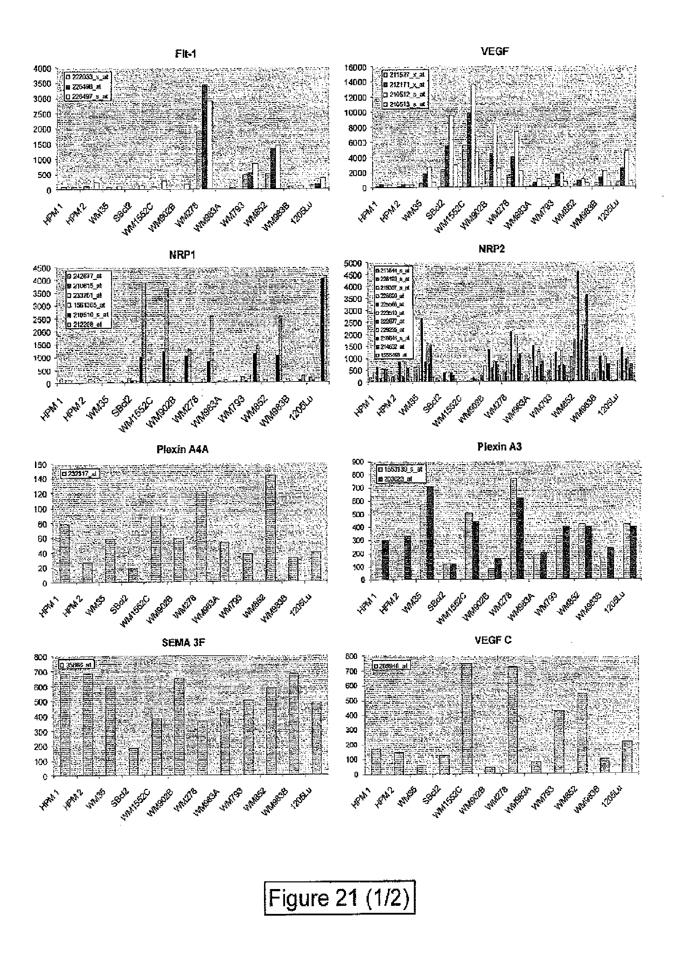

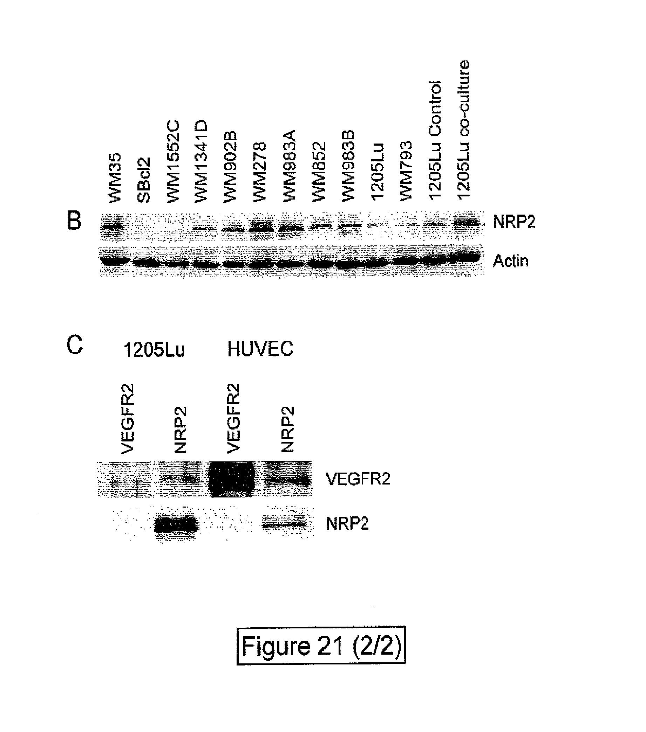

**Figure 21** **(A - C)** shows melanoma cells from varying stages of progression express Neuropilin-2 and related cellular receptors and ligands. Panel A is a graphical depiction of expression profiles of NRP-2, associated ligands, and receptors in melanoma cell lines from varying stages of progression. Panel B is a Western blot showing Neuropilin-2 expression in melanoma cell lines from varying stages of progression. Panel C is an IP- Western blot that shows VEGFR2 is expressed in 1205Lu cells and forms complexes with NRP2.

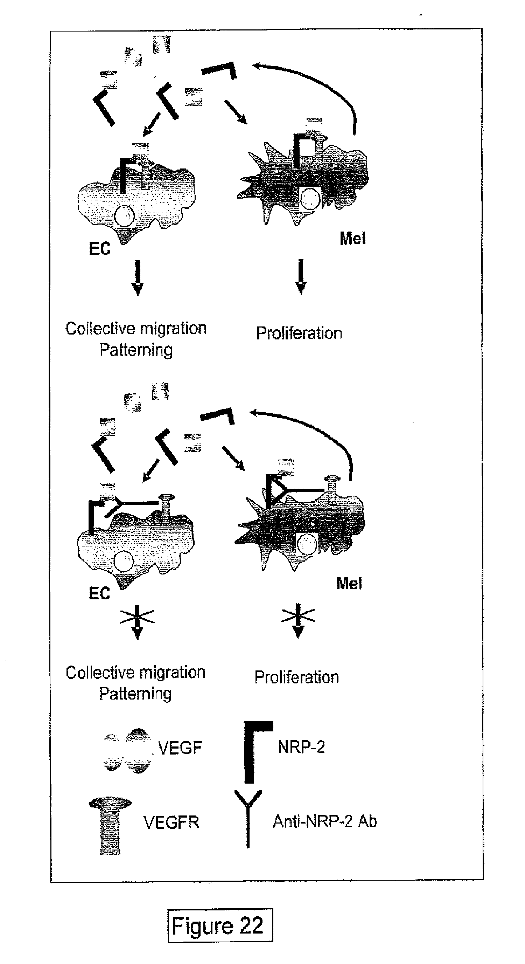

**Figure 22****.** is a schematic showing a model for NRP-2 functions in melanoma-endothelial cell communication and melanoma metastasis.

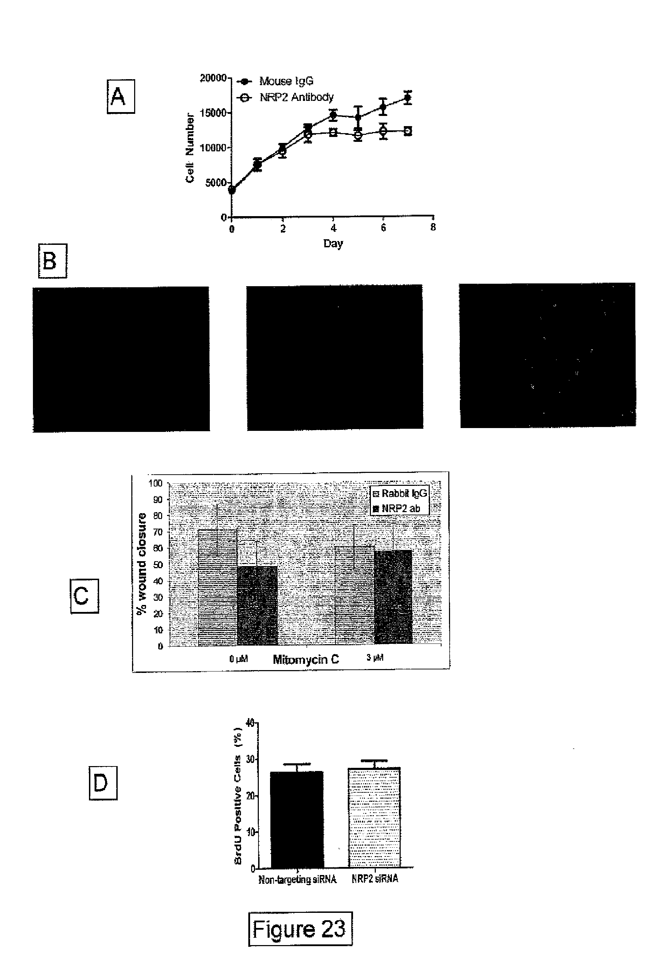

**Figure 23** **(A - D)** shows the effects of Nzp2 gene silencing.

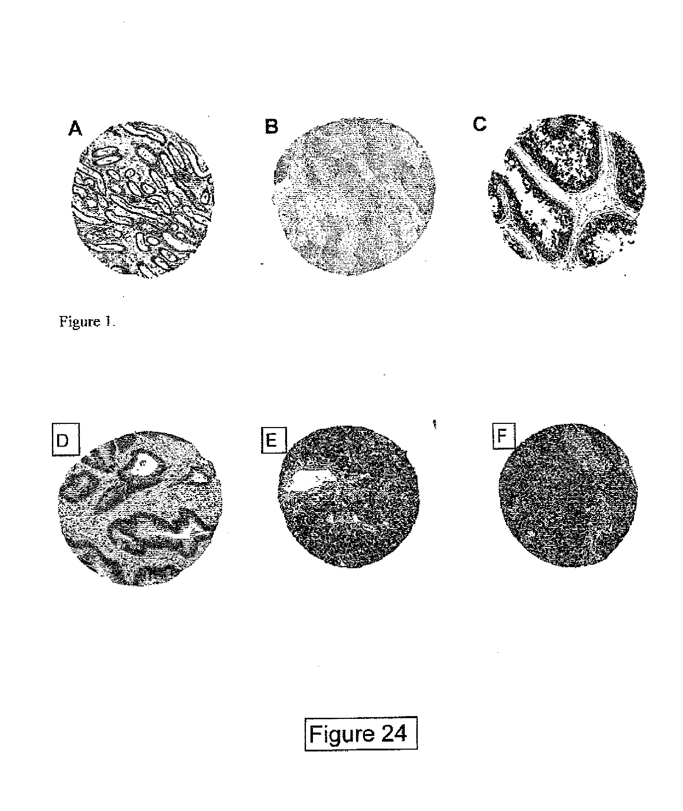

**Figure 24** **(A - F)** show representative staining for Neuropilin-2 in normal human tissues and non-melanocytic human tissues. Panels A - C show representative staining for Neuropilin-2 in normal human tissues and non-melanocytic human tissues. Panel A shows Normal Kidney, Panel B shows Striated Muscle, Panel C shows Testis. Panels D - F show representative staining for Neuropilin-2 in non-melanocytic tumors. Panel D shows Colon Adenocarcinoma, Panel E shows Renal Cell Carcinoma (Clear Cell), Panel F shows Ductal Breast Carcinoma.

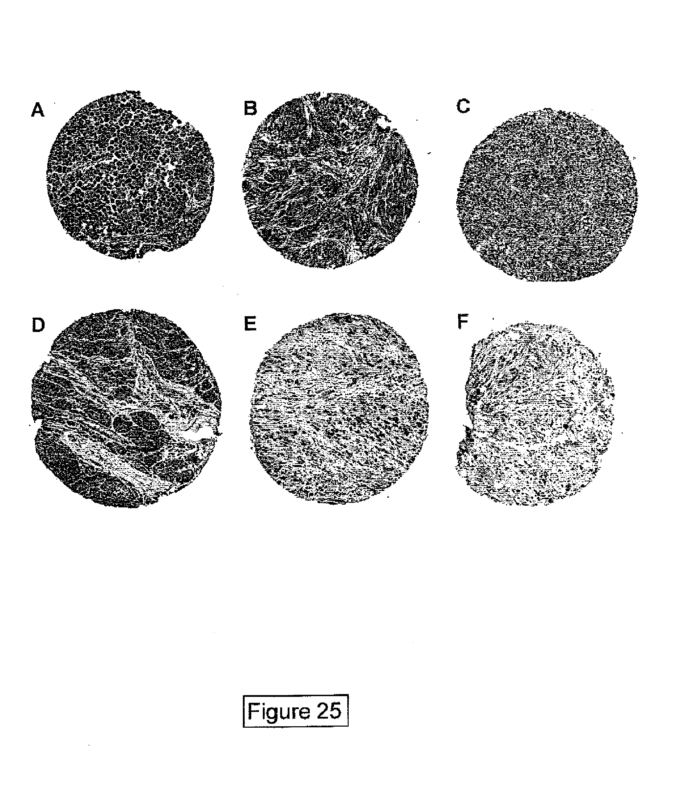

**Figure 25** **(A - F)** shows representative staining for Neuropilin-2 in non-melanocytic tumors. Panel A shows metastatic amelanotic epithelial melanoma, Panel B shows malignant melanoma, Panel C shows metastatic amelanotic small cell malignant melanoma, Panel D shows pigmented epithelial melanoma, Panel E shows spindle cell nodular melanoma, Panel F shows desmoplastic malignant melanoma.

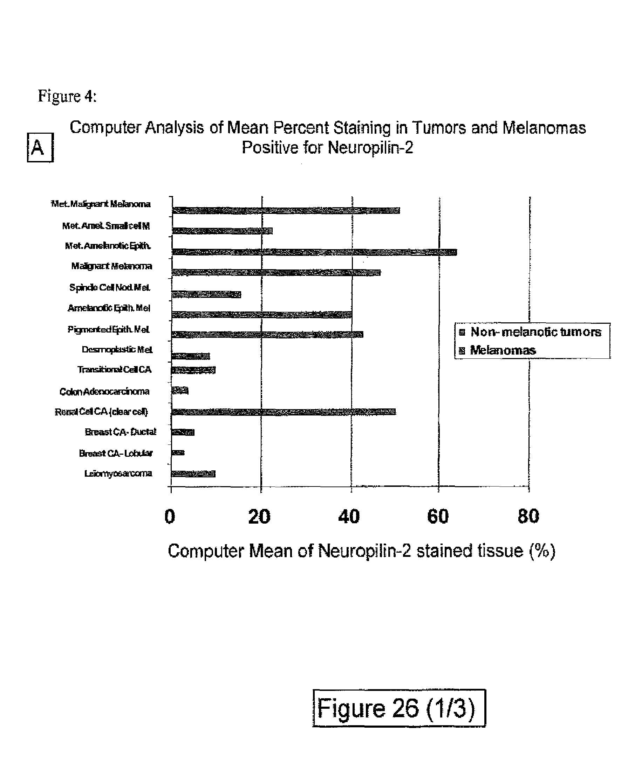

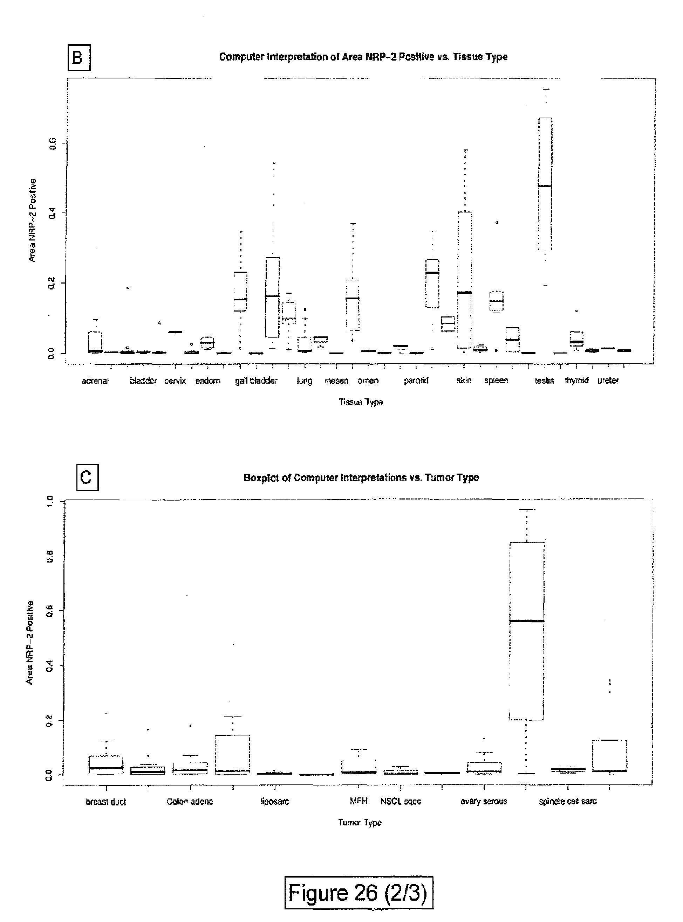

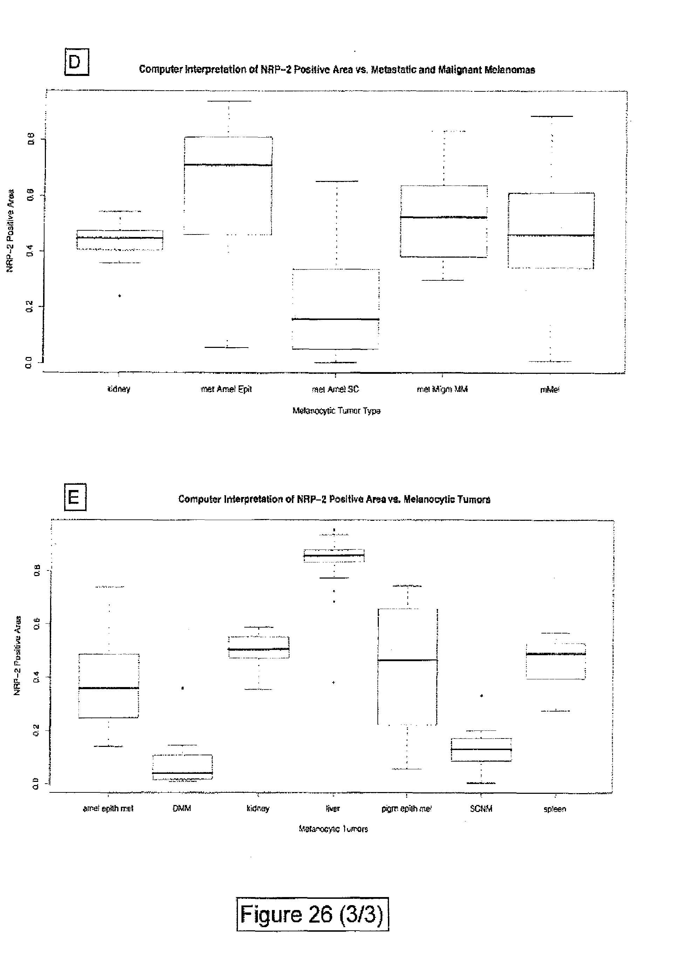

**Figure 26** **(A - E)** shows computer analysis of NRP-2 staining. Panel A is a graphic depiction of quantified tissue staining for Neuropilin-2 in melanocytic Tumors (Green) and Non-Melanocytic Tumors (Pink). Panel B is a graph that shows a computer interpretation of Area NRP-2 positive vs. tissue type. Panel C is a graph that shows a boxplot of the computer interpretation vs. the tumor type. Panel D is a graph that shows a computer interpretation of Area NRP-2 positive vs. metastatic and malignant melanomas. Panel E is a graph that shows a boxplot of the computer interpretation vs. melanocytic tumor type.

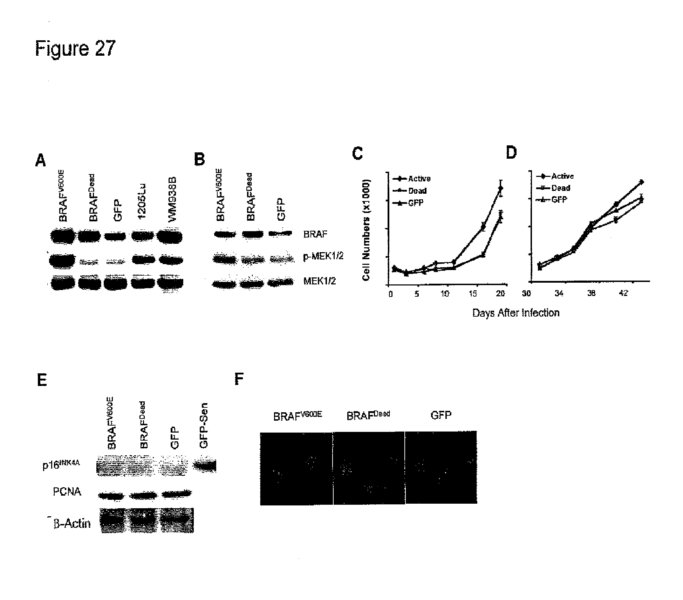

**Figure 27** **(A - F)** shows promotion of cell growth in primary human melanocytes expressing physiologic levels of BRAF^{V600E}. Panels A and B show Western blot analysis of primary human melanocytes infected with lentiviral vectors expressing active BRAF kinase (BRAF^{V600E}), inactive BRAF kinase (BRAF^{Dead}) or GFP and melanoma cell lines expressing BRAF^{V600E} (1205Lu, WM938B). BRAF kinase activity is monitored through MEK phosphorylation (p-MEK1/2) at early (A) passage (day4), and late (B) passage (day 30). Panels C and D are graphs that show the results of growth assays of primary human melanocytes infected with the above-described BRAF kinase mutants at early (C) and late (D) passage. Panel E is a Western blot evaluating p16/INK4a, PCNA, and actin expression in primary human melanocytes expressing active BRAF kinase (BRAF^{V600E}), inactive BRAF kinase (BRAF^{Dead}) or GFP at late timepoints (day 30) following infection. Primary human melanocytes (GFP-Sen) showing replicative senescence (4 to 5 month cultured) were used for positive control of p16/INK4a detection. Panel F is three panels showing telomere FISH assessment for BRAF kinase mutants in primary human melanocytes at late passage (day 30).

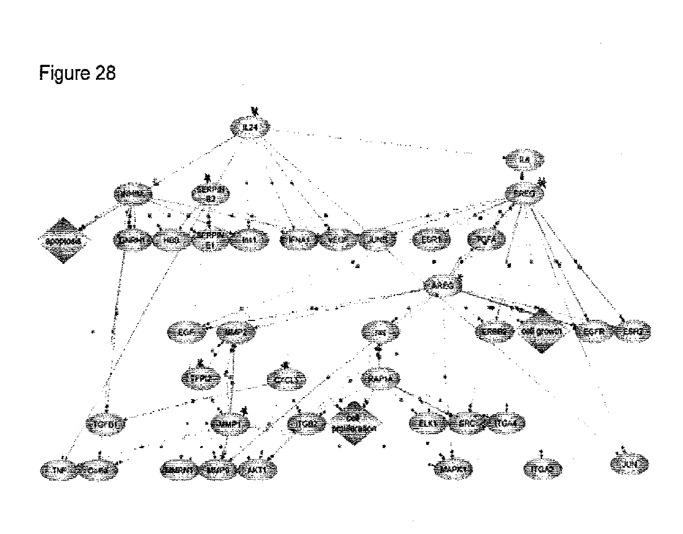

**Figure 28** is a schematic showing network target mapping of BRAF downstream effector genes in primary human melanocytes. Mapping of network targets induced by activated BRAF kinase identifies cell growth, cell proliferation, and apoptosis as cellular processes that are critically mediated by the BRAF gene signature. The ten most highly upregulated BRAF kinase target genes in primary human melanocytes were subjected to network target mapping using a data mining software (Pathway Architect, Stratagene, La Jolla, CA) to detect cellular processes. Eight BRAF effector genes (asterisks) were recognized as nodal genes.

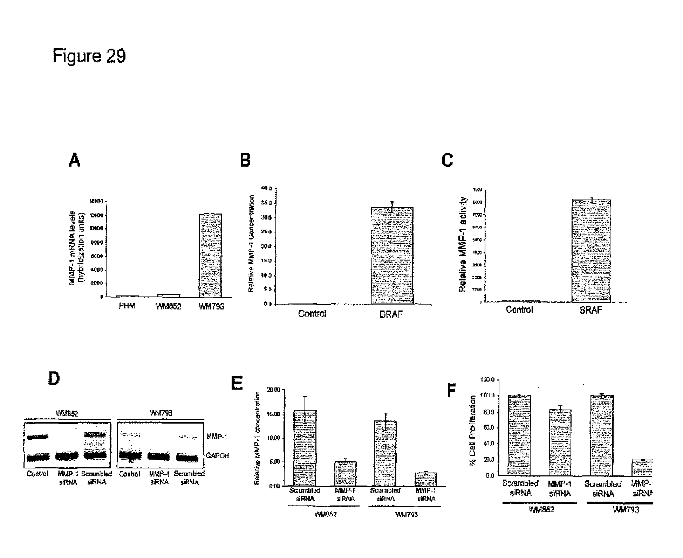

**Figure 29** **(A - F)** shows preferential activation of cell growth by MMP-1 in melanomas expressing mutant versus wildtype BRAF kinase. Panel A is a graph that shows relative MMP-1 mRNA levels in primary human melanocyte and melanomas expressing wildtype BRAF (WM852), or BRAF^{V600E} (WM793). Panel B is a graph that shows relative levels of secreted MMP-1 in conditioned media obtained from primary human melanocytes (uninfected), or those expressing GFP alone (GFP) or BRAF^{V600E} (BRAF) at 72 hours following lentiviral infection. Panel C is a graph that shows relative MMP-1 collagenase activity in conditioned media obtained from primary human melanocyte (uninfected), or those expressing GFP alone (GFP) or BRAF^{V600E} (BRAF) at 72 hours following lentiviral infection. Panel D shows the results of semi-quantitative duplex RT-PCR analysis of MMP-1 expression following gene silencing by siRNA in melanomas possessing either wildtype BRAF (WM852) or BRAF^{V600E} (WM793). Penal E is a graph that shows relative MMP-1 concentration in cell culture media following MMP-1 gene silencing by siRNA versus scrambled control siRNA in melanomas possessing wildtype BRAF (WM852) and BRAF^{V600E} (WM793) cells. Panel F is a graph that shows ³H-thymidine cell proliferation assay of melanomas possessing wildtype BRAF (WM852) and BRAF^{V600E} (WM793) following MMP-1 gene silencing by siRNA versus control scrambled siRNA. Percent cell proliferation shown is a percentage of the control.

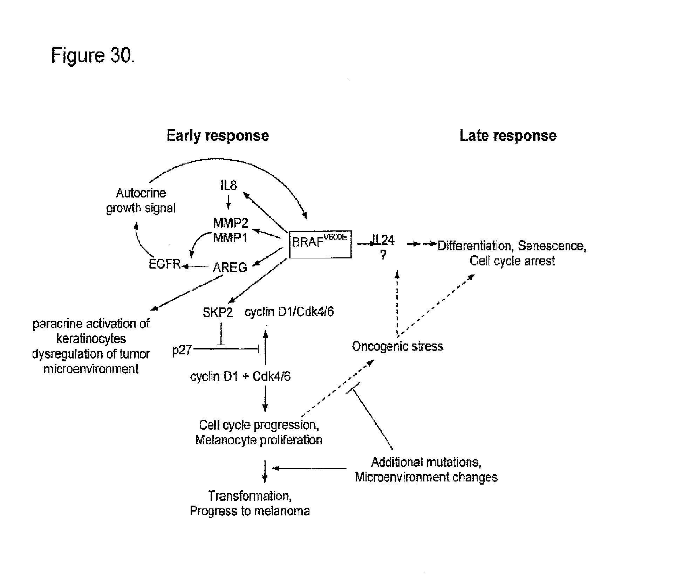

**Figure 30** is a schematic that shows a model for BRAF kinase downstream effector functions in primary human melanocytes and their roles in melanoma development. The model shows that early response BRAF kinase effectors include several growth promoting genes such as MMP-1, amphiregulin, SKP-2 and IL-8. Constitutive activation of BRAF kinase in primary human melanocytes results in an early growth promoting response (pathways at left) characterized by autocrine/paracrine activation of epidermal growth factor receptor (EGFR) signaling via MMP-1 or MMP-2 cleavage of the EGFR ligand, amphiregulin, and activation of Cyclin D1/cdk complexes via SKP-2 mediated proteosomal degradation of p27. Later responses to BRAF kinase in primary human melanocytes trigger a growth arrest mediated, in part, by upregulation of IL-24, as seen in benign nevi. In the case of additional acquired mutations that occur prior to growth arrest of benign nevi, phenotypic outcomes may include premalignant or malignant changes resulting in cellular transformation.

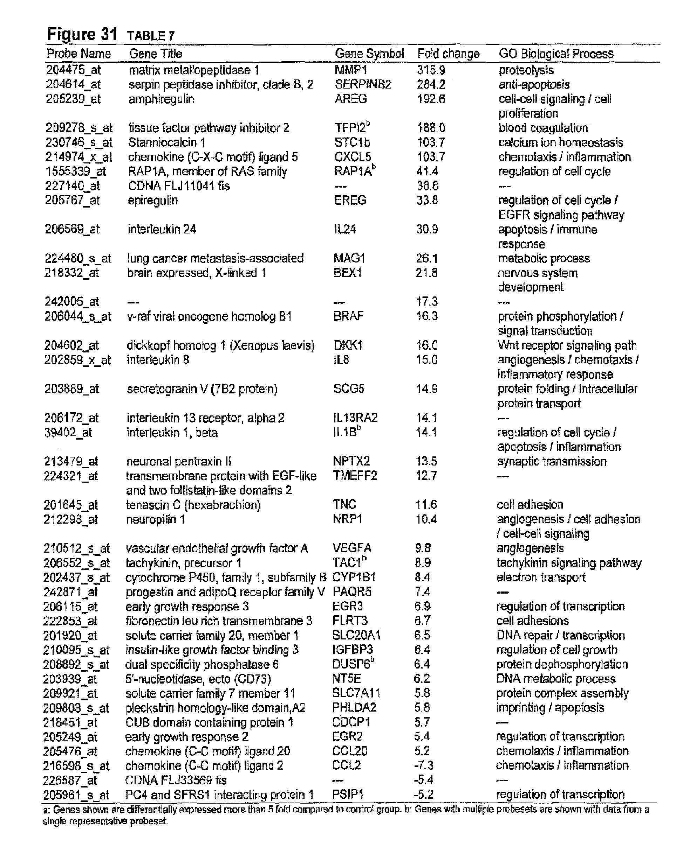

**Figure 31** is a Table (Table 7) showing annotated genes that are differentially expressed by oncogenic BRAF^{V600E} in primary human melanocytes. The genes that are shown are differentially expressed more than 5-fold compared to the control group. Genes with multiple probe sets are shown with data from a single representative probe set.

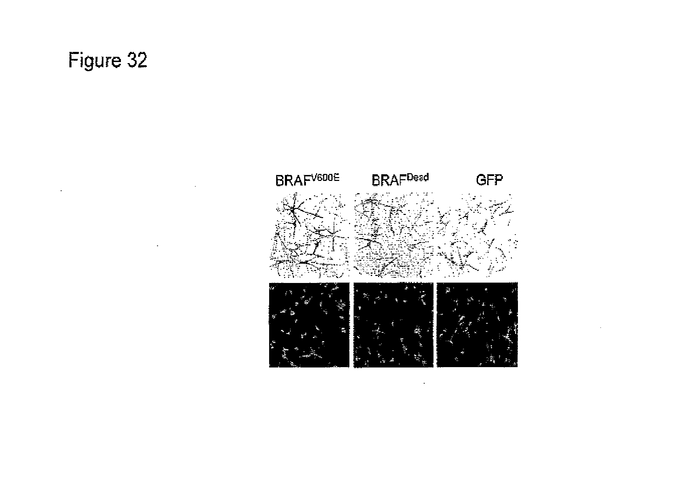

**Figure 32** is a series of 6 panels that show senescence-associated-β-gal (SA-β-gal) activity of primary human melanocytes expressing BRAF kinase mutants at late passage (day 30). No appreciable SA-β-gal activity was noted in any of the transduced cells on day 30. GFP expression is shown (bottom panel) and indicates >95% of cells are expressing the lentiviral vector.

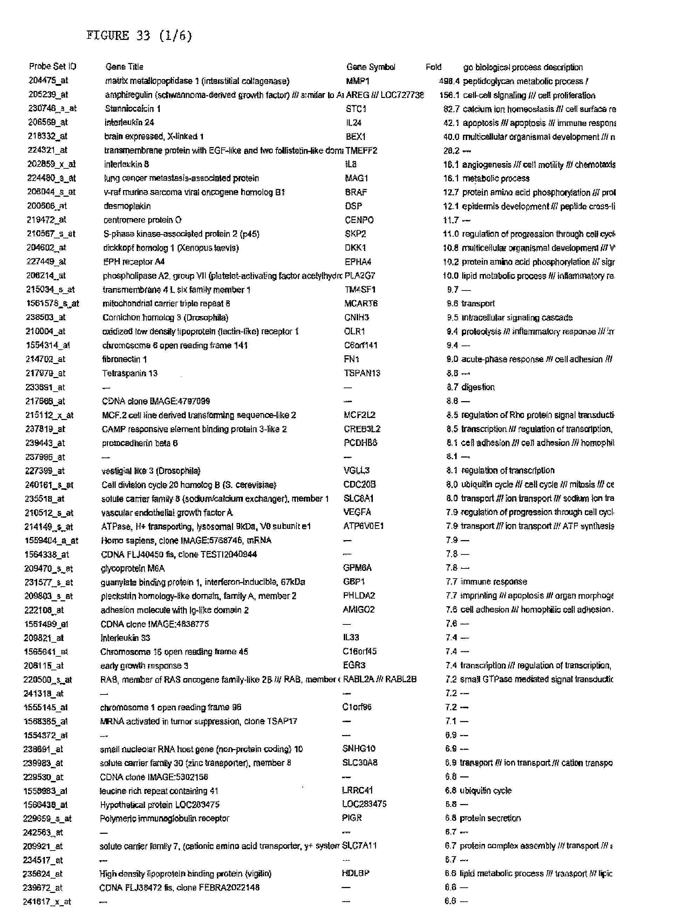

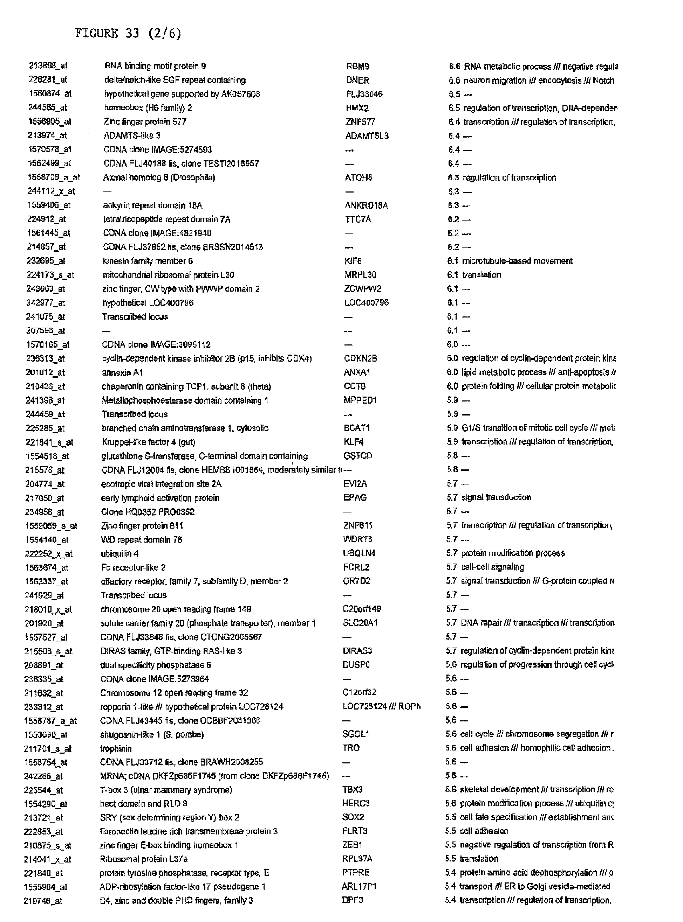

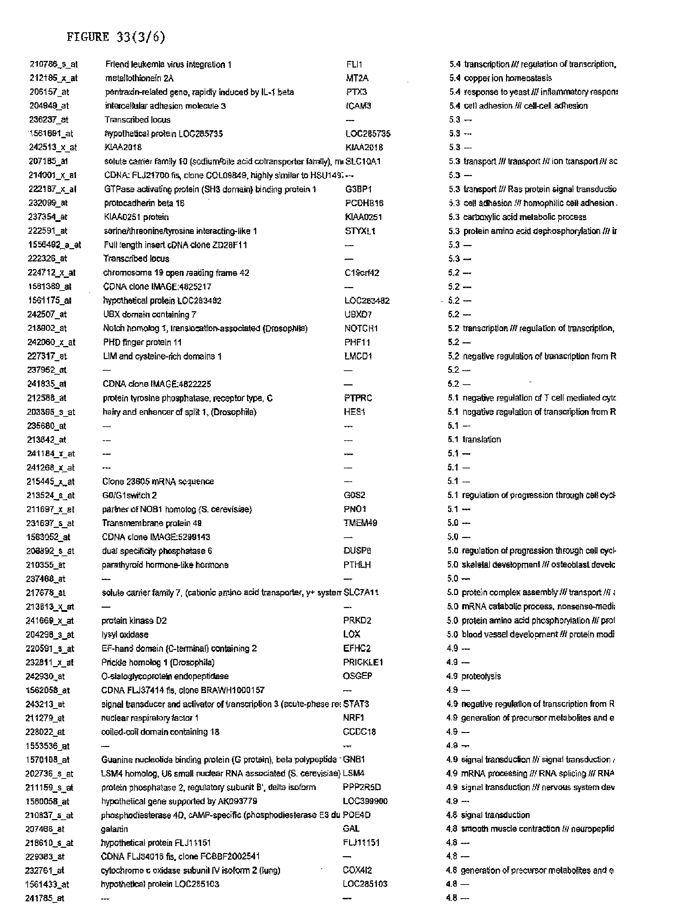

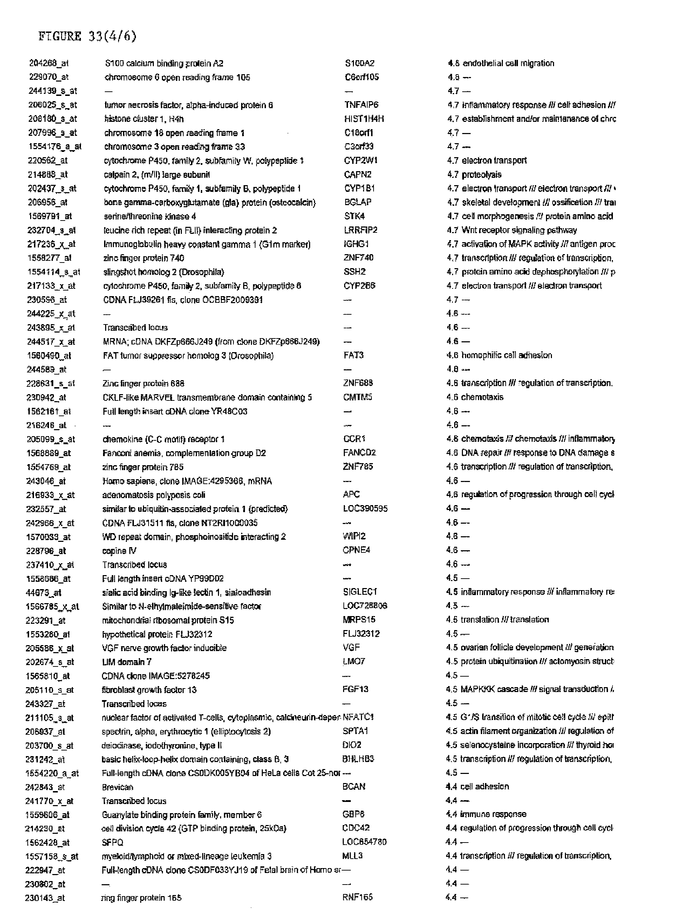

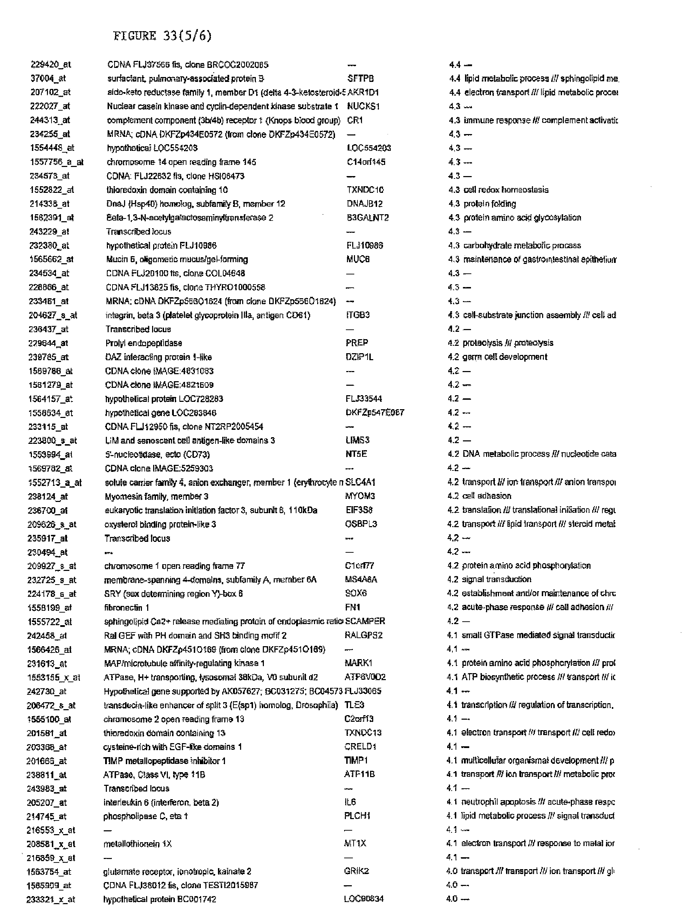

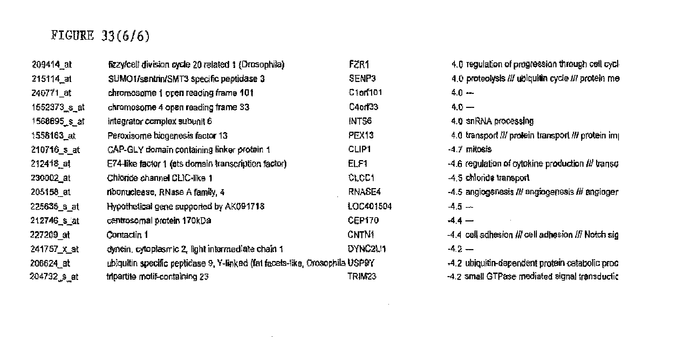

**Figure 33** is a Table (Table 9)showing annotated genes that are differentially expressed by oncogenic BRAF^{V600E} in primary human melanocytes. The genes that are shown is a list of all the genes that are differentially expressed compared to the control group.

Other aspects of the invention are described *infra.*

### DETAILED DESCRIPTION

The present invention provides biomarkers for melanoma status, where the biomarker are correlated with the progression or stage of melanoma. In particular, the invention provides novel biomarkers that may be used to monitor disease onset or progression in patients with known previous diagnosis of melanoma and in patients at high risk for the development of melanoma. These progression-associated genes will be useful as diagnostic/prognostic tumor markers as well as novel therapeutic targets. The invention provides that these biomarkers, used individually, or in combination with other biomarkers from this group or with other diagnostic tests, provide a novel method of determining melanoma status in a subject, and correlating the status of melanoma to the progression or stage of disease.

The present invention presents markers that are differentially present in samples of melanoma subjects and control subjects, and that are differentially present at different stages of melanoma progression, and the application of this discovery in methods and kits for determining melanoma status. These biomarkers are found in samples from melanoma subjects at levels that are different than the levels in samples from subject in whom human melanoma is undetectable. Accordingly, the amount of one or more markers found in a test sample compared to a control, or the presence or absence of one or more markers in the test sample provides useful information regarding the melanoma status of the subject. In particular, the detection of these markers is particularly useful for detecting recurrent melanoma in a patient that has a high risk for recurrent disease.

The present invention also relates to a method for identification of biomarkers for melanoma, with high specificity and sensitivity. Biomarkers were identified that are associated with melanoma status. Biomarkers were identified that are associated with stages of melanoma progression, and can be correlated with melanoma status, progression of disease and risk of recurrence. In particular, certain of the biomarkers identified are specifically upregulated in aggressive melanomas which are melanomas that lead to the greatest mortality, and thus many of these aggressive melanoma genes will function as effective therapeutic targets for invasive melanomas.

The present invention also relates to a method for identification of biomarkers for melanoma that can be used to determine the prognosis of a patient. In particular, biomarkers were identified that can be associated with more aggressive melanoma or less aggressive melanoma.

In accordance with one embodiment of the invention, a series of genes whose expression correlates with melanoma progression and invasion were identified. Preferred up-regulated melanoma progression markers are secreted proteins detectable in circulating blood.. These secreted biomarkers are useful to determine disease onset/progression, to determine prognosis, to determine risk of recurrence and to determine course of therapy in subjects having routine health screenings, routine melanoma screenings, in those suspected of having melanoma, for those with known previous diagnosis of melanoma, and in subjects at high risk for the development of melanoma. The biomarkers described herein are also useful as novel therapeutic targets.

In one embodiment, the identified melanoma biomarkers are useful to predict disease progression. In one embodiment, subjects having a history of melanoma who are at high risk for disease recurrence may be monitored for disease using the instant blood test or other tests described herein. Current disease monitoring is through the use of frequent physical examinations in conjunction with various imaging modalities including CT-scanning, MRI scanning, and PET scanning. Such subject monitoring techniques often detect only grossly-detectable disease which is often difficult to treat. The claimed methods allow for earlier detection of disease recurrence/progression and therefore earlier treatment of subjects with recurrent/progressive disease.

In addition, knowledge of genetic changes that occur in melanoma enable the design and screening for targeted therapeutic agents that interact with the targets. The interaction may be direct or indirect. Therapeutic agents are agents that improve survival in subjects with disease, including advanced disease.

Provided herein are in vitro melanoma model systems and microarray technologies to identify molecular and genetic defects associated with melanoma onset or progression, and to correlate the expression of the biomarkers with progression and stage of disease, thus providing diagnostic and prognostic markers for this disease. Such markers are useful clinically to determine therapeutic strategies for subjects and guide subject treatment

### Definitions

Unless defined otherwise, all technical and scientific terms used herein have the meaning commonly understood by a person skilled in the art to which this invention belongs. The following references provide one of skill with a general definition of many of the terms used in this invention: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale & Marham, The Harper Collins Dictionary of Biology (1991). As used herein, the following terms have the meanings ascribed to them unless specified otherwise.

The term "melanoma status" refers to the status of the disease in the subject. Examples of types of melanoma statuses include, but are not limited to, the subject's risk of melanoma, the presence or absence of disease, the stage of disease in a subject (e.g., stages 0 - IV and recurrent melanoma), and the effectiveness of treatment of disease. Melanoma status may refer to in situ disease or invasive disease. Other statuses and degrees of each status are known in the art.

As used herein, "invasion-specific genes" refers to those genes that are up-regulated or down-regulated in invasive melanomas which are the only melanomas that can become lethal. These invasion specific genes are useful as biomarkers for melanoma and as potential drug targets for treating melanoma. That is the "invasion-specific" will be useful diagnostic markers of melanoma and will be useful predictors of disease outcome (prognostic markers). In addition, secreted tumor markers can be readily detected in subject's serum and will be used as markers of disease status and outcome.

As used herein "inhibitory nucleic acid" is meant a single or double-stranded RNA, siRNA (short interfering RNA), shRNA (short hairpin RNA), or antisense RNA, or a portion thereof, or a mimetic thereof, that when administered to a mammalian cell results in a decrease (e.g., by 10%, 25%, 50%, 75%, or even 90-100%) in the expression of a target gene. Typically, a nucleic acid inhibitor comprises or corresponds to at least a portion of a target nucleic acid molecule, or an ortholog thereof, or comprises at least a portion of the complementary strand of a target nucleic acid molecule.

As used herein "siRNA" refers to small interfering RNA; a siRNA is a double stranded RNA that "corresponds" to or matches a reference or target gene sequence. This matching need not be perfect so long as each strand of the siRNA is capable of binding to at least a portion of the target sequence. SiRNA can be used to inhibit gene expression, see for example Bass, 2001, Nature, 411,428 429; Elbashir et al., 2001, Nature, 411, 494 498; and Zamore et al., Cell 101:25-33 (2000).

As used herein "aggressive tumor genes" refers to those genes that are up-regulated or down-regulated in melanomas that lead to the greatest mortality. In certain embodiments, aggressive tumor genes refers to genes that are upregulated in invasive melanomas. These aggressive tumor genes are useful as biomarkers for melanoma and as potential drug targets for treating melanoma. That is the "aggressive tumor genes" will be useful diagnostic markers of melanoma and will be useful predictors of disease outcome (prognostic markers). In addition, secreted tumor markers can be readily detected in subjects' serum and will be used as markers of disease status and outcome.

As used herein a "tumor of undetermined classification" is meant to refer to an undifferentiated tumor that cannot be classified into a particular tumor type using certain markers. For example, using the markers of the invention may be used to identify a tumor of undetermined classification as a melanoma versus another type of undifferentiated tumor.

As used herein a "melanocytic tumor of undetermined malignant potential" is meant to refer to a melanocytic lesion that may be malignant or benign. In certain cases, the markers of the invention may be used to determine if a lesion has malignant potential, e.g. is a melanoma, or is benign. For example, moles with marked cellular atypia or borderline lesions are particularly amenable to use with markers of the invention to determine if the lesion is melanoma or is benign.

"Gas phase ion spectrometer" refers to an apparatus that detects gas phase ions. Gas phase ion spectrometers include an ion source that supplies gas phase ions. Gas phase ion spectrometers include, for example, mass spectrometers, ion mobility spectrometers, and total ion current measuring devices. "Gas phase ion spectrometry" refers to the use of a gas phase ion spectrometer to detect gas phase ions.

"Mass spectrometer" refers to a gas phase ion spectrometer that measures a parameter that can be translated into mass-to-charge ratios of gas phase ions. Mass spectrometers generally include an ion source and a mass analyzer. Examples of mass spectrometers are time-of-flight, magnetic sector, quadrupole filter, ion trap, ion cyclotron resonance, electrostatic sector analyzer and hybrids of these. "Mass spectrometry" refers to the use of a mass spectrometer to detect gas phase ions.

"Laser desorption mass spectrometer" refers to a mass spectrometer that uses laser energy as a means to desorb, volatilize, and ionize an analyte.

"Tandem mass spectrometer" refers to any mass spectrometer that is capable of performing two successive stages of m/z-based discrimination or measurement of ions, including ions in an ion mixture. The phrase includes mass spectrometers having two mass analyzers that are capable of performing two successive stages of m/z-based discrimination or measurement of ions tandern-in-space. The phrase further includes mass spectrometers having a single mass analyzer that is capable of performing two successive stages of m/z-based discrimination or measurement of ions tandem-in-time. The phrase thus explicitly includes Qq-TOF mass spectrometers, ion trap mass spectrometers, ion trap-TOF mass spectrometers, TOF-TOF mass spectrometers, Fourier transform ion cyclotron resonance mass spectrometers, electrostatic sector magnetic sector mass spectrometers, and combinations thereof.

"Mass analyzer" refers to a sub-assembly of a mass spectrometer that comprises means for measuring a parameter that can be translated into mass-to-charge ratios of gas phase ions. In a time-of-flight mass spectrometer the mass analyzer comprises an ion optic assembly, a flight tube and an ion detector.

"Ion source" refers to a sub-assembly of a gas phase ion spectrometer that provides gas phase ions. In one embodiment, the ion source provides ions through a desorption/ionization process. Such embodiments generally comprise a probe interface that positionally engages a probe in an interrogatable relationship to a source of ionizing energy (e.g., a laser desorption/ionization source) and in concurrent communication at atmospheric or subatmospheric pressure with a detector of a gas phase ion spectrometer.

Forms of ionizing energy for desorbing/ionizing an analyte from a solid phase include, for example: (1) laser energy; (2) fast atoms (used in fast atom bombardment); (3) high energy particles generated via beta decay of radionucleides (used in plasma desorption); and (4) primary ions generating secondary ions (used in secondary ion mass spectrometry). The preferred form of ionizing energy for solid phase analytes is a laser (used in laser desorption/ionization), in particular, nitrogen lasers, Nd-Yag lasers and other pulsed laser sources. "Fluence" refers to the energy delivered per unit area of interrogated image. A high fluence source, such as a laser, will deliver about 1 mJ / mm2 to 50 mJ / mm2. Typically, a sample is placed on the surface of a probe, the probe is engaged with the probe interface and the probe surface is struck with the ionizing energy. The energy desorbs analyte molecules from the surface into the gas phase and ionizes them.

Other forms of ionizing energy for analytes include, for example: (1) electrons that ionize gas phase neutrals; (2) strong electric field to induce ionization from gas phase, solid phase, or liquid phase neutrals; and (3) a source that applies a combination of ionization particles or electric fields with neutral chemicals to induce chemical ionization of solid phase, gas phase, and liquid phase neutrals.

"Solid support" refers to a solid material which can be derivatized with, or otherwise attached to, a capture reagent. Exemplary solid supports include probes, microtiter plates and chromatographic resins.

"Probe" in the context of this invention refers to a device adapted to engage a probe interface of a gas phase ion spectrometer (e.g., a mass spectrometer) and to present an analyte to ionizing energy for ionization and introduction into a gas phase ion spectrometer, such as a mass spectrometer. A "probe" will generally comprise a solid substrate (either flexible or rigid) comprising a sample presenting surface on which an analyte is presented to the source of ionizing energy.

"Surface-enhanced laser desorption/ionization" or "SELDI" refers to a method of desorption/ionization gas phase ion spectrometry (e.g., mass spectrometry) in which the analyte is captured on the surface of a SELDI probe that engages the probe interface of the gas phase ion spectrometer. In "SELDI MS," the gas phase ion spectrometer is a mass spectrometer. SELDI technology is described in, e.g., U.S. patent 5,719,060 (Hutchens and Yip) and U.S. patent 6,225,047 (Hutchens and Yip).

"Surface-Enhanced Affinity Capture" or "SEAC" is a version of SELDI that involves the use of probes comprising an absorbent surface (a "SEAC probe"). "Adsorbent surface" refers to a surface to which is bound an adsorbent (also called a "capture reagent" or an "affinity reagent"). An adsorbent is any material capable of binding an analyte (e.g., a target polypeptide or nucleic acid). "Chromatographic adsorbent" refers to a material typically used in chromatography. Chromatographic adsorbents include, for example, ion exchange materials, metal chelators (e.g., nitriloacetic acid or iminodiaeotic acid), immobilized metal chelates, hydrophobic interaction adsorbents, hydrophilic interaction adsorbents, dyes, simple biomolecules (e.g., nucleotides, amino acids, simple sugars and fatty acids) and mixed mode adsorbents (e.g., hydrophobic attraction/electrostatic repulsion adsorbents). "Biospecific adsorbent" refers an adsorbent comprising a biomolecule, e.g., a nucleic acid molecule (e.g., an aptamer), a polypeptide, a polysaccharide, a lipid, a steroid or a conjugate of these (e.g., a glycoprotein, a lipoprotein, a glycolipid, a nucleic acid (e.g., DNA)-protein conjugate). In certain instances the biospecific adsorbent can be a macromolecular structure such as a multiprotein complex, a biological membrane or a virus. Examples of biospecific adsorbents are antibodies, receptor proteins and nucleic acids. Biospecific adsorbents typically have higher specificity for a target analyte than chromatographic adsorbents. Further examples of adsorbents for use in SELDI can be found in U.S. Patent 6,225,047 (Hutchens and Yip, "Use of retentate chromatography to generate difference maps," May 1, 2001).

In some embodiments, a SEAC probe is provided as a pre-activated surface which can be modified to provide an adsorbent of choice. For example, certain probes are provided with a reactive moiety that is capable of binding a biological molecule through a covalent bond. Epoxide and carbodiimidizole are useful reactive moieties to covalently bind biospecific adsorbents such as antibodies or cellular receptors.

"Adsorption" refers to detectable non-covalent binding of an analyte to an adsorbent or capture reagent.

"Surface-Enhanced Neat Desorption" or "SEND" is a version of SELDI that involves the use of probes comprising energy absorbing molecules chemically bound to the probe surface. ("SEND probe.") "Energy absorbing molecules" ("EAM") refer to molecules that are capable of absorbing energy from a laser desorption/ ionization source and thereafter contributing to desorption and ionization of analyte molecules in contact therewith. The phrase includes molecules used in MALDI, frequently referred to as "matrix", and explicitly includes cinnamic acid derivatives, sinapinic acid ("SPA"), cyano-hydroxy-cinnamic acid ("CHCA") and dihydroxybenzoic acid, ferulic acid, hydroxyacetophenone derivatives, as well as others. It also includes EAMs used in SELDI. SEND is further described in United States patent 5,719,060 and United States patent application 60/408,255, filed September 4, 2002 (Kitagawa, "Monomers And Polymers Having Energy Absorbing Moieties Of Use In Desorption/Ionization Of Analytes").

"Surface-Enhanced Photolabile Attachment and Release" or "SEPAR" is aversion of SELDI that involves the use of probes having moieties attached to the surface that can covalently bind an analyte, and then release the analyte through breaking a photolabile bond in the moiety after exposure to light, e.g., laser light. SEPAR is further described in United States Patent 5,719,060.

"Eluant" or "wash solution" refers to an agent, typically a solution, which is used to affect or modify adsorption of an analyte to an adsorbent surface and/or remove unbound materials from the surface. The elution characteristics of an eluant can depend on, for example, pH, ionic strength, hydrophobicity, degree of chaotropism, detergent strength and temperature.

"Analyte" refers to any component of a sample that is desired to be detected. The term can refer to a single component or a plurality of components in the sample. The "complexity" of a sample adsorbed to an adsorption surface of an affinity capture probe means the number of different protein species that are adsorbed.

"Molecular binding partners" and "specific binding partners" refer to pairs of molecules, typically pairs of biomolecules that exhibit specific binding. Molecular binding partners include, without limitation, receptor and ligand, antibody and antigen, biotin and avidin, and biotin and streptavidin.

"Monitoring" refers to observing and/or recording changes in a continuously varying parameter.

"Biochip" refers to a solid substrate having a generally planar surface to which an adsorbent is attached. Frequently, the surface of the biochip comprises a plurality of addressable locations, each of which location has the adsorbent bound there. Biochips can be adapted to engage a probe interface, and therefore, function as probes.

"Protein biochip" refers to a biochip adapted for the capture of polypeptides. Many protein biochips are described in the art. These include, for example, protein biochips produced by Ciphergen Biosystems (Fremont, CA), Packard BioScience Company (Meriden CT), Zyomyx (Hayward, CA) and Phylos (Lexington, MA). Examples of such protein biochips are described in the following patents or patent applications: U.S. Patent 6,225,047 (Hutchens and Yip, "Use of retentate chromatography to generate difference maps," May 1, 2001); International publication WO 99/51773 (Kuimelis and Wagner, "Addressable protein arrays," October 14, 1999); U.S. Patent 6,329,209 (Wagner et al., "Arrays of protein-capture agents and methods of use thereof," December 11, 2001) and International publication WO 00/56934 (Englert et al., "Continuous porous matrix arrays," September 28, 2000). Protein biochips produced by Ciphergen Biosystems comprise surfaces having chromatographic or biospecific adsorbents attached thereto at addressable locations. Biochips are further described in: WO 00/66265 (Rich et al., "Probes for a Gas Phase Ion Spectrometer," November 9, 2000); WO 00/67293 (Beecher et al., "Sample Holder with Hydrophobic Coating for Gas Phase Mass Spectrometer," November 9, 2000); U.S. patent application US20030032043A1 (Pohl and Papanu, "Latex Based Adsorbent Chip," July 16, 2002) and U.S. patent application 60/350,110 (Um et al., "Hydrophobic Surface Chip," November 8, 2001).

Upon capture on a biochip, analytes can be detected by a variety of detection methods selected from, for example, a gas phase ion spectrometry method, an optical method, an electrochemical method, atomic force microscopy and a radio frequency method. Gas phase ion spectrometry methods are described herein. Of particular interest is the use of mass spectrometry, and in particular, SELDI. Optical methods include, for example, detection of fluorescence, luminescence, chemiluminescence, absorbance, reflectance, transmittance, birefringence or refractive index (e.g., surface plasmon resonance, ellipsometry, a resonant mirror method, a grating coupler waveguide method or interferometry). Optical methods include microscopy (both confocal and non-confocal), imaging methods and non-imaging methods. Immunoassays in various formats (e.g., ELISA) are popular methods for detection of analytes captured on a solid phase. Electrochemical methods include voltametry and amperometry methods. Radio frequency methods include multipolar resonance spectroscopy.

"Marker" or "biomarker" in the context of the present invention refer to a polypeptide (of a particular apparent molecular weight) or nucleic acid, which is differentially present in a sample taken from subjects having human melanoma as compared to a comparable sample taken from control subjects (e.g., a person with a negative diagnosis or undetectable melanoma, normal or healthy subject). The term "'biomarker" is used interchangeably with the term "marker." The biomarkers are identified by, for example, molecular mass in Daltons, and include the masses centered around the identified molecular masses for each marker, affinity binding, nucleic acid detection, etc.

The term "measuring" means methods which include detecting the presence or absence of marker(s) in the sample, quantifying the amount of marker(s) in the sample, and/or qualifying the type of biomarker. Measuring can be accomplished by methods known in the art and those further described herein, including but not limited to microarray analysis (with Significance Analysis of Microarrays (SAM) software), SELDI and immunoassay. Any suitable methods can be used to detect and measure one or more of the markers described herein. These methods include, without limitation, mass spectrometry (e.g., laser desorption/ionization mass spectrometry), fluorescence (e.g. sandwich immunoassay), surface plasmon resonance, ellipsometry and atomic force microscopy.

"Detect" refers to identifying the presence, absence or amount of the object to be detected.

The phrase "differentially present" refers to differences in the quantity and/or the frequency of a marker present in a sample taken from subjects having melanoma as compared to a control subject or a reference subject or sample. For example, some markers described herein are present at an elevated level in samples of subjects compared to samples from control subjects. In contrast, other markers described herein are present at a decreased level in samples of melanoma subjects compared to samples from control subject. Furthermore, a marker can be a polypeptide or a nucleic acid, which is detected at a higher frequency or at a lower frequency in samples of human melanoma subjects compared to samples of control subjects.

A marker can be a polypeptide, which is detected at a higher frequency or at a lower frequency in samples of unaffected tissue from melanoma subjects compared to samples affected tissue from melanoma subjects.

A marker can be a polypeptide, which is detected at a higher frequency or at a lower frequency in samples of human unaffected tissue from melanoma subjects compared to samples of control subjects.

A marker can be a polypeptide, which is detected at a higher frequency or at a lower frequency in samples of human affected tissue from melanoma subjects compared to samples of control subjects.

A marker can be differentially present in terms of quantity, frequency or both.

"Affected tissue," as used herein refers to tissue from a melanoma subject that is grossly diseased tissue (e.g., skin (epidermis and/or dermis) lymph nodes, metastatic sites, e.g., brain, lung, bone, liver, skin) or melanocytes).

"Unaffected tissue," as used herein refers to a tissue from an melanoma subject that is from a portion of tissue that does not have gross disease present, for example tissue that is about 1, 2, 5, 10, 20 or more cm from grossly diseased tissue.

A polypeptide is differentially present between two samples if the amount of the polypeptide or nucleic acid in one sample is statistically significantly different from the amount of the polypeptide or nucleic acid in the other sample. For example, a polypeptide, or nucleic acid is differentially present between the two samples if it is present at least about 25%, at least about 50%, at least about 75%, at least about 100%, 120%, at least about 130%, at least about 150%, at least about 180%, at least about 200%, at least about 300%, at least about 500%, at least about 700%, at least about 900%, or at least about 1000% greater than it is present in the other sample, or if it is detectable in one sample and not detectable in the other.

Alternatively or additionally, a polypeptide or nucleic acid is differentially present between two sets of samples if the frequency of detecting the polypeptide or nucleic acid in the melanoma subjects' samples is statistically significantly higher or lower than in the control samples. For example, a polypeptide or nucleic acid is differentially present between the two sets of samples if it is detected at least about 25%, at least about 50%, at least about 75%, at least about 100%, at least about 120%, at least about 130%, at least about 150%, at least about 180%, at least about 200%, at least about 300%, at least about 500%, at least about 700%, at least about 900%, or at least about 1000% more frequently or less frequently observed in one set of samples than the other set of samples.

"Diagnostic" means identifying the presence or nature of a pathologic condition, i.e., melanoma. Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay, are termed "true negatives." The "specificity" of a diagnostic assay is 1 minus the false positive rate, where the "false positive" rate is defined as the proportion of those without the disease who test positive. While a particular diagnostic method may not provide a definitive diagnosis of a condition, it suffices if the method provides a positive indication that aids in diagnosis.

A "test amount" of a marker refers to an amount of a marker present in a sample being tested. A test amount can be either in absolute amount (e.g., □g/ml) or a relative amount (e.g., relative intensity of signals).

A "diagnostic amount" of a marker refers to an amount of a marker in a subject's sample that is consistent with a diagnosis of melanoma. A diagnostic amount can be either in absolute amount (e.g., µg/ml) or a relative amount (e.g., relative intensity of signals).

A "control amount" of a marker can be any amount or a range of amount, which is to be compared against a test amount of a marker. For example, a control amount of a marker can be the amount of a marker in a person without melanoma. A control amount can be either in absolute amount (e.g., µg/ml) or a relative amount (e.g., relative intensity of signals). As used herein, the term "sensitivity" is the percentage of subjects with a particular disease. For example, in the melanoma group, the biomarkers of the invention have a sensitivity of about 80.0%-98.6%, and preferably a sensitivity of 85%, 87.5%, 90%, 92.5%, 95%, 97%, 98%, 99% or approaching 100%.

As used herein, the term "specificity" is the percentage of subjects correctly identified as having a particular disease i.e., normal or healthy subjects. For example, the specificity is calculated as the number of subjects with a particular disease as compared to non-melanoma subjects (e.g., normal healthy subjects). The specificity of the assays described herein may range from about 80% to 100%. Preferably the specificity is about 90%, 95%, or 100%.

The terms "polypeptide," "peptide" and "protein" are used interchangeably herein to refer to a polymer of amino acid residues. The terms apply to amino acid polymers in which one or more amino acid residue is an analog or mimetic of a corresponding naturally occurring amino acid, as well as to naturally occurring amino acid polymers. Polypeptides can be modified, e.g., by the addition of carbohydrate residues to form glycoproteins. The terms "polypeptide," "peptide" and "protein" include glycoproteins, as well as non-glycoproteins.

"Immunoassay" is an assay that uses an antibody to specifically bind an antigen (e.g., a marker). The immunoassay is characterized by the use of specific binding properties of a particular antibody to isolate, target, and/or quantify the antigen.

"Antibody" refers to a polypeptide ligand substantially encoded by an immunoglobulin gene or immunoglobulin genes, or fragments thereof, which specifically binds and recognizes an epitope (e.g., an antigen). The recognized immunoglobulin genes include the kappa and lambda light chain constant region genes, the alpha, gamma, delta, epsilon and mu heavy chain constant region genes, and the myriad immunoglobulin variable region genes. Antibodies exist, e.g., as intact immunoglobulins or as a number of well-characterized fragments produced by digestion with various peptidases. This includes, e.g., Fab" and F(ab)"2 fragments. The term "antibody," as used herein, also includes antibody fragments either produced by the modification of whole antibodies or those synthesized de novo using recombinant DNA methodologies. It also includes polyclonal antibodies, monoclonal antibodies, chimeric antibodies, humanized antibodies, or single chain antibodies. "Fc" portion of an antibody refers to that portion of an immunoglobulin heavy chain that comprises one or more heavy chain constant region domains, CH1, CH2 and CH3, but does not include the heavy chain variable region.

The phrase "specifically (or selectively) binds" to an antibody or "specifically (or selectively) immunoreactive with," when referring to a protein or peptide, refers to a binding reaction that is determinative of the presence of the protein in a heterogeneous population of proteins and other biologics. Thus, under designated immunoassay conditions, the specified antibodies bind to a particular protein at least two times the background and do not substantially bind in a significant amount to other proteins present in the sample. Specific binding to an antibody under such conditions may require an antibody that is selected for its specificity for a particular protein. For example, polyclonal antibodies raised to marker "X" from specific species such as rat, mouse, or human can be selected to obtain only those polyclonal antibodies that are specifically immunoreactive with marker "X" and not with other proteins, except for polymorphic variants and alleles of marker "X". This selection may be achieved by subtracting out antibodies that cross-react with marker "X" molecules from other species. A variety of immunoassay formats may be used to select antibodies specifically immunoreactive with a particular protein. For example, solid-phase ELISA immunoassays are routinely used to select antibodies specifically immunoreactive with a protein (see, e.g., Harlow & Lane, Antibodies, A Laboratory Manual (1988), for a description of immunoassay formats and conditions that can be used to determine specific immunoreactivity). Typically a specific or selective reaction will be at least twice background signal or noise and more typically more than 10 to 100 times background.

"Managing subject treatment" refers to the behavior of the subject, clinician or physician subsequent to the determination of melanoma status. For example, if the result of the methods of the present invention is inconclusive or there is reason that confirmation of status is necessary, the physician may order more tests. Alternatively, if the status indicates that treatment is appropriate, the physician may schedule the subject for treatment, e.g., surgery, administer one or more therapeutic agents or radiation. Likewise, if the status is negative, e.g., late stage melanoma or if the status is acute, no further action may be warranted. Furthermore, if the results show that treatment has been successful, a maintenance therapy or no further management may be necessary.

### DESCRIPTION OF THE BIOMARKERS

### Melanoma Biomarkers

Described herein is a series of melanoma markers that can be used to predict disease progression and can readily be detected in the circulating serum of patients.

Melanoma biomarkers include any biomarkers as identified in any of Tables 1, 2, 3, 4, 5, 6, 7, and 8 as shown herein.

Melanoma biomarkers, in certain examples, are comprised of one or more of a marker identified in Table 2 or Table 8, or combinations thereof, wherein the biomarker is correlated with a stage of melanoma progression. In particular examples, the biomarker is associated with aggressive melanoma.

Melanoma biomarkers, in certain examples, are comprised of one or more of a marker identified in Table 3, or combinations thereof, wherein the biomarker is correlated with a stage of melanoma progression. In particular examples, the biomarker is associated with less- aggressive melanoma.

Melanoma biomarkers, in certain examples, are comprised of one or more of a marker identified in Table 4, or combinations thereof, wherein the biomarker is correlated with metastatic melanoma. In particular examples, the marker or combinations thereof are down-regulated. In certain cases, the downregulation is correlated with metastatic melanoma.

Melanoma biomarkers, in certain examples, are comprised of one or more of a marker identified in Table 5, or combinations thereof, wherein the biomarker is correlated with invasive or metastatic melanoma. In particular examples, the markers or combinations thereof are overexpressed, and the overexpression is correlated with invasive or metastatic melanoma.

Melanoma biomarkers, in certain examples, are comprised of one or more of a marker identified in Table 6, or combinations thereof, wherein the biomarker is correlated with a stage of melanoma progression. In particular examples, the markers or combinations thereof are overexpressed, and the overexpression is correlated with invasive or metastatic melanoma.

Melanoma biomarkers, in certain examples, are comprised of one or more of a marker identified in Table 7, 8 or 9, or combinations thereof, wherein the biomarker is upregulated in primary melanocytes. In particular examples, the markers or combinations thereof are overexpressed in primary melanocytes. In certain cases, the overexpression is correlated with melanocyte proliferation. The marker may be one or more of, but not limited to, matrix metalloproteinase-1 (MMP-1), SerpinB2, amphiregulin, CXCL5, IL-8, RAP1a and epiregulin.

The sequences of these biomarkers may be found by reference to the Affymetrix reference number and primers for amplifying the biomarkers may be developed by those of skill in the art. Gene expression is elevated in most melanoma profiles, and most likely this elevated expression contributes towards separation of melanoma from normal controls. These genes include those listed herein, and include genes encoding cytokines, chemokines, growth factors, heat shock proteins, transcription factors, MHC molecules, adhesion molecules. cell adhesion, regulation of cell-cell adhesion, cell differentiation, negative regulation of cell differentiation, DNA replication initiation, regulation of apoptosis, angiogenesis, regulation of cell migration, cell proliferation, extracellular matrix organization and intracellular junction organization or maintenance. Some genes have been identified as down-regulated, and may contribute towards separation of melanoma from normal controls. These genes include genes encoding glutathione S-transferase, amino acid production genes, coagulation factors, G protein coupled receptors, and myelin basic protein.

In particular, Neuropilin-2 (Nrp-2) has been ideintified as a mediator of melanoma growth. In particular, Neuropilin-2 (Nrp2) was identified as a gene involved in melanoma-endothelial cell communication and was highly upregulated during this process. Nrp2 represents a novel biomarker for melanoma growth and metastasis, and has diagnostic, prognostic and therapeutic use, as described herein.

In particular, MMP-1 has been identified as a biomarker for melanoma growth. MMP-1 may be a useful therapeutic target in melanomas possessing activating BRAF mutations.

Further biomarkers for melanoma include the proteins or their encoding nucleic acids for the following pathways or cellular processes: melanocyte differentiation, cellular proliferation, melanin biosynthesis.

Corresponding proteins or fragments of proteins for these biomarkers may be represented as intensity peaks in SELDI (surface enhanced laser desorption/ionization) protein chip/mass spectra with molecular masses centered around the values. As discussed above, Markers 1-104 also may be characterized based on affinity for an adsorbent, particularly binding to a cation-exchange or hydrophobic surface under the conditions specified in the Examples, which follow.

The above-identified biomarkers, are examples of biomarkers, as determined by identity, identified by the methods of the invention and serve merely as an illustrative example and are not meant to limit the invention in anyway.

A major advantage of identification of these markers is their high specificity and ability to differentiate between different melanoma states (e.g., between stages 0, I - IV and recurrent melanoma). The biomarkers of the invention are useful as diagnostic markers of melanoma and are useful predictors of disease outcome (prognostic markers).

More specifically, the present invention is based upon the discovery of markers that are differentially present in samples of human melanoma subjects and control subjects, and the application of this discovery in methods and kits for aiding a melanoma diagnosis. Some of these markers are found at an elevated level and/or more frequently in samples from human melanoma subjects compared to a control (e.g., subjects with diseases other than melanoma). Accordingly, the amount of one or more markers found in a test sample compared to a control, or the mere detection of one or more markers in the test sample provides useful information regarding probability of whether a subject being tested has melanoma or not, and/or whether a subject being tested has a particular melanoma subtype or not.

The proteins and nucleic acids of the present invention have a number of other uses. For example, the markers can be used to screen for compounds that modulate the expression of the markers in vitro or in vivo, which compounds in turn may be useful in treating or preventing human melanoma in subjects. In another example, markers can be used to monitor responses to certain treatments of human melanoma. In yet another example, the markers can be used in heredity studies. For instance, certain markers may be genetically linked. This can be determined by, e.g., analyzing samples from a population of human melanoma subjects whose families have a history of melanoma. The results can then be compared with data obtained from, e.g., melanoma subjects whose families do not have a history of melanoma. The markers that are genetically linked may be used as a tool to determine if a subject whose family has a history of melanoma is pre-disposed to having melanoma.

In another aspect, the invention provides methods for detecting markers which are differentially present in the samples of an melanoma subject and a control (e.g., subjects in non-melanoma subjects). The markers can be detected in a number of biological samples. The sample is preferably a biological biopsy sample or a blood sample.

Any suitable methods can be used to detect one or more of the markers described herein. These methods include, without limitation, mass spectrometry (e.g., laser desorption/ionization mass spectrometry), fluorescence (e.g. sandwich immunoassay), surface plasmon resonance, ellipsometry and atomic force microscopy. Methods may further include, by one or more of microarrays, PCR methods, electrospray ionization mass spectrometry (ESI-MS), ESI-MS/MS, ESI-MS/(MS)n, matrix-assisted laser desorption ionization time-of-flight mass spectrometry (MALDI-TOF-MS), surface-enhanced laser desorption/ionization time-of-flight mass spectrometry (SELDI-TOF-MS), desorption/ionization on silicon (DIOS), secondary ion mass spectrometry (SIMS), quadrupole time-of-flight (Q-TOF), atmospheric pressure chemical ionization mass spectrometry (APCI-MS), APCI-MS/MS, APCI-(MS)n, atmospheric pressure photoionization mass spectrometry (APPI-MS), APPI-MS/MS, and APPI-(MS)n, quadrupole mass spectrometry, fourier transform mass spectrometry (FTMS), and ion trap mass spectrometry, where n is an integer greater than zero.

The following example is illustrative of the methods used to identify biomarkers for detection of melanoma. It is not meant to limit or construe the invention in any way. A sample, such as for example, serum from a subject or subject, is immobilized on a biochip. Preferably, the biochip comprises a functionalized, cross-linked polymer in the form of a hydrogel physically attached to the surface of the biochip or covalently attached through a silane to the surface of the biochip. However, any biochip which can bind samples from subjects can be used. The surfaces of the biochips are comprised of, for example, hydrophilic adsorbent to capture hydrophilic proteins (e.g. silicon oxide); carboimidizole functional groups that can react with groups on proteins for covalent binding; epoxide functional groups for covalent binding with proteins (e.g. antibodies, receptors, lectins, heparin, Protein A, biotin/streptavidin and the like); anionic exchange groups; cation exchange groups; metal chelators and the like.

Preferably, samples are pre-fractionated prior to immobilization as discussed below. Analytes or samples captured on the surface of a biochip can be detected by any method known in the art. This includes, for example, mass spectrometry, fluorescence, surface plasmon resonance, ellipsometry and atomic force microscopy. Mass spectrometry, and particularly SELDI mass spectrometry, is a particularly useful method for detection of the biomarkers of this invention. Other methods include, chemical extraction partitioning, ion exchange chromatography, reverse phase liquid chromatography, isoelectric focusing, one-dimensional polyacrylamide gel electrophoresis (PAGE), two-dimensional polyacrylamide gel electrophoresis (2D-PAGE), thin-layer chromatography, gas chromatography, liquid chromatography, and any combination thereof.

Immobilized samples or analytes are preferably subjected to laser ionization and the intensity of signal for mass/charge ratio is detected. The data obtained from the mass/charge ratio signal is transformed into data which is read by any type of computer. An algorithm is executed by the computer user that classifies the data according to user input parameters for detecting signals that represent biomarkers present in, for example, melanoma subjects and are lacking in non-melanoma subject controls. The biomarkers are most preferably identified by their molecular weights.

### TEST SAMPLES

### SUBJECT TYPES

Samples are collected from subjects to establish melanoma status. The subjects may be subjects who have been determined to have a high risk of melanoma based on their family history, a previous treatment, subjects with physical symptoms known to be associated with melanoma, subjects identified through screening assays (e.g., routine melanoma screening) or other techniques. Other subjects include subjects who have melanoma and the test is being used to determine the effectiveness of therapy or treatment they are receiving. Also, subjects could include healthy people who are having a test as part of a routine examination, or to establish baseline levels of the biomarkers. Samples may be collected from subjects who had been diagnosed with melanoma and received treatment to eliminate the melanoma, or perhaps are in remission. As used herein, "melanoma biopsy" refers to tissue from suspected melanoma, tissue from the edges of suspected melanoma or from normal tissue.

### TYPES OF SAMPLE AND PREPARATION OF THE SAMPLE

The markers can be measured in different types of biological samples. The sample is preferably a biological tissue or fluid sample. Examples of biological tissue sample is a blood or biopsy sample, from for example a melanoma biopsy. Examples of a biological fluid sample useful in this invention include blood, blood serum, plasma, vaginal secretions, urine, tears, saliva, urine, tissue, cells, organs, seminal fluids, bone marrow, cerebrospinal fluid, etc. Because the markers are found in blood and melanoma biopsy, these are preferred sample sources for embodiments of the invention.

Nucleic acids may be obtained from the samples in many ways known to one of skill in the art. For example, extraction methods, including for example, solvent extraction, affinity purification and centrifugation. Selective precipitation can also purify nucleic acids. Chromatography methods may also be utilized including, gel filtration, ion exchange, selective adsorption, or affinity binding. The nucleic acids may be, for example, RNA, DNA or may be synthesized into cDNA. The nucleic acids may be detected using microarray techniques that are well known in the art, for example, Affymetrix arrays followed by multidimensional scaling techniques. See R. Ekins and F.W. Chu, Microarrays: their origins and applications. Trends in Biotechnology, 1999, 17, 217-218; D. D. Shoemaker, et al., Experimental annotation of the human genome using microarray technology, Nature Volume 409 Number 6822 Page 922 - 927 (2001) and US Patent 5,750,015.

The markers can be resolved in a sample by using a variety of techniques, e.g., nucleic acid chips, PCR, real time PCR, reverse transcriptase PCR, real time reverse transcriptase PCR, in situ PCR, chromatographic separation coupled with mass spectrometry, protein capture using immobilized antibodies or by traditional immunoassays.

Biomarker expression may also be by PCR methods, including for example, real time PCR. See for example, U.S. Patents 5,723,591; 5,801,155 and 6,084,102 and Higuchi, 1992 and 1993. PCR assays may be done, for example, in a multi-well plate formats or in chips, such as the BioTrove OPEN ARRAY Chips (BioTrove, Woburn, MA).

If desired, the sample can be prepared to enhance detectability of the markers. For example, to increase the detectability of protein markers, a blood serum sample from the subject can be preferably fractionated by, e.g., Cibacron blue agarose chromatography and single stranded DNA affinity chromatography, anion exchange chromatography, affinity chromatography (e.g., with antibodies) and the like. The method of fractionation depends on the type of detection method used. Any method that enriches for the protein of interest can be used. Typically, preparation involves fractionation of the sample and collection of fractions determined to contain the biomarkers. Methods of pre-fractionation include, for example, size exclusion chromatography, ion exchange chromatography, heparin chromatography, affinity chromatography, sequential extraction, gel electrophoresis and liquid chromatography. The analytes also may be modified prior to detection. These methods are useful to simplify the sample for further analysis. For example, it can be useful to remove high abundance proteins, such as albumin, from blood before analysis.

In one embodiment, a sample can be pre-fractionated according to size of proteins in a sample using size exclusion chromatography. For a biological sample wherein the amount of sample available is small, preferably a size selection spin column is used. For example, a K30 spin column (available from Princeton Separation, Ciphergen Biosystems, Inc., etc.) can be used. In general, the first fraction that is eluted from the column ("fraction 1") has the highest percentage of high molecular weight proteins; fraction 2 has a lower percentage of high molecular weight proteins; fraction 3 has even a lower percentage of high molecular weight proteins; fraction 4 has the lowest amount of large proteins; and so on. Each fraction can then be analyzed by gas phase ion spectrometry for the detection of markers.

In another embodiment, a sample can be pre-fractionated by anion exchange chromatography. Anion exchange chromatography allows pre-fractionation of the proteins in a sample roughly according to their charge characteristics. For example, a Q anion-exchange resin can be used (e.g., Q HyperD F, Biosepra), and a sample can be sequentially eluted with eluants having different pH's. Anion exchange chromatography allows separation of biomolecules in a sample that are more negatively charged from other types of biomolecules. Proteins that are eluted with an eluant having a high pH is likely to be weakly negatively charged, and a fraction that is eluted with an eluant having a low pH is likely to be strongly negatively charged. Thus, in addition to reducing complexity of a sample, anion exchange chromatography separates proteins according to their binding characteristics.

In yet another embodiment, a sample can be pre-fractionated by heparin chromatography. Heparin chromatography allows pre-fractionation of the markers in a sample also on the basis of affinity interaction with heparin and charge characteristics. Heparin, a sulfated mucopolysaccharide, will bind markers with positively charged moieties and a sample can be sequentially eluted with eluants having different pH's or salt concentrations. Markers eluted with an eluant having a low pH are more likely to be weakly positively charged. Markers eluted with an eluant having a high pH are more likely to be strongly positively charged. Thus, heparin chromatography also reduces the complexity of a sample and separates markers according to their binding characteristics.

In yet another embodiment, a sample can be pre-fractionated by removing proteins that are present in a high quantity or that may interfere with the detection of markers in a sample. For example, in a blood serum sample, serum albumin is present in a high quantity and may obscure the analysis of markers. Thus, a blood serum sample can be pre-fractionated by removing serum albumin. Serum albumin can be removed using a substrate that comprises adsorbents that specifically bind serum albumin. For example, a column which comprises, e.g., Cibacron blue agarose (which has a high affinity for serum albumin) or anti-serum albumin antibodies can be used.

In yet another embodiment, a sample can be pre-fractionated by isolating proteins that have a specific characteristic, e.g. are glycosylated. For example, a blood serum sample can be fractionated by passing the sample over a lectin chromatography column (which has a high affinity for sugars). Glycosylated proteins will bind to the lectin column and non-glycosylated proteins will pass through the flow through. Glycosylated proteins are then eluted from the lectin column with an eluant containing a sugar, e.g., N-acetyl-glucosamine and are available for further analysis.

Many types of affinity adsorbents exist which are suitable for pre-fractionating blood serum samples. An example of one other type of affinity chromatography available to pre-fractionate a sample is a single stranded DNA spin column. These columns bind proteins which are basic or positively charged. Bound proteins are then eluted from the column using eluants containing denaturants or high pH.

Thus there are many ways to reduce the complexity of a sample based on the binding properties of the proteins in the sample, or the characteristics of the proteins in the sample.

In yet another embodiment, a sample can be fractionated using a sequential extraction protocol. In sequential extraction, a sample is exposed to a series of adsorbents to extract different types of biomolecules from a sample. For example, a sample is applied to a first adsorbent to extract certain proteins, and an eluant containing non-adsorbent proteins (i.e., proteins that did not bind to the first adsorbent) is collected. Then, the fraction is exposed to a second adsorbent. This further extracts various proteins from the fraction. This second fraction is then exposed to a third adsorbent, and so on.

Any suitable materials and methods can be used to perform sequential extraction of a sample. For example, a series of spin columns comprising different adsorbents can be used. In another example, a multi-well comprising different adsorbents at its bottom can be used. In another example, sequential extraction can be performed on a probe adapted for use in a gas phase ion spectrometer, wherein the probe surface comprises adsorbents for binding biomolecules. In this embodiment, the sample is applied to a first adsorbent on the probe, which is subsequently washed with an eluant. Markers that do not bind to the first adsorbent is removed with an eluant. The markers that are in the fraction can be applied to a second adsorbent on the probe, and so forth. The advantage of performing sequential extraction on a gas phase ion spectrometer probe is that markers that bind to various adsorbents at every stage of the sequential extraction protocol can be analyzed directly using a gas phase ion spectrometer.

In yet another embodiment, biomolecules in a sample can be separated by high-resolution electrophoresis, e.g., one or two-dimensional gel electrophoresis. A fraction containing a marker can be isolated and further analyzed by gas phase ion spectrometry. Preferably, two-dimensional gel electrophoresis is used to generate two-dimensional array of spots of biomolecules, including one or more markers. See, e.g., Jungblut and Thiede, Mass Spectr. Rev. 16:145-162 (1997).

The two-dimensional gel electrophoresis can be performed using methods known in the art. See, e.g., Deutscher ed., Methods In Enzymology vol. 182. Typically, biomolecules in a sample are separated by, e.g., isoelectric focusing, during which biomolecules in a sample are separated in a pH gradient until they reach a spot where their net charge is zero (i.e., isoelectric point). This first separation step results in one-dimensional array of biomolecules. The biomolecules in one- dimensional array is further separated using a technique generally distinct from that used in the first separation step. For example, in the second dimension, biomolecules separated by isoelectric focusing are further separated using a polyacrylamide gel, such as polyacrylamide gel electrophoresis in the presence of sodium dodecyl sulfate (SDS-PAGE). SDS-PAGE gel allows further separation based on molecular mass of biomolecules. Typically, two-dimensional gel electrophoresis can separate chemically different biomolecules in the molecular mass range from 1000-200,000 Da within complex mixtures.

Biomolecules in the two-dimensional array can be detected using any suitable methods known in the art. For example, biomolecules in a gel can be labeled or stained (e.g., Coomassie Blue or silver staining). If gel electrophoresis generates spots that correspond to the molecular weight of one or more markers of the invention, the spot can be is further analyzed by gas phase ion spectrometry. For example, spots can be excised from the gel and analyzed by gas phase ion spectrometry. Alternatively, the gel containing biomolecules can be transferred to an inert membrane by applying an electric field. Then a spot on the membrane that approximately corresponds to the molecular weight of a marker can be analyzed by gas phase ion spectrometry. In gas phase ion spectrometry, the spots can be analyzed using any suitable techniques, such as MALDI or SELDI (e.g., using ProteinChip® array) as described in detail below.

Prior to gas phase ion spectrometry analysis, it may be desirable to cleave biomolecules in the spot into smaller fragments using cleaving reagents, such as proteases (e.g., trypsin). The digestion of biomolecules into small fragments provides a mass fingerprint of the biomolecules in the spot, which can be used to determine the identity of markers if desired.

In yet another embodiment, high performance liquid chromatography (HPLC) can be used to separate a mixture of biomolecules in a sample based on their different physical properties, such as polarity, charge and size. HPLC instruments typically consist of a reservoir of mobile phase, a pump, an injector, a separation column, and a detector. Biomolecules in a sample are separated by injecting an aliquot of the sample onto the column. Different biomolecules in the mixture pass through the column at different rates due to differences in their partitioning behavior between the mobile liquid phase and the stationary phase. A fraction that corresponds to the molecular weight and/or physical properties of one or more markers can be collected. The fraction can then be analyzed by gas phase ion spectrometry to detect markers. For example, the spots can be analyzed using either MALDI or SELDI (e.g., using ProteinChip® array) as described in detail below.

Optionally, a marker can be modified before analysis to improve its resolution or to determine its identity. For example, the markers may be subject to proteolytic digestion before analysis. Any protease can be used. Proteases, such as trypsin, that are likely to cleave the markers into a discrete number of fragments are particularly useful. The fragments that result from digestion function as a fingerprint for the markers, thereby enabling their detection indirectly. This is particularly useful where there are markers with similar molecular masses that might be confused for the marker in question. Also, proteolytic fragmentation is useful for high molecular weight markers because smaller markers are more easily resolved by mass spectrometry. In another example, biomolecules can be modified to improve detection resolution. For instance, neuraminidase can be used to remove terminal sialic acid residues from glycoproteins to improve binding to an anionic adsorbent (e.g., cationic exchange ProteinChip® arrays) and to improve detection resolution. In another example, the markers can be modified by the attachment of a tag of particular molecular weight that specifically bind to molecular markers, further distinguishing them. Optionally, after detecting such modified markers, the identity of the markers can be further determined by matching the physical and chemical characteristics of the modified markers in a protein database (e.g., SwissProt).

### DETECTION AND MEASUREMENT OF MARKERS

Once captured on a substrate, e.g., biochip or antibody, any suitable method can be used to measure a marker or markers in a sample. For example, markers can be detected and/or measured by a variety of detection methods including for example, gas phase ion spectrometry methods, optical methods, electrochemical methods, atomic force microscopy, radio frequency methods, surface plasmon resonance, ellipsometry and atomic force microscopy.

### SELDI

One preferred method of detection and/or measurement of the biomarkers uses mass spectrometry, and in particular, "Surface-enhanced laser desorption/ionization" or "SELDI". SELDI refers to a method of desorption/ionization gas phase ion spectrometry (e.g., mass spectrometry) in which the analyte is captured on the surface of a SELDI probe that engages the probe interface. In "SELDI MS," the gas phase ion spectrometer is a mass spectrometer. SELDI technology is described in more detail above and as follows.

Preferably, a laser desorption time-of-flight mass spectrometer is used in embodiments of the invention. In laser desorption mass spectrometry, a substrate or a probe comprising markers is introduced into an inlet system. The markers are desorbed and ionized into the gas phase by laser from the ionization source. The ions generated are collected by an ion optic assembly, and then in a time-of-flight mass analyzer, ions are accelerated through a short high voltage field and let drift into a high vacuum chamber. At the far end of the high vacuum chamber, the accelerated ions strike a sensitive detector surface at a different time. Since the time-of-flight is a function of the mass of the ions, the elapsed time between ion formation and ion detector impact can be used to identify the presence or absence of markers of specific mass to charge ratio.

Markers on the substrate surface can be desorbed and ionized using gas phase ion spectrometry. Any suitable gas phase ion spectrometers can be used as long as it allows markers on the substrate to be resolved. Preferably, gas phase ion spectrometers allow quantitation of markers.

In one embodiment, a gas phase ion spectrometer is a mass spectrometer. In a typical mass spectrometer, a substrate or a probe comprising markers on its surface is introduced into an inlet system of the mass spectrometer. The markers are then desorbed by a desorption source such as a laser, fast atom bombardment, high energy plasma, electrospray ionization, thermospray ionization, liquid secondary ion MS, field desorption, etc. The generated desorbed, volatilized species consist of preformed ions or neutrals which are ionized as a direct consequence of the desorption event. Generated ions are collected by an ion optic assembly, and then a mass analyzer disperses and analyzes the passing ions. The ions exiting the mass analyzer are detected by a detector. The detector then translates information of the detected ions into mass-to-charge ratios. Detection of the presence of markers or other substances will typically involve detection of signal intensity. This, in turn, can reflect the quantity and character of markers bound to the substrate. Any of the components of a mass spectrometer (e.g., a desorption source, a mass analyzer, a detector, etc.) can be combined with other suitable components described herein or others known in the art in embodiments of the invention.

Preferably, a laser desorption time-of-flight mass spectrometer is used in embodiments of the invention. In laser desorption mass spectrometry, a substrate or a probe comprising markers is introduced into an inlet system. The markers are desorbed and ionized into the gas phase by laser from the ionization source. The ions generated are collected by an ion optic assembly, and then in a time-of-flight mass analyzer, ions are accelerated through a short high voltage field and let drift into a high vacuum chamber. At the far end of the high vacuum chamber, the accelerated ions strike a sensitive detector surface at a different time. Since the time-of-flight is a function of the mass of the ions, the elapsed time between ion formation and ion detector impact can be used to identify the presence or absence of markers of specific mass to charge ratio.

In another embodiment, an ion mobility spectrometer can be used to detect markers. The principle of ion mobility spectrometry is based on different mobility of ions. Specifically, ions of a sample produced by ionization move at different rates, due to their difference in, e.g., mass, charge, or shape, through a tube under the influence of an electric field. The ions (typically in the form of a current) are registered at the detector which can then be used to identify a marker or other substances in a sample. One advantage of ion mobility spectrometry is that it can operate at atmospheric pressure. In yet another embodiment, a total ion current measuring device can be used to detect and characterize markers. This device can be used when the substrate has a only a single type of marker. When a single type of marker is on the substrate, the total current generated from the ionized marker reflects the quantity and other characteristics of the marker. The total ion current produced by the marker can then be compared to a control (e.g., a total ion current of a known compound). The quantity or other characteristics of the marker can then be determined.

### IMMUNOASSAY

In another embodiment, an immunoassay can be used to detect and analyze markers in a sample. This method comprises: (a) providing an antibody that specifically binds to a marker; (b) contacting a sample with the antibody; and (c) detecting the presence of a complex of the antibody bound to the marker in the sample.

An immunoassay is an assay that uses an antibody to specifically bind an antigen (e.g., a marker). The immunoassay is characterized by the use of specific binding properties of a particular antibody to isolate, target, and/or quantify the antigen. The phrase "specifically (or selectively) binds" to an antibody or "specifically (or selectively) immunoreactive with," when referring to a protein or peptide, refers to a binding reaction that is determinative of the presence of the protein in a heterogeneous population of proteins and other biologics. Thus, under designated immunoassay conditions, the specified antibodies bind to a particular protein at least two times the background and do not substantially bind in a Significant amount to other proteins present in the sample. Specific binding to an antibody under such conditions may require an antibody that is selected for its specificity for a particular protein. For example, polyclonal antibodies raised to a marker from specific species such as rat, mouse, or human can be selected to obtain only those polyclonal antibodies that are specifically immunoreactive with that marker and not with other proteins, except for polymorphic variants and alleles of the marker. This selection may be achieved by subtracting out antibodies that cross-react with the marker molecules from other species.

Using the purified markers or their nucleic acid sequences, antibodies that specifically bind to a marker can be prepared using any suitable methods known in the art. See, e.g., Coligan, Current Protocols in Immunology (1991); Harlow & Lane, Antibodies: A Laboratory Manual (1988); Coding, Monoclonal Antibodies: Principles and Practice (2d ed. 1986); and Kohler & Milstein, Nature 256:495-497 (1975). Such techniques include, but are not limited to, antibody preparation by selection of antibodies from libraries of recombinant antibodies in phage or similar vectors, as well as preparation of polyclonal and monoclonal antibodies by immunizing rabbits or mice (see, e.g., Huse et al., Science 246:1275-1281 (1989); Ward et al., Nature 341:544-546 (1989)). Typically a specific or selective reaction will be at least twice background signal or noise and more typically more than 10 to 100 times background.

Generally, a sample obtained from a subject can be contacted with the antibody that specifically binds the marker. Optionally, the antibody can be fixed to a solid support to facilitate washing and subsequent isolation of the complex, prior to contacting the antibody with a sample. Examples of solid supports include glass or plastic in the form of, e.g., a microtiter plate, a stick, a bead, or a microbead. Antibodies can also be attached to a probe substrate or ProteinChip® array described above. The sample is preferably a biological fluid sample taken from a subject. Examples of biological fluid samples include blood, serum, plasma, nipple aspirate, urine, tears, saliva etc. In a preferred embodiment, the biological fluid comprises blood serum. The sample can be diluted with a suitable eluant before contacting the sample to the antibody.

After incubating the sample with antibodies, the mixture is washed and the antibody-marker complex formed can be detected. This can be accomplished by incubating the washed mixture with a detection reagent. This detection reagent may be, e.g., a second antibody which is labeled with a detectable label. Exemplary detectable labels include magnetic beads (e.g., DYNABEADSTM), fluorescent dyes, radiolabels, enzymes (e.g., horse radish peroxide, alkaline phosphatase and others commonly used in an ELISA), and colorimetric labels such as colloidal gold or colored glass or plastic beads. Alternatively, the marker in the sample can be detected using an indirect assay, wherein, for example, a second, labeled antibody is used to detect bound marker-specific antibody, and/or in a competition or inhibition assay wherein, for example, a monoclonal antibody which binds to a distinct epitope of the marker is incubated simultaneously with the mixture.

In certain examples radiolabelled antibodies to any of the biomarkers can be used as an imaging tool for melanoma.

Methods for measuring the amount of, or presence of, antibody-marker complex include, for example, detection of fluorescence, luminescence, chemiluninescence, absorbance, reflectance, transmittance, birefringence or refractive index (e.g., surface plasmon resonance, ellipsometry, a resonant mirror method, a grating coupler waveguide method or interferometry). Optical methods include microscopy (both confocal and non-confocal), imaging methods and non-imaging methods. Electrochemical methods include voltametry and amperometry methods. Radio frequency methods include multipolar resonance spectroscopy. Methods for performing these assays are readily known in the art. Useful assays include, for example, an enzyme immune assay (EIA) such as enzyme-linked immunosorbent assay (ELISA), a radioimmune assay (RIA), a Western blot assay, or a slot blot assay. These methods are also described in, e.g., Methods in Cell Biology: Antibodies in Cell Biology, volume 37 (Asai, ed. 1993); Basic and Clinical Immunology (Stites & Terr, eds., 7th ed. 1991); and Harlow & Lane, supra.

Throughout the assays, incubation and/or washing steps may be required after each combination of reagents. Incubation steps can vary from about 5 seconds to several hours, preferably from about 5 minutes to about 24 hours. However, the incubation time will depend upon the assay format, marker, volume of solution, concentrations and the like. Usually the assays will be carried out at ambient temperature, although they can be conducted over a range of temperatures, such as 10°C to 40°C.

Immunoassays can be used to determine presence or absence of a marker in a sample as well as the quantity of a marker in a sample. The amount of an antibody-marker complex can be determined by comparing to a standard. A standard can be, e.g., a known compound or another protein known to be present in a sample. As noted above, the test amount of marker need not be measured in absolute units, as long as the unit of measurement can be compared to a control.

The methods for detecting these markers in a sample have many applications. For example, one or more markers can be measured to aid melanoma diagnosis or prognosis. In another example, the methods for detection of the markers can be used to monitor responses in a subject to melanoma treatment. In another example, the methods for detecting markers can be used to assay for and to identify compounds that modulate expression of these markers in vivo or in vitro. In a preferred example, the biomarkers are used to differentiate between the different stages of tumor progression, thus aiding in determining appropriate treatment and extent of metastasis of the tumor.

### USE OF MODIFIED FORMS OF A BIOMARKER

It has been found that proteins frequently exist in a sample in a plurality of different forms characterized by a detectably different mass. These forms can result from either, or both, of pre- and post-translational modification. Pre-translational modified forms include allelic variants, slice variants and RNA editing forms. Post-translationally modified forms include forms resulting from proteolytic cleavage (e.g., fragments of a parent protein), glycosylation, phosphorylation, lipidation, oxidation, methylation, cystinylation, sulphonation and acetylation. The collection of proteins including a specific protein and all modified forms of it is referred to herein as a "protein cluster." The collection of all modified forms of a specific protein, excluding the specific protein, itself, is referred to herein as a "modified protein cluster." Modified forms of any biomarkers of this invention (including any of Markers 1-104) also maybe used, themselves, as biomarkers. In certain cases the modified forms may exhibit better discriminatory power in diagnosis than the specific forms set forth herein.

Modified forms of a biomarker including any of the Markers as described herein can be initially detected by any methodology that can detect and distinguish the modified from the biomarker. A preferred method for initial detection involves first capturing the biomarker and modified forms of it, e.g., with biospecific capture reagents, and then detecting the captured proteins by mass spectrometry. More specifically, the proteins are captured using biospecific capture reagents, such as antibodies, aptamers or Affibodies that recognize the biomarker and modified forms of it. This method also will also result in the capture of protein interactors that are bound to the proteins or that are otherwise recognized by antibodies and that, themselves, can be biomarkers. Preferably, the biospecific capture reagents are bound to a solid phase. Then, the captured proteins can be detected by SELDI mass spectrometry or by eluting the proteins from the capture reagent and detecting the eluted proteins by traditional MALDI or by SELDI. The use of mass spectrometry is especially attractive because it can distinguish and quantify modified forms of a protein based on mass and without the need for labeling.

Preferably, the biospecific capture reagent is bound to a solid phase, such as a bead, a plate, a membrane or a chip. Methods of coupling biomolecules, such as antibodies, to a solid phase are well known in the art. They can employ, for example, bifunctional linking agents, or the solid phase can be derivatized with a reactive group, such as an epoxide or an imidizole, that will bind the molecule on contact. Biospecific capture reagents against different target proteins can be mixed in the same place, or they can be attached to solid phases in different physical or addressable locations. For example, one can load multiple columns with derivatized beads, each column able to capture a single protein cluster. Alternatively, one can pack a single column with different beads derivatized with capture reagents against a variety of protein clusters, thereby capturing all the analytes in a single place. Accordingly, antibody-derivatized bead-based technologies, such as xMAP technology of Luminex (Austin, TX) can be used to detect the protein clusters. However, the biospecific capture reagents must be specifically directed toward the members of a cluster in order to differentiate them.

In yet another embodiment, the surfaces of biochips can be derivatized with the capture reagents directed against protein clusters either in the same location or in physically different addressable locations. One advantage of capturing different clusters in different addressable locations is that the analysis becomes simpler.

After identification of modified forms of a protein and correlation with the clinical parameter of interest, the modified form can be used as a biomarker in any of the methods of this invention. At this point, detection of the modified from can be accomplished by any specific detection methodology including affinity capture followed by mass spectrometry, or traditional immunoassay directed specifically the modified form. Immunoassay requires biospecific capture reagents, such as antibodies, to capture the analytes. Furthermore, if the assay must be designed to specifically distinguish protein and modified forms of protein.

This can be done, for example, by employing a sandwich assay in which one antibody captures more than one form and second, distinctly labeled antibodies, specifically bind, and provide distinct detection of, the various forms. Antibodies can be produced by immunizing animals with the biomolecules. This invention contemplates traditional immunoassays including, for example, sandwich immunoassays including ELISA or fluorescence-based immunoassays, as well as other enzyme immunoassays.

### DATA ANALYSIS

The methods for detecting these markers in a sample have many applications. For example, one or more markers can be measured to aid human melanoma diagnosis or prognosis. In another example, the methods for detection of the markers can be used to monitor responses in a subject to melanoma treatment. In another example, the methods for detecting markers can be used to assay for and to identify compounds that modulate expression of these markers in vivo or in vitro.

Differentiation of non-melanoma and melanoma status may be by the detection of one or more of the Markers identified in Tables 1 - 9 or the Markers described as proteins or pathways for melanoma. For example, an exemplary marker that may independently discriminate between melanoma status is Nrp-2 or MMP-1. Combinations of markers are also useful in the methods of the invention for the determination melanoma and melanoma status. Markers may be detected, determine, monitored in a sample by molecular biological methods, including, immunological, arrays (nucleic acid, protein), PCR methods (real-time, reverse transcriptase, PCR).

Detection of markers can be analyzed using any suitable means, including arrays. Nucleic acid arrays may be analyzed using software, for example, Applied Maths, Belgium. GenExplore™ : 2-way cluster analysis, principal component analysis, discriminant analysis, self-organizing maps; BioDiscovery, Inc., Los Angeles, California (ImaGene™, special image processing and data extraction software, powered by MatLab®; GeneSight: hierarchical clustering, artificial neural network (SOM), principal component analysis, time series; AutoGene™; CloneTracker™); GeneData AG (Basel, Switzerland); Molecular Pattern Recognition web site at MIT's Whitehead Genome Center; Rosetta Inpharmatics, Kirkland, Washington. Resolver™ Expression Data Analysis System; Scanalytics, Inc. , Fairfax, VA. Its MicroArray Suite enables researchers to acquire, visualize, process, and analyze gene expression Microarray data; TIGR (The Institute for Genome Research) offers software tools (free for academic institutions) for array analysis. For example, see also Eisen MB, Brown PO., Methods Enzymol. 1999;303:179-205.

Detection of markers can be analyzed using any suitable means. In one embodiment, the four-step data reduction algorithm developed by B. Ryu is utilized (Ryu B, Jones J, Blades NJ, Pannigiani G, Hollingsworth MA and Hruban R. Relationships and Differentially Expressed Genes among Pancreatic Cancers examined by Large-scale Serial Analysis of Gene Expression. Cancer Res 2002; 62:819-826). For example, group comparison was performed using Student's t test. Next, fold differences were evaluated and only those genes with a five-fold or greater expression were kept. The third step involves filtration by sample criteria. However, this step was not applied in this circumstance as expression profiling data from primary melanoma tissue samples was not yet available. In the final step, the genes were further reduced according to the degree of up-regulated expression (Norgauer J, Metzner B, Schraufstatter I. Expression and growth-promoting function of the IL-8 receptor beta in human melanoma cells. J Immunol. 1996; 156(3):1132-1137).

In one embodiment, data generated, for example, by desorption is analyzed with the use of a programmable digital computer. The computer program generally contains a readable medium that stores codes. Certain code can be devoted to memory that includes the location of each feature on a probe, the identity of the adsorbent at that feature and the elution conditions used to wash the adsorbent. The computer also contains code that receives as input, data on the strength of the signal at various molecular masses received from a particular addressable location on the probe. This data can indicate the number of markers detected, including the strength of the signal generated by each marker.

Data analysis can include the steps of determining signal strength (e.g., height of peaks) of a marker detected and removing "outliers" (data deviating from a predetermined statistical distribution). The observed peaks can be normalized, a process whereby the height of each peak relative to some reference is calculated. For example, a reference can be background noise generated by instrument and chemicals (e.g., energy absorbing molecule) which is set as zero in the scale. Then the signal strength detected for each marker or other biomolecules can be displayed in the form of relative intensities in the scale desired (e.g., 100). Alternatively, a standard (e.g., a serum protein) may be admitted with the sample so that a peak from the standard can be used as a reference to calculate relative intensities of the signals observed for each marker or other markers detected.

The computer can transform the resulting data into various formats for displaying. In one format, referred to as "spectrum view or retentate map," a standard spectral view can be displayed, wherein the view depicts the quantity of marker reaching the detector at each particular molecular weight. In another format, referred to as "peak map," only the peak height and mass information are retained from the spectrum view, yielding a cleaner image and enabling markers with nearly identical molecular weights to be more easily seen. In yet another format, referred to as "gel view," each mass from the peak view can be converted into a grayscale image based on the height of each peak, resulting in an appearance similar to bands on electrophoretic gels. In yet another format, referred to as "3-D overlays," several spectra can be overlaid to study subtle changes in relative peak heights. In yet another format, referred to as "difference map view," two or more spectra can be compared, conveniently highlighting unique markers and markers which are up- or down-regulated between samples. Marker profiles (spectra) from any two samples may be compared visually. In yet another format, Spotfire Scatter Plot can be used, wherein markers that are detected are plotted as a dot in a plot, wherein one axis of the plot represents the apparent molecular of the markers detected and another axis represents the signal intensity of markers detected. For each sample, markers that are detected and the amount of markers present in the sample can be saved in a computer readable medium. This data can then be compared to a control (e.g., a profile or quantity of markers detected in control, e.g., men in whom human melanoma is undetectable).

When the sample is measured and data is generated, e.g., by mass spectrometry, the data is then analyzed by a computer software program. Generally, the software can comprise code that converts signal from the mass spectrometer into computer readable form. The software also can include code that applies an algorithm to the analysis of the signal to determine whether the signal represents a "peak" in the signal corresponding to a marker of this invention, or other useful markers. The software also can include code that executes an algorithm that compares signal from a test sample to a typical signal characteristic of "normal" and melanoma and determines the closeness of fit between the two signals. The software also can include code indicating which the test sample is closest to, thereby providing a probable diagnosis.

In preferred methods of the present invention, multiple biomarkers are measured. The use of multiple biomarkers increases the predictive value of the test and provides greater utility in diagnosis, toxicology, subject stratification and subject monitoring. The process called "Pattern recognition" detects the patterns formed by multiple biomarkers greatly improves the sensitivity and specificity of clinical proteomics for predictive medicine. Subtle variations in data from clinical samples, e.g., obtained using SELDI, indicate that certain patterns of protein expression can predict phenotypes such as the presence or absence of a certain disease, a particular stage of melanoma progression, or a positive or adverse response to drug treatments.

Data generation in mass spectrometry begins with the detection of ions by an ion detector as described above. Ions that strike the detector generate an electric potential that is digitized by a high speed time-array recording device that digitally captures the analog signal. Ciphergen's ProteinChip® system employs an analog-to-digital converter (ADC) to accomplish this. The ADC integrates detector output at regularly spaced time intervals into time-dependent bins. The time intervals typically are one to four nanoseconds long. Furthermore, the time-of-flight spectrum ultimately analyzed typically does not represent the signal from a single pulse of ionizing energy against a sample, but rather the sum of signals from a number of pulses. This reduces noise and increases dynamic range. This time-of-flight data is then subject to data processing. In Ciphergen's ProteinChip® software, data processing typically includes TOF-to-M/Z transformation, baseline subtraction, high frequency noise filtering.

TOF-to-M/Z transformation involves the application of an algorithm that transforms times-of-flight into mass-to-charge ratio (M/Z). In this step, the signals are converted from the time domain to the mass domain. That is, each time-of flight is converted into mass-to-charge ratio, or M/Z. Calibration can be done internally or externally. In internal calibration, the sample analyzed contains one or more analytes of known M/Z. Signal peaks at times-of-flight representing these massed analytes are assigned the known M/Z. Based on these assigned M/Z ratios, parameters are calculated for a mathematical function that converts times-of-flight to M/Z. In external calibration, a function that converts times-of-flight to M/Z, such as one created by prior internal calibration, is applied to a time-of-flight spectrum without the use of internal calibrants.

Baseline subtraction improves data quantification by eliminating artificial, reproducible instrument offsets that perturb the spectrum. It involves calculating a spectrum baseline using an algorithm that incorporates parameters such as peak width, and then subtracting the baseline from the mass spectrum.

High frequency noise signals are eliminated by the application of a smoothing function. A typical smoothing function applies a moving average function to each time-dependent bin. In an improved version, the moving average filter is a variable width digital filter in which the bandwidth of the filter varies as a function of, e.g., peak bandwidth, generally becoming broader with increased time-of-flight. See, e.g., WO 00/70648, November 23, 2000 (Gavin et aL, "Variable Width Digital Filter for Time-of-flight Mass Spectrometry").

Analysis generally involves the identification of peaks in the spectrum that represent signal from an analyte. Peak selection can, of course, be done by eye. However, software is available as part of Ciphergen's ProteinChip® software that can automate the detection of peaks. In general, this software functions by identifying signals having a signal-to-noise ratio above a selected threshold and labeling the mass of the peak at the centroid of the peak signal. In one useful application many spectra are compared to identify identical peaks present in some selected percentage of the mass spectra. One version of this software clusters all peaks appearing in the various spectra within a defined mass range, and assigns a mass (M/Z) to all the peaks that are near the mid-point of the mass (M/Z) cluster.

Peak data from one or more spectra can be subject to further analysis by, for example, creating a spreadsheet in which each row represents a particular mass spectrum, each column represents a peak in the spectra defined by mass, and each cell includes the intensity of the peak in that particular spectrum. Various statistical or pattern recognition approaches can applied to the data.

The spectra that are generated in embodiments of the invention can be classified using a pattern recognition process that uses a classification model. In some embodiments, data derived from the spectra (e.g., mass spectra or time-of-flight spectra) that are generated using samples such as "known samples" can then be used to "train" a classification model. A "known sample" is a sample that is pre-classified (e.g., melanoma or not melanoma). Data derived from the spectra (e.g., mass spectra or time-of-flight spectra) that are generated using samples such as "known samples" can then be used to "train" a classification model. A "known sample" is a sample that is pre-classified The data that are derived from the spectra and are used to form the classification model can be referred to as a "training data set". Once trained, the classification model can recognize patterns in data derived from spectra generated using unknown samples. The classification model can then be used to classify the unknown samples into classes. This can be useful, for example, in predicting whether or not a particular biological sample is associated with a certain biological condition (e.g., diseased vs. non diseased).

The training data set that is used to form the classification model may comprise raw data or pre-processed data. In some embodiments, raw data can be obtained directly from time-of-flight spectra or mass spectra, and then may be optionally "pre-processed" in any suitable manner. For example, signals above a predetermined signal-to-noise ratio can be selected so that a subset of peaks in a spectrum is selected, rather than selecting all peaks in a spectrum. In another example, a predetermined number of peak "clusters" at a common value (e.g., a particular time-of-flight value or mass-to-charge ratio value) can be used to select peaks. Illustratively, if a peak at a given mass-to-charge ratio is in less than 50% of the mass spectra in a group of mass spectra, then the peak at that mass-to-charge ratio can be omitted from the training data set. Pre-processing steps such as these can be used to reduce the amount of data that is used to train the classification model.

Classification models can be formed using any suitable statistical classification (or "learning") method that attempts to segregate bodies of data into classes based on objective parameters present in the data. Classification methods may be either supervised or unsupervised. Examples of supervised and unsupervised classification processes are described in Jain, "Statistical Pattern Recognition: A Review", IEEE Transactions on Pattern Analysis and Machine Intelligence, Vol. 22, No. 1, January 2000, which is herein incorporated by reference in its entirety.

In supervised classification, training data containing examples of known categories are presented to a learning mechanism, which learns one more sets of relationships that define each of the known classes. New data may then be applied to the learning mechanism, which then classifies the new data using the learned relationships. Examples of supervised classification processes include linear regression processes (e.g., multiple linear regression (MLR), partial least squares (PLS) regression and principal components regression (PCR)), binary decision trees (e.g., recursive partitioning processes such as CART - classification and regression trees), artificial neural networks such as back propagation networks, discriminant analyses (e.g., Bayesian classifier or Fischer analysis), logistic classifiers, and support vector classifiers (support vector machines).

A preferred supervised classification method is a recursive partitioning process. Recursive partitioning processes use recursive partitioning trees to classify spectra derived from unknown samples. Further details about recursive partitioning processes are provided in U.S. 2002 0138208 A1 (Paulse et al., "Method for analyzing mass spectra," September 26, 2002.

In other embodiments, the classification models that are created can be formed using unsupervised learning methods. Unsupervised classification attempts to learn classifications based on similarities in the training data set, without pre classifying the spectra from which the training data set was derived. Unsupervised learning methods include cluster analyses. A cluster analysis attempts to divide the data into "clusters" or groups that ideally should have members that are very similar to each other, and very dissimilar to members of other clusters. Similarity is then measured using some distance metric, which measures the distance between data items, and clusters together data items that are closer to each other. Clustering techniques include the MacQueen's K-means algorithm and the Kohonen's Self-Organizing Map algorithm.

Learning algorithms asserted for use in classifying biological information are described in, for example, WO 01/31580 (Barnhill et al., "Methods and devices for identifying patterns in biological systems and methods of use thereof," May 3, 2001); U.S. 2002/0193950 A1 (Gavin et al., "Method or analyzing mass spectra," December 19, 2002); U.S. 2003/0004402 A1 (Hitt et al., "Process for discriminating between biological states based on hidden patterns from biological data," January 2,2003); and U.S. 2003/0055615 A1 (Zhang and Zhang, "Systems and methods for processing biological expression data" March 20, 2003).

More specifically, to obtain the biomarkers the peak intensity data of samples from subjects, e.g., melanoma subjects, and healthy controls are used as a "discovery set." This data were combined and randomly divided into a training set and a test set to construct and test multivariate predictive models using a non-linear version of Unified Maximum Separability Analysis ("USMA") classifiers. Details of USMA classifiers are described in U.S. 2003/0055615 A1.

The invention provides methods for aiding a human melanoma diagnosis using one or more markers, for example Markers in the tables and figures which follow, and including one or more Markers as specified herein. In particular, Nrp-2 and MMP-1 are usful markers for more aggressive melanoma. These markers can be used alone, in combination with other markers in any set, or with entirely different markers in aiding human melanoma diagnosis. The markers are differentially present in samples of a human melanoma subject and a normal subject in whom human melanoma is undetectable. For example, some of the markers are expressed at an elevated level and/or are present at a higher frequency in human melanoma subjects than in normal subjects, while some of the markers are expressed at a decreased level and/or are present at a lower frequency in human melanoma subjects than in normal subjects. Therefore, detection of one or more of these markers in a person would provide useful information regarding the probability that the person may have melanoma.

### DIFFERENTIATION BETWEEN NORMAL AND UNAFFECTED DISEASE TISSUE

The invention provides methods for aiding a human melanoma, diagnosis using one or more markers, for example the markers as described in the tables and figures herein, and including one or more of the markers identified in the Tables 1-8, and Table 9, as specified herein. These markers can be used alone, in combination with other markers in any set, or with entirely different markers in aiding human melanoma diagnosis. The markers are, differentially present in samples of a human melanoma subject and a normal subject in whom human melanoma is undetectable. For example, some of the markers are expressed at an elevated level and/or are present at a higher frequency in human melanoma subjects than in normal subjects, while some of the markers are expressed at a decreased level and/or are present at a lower frequency in human melanoma subjects than in normal subjects. Some of the markers are present at higher levels in more aggressive melanomas. Some of the markers are present at higher levels in less aggressive melanomas. Therefore, detection of one or more of these markers in a person would provide useful information regarding the probability that the person may have melanoma.

In a preferred embodiment, a biological sample is collected from a subject and then either left unfractionated, or fractionated using an anion exchange resin as described above. The biomarkers in the sample are captured using an ProteinChip array. The markers are then detected using SELDI The results are then entered into a computer system, which contains an algorithm that is designed using the same parameters that were used in the learning algorithm and classification algorithm to originally determine the biomarkers. The algorithms produces a diagnosis based upon the data received relating to each biomarker.

The diagnosis is determined by examining the data produced from the tests with algorithms that are developed using the biomarkers. The algorithms depend on the particulars of the test protocol used to detect the biomarkers. These particulars include, for example, sample preparation, chip type and mass spectrometer parameters. If the test parameters change, the algorithm must change. Similarly, if the algorithm changes, the test protocol must change.

In another embodiment, the sample is collected from the subject. The biomarkers are captured using an antibody ProteinChip array as described above. The markers are detected using a biospecific SELDI test system. The results are then entered into a computer system, which contains an algorithm that is designed using the same parameters that were used in the learning algorithm and classification algorithm to originally determine the biomarkers. The algorithm produces a diagnosis based upon the data received relating to each biomarker.

In yet other preferred embodiments, the markers are captured and tested using non-SELDI formats. In one example, the sample is collected from the subject. The biomarkers are captured on a substrate using other known means, e.g., antibodies to the markers. The markers are detected using methods known in the art, e.g., optical methods and refractive index. Examples of optical methods include detection of fluorescence, e.g., ELISA. Examples of refractive index include surface plasmon resonance. The results for the markers are then subjected to an algorithm, which may or may not require artificial intelligence. The algorithm produces a diagnosis based upon the data received relating to each biomarker.

In any of the above methods, the data from the sample maybe fed directly from the detection means into a computer containing the diagnostic algorithm. Alternatively, the data obtained can be fed manually, or via an automated means, into a separate computer that contains the diagnostic algorithm.

Accordingly, embodiments of the invention include methods for aiding a human melanoma diagnosis, wherein the method comprises: (a) detecting at least one marker in a sample, wherein the marker is selected from any of the markers identifies in any one of Tables 1-8; and (b) correlating the detection of the marker or markers with a probable diagnosis of human melanoma. The correlation may take into account the amount of the marker or markers in the sample compared to a control amount of the marker or markers (up or down regulation of the marker or markers) (e.g., in normal subjects in whom human melanoma is undetectable). The correlation may take into account the presence or absence of the markers in a test sample and the frequency of detection of the same markers in a control. The correlation may take into account both of such factors to facilitate determination of whether a subject has a human melanoma or not.

In a preferred embodiment, any one of the markers identified in any one of Tables 1 - 8 are used to make a correlation with melanoma, wherein the melanoma may be any subtype, e.g., superficial spreading, nodular, acrolentiginous, and lentigo maligna.

In one example, the biomarker is selected from one or more markers identified in Table 2 or Table 8, or combinations thereof.

In another example, the biomarker is selected from one of more markers identified in Table 4, or combinations thereof.

In another example, the biomarker is selected from one or more markers identified in Table 5, or combinations thereof.

In another example, the biomarker is selected from one or more markers identified in Table 6, or combinations thereof.

In another example, the biomarker is selected from one or more markers identified in Table 7, 8 or 9.

In another example, the biomarker is NRP-2 or MMP-1.

Thus, detection of biomarkers that correlate, for example, with aggressive melanoma, will provide a methods for aiding a human melanoma diagnosis, where the diagnosis is of a more aggressive form of melanoma

In certain cases, the measurement of biomarker expression is used to classify a subject as a low or high risk for melanoma recurrence.

In other cases, biomarker detection is used to determine a course of treatment for a subject.

Any suitable samples can be obtained from a subject to detect markers. Preferably, a sample is a blood sample or a tissue biopsy. If desired, the sample can be prepared as described above to enhance detectability of the markers. For example, to increase the detectability of markers, a sample from the subject can be preferably fractionated by, e.g., Cibacron blue agarose chromatography and single stranded DNA affinity chromatography, anion exchange chromatography and the like. Sample preparations, such as pre-fractionation protocols, are optional and may not be necessary to enhance detectability of markers depending on the methods of detection used. For example, sample preparation may be unnecessary if antibodies that specifically bind markers are used to detect the presence of markers in a sample.

Processes for the purification of a biomarker include fractioning a sample, as described herein, for example, by size-exclusion chromatography and collecting a fraction that includes one or more biomarkers; and/or fractionating a sample comprising the one or more biomarkers by anion exchange chromatography and collecting a fraction that includes one or more biomarkers, wherein the biomarker is selected from one or more of the biomarkes as described herein.

### DIAGNOSIS OF SUBJECT AND DETERMINATION OF MELANOMA STATUS

Any biomarker, individually, is useful in aiding in the determination of melanoma status. First, the selected biomarker is measured in a subject sample using the methods described herein, e.g., capture on a SELDI biochip followed by detection by mass spectrometry or by immunoassay or microarray. Then, the measurement is compared with a diagnostic amount or control that distinguishes a melanoma status from a non-melanoma status. The diagnostic amount will reflect the information herein that a particular biomarker is up-regulated or down-regulated in a melanoma status compared with a non-melanoma status. As is well understood in the art, the particular diagnostic amount used can be adjusted to increase sensitivity or specificity of the diagnostic assay depending on the preference of the diagnostician. The test amount as compared with the diagnostic amount thus indicates melanoma status.

In a preferred embodiment, any one of the markers identified in any one of Tables 1 - 8 are used to make a correlation with melanoma, wherein the melanoma may be any subtype, e.g., superficial spreading, nodular, acrolentiginous, and lentigo maligna.

In one example, the biomarker is selected from one or more markers identified in Table 2 or Table 8, or combinations thereof.

In another example, the biomarker is selected from one of more markers identified in Table 4, or combinations thereof.

In another example, the biomarker is selected from one or more markers identified in Table 5, or combinations thereof.

In another example, the biomarker is selected from one or more markers identified in Table 6, or combinations thereof.

In another example, the biomarker is selected from one or more markers identified in Table 7, 8 or 9.

In another example, the biomarker is NRP-2 or MMP-1.

Thus, the biomarker that is expressed can be used to correlate melanoma status with disease progression.

While individual biomarkers are useful diagnostic markers, combination of biomarkers provide predictive value as well. Specifically, the detection of a plurality of markers in a sample increases the percentage of true positive and true negative diagnoses and would decrease the percentage of false positive or false negative diagnoses. Thus, methods of the present invention comprise the measurement of more than one biomarker.

The detection of the marker or markers is then correlated with a probable diagnosis of melanoma. In some embodiments, the detection of the mere presence or absence of a marker, without quantifying the amount of marker, is useful and can be correlated with a probable diagnosis of melanoma. For example, markers as identified from any one of Tables 1 - 8 or Table 9, can be more frequently detected in human melanoma subjects than in normal subjects. A mere detection of one or more of these markers in a subject being tested indicates that the subject has a higher probability of having melanoma.

In other embodiments, the measurement of markers can involve quantifying the markers to correlate the detection of markers with a probable diagnosis of melanoma. Thus, if the amount of the markers detected in a subject being tested is different compared to a control amount (i.e., higher or lower than the control, depending on the marker), then the subject being tested has a higher probability of having melanoma.

The correlation may take into account the amount of the marker or markers in the sample compared to a control amount of the marker or markers (up or down regulation of the marker or markers) (e.g., in normal subjects or in non-melanoma subjects such as where melanoma is undetectable). A control can be, e.g., the average or median amount of marker present in comparable samples of normal subjects in normal subjects or in non-melanoma subjects such as where melanoma is undetectable. The control amount is measured under the same or substantially similar experimental conditions as in measuring the test amount. The correlation may take into account the presence or absence of the markers in a test sample and the frequency of detection of the same markers in a control. The correlation may take into account both of such factors to facilitate determination of melanoma status.

In certain embodiments of the methods of qualifying melanoma status, the methods further comprise managing subject treatment based on the status. As before the management of the subject describes the actions of the physician or clinician subsequent to determining melanoma status. For example, if the result of the methods of the present invention is inconclusive or there is reason that confirmation of status is necessary, the physician may order more tests (e.g., CT_scans, PET scans,MRI scans, PET-CT scans, X-rays, biopsies, blood tests (LFTs, LDH). Alternatively, if the status indicates that treatment is appropriate, the physician may schedule the subject for treatment. In other instances, the subject may receive therapeutic treatments, either in lieu of, or in addition to, surgery. No further action may be warranted. Furthermore, if the results show that treatment has been successful, a maintenance therapy or no further management may be necessary.

Therapeutic agents may include, one or more of fotemustine, dacarbazine, interferon, citsplatin, tamoxifen, interleukin-2, ifn alfa, vinblastin, or orcarmubris.

The invention also provides for such methods where the biomarkers (or specific combination of biomarkers) are measured again after subject management. In these cases, the methods are used to monitor the status of the melanoma, e.g., response to melanoma treatment, remission of the disease or progression of the disease. Because of the ease of use of the methods and the lack of invasiveness of the methods, the methods can be repeated after each treatment the subject receives. This allows the physician to follow the effectiveness of the course of treatment. If the results show that the treatment is not effective, the course of treatment can be altered accordingly. This enables the physician to be flexible in the treatment options.

In another example, the methods for detecting markers can be used to assay for and to identify compounds that modulate expression of these markers in vivo or in vitro.

The methods of the present invention have other applications as well. For example, the markers can be used to screen for compounds that modulate the expression of the markers in vitro or in vivo, which compounds in turn may be useful in treating or preventing melanoma in subjects. In another example, the markers can be used to monitor the response to treatments for melanoma. In yet another example, the markers can be used in heredity studies to determine if the subject is at risk for developing melanoma. For instance, certain markers may be genetically linked. This can be determined by, e.g., analyzing samples from a population of melanoma subjects whose families have a history of melanoma. The results can then be compared with data obtained from, e.g., melanoma subjects whose families do not have a history of melanoma. The markers that are genetically linked may be used as a tool to determine if a subject whose family has a history of melanoma is pre-disposed to having melanoma.

In a preferred embodiment of the invention, a diagnosis based on the presence or absence in a test subject of any the biomarkers of this invention is communicated to the subject as soon as possible after the diagnosis is obtained. The diagnosis may be communicated to the subject by the subject's treating physician. Alternatively, the diagnosis may be sent to a test subject by email or communicated to the subject by phone. A computer may be used to communicate the diagnosis by email or phone. In certain embodiments, the message containing results of a diagnostic test may be generated and delivered automatically to the subject using a combination of computer hardware and software which will be familiar to artisans skilled in telecommunications. One example of a healthcare-oriented communications system is described in U.S. Patent Number 6,283,761; however, the present invention is not limited to methods which utilize this particular communications system. In certain embodiments of the methods of the invention, all or some of the method steps, including the assaying of samples, diagnosing of diseases, and communicating of assay results or diagnoses, may be carried out in diverse (e.g., foreign) jurisdictions.

Methods of the invention for determining the melanoma status of a subject, include for example, obtaining a biomarker profile from a sample taken from the subject; and comparing the subject's biomarker profile to a reference biomarker profile obtained from a reference population, wherein the comparison is capable of classifying the subject as belonging to or not belonging to the reference population; wherein the subject's biomarker profile and the reference biomarker profile comprise one or more markers as described herein.

The method may further comprise repeating the method at least once, wherein the subject's biomarker profile is obtained from a separate sample taken each time the method is repeated.

Samples from the subject may be taken at any time, for example, the samples may be taken 24 hours apart or any other time determined useful.

Such comparisons of the biomarker profiles can determine melanoma status in the subject with an accuracy of at least about 60%, 70%, 80%, 90%, 95%, and approaching 100% as shown in the examples which follow.

The reference biomarker profile can be obtained from a population comprising a single subject, at least two subjects, at least 20 subjects or more. The number of subjects will depend, in part, on the number of available subjects, and the power of the statistical analysis necessary.

A method of treating melanoma comprising administering to a subject suffering from or at risk of developing melanoma a therapeutically effective amount of a compound capable of modulating the expression or activity of one or more of the biomarkers of Tables 1-3.

A method of treating a condition in a subject comprising administering to a subject a therapeutically effective amount of a compound which modulates the expression or activity of one or more of the biomarkers from Table 1-8.

Compounds useful in methods disclosed herein include, for example, fotemustine, dacarbazine, interferon, cisplatin, tamoxifen, interleukin-2, ifn alfa, vinblastin, or orcarmubris.

The invention includes methods of qualifying melanoma status in a subject comprising:
(a) measuring at least one biomarker in a sample from the subject, wherein the biomarker is selected from one or more of the biomarkers from one or more of Tables 1-8 and
(b) correlating the measurement with melanoma status.

The method may also comprise the step of measuring the at least one biomarker after subject management.

In a preferred embodiment, any one of the markers identified in any one of Tables 1 - 8 are used to make a correlation with melanoma, wherein the melanoma may be any subtype, e.g., superficial spreading, nodular, acrolentiginous, and lentigo maligna.

In one example, the biomarker is selected from one or more markers identified in Table 2 or Table 8, or combinations thereof.

In another example, the biomarker is selected from one of more markers identified in Table 4, or combinations thereof.

In another example, the biomarker is selected from one or more markers identified in Table 5, or combinations thereof.

In another example, the biomarker is selected from one or more markers identified in Table 6, or combinations thereof.

In another example, the biomarker is selected from one or more markers identified in Table 7, 8 or 9.

In another example, the biomarker is NRP-2 or MMP-1.

Optionally, the methods of the invention may further comprise generating data on immobilized subject samples on a biochip, by subjecting the biochip to laser ionization and detecting intensity of signal for mass/charge ratio; and transforming the data into computer readable form; and executing an algorithm that classifies the data according to user input parameters, for detecting signals that represent biomarkers present in melanoma subjects and are lacking in non-melanoma subject controls.

Types or stages of melanoma that may be identified or differentiated from one another according to this method include, stages 0, I-IV and recurrent melanoma.

### ANTIBODIES

Antibodies are well known to those of ordinary skill in the science of immunology. As used herein, the term "antibody" means not only intact antibody molecules, but also fragments of antibody molecules that retain immunogen binding ability. Such fragments are also well known in the art and are regularly employed both *in vitro* and *in vivo.* Accordingly, as used herein, the term "antibody" means not only intact immunoglobulin molecules but also the well-known active fragments F(ab')₂, and Fab. F(ab')₂, and Fab fragments which lack the Fc fragment of intact antibody, clear more rapidly from the circulation, and may have less non-specific tissue binding of an intact antibody (Wahl et al., J. Nucl. Med. 24:316-325 (1983). The antibodies of the invention comprise whole native antibodies, bispecific antibodies; chimeric antibodies; Fab, Fab', single chain V region fragments (scFv) and fusion polypeptides.

In one embodiment, an antibody that binds Nrp-2 polypeptide (e.g., Nrp-2 or a Nrp-2 variant) is monoclonal. Alternatively, the anti-Nrp-2 antibody is a polyclonal antibody. The preparation and use of polyclonal antibodies are also known the skilled artisan. The invention also encompasses hybrid antibodies, in which one pair of heavy and light chains is obtained from a first antibody, while the other pair of heavy and light chains is obtained from a different second antibody. Such hybrids may also be formed using humanized heavy and light chains. Such antibodies are often referred to as "chimeric" antibodies.

In general, intact antibodies are said to contain "Fc" and "Fab" regions. The Fc regions are involved in complement activation and are not involved in antigen binding. An antibody from which the Fc' region has been enzymatically cleaved, or which has been produced without the Fc' region, designated an "F(ab')₂" fragment, retains both of the antigen binding sites of the intact antibody. Similarly, an antibody from which the Fc region has been enzymatically cleaved, or which has been produced without the Fc region, designated an "Fab"' fragment, retains one of the antigen binding sites of the intact antibody. Fab' fragments consist of a covalently bound antibody light chain and a portion of the antibody heavy chain, denoted "Fd." The Fd fragments are the major determinants of antibody specificity (a single Fd fragment may be associated with up to ten different light chains without altering antibody specificity). Isolated Fd fragments retain the ability to specifically bind to immunogenic epitopes.

Antibodies can be made by any of the methods known in the art utilizing Nrp-2 polypeptides (e.g., Nrp-2 or an Nrp-2 variant), or immunogenic fragments thereof, as an immunogen. One method of obtaining antibodies is to immunize suitable host animals with an immunogen and to follow standard procedures for polyclonal or monoclonal antibody production. The immunogen will facilitate presentation of the immunogen on the cell surface. Immunization of a suitable host can be carried out in a number of ways. Nucleic acid sequences encoding an Nrp-2 polypeptide, or immunogenic fragments thereof, can be provided to the host in a delivery vehicle that is taken up by immune cells of the host. The cells will in turn express the receptor on the cell surface generating an immunogenic response in the host. Alternatively, nucleic acid sequences encoding a Nrp-2 polypeptide, or immunogenic fragments thereof, can be expressed in cells *in vitro,* followed by isolation of the receptor and administration of the receptor to a suitable host in which antibodies are raised.

Using either approach, antibodies can then be purified from the host. Antibody purification methods may include salt precipitation (for example, with ammonium sulfate), ion exchange chromatography (for example, on a cationic or anionic exchange column preferably run at neutral pH and eluted with step gradients of increasing ionic strength), gel filtration chromatography (including gel filtration HPLC), and chromatography on affinity resins such as protein A, protein G, hydroxyapatite, and anti-immunoglobulin.

Antibodies can be conveniently produced from hybridoma cells engineered to express the antibody. Methods of making hybridomas are well known in the art. The hybridoma cells can be cultured in a suitable medium, and spent medium can be used as an antibody source. Polynucleotides encoding the antibody of interest can in turn be obtained from the hybridoma that produces the antibody, and then the antibody may be produced synthetically or recombinantly from these DNA sequences. For the production of large amounts of antibody, it is generally more convenient to obtain an ascites fluid. The method of raising ascites generally comprises injecting hybridoma cells into an immunologically naive histocompatible or immunotolerant mammal, especially a mouse. The mammal may be primed for ascites production by prior administration of a suitable composition; e.g., Pristane.

Monoclonal antibodies (Mabs) produced by methods of the invention can be "humanized" by methods known in the art. "Humanized" antibodies are antibodies in which at least part of the sequence has been altered from its initial form to render it more like human immunoglobulins. Techniques to humanize antibodies are particularly useful when non-human animal (e.g., murine) antibodies are generated. Examples of methods for humanizing a murine antibody are provided in U.S. Patent Nos. 4,816,567, 5,530,101, 5,225,539, 5,585,089, 5,693,762 and 5,859,205.

### INHIBITORY NUCLEIC ACIDS

The invention encompasses the use of inhibitory nucleic acids. Inhibitory nucleic acids may be designed based on identification of biomarkers that indicate melanoma status and progression of disease in a subject.

In certain preferred examples, the invention features Nrp-2 or MMP-1 inhibitory nucleic acid molecules.

Nrp-2 or MMP-1 inhibitory nucleic acid molecules are essentially nucleobase oligomers that may be employed as single-stranded or double-stranded nucleic acid molecule to decrease Nrp-2 or MMP-1 expression. In one approach, the Nrp-2 or MMP-1 inhibitory nucleic acid molecule is a double-stranded RNA used for RNA interference (RNAi)-mediated knock-down of Nrp-2 or MMP-1 gene expression. In one embodiment, a double-stranded RNA (dsRNA) molecule is made that includes between eight and twenty-five (e.g., 8, 10, 12, 15, 16, 17, 18, 19, 20, 21, 22, 23, 24, 25) consecutive n-aclcobases of a nucleobase oligomer of the invention. The dsRNA can be two complementary strands of RNA that have duplexed, or a single RNA strand that has self-duplexed (small hairpin (sh)RNA). Typically, dsRNAs are about 21 or 22 base pairs, but may be shorter or longer (up to about 29 nucleobases) if desired. Double stranded RNA can be made using standard techniques (e.g., chemical synthesis or in vitro transcription). Kits are available, for example, from Ambion (Austin, Tex.) and Epicentre (Madison, Wis.). Methods for expressing dsRNA in mammalian cells are described in Brummelkamp et al. Science 296:550-553, 2002; Paddison et al. Genes & Devel. 16:948-958, 2002. Paul et al. Nature Biotechnol. 20:505-508, 2002; Sui et al. Proc. Natl. Acad. Sci. USA 99:5515-5520, 2002; Yu et al. Proc. Natl. Acad. Sci. USA 99:6047-6052, 2002; Miyagishi et al. Nature Biotechnol. 20:497-500, 2002; and Lee et al. Nature Biotechnol. 20:500-505 2002, each of which is hereby incorporated by reference. An inhibitory nucleic acid molecule that "corresponds" to an Nrp-2 or MMP-1 gene comprises at least a fragment of the double-stranded gene, such that each strand of the double-stranded inhibitory nucleic acid molecule is capable of binding to the complementary strand of the target Nrp-2 or MMP-1 gene. The inhibitory nucleic acid molecule need not have perfect correspondence to the reference Nrp-2 or MMP-1 sequence. In one embodiment, an siRNA has at least about 85%, 90%, 95%, 96%, 97%, 98%, or even 99% sequence identity with the target nucleic acid. For example, a 19 base pair duplex having 1-2 base pair mismatch is considered useful in the methods of the invention. In other embodiments, the nucleobase sequence of the inhibitory nucleic acid molecule exhibits 1, 2, 3, 4, 5 or more mismatches.

The inhibitory nucleic acid molecules provided by the invention are not limited to siRNAs, but include any nucleic acid molecule sufficient to decrease the expression of an Nrp-2 or MMP-1 nucleic acid molecule or polypeptide. Each of the DNA sequences provided herein may be used, for example, in the discovery and development of therapeutic antisense nucleic acid molecule to decrease the expression of Nrp-2 or MMP-1. The invention further provides catalytic RNA molecules or ribozymes. Such catalytic RNA molecules can be used to inhibit expression of an Nrp-2 or MMP-1 nucleic acid molecule *in vivo.* The inclusion of ribozyme sequences within an antisense RNA confers RNA-cleaving activity upon the molecule, thereby increasing the activity of the constructs. The design and use of target RNA-specific ribozymes is described in Haseloff et al., Nature 334:585-591. 1988, and U.S. Patent Application Publication No. 2003/0003469 A1, each of which is incorporated by reference. In various embodiments of this invention, the catalytic nucleic acid molecule is formed in a hammerhead or hairpin motif. Examples of such hammerhead motifs are described by Rossi et al., Aids Research and Human Retroviruses, 8:183, 1992. Example of hairpin motifs are described by Hampel et al., "RNA Catalyst for Cleaving Specific RNA Sequences," filed Sep. 20, 1989, which is a continuation-in-part of U.S. Ser. No. 07/247,100 filed Sep. 20, 1988, Hampel and Tritz, Biochemistry, 28:4929, 1989, and Hampel et al., Nucleic Acids Research, 18: 299, 1990. These specific motifs are not limiting in the invention and those skilled in the art will recognize that all that is important in an enzymatic nucleic acid molecule of this invention is that it has a specific substrate binding site which is complementary to one or more of the target gene RNA regions, and that it have nucleotide sequences within or surrounding that substrate binding site which impart an RNA cleaving activity to the molecule.

After a subject is diagnosed as having melanoma, or at risk for recurrence of melanoma, a method of treatment is selected.

In one embodiment, the inhibitory nucleic acid molecules of the invention are administered systemically in dosages between about 1 and 100 mg/kg (e.g., 1,5, 10, 20, 25, 50, 75, and 100 mg/kg). In other embodiments, the dosage ranges from between about 25 and 500 mg/m²/day. Desirably, a human patient having melanoma receives a dosage between about 50 and 300 mg/m²/day (e.g., 50, 75, 100, 125, 150, 175, 200, 250, 275, and 300).

### MODIFIED INHIBITORY NUCLEIC ACID MOLECULES

A desirable inhibitory nucleic acid molecule is one based on 2'-modified oligonucleotides containing oligodeoxynucleotide gaps with some or all internucleotide linkages modified to phosphorothioates for nuclease resistance. The presence of methylphosphonate modifications increases the affinity of the oligonucleotide for its target RNA and thus reduces the IC₅₀. This modification also increases the nuclease resistance of the modified oligonucleotide. It is understood that the methods and reagents of the present invention may be used in conjunction with any technologies that may be developed to enhance the stability or efficacy of an inhibitory nucleic acid molecule.

Inhibitory nucleic acid molecules include nucleobase oligomers containing modified backbones or non-natural internucleoside linkages. Oligomers having modified backbones include those that retain a phosphorus atom in the backbone and those that do not have a phosphorus atom in the backbone. For the purposes of this specification, modified oligonucleotides that do not have a phosphorus atom in their internucleoside backbone are also considered to be nucleobase oligomers. Nucleobase oligomers that have modified oligonucleotide backbones include, for example, phosphorothioates, chiral phosphorothioates, phosphorodithioates, phosphotriesters, aminoalkyl-phosphotriesters, methyl and other alkyl plaosphonates including 3'-alkylene phosphonates and chiral phosphonates, phosphinates, phosphoramidates, thionophosphoramidates, thionoalkylphosphonates, thionoalkylphosphotriest- ers, and boranophosphates. Various salts, mixed salts and free acid forms are also included. Representative United States patents that teach the preparation of the above phosphorus-containing linkages include, but are not limited to, U.S. Pat. Nos. 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; and 5,625,050, each of which is herein incorporated by reference.

Nucleobase oligomers having modified oligonucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH₂ component parts. Representative United States patents that teach the preparation of the above oligonucleotides include, but are not limited to, U.S. Pat. Nos. 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; and 5,677,439, each of which is herein incorporated by reference.

Nucleobase oligomers may also contain one or more substituted sugar moieties. Such modifications include 2'-O-methyl and 2'-methoxyethoxy modifications. Another desirable modification is 2'-dimethylaminooxyethoxy, 2'-aminopropoxy and 2'-fluoro. Similar modifications may also be made at other positions on an oligonucleotide or other nucleobase oligomer, particularly the 3' position of the sugar on the 3' terminal nucleotide. Nucleobase oligomers may also have sugar mimetics such as cyclobutyl moieties in place of the pentofuranosyl sugar. Representative United States patents that teach the preparation of such modified sugar structures include, but are not limited to, U.S. Pat Nos. 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; and 5,700,920, each of which is herein incorporated by reference in its entirety.

In other nucleobase oligomers, both the sugar and the internucleoside linkage, i.e., the backbone, are replaced with novel groups. The nucleobase units are maintained for hybridization with an Nrp-2 or MMP-1 nucleic acid molecule. Methods for making and using these nucleobase oligomers are described, for example, in "Peptide Nucleic Acids (PNA): Protocols and Applications" Ed. P. E. Nielsen, Horizon Press, Norfolk, United Kingdom, 1999. Representative United States patents that teach the preparation of PNAs include, but are not limited to, U.S. Pat. Nos. 5,539,082; 5,714,331; and 5,719,262, each of which is herein incorporated by reference. Further teaching of PNA compounds can be found in Nielsen et al., Science, 1991, 254, 1497-1500.

### DELIVERY OF NUCLEOBASE OLIGOMERS

Naked oligonucleotides are capable of entering tumor cells and inhibiting the expression of Nrp-2 or MMP-1. Nonetheless, it may be desirable to utilize a formulation that aids in the delivery of an inhibitory nucleic acid molecule or other nucleobase oligomers to cells (see, e.g., U.S. Pat. Nos. 5,656,611, 5,753,613, 5,785,992, 6,120,798, 6,221,959, 6,346,613, and 6,353,055, each of which is hereby incorporated by reference).

### POLYNUCLEOTIDE THERAPY

Polynucleotide therapy featuring a polynucleotide encoding an inhibitory nucleic acid molecule or analog thereof is another therapeutic approach for treating melanoma in a subject. For example, the inhibitory nucleic acid molecule may be selected from the biomarkers described herein.

Polynucleotide therapy featuring polynucleotide encoding a Nrp-2 or MMP-1 inhibitory nucleic acid molecule or analog thereof is another therapeutic approach for treating melanoma in a subject. Expression vectors encoding inhibitory nucleic acid molecules can be delivered to cells of a subject having melanoma. The nucleic acid molecules must be delivered to the cells of a subject in a form in which they can be taken up and are advantageously expressed so that therapeutically effective levels can be achieved.

Methods for delivery of the polynucleotides to the cell according to the invention include using a delivery system such as liposomes, polymers, microspheres, gene therapy vectors, and naked DNA vectors.

Transducing viral (e.g., retroviral, adenoviral, lentiviral and adeno-associated viral) vectors can be used for somatic cell gene therapy, especially because of their high efficiency of infection and stable integration and expression (see, e.g., Cayouette et al., Human Gene Therapy 8:423-430, 1997; Kido et al., Current Eye Research 15:833-844, 1996; Bloomer et al., Journal of Virology 71:6641-6649, 1997; Naldini et al., Science 272:263-267, 1996; and Miyoshi et al., Proc. Natl. Acad. Sci. U.S.A. 94:103 19, 199 7). For example, a polynucleotide encoding a Nrp-2 or MMP-1 inhibitory nucleic acid molecule, can be cloned into a retroviral vector and expression can be driven from its endogenous promoter, from the retroviral long terminal repeat, or from a promoter specific for a target cell type of interest. Other viral vectors that can be used include, for example, a vaccinia virus, a bovine papilloma virus, or a herpes virus, such as Epstein-Barr Virus (also see, for example, the vectors of Miller, Human Gene Therapy 15-14, 1990; Friedman, Science 244:1275-1281, 1989; Eglitis et al., BioTechniques 6:608-614, 1988; Tolstoshev et al., Current Opinion in Biotechnology 1:55-61,1990; Sharp, The Lancet 337:1277-1278, 1991; Cornetta et al., Nucleic Acid Research and Molecular Biology 36:311-322, 1987; Anderson, Science 226:401-409, 1984; Moen, Blood Cells 17:407-416, 1991; Miller et al., Biotechnology 7:980-990, 1989; Le Gal La Salle et al., Science 259:988-990, 1993; and Johnson, Chest 107:77S-83S-, 1995). Retroviral vectors are particularly well developed and have been used in clinical settings (Rosenberg et al., N. Engl. J. Med 323:370, 1990; Anderson et al., U.S. Pat. No.5,399,346).

Non-viral approaches can also be employed for the introduction of an Nrp-2 or MMP-1 inhibitory nucleic acid molecule therapeutic to a cell of a patient diagnosed as having a neoplasia. For example, a Nrp-2 inhibitory nucleic acid molecule can be introduced into a cell by administering the nucleic acid in the presence of lipofection (Feigner et al., Proc. Natl. Acad. Sci. U.S.A. 84:7413, 1987; Ono et al., Neuroscience Letters 17:259, 1990; Brigham et al., Am. J. Med. Sci. 298:278, 1989; Staubinger et al., Methods in Enzymology 101:512, 1983), asialoorosomucoid-polylysine conjugation (Wu et al., Journal of Biological Chemistry 263:14621, 1988; Wu et al., Journal of Biological Chemistry 264:16985, 1989), or by microinjection under surgical conditions (Wolff et al., Science 247:1465, 1990). Preferably the Nrp-2 or MMP-1 inhibitory nucleic acid molecules are administered in combination with a liposome and protamine.

Gene transfer can also be achieved using non-viral means involving transfection *in vitro.* Such methods include the use of calcium phosphate, DEAE dextran, electroporation, and protoplast fusion. Liposomes can also be potentially beneficial for delivery of DNA into a cell.

Nrp-2 or MMP-1 inhibitory nucleic acid molecule expression for use in polynucleotide therapy methods can be directed from any suitable promoter (e.g., the human cytomegalovirus (CMV), simian virus 40 (SV40), or metallothionein promoters), and regulated by any appropriate mammalian regulatory element. For example, if desired, enhancers known to preferentially direct gene expression in specific cell types can be used to direct the expression of a nucleic acid. The enhancers used can include, without limitation, those that are characterized as tissue- or cell-specific enhancers.

For any particular subject, the specific dosage regimes should be adjusted over time according to the individual need and the professional judgment of the person administering or supervising the administration of the compositions.

### KITS

In one aspect, the invention provides kits for the analysis of melanoma status. The kits include PCR primers for at least one marker selected from those identified in one or more of Tables 1 - 8. In preferred embodiments, the kit includes more than two or three markers selected from those identified in one or more of Tales 1- 8. The kit may further include instructions for use and correlation of the maker with disease status. The kit may also include a DNA array containing the complement of one or more of the Markers selected from one or more of Tables 1 - 8, reagents, and/or enzymes for amplifying or isolating sample DNA. The kits may include reagents for real-time PCR, for example, TaqMan probes and/or primers, and enzymes.

In yet another aspect, the invention provides kits for qualifying melanoma status and/or aiding a diagnosis of human melanoma, wherein the kits can be used to detect the markers of the present invention. For example, the kits can be used to detect any one or more of the markers described herein, which markers are differentially present in samples of melanoma subjects and normal subjects. The kits of the invention have many applications. For example, the kits can be used to differentiate if a subject has melanoma or has a negative diagnosis, thus aiding a human melanoma diagnosis. In another example, the kits can be used to identify compounds that modulate expression of one or more of the markers in in vitro or in vivo animal models for melanoma.

In one embodiment, a kit comprises: (a) a substrate comprising an adsorbent thereon, wherein the adsorbent is suitable for binding a marker, and (b) instructions to detect the marker or markers by contacting a sample with the adsorbent and detecting the marker or markers retained by the adsorbent. In some embodiments, the kit may comprise an eluant (as an alternative or in combination with instructions) or instructions for making an eluant, wherein the combination of the adsorbent and the eluant allows detection of the markers using gas phase ion spectrometry.

Such kits can be prepared from the materials described above, and the previous discussion of these materials (e.g., probe substrates, adsorbents, washing solutions, etc.) is fully applicable to this section and will not be repeated.

In another embodiment, the kit may comprise a first substrate comprising an adsorbent thereon (e.g., a particle functionalized with an adsorbent) and a second substrate onto which the first substrate can be positioned to form a probe, which is removably insertable into a gas phase ion spectrometer. In other embodiments, the kit may comprise a single substrate, which is in the form of a removably insertable probe with adsorbents on the substrate. In yet another embodiment, the kit may further comprise a pre-fractionation spin column (e.g., Cibacron blue agarose column, anti-HSA agarose column, K-30 size exclusion column, Q-anion exchange spin column, single stranded DNA column, lectin column, etc.).

In another embodiment, a kit comprises (a) an antibody that specifically binds to a marker; and (b) a detection reagent. An antibody may be, for example, an Nrp-2 antibody. Such kits can be prepared from the materials described above, and the previous discussion regarding the materials (e.g., antibodies, detection reagents, immobilized supports, etc.) is fully applicable to this section and will not be repeated. Optionally, the kit may further comprise pre-fractionation spin columns. In some embodiments, the kit may further comprise instructions for suitable operation parameters in the form of a label or a separate insert.

Optionally, the kit may further comprise a standard or control information so that the test sample can be compared with the control information standard to determine if the test amount of a marker detected in a sample is a diagnostic amount consistent with a diagnosis of melanoma.

### MELANOMA CANDIDATE GENES AND USE OF BIOMARKERS FOR MELANOMA IN SCREENING ASSAYS

In one aspect the invention also includes melanoma candidate genes, which are useful as therapeutic targets. These genes include, for example, those listed herein, e.g. markers identified in one or more of Tables 1 - 8.

In particular, as described, Nrp-2 and MMP-1 represent useful therpautic targets.

Other candidate genes may be identified by, for example, the methods described herein. Useful methods to identify melanoma candidate genes useful as candidate drug targets include those used to identify the candidate therapeutic targets.

Candidate genes useful as therapeutic agents include those that are up- and down-regulated in melanoma compared to controls or reference samples. Candidate genes may also be useful as therapeutic agents, for example, for gene replacement therapy of down-regulated genes and proteins.

The methods of the present invention have other applications as well. For example, the biomarkers can be used to screen for compounds that modulate the expression of the biomarkers in vitro or in vivo, which compounds in turn may be useful in treating or preventing melanoma in subjects. In another example, the biomarkers can be used to monitor the response to treatments for melanoma. In yet another example, the biomarkers can be used in heredity studies to determine if the subject is at risk for developing melanoma.

Thus, for example, the kits of this invention could include a solid substrate having a hydrophobic function, such as a protein biochip (e.g., a Ciphergen ProteinChip array) and a buffer for washing the substrate, as well as instructions providing a protocol to measure the biomarkers of this invention on the chip and to use these measurements to diagnose melanoma.

Method for identifying a candidate compound for treating melanoma may comprise, for example, contacting one or more of the biomarkers of Tables 1 - 3 with a test compound; and determining whether the test compound interacts with the biomarker, wherein a compound that interacts with the biomarker is identified as a candidate compound for treating melanoma.

Compounds suitable for therapeutic testing may be screened initially by identifying compounds which interact with one or more biomarkers listed in identified herein. By way of example, screening might include recombinantly expressing a biomarker of this invention, purifying the biomarker, and affixing the biomarker to a substrate. Test compounds would then be contacted with the substrate, typically in aqueous conditions, and interactions between the test compound and the biomarker are measured, for example, by measuring elution rates as a function of salt concentration. Certain proteins may recognize and cleave one or more biomarkers of this invention, in which case the proteins may be detected by monitoring the digestion of one or more biomarkers in a standard assay, e.g., by gel electrophoresis of the proteins.

In a related embodiment, the ability of a test compound to inhibit the activity of one or more of the biomarkers of this invention may be measured. One of skill in the art will recognize that the techniques used to measure the activity of a particular biomarker will vary depending on the function and properties of the biomarker. For example, an enzymatic activity of a biomarker may be assayed provided that an appropriate substrate is available and provided that the concentration of the substrate or the appearance of the reaction product is readily measurable. The ability of potentially therapeutic test compounds to inhibit or enhance the activity of a given biomarker may be determined by measuring the rates of catalysis in the presence or absence of the test compounds. The ability of a test compound to interfere with a non-enzymatic (e.g., structural) function or activity of one of the biomarkers of this invention may also be measured. For example, the self-assembly of a multi-protein complex which includes one of the biomarkers of this invention may be monitored by spectroscopy in the presence or absence of a test compound. Alternatively, if the biomarker is a non-enzymatic enhancer of transcription, test compounds which interfere with the ability of the biomarker to enhance transcription may be identified by measuring the levels of biomarker-dependent transcription in vivo or in vitro in the presence and absence of the test compound.

Test compounds capable of modulating the activity of any of the biomarkers of this invention may be administered to subjects who are suffering from or are at risk of developing melanoma. For example, the administration of a test compound which increases the activity of a particular biomarker may decrease the risk of melanoma in a subject if the activity of the particular biomarker in vivo prevents the accumulation of proteins for melanoma. Conversely, the administration of a test compound which decreases the activity of a particular biomarker may decrease the risk of melanoma in a subject if the increased activity of the biomarker is responsible, at least in part, for the onset of melanoma.

At the clinical level, screening a test compound includes obtaining samples from test subjects before and after the subjects have been exposed to a test compound. The levels in the samples of one or more of the biomarkers of this invention may be measured and analyzed to determine whether the levels of the biomarkers change after exposure to a test compound. The samples may be analyzed by mass spectrometry, as described herein, or the samples may be analyzed by any appropriate means known to one of skill in the art. For example, the levels of one or more of the biomarkers of this invention may be measured directly by Western blot using radio- or fluorescently-labeled antibodies which specifically bind to the biomarkers. Alternatively, changes in the levels of mRNA encoding the one or more biomarkers may be measured and correlated with the administration of a given test compound to a subject. In a further embodiment, the changes in the level of expression of one or more of the biomarkers may be measured using in vitro methods and materials. For example, human tissue cultured cells which express, or are capable of expressing, one or more of the biomarkers of this invention may be contacted with test compounds. Subjects who have been treated with test compounds will be routinely examined for any physiological effects which may result from the treatment. In particular, the test compounds will be evaluated for their ability to decrease disease likelihood in a subject. Alternatively, if the test compounds are administered to subjects who have previously been diagnosed with melanoma, test compounds will be screened for their ability to slow or stop the progression of the disease.

Methods of identifying therapeutic targets for melanoma generally comprise comparing an expression profile of a melanoma cell with an expression profile of one a reference cell, wherein the comparison is capable of classifying proteins or transcripts in the profile as being associated with melanoma invasion.

Reference cells may be normal cells (cells that are not melanoma cells) or melanoma cells a different stage from the melanoma cells being compared to. The reference cells may be primary cultured cells, fresh blood cells, established cell lines or other cells determined to be appropriate to one of skill in the art. Transcripts and proteins associated with melanoma invasion include cells that differentiate between the states of melanoma and between normal and melanoma cell lines. The transcripts and proteins may also differentiate between melanoma and other forms of cancer. The proteins may be secreted proteins, such that they are easily detectable from a blood sample.

### CLASSIFICATION ALGORITHMS

A dataset can be analyzed by multiple classification algorithms. Some classification algorithms provide discrete rules for classification; others provide probability estimates of a certain outcome (class). In the latter case, the decision (diagnosis) is made based on the class with the highest probability. For example, consider the three-class problem: healthy, benign, and melanoma. Suppose that a classification algorithm (e.g. Nearest neighbor) is constructed and applied to sample A, and the probability of the sample being healthy is 0, benign is 33%, and melanoma is 67%. Sample A would be diagnosed as being melanoma. This approach, however, does not take into account any "fuzziness" in the diagnosis, e.g., that there was a certain probability that the sample was benign. Therefore, the diagnosis would be the same as for sample B, which has a probability of 0 of being healthy or benign and a probability of 1 of being melanoma.

### EXAMPLES

This invention is further illustrated by the following examples, which should not be construed as limiting. All documents mentioned herein are incorporated herein by reference.

Melanoma is a disease with high metastatic potential even at very early stages of development. The key to improved patient survival is early diagnosis and treatment of melanoma, even in the metastatic setting. There are currently no tests to accurately predict patient outcome for early stage disease, and no blood tests that readily indicate disease recurrence/progression.

Tumor markers can be identified due to overexpression of their mRNA in affected cells and many tumor markers in clinical use today were originally identified in such a manner. It is assumed that melanoma, like other tumors, evolves due to a sequence of genetic and epigenetic events that occur over time. While the genetic events that occur in the evolution of other tumors like colon and breast cancers have been thoroughly investigated, systematic evaluation of melanoma for such genetic or epigenetic changes has lagged behind. This is due, in large part, to the lack of available tissue for investigation of progressive melanocytic lesions and a relative uncertainty regarding the true precursor lesions in melanoma. Recently, a series of melanoma cell lines were prepared from primary human tumors of varying degrees of malignant progression. These lines have shown a remarkable recapitulation of expression patterns seen in primary human tissue and have therefore been used to initially assess gene expression profiles from melanomas at different stages of malignant progression. Genes identified as being differentially expressed in a stage-specific fashion will be further analyzed using clinical outcomes data in order to determine markers that may be of prognostic utility. Markers deemed to be of greatest clinical relevance will be further analyzed in in-vivo assessments. It is expected that these gene profiling studies will allow us to identify novel tumor markers that can be used to more accurately diagnose melanoma, stage melanoma patients, determine clinical outcomes, and develop targeted therapies.

The experiments described herein identify an expanded series of melanoma markers that are likely to predict disease progression, several of which can readily be detected in the circulating serum of patients. Thus, patients with a history of melanoma who are at high risk for disease recurrence may be monitored for disease using a simple, readily-available blood test. Current disease monitoring is through the use of frequent physical examinations in conjunction with various imaging modalities including CT-scanning, MRI scanning, and PET scanning. Such patient monitoring techniques often detect only grossly-detectable disease which is often difficult to treat. Thus, blood and tissue tests will allow for earlier detection of disease recurrence and/or progression and therefore earlier treatment of patients with recurrent and/or progressive disease. Since the genes identified are specifically upregulated in aggressive melanomas which are melanomas that lead to the greatest mortality, it is expected that many of these "aggressive melanoma genes" will function as effective therapeutic targets for invasive melanomas. Accordingly, new tumor markers and therapies can be developed based on their relevance to disease onset and progression. Simple therapies can developed, initially as humanized antibodies to cell surface markers and/or secreted protein, with later development of targeted small molecules as indicated from preclinical and early clinical trials.

Described herein are biomarkers for melanoma status, where the biomarkers can be correlated with a stage or a status of melanoma progression. Thus, the biomarkers provide a way to correlate expression with disease state, and thus provide diagnostic, prognostic and therapeutic potential that was heretofore unrecognized.

### Example 1. Identification of novel melanoma markers using gene expression profiling

### Assessment of gene expression profiles of melanoma cell lines from varying stages of malignant progression

A major problem with evaluating expression profiles from melanocytic lesions and melanomas has been the lack of primary tissue specimens from which intact RNA can be readily extracted. Although recent work using expression profiling has allowed for subclassification of melanomas based on global transcript patterns (Bittner M, et al. (2000) Molecular classification of cutaneous malignant melanoma by gene expression profiling. Nature 406: 536-540), these data were of limited utility since the primary specimens were from advanced stages of disease and mRNAs used for analysis were not obtained from microdissected specimens thus analysis included transcript from non-melanocytic cells which may have skewed the data obtained. Others have evaluated metastatic melanoma cell lines by gene expression profiling (Smith AP, et al. (2004) SAGE identification and fluorescence imaging analysis of genes and transcripts in melanomas and precursor lesions. Cancer Biol Ther 3: 104-109) and have been able to confirn the significance of particular genetic alterations in late-stage melanomas using this method. Since standard analysis of melanomas and other melanocytic lesions involves hematoxylin and eosin (H&E) staining of formalin-fixed, paraffin-embedded tissues, the availability of early primary melanocytic lesions for microarray analysis is limited. It is assumed that genes which are overexpressed in primary melanocytic lesions will analogously be overexpressed in secondary melanoma sources derived from patient tumors. Ten melanoma cell lines were used that were derived from varying stages of malignant progression (Weeraratna AT, et al. (2004) Generation and analysis of melanoma SAGE libraries: SAGE advice on the melanoma transcriptome. Oncogene 23: 2264-2274), and initial studies were performed to assess the expression of genes of interest. Since it was found that expression of the HLH protein Id1 was upregulated in radial growth phase melanomas using in-situ hybridization on primary melanomas (Rumpler G, et al. (2003) Identification of differentially expressed genes in models of melanoma progression by cDNA array analysis: SPARC, MIF and a novel cathepsin protease characterize aggressive phenotypes. Exp Dermatol 12: 761-771), it was of further interest to assess whether this expression pattern would be recapitulated in the cell lines obtained. Idl was found to be expressed in all three radial growth phase cell lines obtained by both transcript and protein analysis and to be undetectable in any of the later stage melanomas or primary human melanocytes. This data suggested that the cell lines could be used to screen for genes of interest that are aberrantly expressed during the process of melanoma development and progression. Since the progression from radial growth phase (RGP) to vertical growth phase (VGP) is critical for the development of metastatic disease, identification of markers to delineate the growth phase of a particular melanocytic lesion can be used in determining prognosis and treatment strategies.

### Gene expression profiling of melanoma cell lines

The expression profiles of 10 melanoma cell lines from various stages of malignant progression (3 radial-growth phase (RGP), 1 early vertical-growth phase (VGP), 3 late VGP, 3 metastatic melanomas) as well as pools of primary human melanocytes derived from neonatal foreskins, using the Affimetrix Human Genome U133 Set, have been evaluated. This set consists of 2 gene chip arrays which contain nearly 45,000 probe sets that represent more than 39,000 transcripts including 33,000 well-substantiated human genes and over 10,000 ESTs. mRNAs were prepared from all cell lines using the TRIzol protocol for RNA isolation. Total RNA was purified using a QIAGEN RNeasy protocol. To ensure intact RNA, samples were evaluated on an Agilent 2100 BioAnalyzer. Once RNA quality was assured, all specimens were analyzed at the Johns Hopkins Medical Institutions (JHMI) Microarray Core Facility. This facility labels and hybridizes all RNAs to the U 133 set described above and raw data is provided for analysis. A sophisticated software package is available for analyzing these gene expression profiles (GeneExpress). Data was evaluated for trend using the GeneExpress analysis program.

### Gene expression criteria for choosing candidates for further study

Genes that are ~3 to 10-fold or more elevated or repressed in melanoma cell lines and primary human melanomas are selected by Affymetrix chip array data relative to primary human melanocytes or serial stages of melanoma progression for further analysis, following confirmation of the gene expression patterns of overexpressed or repressed gene candidates using RT-PCR on cDNAs from the primary melanomas, human melanocytes and melanoma cell lines that are evaluated. Since Affymetrix data provides quantitative information on transcript levels, quantitative Real-time RT-PCR will generally not be needed but can be used if a candidate gene is expressed in the primary human melanocytes at a reduced but not absent level compared to the melanoma cell lines and primary melanomas and vice-versa. In such cases, accurate quantification of transcript level will be used to decide whether to proceed to further studies.

Gene expression profiling studies on 10 melanoma cell lines derived from different stages of malignant progression were evaluated using Affymetrix microarray chips (U133 plus 2.0). Ten melanoma cell lines (3 cell lines with radial growth phase-like (RGP), 4 cell lines with vertical growth phase-like (VGP) phenotypes and 3 metastatic (MM) cell lines) were subjected to gene expression profile analysis. Extensive phenotypic characterizations of these cell lines including AJCC stage/pathology, level/thickness, mitotic rate, growth characteristics in soft agar, and growth factor dependency have been assessed (Weeraratna AT, et al. 2004, as above).

Unsupervised hierarchical clustering of the gene profiles of all cell lines described showed that the most similar global gene expression patterns are observed between VGP6 and MM9 as well as between VGP7 and MM10. This was described in US Application no. 11/794,832, filed July 6, 2007, incorporated by reference in its entirety herein and is shown in the Table in Figure 2. Since those samples were serially derived from the same patients (Pt A= VGP6/MM9, Pt B=VGP7/MM10), a possibility is that individual variation in gene profiling is a more decisive factor than disease stage variation in determining global gene expression patterns (Figure 1). RGP melanomas are also abserved to have distinctive gene expression profiles relative to VGP melanomas and MMs. This suggests that VGP and MM cell lines are more alike with regarding to their global gene expression patterns than their non-invasive predecessor lesions. The similarity of global gene expression pattern between VGP and MM samples relative to RGP also can be speculated from the paired analysis group matched samples. Approximately 60% of genes differentially expressed in VGP compared to RGP are also differentially expressed in MM (1,092 out of 1,850 genes and ESTs from VGP also differentially expressed in MM). This fact may indicate that melanoma progression from RGP to VGP requires more extensive genetic alterations than VGP to MM progression. In addition, the gene expression profile similarity between VGP and MM suggests that VGP melanomas may be fully equipped to become metastatic at the onset but require appropriate environmental cues for metastasis to occur.

Additional analyses of the gene expression profiles obtained have been carried out, which allow for the correlation of the biomarkers identified with a stage of melanoma progression. Such correlation with stage of melanoma progression was heretofore not described, and provides novel therapeutic and diagnostic and prognostic methods and compositions.

Significance Analysis of Microarrays (SAM) was performed that would allow for additional clustering of genes if interest. This data is provided in Figure 3 for early, less aggressive melanoma cell lines versus metastatic melanoma cell lines (which cluster with primary human melanocytes).

Unsupervised hierarchical clustering of the data analyzed above provides the basis for undertaking this SAM analysis. In Figure 4, the clustering tree is included along with an example of a gene found to be down-regulated in aggressive melanoma (ATF3). Note primary human melanocytes (HPM 1 and HPM 2) cluster along with aggressive melanomas.

Genes associated with aggressive melanomas as indicated by the clustering data above are included in the Table shown in Figure 5. It might be expected that genes identified in this screen may be useful diagnostic/prognostic markers and ultimately useful therapeutic targets for advanced melanoma. A list of genes associated with less aggressive melanomas is shown in the Table in Figure 6.

Melanoma cell line gene expression profiles were further evaluated to define genes specific to primary human melanocytes versus aggressive melanomas which clustered together within the greater hierarchical tree. SAM analysis for this clustering of gene expression profiles is indicated below (Figure 7). Note that there are only genes found to be downregulated in metastatic melanoma cell lines versus primary human melanocytes with no genes found to be elevated in expression in tumor cell lines. This is a result which will be of diagnostic and therapeutic utility as the functional significance of these genes is evaluated. Figure 9 is a Table that shows a list of pro-invasive genes associated with melanoma invasion and metastasis.

Given the above gene clustering and SAM data, a final analysis of all gene expression profiling data was undertaken to further define invasion-specific genes. The four-step data reduction algorithm shown in Figure 1 has allowed the definition of a final wholly-inclusive set of melanoma invasion-associated genes shown in the Table in Figure 8. It is expected that the genes that are included in the Table shown in Figure 8 will likely be prognostic melanoma markers as well as potential therapeutic targets.

### Example 2. (Molecular Markers of Melanoma Progression

Previous studies of primary human melanomas have identified gene signatures associated with tumor progression (Haqq C, et al. (2005); The gene expression signatures of melanoma progression. Proc Natl Acad Sci U S A 102: 6092-6097; Winnepenninckx V, et al. (2006) Gene expression profiling of primary cutaneous melanoma and clinical outcome.J Natl Cancer Inst 98: 472-482; Jaeger J, et al. (2007) Gene expression signatures for tumor progression, tumor subtype, and tumor thickness in laser-microdissected melanoma tissues. Clin Cancer Res 13: 806-815). These signatures included upregulation of cell cycle regulatory proteins, mitotic checkpoint genes, genes involved in DNA replication and repair, and cellular stress response genes in addition to loss of genes promoting apoptosis; however, few of the genes identified in these studies were concordant suggesting limitations due to tumor variability. Since better knowledge of gene expression signatures associated with melanoma progression may identify improved screening tools and therapeutic strategies, the experiments described herein make use of high density cDNA microarrays for gene expression profiling of genetically well-defined melanoma cell lines isolated from distinctive stages of tumor progression. Novel data reduction algorithms were used to identify gene signatures associated with tumor invasion and metastasis.

Described herein are unique sets of gene expression signatures that are associated with melanoma progression. These sets of gene expression signatures can be correlated with stages or steps of melanoma progression, and provide novel prognostic, diagnostic or therapeutic opportunities. Many of these pathways have previously been implicated in melanoma progression; however, the specific signature genes identified in the stages of disease progression are novel. These particular progression-associated genes may reflect the underlying molecular mechanisms of the various phases in the known tumor progression pathways of melanoma. As such, the pathways and molecules identified in this study have the potential to be utilized as therapeutic targets for melanoma as well as novel molecular markers for melanoma progression. Moreover, these studies support the use of renewable sources of tumor cells, such as informative tumor cell lines, for the early identification of genes associated with malignant progression which can be subsequently validated using more precious primary tissue specimens.

### Evaluation of gene expression profiles from melanoma cells lines of varying stages of progression identifies a signature for aggressive melanomas

In order to define gene expression patterns during the course of melanoma development and progression, a series of primary and metastatic melanoma cells derived from lesions of discrete phases of melanoma progression as well as pools of primary human melanocytes were evaluated. Tumor cell lines derived from three radial growth phase (RGP) melanomas (WM35, SBC12, and WM1552C), four vertical growth phase (VGP) melanomas (WM902B, WM278, WM983A, and WM793), and three metastatic melanomas (WM852, WM983B, 1205Lu) were evaluated. These cell lines possess a notable ability to recapitulate the clinical stages of disease from which they were derived (Hsu M-Y ED, Herlyn M (1999) The Wistar (WM) melanoma cell lines; Palsson, Masters, eds. London: Kluwer Acad. Publ. pp 259-274; Satyamoorthy K, et al. (1997) Melanoma cell lines from different stages of progression and their biological and molecular analyses. Melanoma Res 7 Suppl 2: S35-42), and have been characterized with respect to tumorigenicity and metastasis (Meier F, et al. (2000) Human melanoma progression in skin reconstructs : biological significance of bFGF. AmJ Pathol 156: 193-200; Mancianti ML , Herlyn M (1989) Tumor progression in melanoma: the biology of epidermal melanocyte in vitro; ContiCJea, ed. New York: Raven Press. pp 369; Herlyn D, et al. (1991) Experimental model of human melanoma metastasis. Cancer Treat Res 54: 105-118; Valyi-Nagy I, et al. (1991) The human melanocyte system as a model for studies on tumor progression. Basic Life Sci 57: 315-326) cellular growth characteristics including life span, growth factor dependency, anchorage-independent growth (Hsu, 1999, as above); and pigmentation and morphology (Herlyn M, et al. (1985) Characteristics of cultured human melanocytes isolated from different stages of tumor progression. Cancer Res 45: 5670-5676). In addition, cytogenetic analyses in these cell lines, including non-random abnormalities such as deletions, translocations, and amplifications have been well-documented and suggest high relevance to the primary tumor of origin (reviewed in (Hsu (1999) as above).

Global gene expression patterns were obtained using Affymetrix gene chips and comparison of gene expression profiles was performed using hierarchical clustering analysis. This clustering analysis identified two distinct groups of melanoma cell lines based on the similarity of their expression patterns, separating radial growth phase (RGP) and metastatic melanomas (MM) (Figure 10A-D); however, vertical growth phase (VGP) melanomas failed to form a distinctive cluster (Figure 10A). The first group, characterized as "less-aggressive" primary melanomas (designated as Group1), included all three RGP melanomas (WM35, Sbcl2, and WM1552C) and two VGP melanomas (WM902B and WM278). The second group, characterized as "more-aggressive" melanomas (designated as Group2), included all three metastatic melanomas (WM853, WM983B, and 1205Lu) and two VGP melanomas (WM983B and WM793). Additional cluster analyses with different linkage matrices produced similar results (data not shown). Of note, only 2 of the 10 cell lines (Sbcl2 and WM853) were found to be wildtype for BRAF kinase, and this genotype failed to demonstrate a notable cluster in the hierarchical analyses.

Next, in order to identify a cohort of genes differentially expressed between the defined groups of melanomas, the gene expression array dataset was subjected to the microarray data analysis program Significance Analysis of Microarray (SAM) (Tusher VG, Tibshirani R, Chu G (2001) Significance analysis of microarrays applied to the ionizing radiation response. Proc Natl Acad Sci U S A 98: 5116-5121). This analysis resulted in 142 differentially expressed probesets with a 3.5 % false discovery rate (Figure 10B and 10C). In total, 89 probe sets representing 65 well-defined genes were found to be upregulated in more-aggressive (Group 2) versus less-aggressive (Group 1) melanomas, and 53 probe sets representing 37 well-defined genes were found to be downregulated (Figure 5). When more stringent criteria were applied (well-characterized genes which are differentially expressed greater than 4-fold) to this signature, 21 upregulated and 5 downregulated genes in the more-aggressive melanoma cells were identified (Figure 10C).

Of note, the set of genes highly expressed in more-aggressive melanomas includes many novel genes with reported functional roles in cell cycle regulation and proliferation such as ZWINT, CDCA2, NCAPH, NCAPG, NCAPG2, PBK, NUSAP1, BIRC5, ESCO2, HELLS, MELK, and CDKN2C (Obuse C, et al. (2004) A conserved Mis 12 centromere complex is linked to heterochromatic HP1 and outer kinetochore protein Zwint-1. Nat Cell Biol 6: 1135-1141; Trinkle-Mulcahy L, et al. (2006) Repo-Man recruits PP1 gamma to chromatin and is essential for cell viability. J Cell Biol 172: 679-692; Hirano T, et al. (1997) Condensins, chromosome condensation protein complexes containing XCAP-C, XCAP-E and a Xenopus homolog of the Drosophila Barren protein. Cell 89: 511-521; Kimura K, et al. (2001) Chromosome condensation by a human condensin complex in Xenopus egg extracts.J Biol Chem 276: 5417-5420; Ono T, et al. (2003) Differential contributions of condensin I and condensin II to mitotic chromosome architecture in vertebrate cells. Cell 115: 109-121; Simons-Evelyn M, et al. (2001) PBK/TOPK is a novel mitotic kinase which is upregulated in Burkitt's lymphoma and other highly proliferative malignant cells. Blood Cells Mol Dis 27: 825-829; Raemaekers T, et al. (2003) NuSAP, a novel microtubule-associated protein involved in mitotic spindle organization.J Cell Biol 162: 1017-1029; Li F, et al. (1998) Control of apoptosis and mitotic spindle checkpoint by survivin. Nature 396: 580-584; Vega H, et al. (2005) Roberts syndrome is caused by mutations in ESCO2, a human homolog of yeast ECO1 that is essential for the establishment of sister chromatid cohesion. Nat Genet 37: 468-470; Sun LQ, et al. (2004) Growth retardation and premature aging phenotypes in mice with disruption of the SNF2-like gene, PASG. Genes Dev 18: 1035-1046; Beullens M, et al. (2005) Substrate specificity and activity regulation of protein kinase MELK. J Biol Chem 280: 40003-40011), as well as genes that are involved DNA replication and repair processes including GINS1, GINS4, RAD54L, TYMS, and DHFR (Ueno M, et al. (2005) PSF1 is essential for early embryogenesis in mice. Mol Cell Biol 25: 10528-10532; Kong L, et al. (2006) Identification and characterization of mouse PSF1-binding protein, SLD5. Biochem Biophys Res Commun 339: 1204-1207; Banerjee D, et al. (2002) Novel aspects of resistance to drugs targeted to dihydrofolate reductase and thymidylate synthase. Biochim Biophys Acta 1587: 164-173). This is shown in Table 8, below. Differential expression of these genes was validated by quantitative real-time RT-PCR (Figure 10D).

**Table 8**

| Probe Set ID | Gene Title | Gene Symbol | Fold^{b} | Function |
|---|---|---|---|---|
| 227350_at | Hellcase, lymphold-specific | HELLS^{c} | 8.5 | cell proliferation |
| 202589_at | thymidylate synthetase | TYMS | 7.2 | DNA replication, DNA repair |
| 204558_at | RAD54-like (S. cerevisiae) | RAD54L | 6.2 | DNA repair, response to DNA damage |
| 204825_at | maternal embryonic leucine phosphorylation zipper kinase | MELK | 5.9 | Mitosis, protein |
| 204159_at | cyclin-dependent kinase inhibitor 2C (p18) | CDKN2C | 5.8 | cell cycle |
| 212949_at | barren homolog 1 (Drosophila) | NCAPH | 5.8 | cell cycle, chromosome condensation |
| 219588_s_at | leucine zipper protein 5 | NCAPG2 | 4.6 | cell cycle, chromosome condensation |
| 218663_at | chromosome condensation protein G | HCAPG1 | 4.5 | cell cycle, chromosome condensation |
| 211767_at | GINS complex subunit 4 (Sid5 homolog) | GINS4 | 5.6 | DNA replication, cell proliferation |
| 206102_at | GINS complex subunit 1 (Psf1 proliferation homolog) | GINS1 | 4.7 | DNA replication, cell |
| 202095_s_at | baculoviral IAP repeat- containing 5 | BIRC5 | 5.1 | G2/M transition cell cycle |
| 219148_at | PDZ binding kinase | PBK | 5.1 | mitosis, protein phosphorylation |
| 235178_x_at | establishment of cohesion 1 homolog 2 | ESCO2 | 5.1 | cell cycle |
| 226661_at | cell division cycle associated 2 | CDCA2^{c} | 4.9 | cell cycle |
| 204026_s_at | ZW10 interactor | ZWINT | 4.7 | cell cycle, spindle organization |
| 202534_x_at | dihydrofolate reductase | DHFR | 4.5 | DNA replication, nucleotide metab olism |
| 218039_at | nucleolar and spindie associated protein 1 | NUSAP1 | 4.1 | cell cycle, chromosome *condensation* |
| 1555788_a_at | tribbles homolog 3 (Drosophila)TRIB3^{c} | | ²6.7 | anti-proliferation, apoptosis |

| | | | | |
|---|---|---|---|---|
| ^{a}Genes with greater than four-fold differential expression are shown. ^{b}Fold represents average expression ratio of group 2 over Group1. ^{c}Genes with multiple probesets are shown with data from a single representative probeset. dol:10.1371/journal.pone.0000594.t001 | | | | |

### Identification of a signature gene expression in aggressive melanomas

Since an aim of these studies was defining melanoma progression signatures, and all melanomas are initiated in primary human melanocytes, the expression profiling data was evaluated in the context of cultured neonatal primary human melanocytes (Figure 11A-D). Surprisingly, when two pools of short-term cultured primary human melanocytes (HPM1 and HPM2) were included in the previously employed hierarchical clustering protocol, the global gene expression pattern of the normal melanocytes was found to be more similar to that of the more-aggressive melanomas (Group 2) than the less-aggressive melanomas (Group 1) (Figure 11A). Since early cultures of primary human melanocytes derived from neonatal foreskins divide rapidly yet possess a normal differentiation program, it was reasoned that the similarities of these cells to more aggressive tumors was likely due to their proliferative potential. In order to test this hypothesis gene expression profiles of more-aggressive melanoma cells (Group 2) were compared to those of short-term cultured primary human melanocytes. Expression profiles were subjected to SAM analysis which identified a cohort of differentially expressed genes with a 0.85% false discovery rate. Remarkably, all differentially expressed genes were found to be down-regulated genes in aggressive melanoma cells versus primary human melanocytes, suggesting that loss of specific gene signatures may be a key event in the development of advanced melanomas (Figure 11B). Further assessment of all melanoma expression profiles using Tree View revealed that the majority of these melanoma-associated genes are also down-regulated in the less-aggressive primary melanomas (Group 1) (Figure 11C). This gene signature is comprised of critical mediators of cellular adhesion and melanocyte development and differentiation and includes:, CDH1, c-KIT, FAX3, CITED-1/MSG1, TYR, MELANA, MC1R, and OCA2 (Nishimura EK, et al. (1999) Regulation of E-and P-cadherin expression correlated with melanocyte migration and diversification. Dev Biol 215: 155-166; Pla P, et al. (2001) Cadherins in neural crest cell development and transformation.J Cell Physiol 189: 121-132; Mackenzie MA, et al. (1997) Activation of the receptor tyrosine kinase Kit is required for the proliferation of melanoblasts in the mouse embryo. Dev Biol 192: 99-107; Hornyak TJ, et al. (2001) Transcription factors in melanocyte development: distinct roles for Pax-3 and Mitf. Mech Dev 101: 47-59; Vachtenheim J, Novotna H (1999) Expression of genes for microphthalmia isoforms, Pax3 and MSG1, in human melanomas. Cell Mol Biol (Noisy-le-grand) 45: 1075-1082; Spritz RA (1994) Molecular genetics of oculocutaneous albinism. Hum Mol Genet 3 Spec No: 1469-1475; Du J, et al. (2003) MLANA/MART1 and SILV/PMEL17/GP100 are transcriptionally regulated by MITF in melanocytes and melanoma. AmJ Pathol 163: 333-343; Suzuki I, et al. (1996) Binding of melanotropic hormones to the melanocortin receptor MC IR on human melanocytes stimulates proliferation and melanogenesis. Endocrinology 137: 1627-1633. The results are shown in the Table shown in Figure 8. While such a loss of cellular adhesion by E-cadherin and P-cadherin has been documented in melanoma (reviewed in (Bonitsis N, et aL (2006) The role of cadherin/catenin complex in malignant melanoma. Exp Oncol 28: 187-193.), this signature identifies specific defects in the intrinsic melanocyte development program that may contribute to melanoma development. In addition, genes with tumor suppressor and metastasis suppressor functions (DPP4, SYK) are included in this melanoma signature (Wesley UV, et al. (2005) Dipeptidyl peptidase inhibits malignant phenotype of prostate cancer cells by blocking basic fibroblast growth factor signaling pathway. Cancer Res 65: 1325-1334; Coopman PJ, Mueller SC (2006) The Syk tyrosine kinase: A new negative regulator in tumor growth and progression. Cancer Lett 241: 159-173). Significant down-regulation of these genes in the aggressive metastatic melanoma cells was validated using semi-quantitative duplex RT-PCR (Figure 11D). Furthermore, this differentially expressed "melanoma signature" contains many genes whose functional roles in melanoma progression have not been well characterized and may provide novel insights into the early development of melanoma from primary melanocytes.

### Identification of an invasion-specific gene signature for melanoma

A current melanoma progression model suggests the sequential evolution of primary in situ tumors and minimally invasive tumors which are termed "radial growth phase" (RGP), followed by a subsequent conversion to a more aggressive "vertical growth phase" (VGP), in which tumor cells are programmed to cross the epidermal basement membrane and invade vertically into the dermis. It has been postulated that the VGP is the critical stage in which a tumor gains metastatic capacity. Thus, a comparison was made between the gene expression profiles of RGP and VGP melanomas using a uniquely designed data reduction algorithm in order to identify genes that are likely to be relevant to this critical invasive phenotype (Figure 2). This melanoma invasion-specific signature is notably characterized by the inclusion of several genes involved in chemotaxis and the inflammatory response (CXCL1, CXCL2, IL8, and IL6), cell adhesion (HNT, ITGA4, ITGB8, CSPG2, ZP4, and FLRT3), and extracellular matrix organization (MMP1, COL4A1, COL4A2, and COL5A2) (Figure 2). These genes and their relative expression profiles are depicted in Figure 12B. These cellular processes have previously been implicated in tumor progression for a wide variety of malignancies including melanoma and are felt to be essential components of tumor invasion and metastasis. In addition, many of these invasion-specific signature genes are also upregulated in metastatic melanomas (Figure 12B). The differential expressions were validated on the selected genes using semi-quantitative duplex-PCR analysis (Figure 12C).

Since the melanoma invasion-specific signature was associated with common functions of matrix invasion/inflammation/cell migration it was next investigated whether a common upstream regulatory pathway might link these signature genes. The eight most highly up-regulated genes from the melanoma invasion-specific signature were selected for further evaluation and gene promoter sequences were analyzed to identify transcription factor binding cis elements. This promoter analysis yielded a profile of transcription factors with common sequence elements in the signature genes. The most ubiquitous cis elements among the gene promoters evaluated were E12, E47, GCN4, GR, HES-1, IL-6, MEF-2, NF-KB, N-Oct-3, PU. 1, RAR-alpha1, SRF, and the basal gene transcriptional complex components of TFIID, TBP, and TBF1. The NF-KB binding sequence was identified in 7 out of 8 of the most upregulated invasion-specific signature genes (Figure 12D). The NF-KB pathway as a mediator of melanoma invasion was of particular interest since the most highly upregulated invasion-specific genes, CXCL-1 and IL-8, have previously been reported to be activated by NF-KB and had previously been implicated in melanoma progression (reviewed in Ueda Y, Richmond A (2006) NF-kappaB activation in melanoma. Pigment Cell Res 19: 112-124). Given the consequences of NF-KB activation in a cell, NF-KB function is highly regulated by specific cytosolic inhibitory activities which prevent inappropriate NF-KB activation and shuttling to the nucleus. Thus, only nuclear NF-KB is considered to be functionally activated. In order to evaluate NF-KB function in the tumor cell lines, NF-KB cellular-localization was used as a surrogate marker for NF-KB activity. Invasive (VGP) melanomas posses both cytosolic (inactive) and nuclear (active) localization of NF-KB, while non-invasive (RGP) melanomas possess NF-KB confined to the cytosolic compartment suggesting specific activation of NF-KB during melanoma progression (Figure 12E).

Here, a series of well-defined melanoma cell lines from varying stages of malignant progression have been used to assess molecular signatures associated with disease progression. These cell lines have undergone extensive characterization of their tumorigenic potential and invasion capacity (Meier, 2000, as above; Hsu MY, et al. (1998) Adenoviral gene transfer of beta3 integrin subunit induces conversion from radial to vertical growth phase in primary human melanoma. AmJ Pathol 153: 1435-1442), and have been shown to possess a remarkable ability to recapitulate the clinical stages of disease from which they were derived. The resutls show that unsupervised hierarchical clustering of global gene expression profiles of melanoma cell lines allows for the classification of tumor cells into 2 groups (Figure 10A), defined as less-aggressive (Group 1) and more-aggressive (Group 2) melanomas. While all radial growth phase melanomas clustered in Group 1, and all metastatic melanomas clustered in Group 2, vertical growth phase melanomas failed to form a distinctive cluster suggesting that vertical growth phase melanomas may be considered to be a transient or transition phase within the current melanoma progression model (Clark WHJr, et al. (1984) A study of tumor progression: the precursor lesions of superficial spreading and nodular melanoma. Hum Pathol 15: 1147-1165). Aggressive (Group 2) melanomas were characterized by upregulation of genes associated with cell cycle progression, DNA replication and repair, and altered expression of apoptosis-related genes including upregulation of the antiapoptotic gene BIRC5/survivin (Grossman D, Altieri DC (2001) Drug resistance in melanoma: mechanisms, apoptosis, and new potential therapeutic targets. Cancer Metastasis Rev 20: 3-11), and downregulation of the novel stress-associated apoptosis inducer TRIB3 (Ohoka N, et al. (2005) TRB3, a novel ER stress-inducible gene, is induced via ATF4-CHOP pathway and is involved in cell death. EMBOJ 24: 1243-1255), (Figure 13). These signature genes for melanoma progression are remarkably similar to those obtained from recent large-scale studies using primary human melanomas and suggest high correlation with alterations seen in primary tumor specimens (Winnepenminckx V, 2006, as above). Notably, a dominant signature associated with BRAF kinase mutations was not identified, which may be reflective of the relative infrequency of wildtype BRAF in these cell lines. As a whole, this gene signature suggests a series of molecular alterations occur in aggressive melanomas that promote melanoma cell growth, survival and apoptotic resistance which contribute to the unresponsiveness of melanomas to traditional chemotherapeutic agents (Atkins MB (1997) The treatment of metastatic melanoma with chemotherapy and biologics. Curr Opin Oncol 9: 205-213).

While gene signatures associated with aggressive melanomas provide insights into molecular pathways important for tumor progression, further analysis of these tumor cell lines in conjunction with expression profiles from primary human melanocytes using SAM analysis revealed a striking signature characterized exclusively by gene loss in melanomas and primarily by loss of cellular adhesion and melanocyte differentiation-associated genes (Figures 11B, 11C). This melanoma-associated signature may define critical molecular mechanisms involved in melanocyte development and differentiation which distinguish these tumor cells from their primary cell of origin. In fact, the identification of several genes in this signature with established functional roles in melanocyte differentiation and melanin biosynthesis such as CDH3, CDH1, c-KIT, FAX3, CITED-1/MSG1, TYR, MELANA, MC1R, and OCA2, supports this notion. Moreover, this signature has identified two tumor suppressor genes, DPP4 and SYK, whose downregulation has previously been implicated in melanoma development (Houghton AN, et al. (1988) Cell surface antigens of human melanocytes and melanoma. Expression of adenosine deaminase binding protein is extinguished with melanocyte transformation.J Exp Med 167: 197-212; Roesch A, et al. (2006) Loss of dipeptidyl peptidase IV immunostaining discriminates malignant melanomas from deep penetrating nevi. Mod Pathol 19: 1378-1385; Muthusamy V, et al. (2006) Epigenetic silencing of novel tumor suppressors in malignant melanoma. Cancer Res 66: 11187-11193).

Finally, evaluation of an invasion-specific signature for melanoma identified dominant gene activation by the transcription factor, NF-KB. Constitutive activation of NF-KB and an inflammatory response is an emerging hallmark of various tumor types (Karin M (2006) Nuclear factor-kappaB in cancer development and progression. Nature 441: 431-436). In addition, NF-KB has specifically been implicated in the development of invasive aggressive melanomas through autocrine and paracrine mechanisms (reviewed in Ueda et al. 2006). The melanoma invasion signature described herein is associated with upregulation of critical NF-KB effectors including CXCL1, FMN2, MMP1, IL-8, IGFBP3 which have been implicated in the regulation of tumor cell proliferation, motility, migration, and/or invasion (Figure 12D). In addition, the putative NF-KB target gene GAGE7B which was identified in the melanoma invasion-specific signature, has been associated with apoptotic resistance and worse prognosis in other tumors (Cilensek ZM, et al. (2002) A member of the GAGE family of tumor antigens is an anti-apoptotic gene that confers resistance to Fas/CD95/APO-1, Interferon-gamma, taxol and gamma-irradiation. Cancer Biol Ther 1: 380-387). In addition, several of the invasion-specific signature genes are chemokines including CXCL1, CXCL2, and IL-8 which have been implicated in the promotion of tumor-associated angiogenesis, a critical feature of invasive tumors (Rofstad EK, Halsor EF (2000) Vascular endothelial growth factor, interleukin 8, platelet-derived endothelial cell growth factor, and basic fibroblast growth factor promote angiogenesis and metastasis in human melanoma xenografts. Cancer Res 60: 4932-4938).

In summary, the gene expression profiling studies of melanoma cell lines from varying stages of malignant progression and primary human melanocytes have identified several important melanoma signatures including: 1) Aggressive melanomas are characterized by upregulation of genes associated with cell cycle progression, DNA replication and repair and apoptotic resistance as well as loss of genes associated with apoptotic susceptibility, 2) Melanomas notably differ from their cell of origin, primary human melanocytes, due to a loss of cellular adhesion and differentiation-associated genes, and 3) Invasive melanomas are characterized by a signature indicative of global activation of NF-KB and downstream effector genes associated with tumor cell migration, invasion, chemotaxis, and proliferation. Since pathways associated with tumor progression may have clinical utility as prognostic tumor markers and therapeutic targets, it is expected that novel melanoma signature genes identified in this study will be further developed forsuch translational endpoints. Moreover, the important information regarding melanoma biology gleaned from these studies on renewable cell resources cannot be understated. A major roadblock to advances in melanoma therapy has been the relative paucity of informative tissue specimens available for analysis in profiling studies as well as the notoriously heterogeneous nature of this malignancy. The use of surrogate tissue resources including tumor cell lines for the early discovery phases in melanoma, as used in this study, will undoubtedly allow for the conservation of precious tissue specimens for use in more advanced validation studies. It is expected that the novel melanoma progression-associated genes identified in this study will provide new insights into the molecular defects associated with this malignancy and ultimately pave the way for the development of new melanoma biomarkers and novel targeted therapies.

### Methods

The above-described examples were carried out using, but not limited to, the following materials and methods:

### Cells

Ten melanoma cell lines (WM35, SBC12, and WM1552C, WM902B, WM278, WM983A, and WM793, WM852, WM983B, 1205Lu) were obtained from M. Herlyn (The Wistar Institute, Philadelphia, PA). These cell lines were maintained in modified complete melanocyte growth medium (Cell Application Inc., San Diego, CA) which lacked 12-O - tetradecanoyl phorbol-13-acetate and was supplemented with 2 % fetal bovine serum. Normal human primary melanocytes were isolated from neonatal foreskins and grown in complete melanocyte growth medium (Cell Applications, Cat. No., 135-500). The complete melanocyte growth medium consists of the melanocyte basal medium (Cell Applications, Cat. No. 134-500) and growth supplement cocktails containing hydrocortisone (0.5 mg/ml), insulin (5 mg/ml), 12-O-tetradecanoylphorbol-13-acetate (10 ng/ml), bovine pituitary extract (21 mg/ml), bFGF (1 ng/ml), heparin (1 mg/ml), FBS (0.5 %), gentamycin sulfate (50 mg/ml), amphotericin B (5 ng/ml), and NaCl (45 mM).

### Gene Expression Profiling

Total RNA was isolated from exponentially growing melanoma cell lines using RNeasy column purification per manufacturer's protocol (QIAGEN). Two sets of short-term cultured (2 to 3 passage numbers) normal human melanocytes were prepared from neo-natal foreskins. In order to minimize genetic variability melanocytes from 4-5 individuals were pooled for each culture. Total RNA from normal melanocytes was extracted and purified by a combination of phase extraction and chromatography using TRIzol reagent (INVITROGEN Life Technologies Inc.) and RNeasy columns (QIAGEN) in order to remove melanin. In brief, exponentially growing melanocytes were lysed with TRIzol reagent and lysate was incubated at 65°C for 2 minutes to inactivate melanin. Lysate was then subjected to phase extraction and RNeasy column purification. RNA quality checks, double strand complementary DNA synthesis, hybridization with Human Genome U133 Plus 2.0 Array Chips (AFFYMETRIX Inc. Santa Clara, CA), and initial data extraction were performed at The Gene Array Core Facility in the Malaria Research Institute (JHMRI) at The Johns Hopkins Bloomberg School of Public Health (on the world wide web at malaria.jhsph.edu/jhmri/resources_education/gene_array_core).

### Data Extraction and Statistical Analysis

SAM [19], Gene Cluster 3.0 [58] and TreeView (on the world wide web at bonsai.ims. u-tokyo.ac.jp/,mdehoon/software/cluster/index.html), Access, and Excel (MICROSOFT, Seattle, WA) programs were used. For all of the statistical analysis beyond the initial description of datasets, microarray data were normalized (Dataset S1) and a subset of the 12 microarray data (10 from melanoma cell lines and 2 from normal human melanocytes) was obtained by filtering to require each gene probe to have at least one observation in the expression intensity resulting in a 'present' call from all 12 samples. This produced a subset of data containing a total of 32,632 affymetrix gene probes (Dataset S2). This filtered subset of data was used for all of the additional analysis.

### Cluster Analysis

Unsupervised hierarchical clustering analysis was performed on the subset of data (without log transformation) with Gene Cluster 3.0 (http://bonsai.ims.u¬tokyo.ac.jp/,mdehoon/software/cluster/index.html) by using the correlation (uncentered) similarity metric and centeroid linkage clustering method. The resulting tree-images were visualized using Java TreeView. Statistical Analysis of Microarray(SAM). SAM was performed on the subset of array data without log transformation using SAM software package. Groups are defined based on the hierarchical clustering; for example, group 1 = less-aggressive primary melanomas (RGP melanomas: WM35, Sbcl2, WM1552C and VGP melanomas: WM902B and WM278), and group 2 = aggressive metastatic melanomas (Metastatic melanomas: WM852, WM983B, and 1205Lu; and VGP melanomas: WM983A and WM793). Delta was chosen to limit the output gene list so that minimum predicted false-positives would be included. Three-step Data Reduction Algorithm. In order to identify melanoma invasion-specific gene signature, uniquely designed three-step data reduction algorithm was applied to the subset of expression data. First step is that the proveset should be called as 'present' in three samples out of four VGP melanoma cells and two samples out of three RGP melanoma cells. Second step is that the candidate proveset should be expressed five folds or more in VGP melanoma cell lines than RGP melanoma cell lines. The last step is that the gene probesets were retained only when the expression level is greater than three folds in VGP melanoma cell lines compared to that of primary human melanocytes. The last step is implemented because the candidate gene expression level should be higher if the gene products have certain degree of functional roles in the invasion processes of malignant melanoma. The probesets, those that pass through three-step filtration criteria are subjected to probeset to gene mapping using NetAffx, a web interface program from Affymetrix Inc. Gene annotation for the gene nane, gene symbol, and GO Biological Procession Analysis also performed by the NetAffx.

### Quantitative Real-time PCR

cDNA was generated by using the SuperScriptTM First-Strand Synthesis System for RT-PCR according to manufacturers instructions (INVITROGEN, Carlsbad, CA). Quantitative real-time PCR was performed with an Applied Biosystems Prism 7900 HT Sequence Detection System using SYBR Green PCR Master Mix (APPLIED BIOSYSTEMS, Foster City, CA). The thermal cycling conditions for quantitative real-time RT-PCR analysis to validate gene expression changes were as follows: hold for 10 minutes at 95°C, followed by three-step PCR for 40 cycles of 95°C for 15 seconds, 55°C to 60°C for 25 seconds, and 72°C for 30 seconds. Optimal annealing temperatures were predetermined to ensure single amplified product. All samples were performed in triplicate. Amplification data were analyzed with an Applied Biosystems Prism Sequencer Detection Software Version 2.3 (APPLIED BIOSYSTEMS, Forster City, CA). Human GAPDH gene was used as endogenous control. To normalize the relative expression of the genes of interest to the GAPDH control, standard curves were prepared for each gene and GAPDH in each experiment.

### Semi-quantitative Duplex PCR

Semi-quantitative duplex RT-PCR was performed by an MJ Research Programmable Thermal Controller (PTC-100, Inc., Watertown, MA) and the amplified products were separated on an agarose gel. The duplex PCR utilized 20 bp oligonucleotides to amplify regions of 300-400 bp from the genes of interest. Intitial optimization experiments were conducted to establish the most favorable primer concentrations between the genes of interest and internal control GAPDH, yielding 0.8 mM and 0.04 mM, re-spectively. The PCR was carried out in a total volume of 25 mL, containing 2.5 mL of 10X PCR Buffer (containing 15 mM MgCl2), 0.2 mM dNTPs, and 0.3 ul AmpliTaq Gold DNA Polymerase (Applied Biosystems, Foster City, CA). Thirty to thirty-five amplification cycles were performed by an MJ Research Programmable Thermal Controller (PTC-100, Inc., Watertown, MA), using a denaturing temperature of 95°C for 25 seconds, an annealing temperature varying between 55°C-60°C (depending on gene) for 30 seconds, and primer extension at 72°C for 30 seconds. Each amplification experiment also included two negative PCR controls, a no-RNA control from reverse transcrip¬tion procedures and a no-cDNA water control. Following amplification, 25 mL of the samples were separated via electro¬phoresis on a 3% agarose gel. The primer sequences were designed by using Primer3, primer analysis software (on the world wide web at frodo. wi.mit.edu/cgi-bin/primer3/primer3_www.cgi), yielded only one amplified product and had the following sequences:

| | |
|---|---|
| MELK | Forward: 5'-TGGCTCTCTCCCAGTAGCAT-3' |
| | Reverse: 5'-TAGCACTGGCTTGTCCACAG-3' |
| GINS4 | Forward: 5'-CAGAGAGTTCATGGCGAACA-3' |
| | Reverse: 5'-CTCCCAAAGTGCTGGGATTA-3' |
| NCAPG | Forward: 5'-TATTGGTGTGCCCTTTGTGA-3' |
| | Reverse: 5'-CAGGGATATTGGGATTGTGG-3' |
| CDH3 | Forward: 5'-ACGACACCCTCTTGGTGTTC-3' |
| | Reverse: 5'-GTCAAACTGCCCACATTCCT-3' |
| KIT | Forward: 5'-TGACTTACGACAGGCTCGTG-3' |
| | Reverse: 5'-AAGGAGTGAACAGGGTGTGG-3' |
| DPP4 | Forward: 5'-CAAATTGAAGCAGCCAGACA-3' |
| | Reverse: 5'-CAGGGCTTTGGAGATCTGAG-3' |
| SYR | Forward: 5'-GAAGCCATATCGAGGGATGA-3' |
| | Reverse: 5'-TGACAAGTTGTGGGCATGTT-3' |
| CXCL1 | Forward: 5'-TGTTTGAGCATCGCTTAGGA-3' |
| | Reverse: 5'-GATCTCATTGGCCATTTGCT-3' |
| CXCL2 | Forward: 5'-TTGCGCCTAATGTGTTTGAG-3' |
| | Reverse: 5'-ATACATTTCCCTGCCGTCAC-3' |
| IL8 | Forward: 5'-AGGGTTGCCAGATGCAATAC-3' |
| | Reverse: 5'-AGCAGACTAGGGTTGCCAGA-3' |
| IGFBP3 | Forward: 5'-GCTACAGCATGCAGAGCAAG-3' |
| | Reverse: 5'-AACATGTGGTGAGCATTCCA-3' |
| GAPDH | Forward: 5'-GATCATCAGCAATGCCTCCT-3' |
| | Reverse: 5'-TTCAGCTCAGGGATGACCTT-3' |

### Immunofluorescence Labeling

RGP and VGP Cells were plated on glass slides and cultured in melanocyte growth media overnight without any stimulation. Cells were fixed at room temperature for 15 minutes using 3.5% paraformaldehyde solution. Cells were washed briefly with PBS and then permeabiized with either 0.5% Triton X-100 for 10 minutes or 20°C cooled methanol for 15 minutes. Slides were blocked with 16% normal goat serum (SANTA CRUZ BIOTECH, Santa Cruz, CA) for 1 hour and then incubated with rabbit polyclonal IgG p65 antibody (SANTA CRUZ BIOTECH, Santa Cruz, CA) at 1:100 dilution. Subsequent to overnight incubation at 4°C, the slides were washed with PBS and incubated with goat anti-rabbit IgG-Alexa 594 (MOLECULAR PROBES Eugene, OR) at 1:200 dilution at room temperature for 1 hour. Stained slides were washed with PBS and viewed under a fluorescence microscope (Eclipse TS100, NIKON, Tokyo, Japan).

### Gene Transcription Promoter Analysis

Transcription factor binding cis element sequence profiling in the selected gene promoter was performed by using a web tool known as TESS (Transcription Element Search System, available on the world wide web at cbil. upenn.edu/tess). Each genes promoter sequences from the transcription start site up to 2.0 kb of upstream of the genes were subjected to the TESS and screened by TRANSFAC database to identify matched consensus sequences of known DNA binding transcription factors.

### Example 3. Assessment of Tumor Enodthelial Cell Communication: Identification of Biomarkers and Therapeutic Targets.

Tumor metastasis is the cause of >90% of solid tumor deaths (Gupta and Massague, (2006). Cancer metastasis: building a framework. Cell 127, 679-695). Metastatic disease is particularly onerous in patients with melanoma where the average life expectancy of patients with advanced disease is 6-9 months (Balch et al., (2001). Final version of the American Joint Committee on Cancer staging system for cutaneous melanoma. J Clin Oncol 19, 3635-3648). In order for metastasis to occur, cells must dissociate from the primary tumor, move through the surrounding tissue, locate and intravasate into a blood vessel, adhere to and extravasate from the vessel, adapt to a new microenvironment and undergo mitosis to form a new tumor at the secondary site ((2003). The pathogenesis of cancer metastasis: the 'seed and soil' hypothesis revisited. Nat Rev Cancer 3, 453-458). While there is a clear set of discrete steps that must take place in order for metastasis to occur, the precise molecular events required for each step for any given malignancy remain uncertain (reviewed in (Gupta and Massague, 2006; Nguyen and Massague, (2007). Genetic determinants of cancer metastasis. Nature reviews 8, 341-352). Given the complexity of the metastatic process, the majority of studies to date have focused on either a particular metastasis-associated cellular event in intimate detail or the more global process of metastasis in a highly observational system. Communication networks between the tumor and its surrounding microenvironment have garnered much attention with mounting evidence supporting a critical role in metastasis (reviewed in (Chambers et al., (2002). Dissemination and growth of cancer cells in metastatic sites. Nat Rev Cancer 2, 563-572; Kalluri and Zeisberg, (2006). Fibroblasts in cancer. Nat Rev Cancer 6, 392-401; Liotta and Kohn, (2001). The microenvironment of the tumour-host interface. Nature 411, 375-379). Over the past decade in vivo and in vitro model systems have been developed to better assess these particular communication networks (Anderson et al., (2006). Tumor morphology and phenotypic evolution driven by selective pressure from the microenvironment. Cell 127, 905-915.; Bockhorn et al., (2007). Active versus passive mechanisms in metastasis: do cancer cells crawl into vessels, or are they pushed? Lancet Oncol 8, 444-448; Shioda et al., (1997). Early events of metastasis in the microcirculation involve changes in gene expression of cancer cells. Tracking mRNA levels of metastasizing cancer cells in the chick embryo chorioallantoic membrane. Am J Pathol 150, 2099-2112).

In order for a primary tumor to metastasize, critical communication must take place between the tumor cell and its surrounding vasculature. Previous attempts to evaluate this communication network have assessed indirect signaling between tumor and endothelial cells through soluble factors (Beierle et al., (2004). Expression of VEGF receptors in cocultured neuroblastoma cells. The Journal of surgical research 119, 56-65; Liang et al., (2006). Screening and identification of vascular-endothelial-cell-specific binding peptide in gastric cancer. Journal of molecular medicine (Berlin, Germany) 84, 764-773; Ye and Yuan, (2007). Inhibition of p38 MAPK reduces tumor conditioned medium-induced angiogenesis in co-cultured human umbilical vein endothelial cells and fibroblasts. Bioscience, biotechnology, and biochemistry 71, 1162-1169), used a direct two-cell co-culture system to evaluate specific genes of interest (Ghosh et al., (2007). Use of multicellular tumor spheroids to dissect endothelial cell-tumor cell interactions: a role for T-cadherin in tumor angiogenesis. FEBS Lett 581, 4523-4528; Hatfield et aL, (2006). Microvascular endothelial cells increase proliferation and inhibit apoptosis of native human acute myelogenous leukemia blasts. International journal of cancer Journal international du cancer 119, 2313-2321; Hu et al., (2007). Calcium-activated potassium channels mediated blood-brain tumor barrier opening in a rat metastatic brain tumor model. Molecular cancer 6,22; Kargozaran et al., (2007). A role for endothelial-derived matrix metalloproteinase-2 in breast cancer cell transmigration across the endothelial-basement membrane barrier. Clinical & experimental metastasis 24, 495-502; Naidoo and Raidoo, (2006). Tissue kallikrein and kinin receptor expression in an angiogenic co-culture neuroblastoma model. Metabolic brain disease 21, 253-265), limited assessments to endothelial cells (Khodarev et al., (2003). Tumour-endothelium interactions in co-culture: coordinated changes of gene expression profiles and phenotypic properties of endothelial cells. Journal of cell science 116, 1013-1022; Penalva et al., (2004). RNA-binding proteins to assess gene expression states of co-cultivated cells in response to tumor cells. Molecular cancer 3, 24), or evaluated effects of a particular drug on the system (Depasquale and Wheatley, (2006). Action of Lovastatin (Mevinolin) on an in vitro model of angiogenesis and its co-culture with malignant melanoma cell lines. Cancer cell international 6, 9; Neuzil et al., (2007). alpha-Tocopheryl succinate inhibits angiogenesis by disrupting paracrine FGF2 signalling. FEBS Lett 581, 4611-4615). Melanomas are highly aggressive tumors with a known propensity for early metastasis. Given the complex nature of metastasis, a two-dimensional co-culture system comprised of metastatic melanoma cells and human umbilical vein endothelial cells (HUVECs) has been chosen in the experiments described herein to reduce the process to critical interactions between a tumor and its associated vasculature. Using gene expression profiling, these studies allowed for the definition of a global melanoma-endothelial cell communication network which is likely to mediate melanoma metastasis. Among the top genes upregulated in melanoma cells during crosstalk with endothelial cells is the cell surface receptor, neuropilin-2 (Nrp2).

Neuropilins are transmembrane glycoproteins that modulate the development of the nervous and vascular systems ((Bielenberg et al., 2006; Favier et al., (2006). Neuropilin-2 interacts with VEGFR-2 and VEGFR-3 and promotes human endothelial cell survival and migration. Blood 108, 1243-1250; Staton et al., (2007). Neuropilins in physiological and pathological angiogenesis. The Journal of pathology 212, 237-248). They function as co-receptors for the vascular endothelial growth factor receptors and the plexins and bind two known ligands with distinct functions: class 3 semaphorins, which are involved in axonal guidance; and vascular endothelial growth factor (VEGF) family members involved in promoting angiogenesis. Neuropilin-2 is expressed by venous and lymphatic endothelial cells and can bind the lymphangiogenesis-associated ligand, VEGF-C. Blocking Nrp2 function has recently been shown to inhibit tumor metastasis through effects on lymphendothelial cell migration and tumor-associated lymphangiogenesis (Caunt et al., (2008). Blocking neuropilin-2 function inhibits tumor cell metastasis. Cancer Cell 13, 331-342). Neuropilins are expressed in a variety of cancers (Bielenberg and Klagsbrun, (2007). Targeting endothelial and tumor cells with semaphorins. Cancer Metastasis Rev 26, 421-431; Bielenberg et al., (2004). Semaphorin 3F, a chemorepulsant for endothelial cells, induces a poorly vascularized, encapsulated, nonmetastatic tumor phenotype. J Clin Invest 114, 1260-1271; Chen et al., (2005). Roles of neuropilins in neuronal development, angiogenesis, and cancers. World journal of surgery 29, 271-275; Ellis, (2006). The role of neuropilins in cancer. Mol Cancer Ther 5, 1099-1107; Guttmann-Raviv et al., (2006). The neuropilins and their role in tumorigenesis and tumor progression. Cancer Lett 231, 1-11; Klagsbrun et al., (2002). The role of neuropilin in vascular and tumor biology. Advances in experimental medicine and biology 515, 33-48). While Nrp1 is generally expressed strongly in epithelial tumors, Nrp2 is more highly expressed in tumor cells of neural origin including glioblastomas, melanomas, and neuroblastomas, in addition to osteosarcomas, bladder, pancreatic, and lung tumors. In melanoma, exogenous expression of the NRP2 ligand Semaphorin 3F (Sema 3F) in tumor xenografts has been shown to inhibit tumor cell migration and metastasis to lymph nodes and lung without significant effects on tumor cell growth, leading to poorly vascularized tumors (Bielenberg et al., 2004). In contrast, others have demonstrated inhibition of melanoma cell growth by Sema 3F (Chabbert-de Ponnat et al., (2006). Antiproliferative effect of semaphorin 3F on human melanoma cell lines. J Invest Dermatol 126, 2343-2345).

In the experiments described herein, a screen for genes involved in melanoma-endothelial cell communication was undertaken to assess molecular pathways involved in melanoma metastasis. Neuropilin-2 (Nrp2) was identified as a gene involved in melanoma-endothelial cell communication, and was found to be highly upregulated during this process. The data presented herein show novel quantifiable assessments of melanoma-endothelial cell crosstalk demonstrate a vital role for Nrp2 in this process. Further evaluations of Nrp2 function in melanoma suggest that Nrp2 is a critical mediator of melanoma growth and specific neutralizing antibodies to NRP2 completely and irreversibly inhibit melanoma growth. Thus, Nrp2 may be an important target for melanoma growth and metastasis, and may be a useful therapeutic target for patients with advanced melanoma.

### Melanoma-Endothelial Cell Crosstalk in vitro

In order to define the molecular determinants of melanoma metastasis an in vitro two-dimensional co-culture system of melanoma cells and endothelial cells was performed, and the process was limited to critical interactions between the tumor and its associated vasculature. The initial evaluation of this process involved the simple observation of fluorescently labeled tumor and endothelial cells separated by a physical barrier (Figure 14A). Metastatic melanoma cells, GFP-1205Lu (green) were allowed to migrate toward a central clone of endothelial cells, RFP-HUVECs (red) and observed over time. In the process of evaluating these studies, it was noted that the interaction of endothelial cells with tumor cells at the cell-cell interface allowed for the directed assembly of endothelial cells into network-like structures that would be analogous to vessel-like structures in three dimensions (Figure 14B). This structured assembly of endothelial cells differed from that observed with endothelial cells alone (Figure 14F) or with migration of tumor cells into an endothelial cell pool. Next, the nature of this interaction over time was evaluated through direct co-culture of melanoma and endothelial cells. minimal network formation at 6 hours of co-culture was noted (Figure 14C) but significant network formation by endothelial cells at 24 hours (Figure 14D) and 48 hours (Figure 14E) of co-culture. Since the co-culture system allowed the observation of evidence for melanoma-endothelial cell crosstalk, the determination of whether this crosstalk "readout" required direct cell-cell communication or could be mediated through secreted factors was next explored. It was found that incubation of HUVECs in either basal medium alone or HUVEC-conditioned media for 48 hours failed to elicit HUVEC patterning in vitro (Figures 14G, 14H). However, incubation of HUVECs in either melanoma cell-conditioned media or co-culture conditioned media allowed for similar degrees of HUVEC patterning at 48 hours of incubation (Figures 14I, 14J) suggesting that this particular measure of melanoma-HUVEC crosstalk was mediated by a tumor-secreted factor.

### Cellular Crosstalk Between Melanoma Cells and Endothelial Cells Increases During Tumor Progression

Given the striking evidence of cellular crosstalk between the metastatic melanoma cell line, 1205Lu, and HUVEC endothelial cells, it was next evaluated whether such crosstalk was influenced by stage of melanoma progression or whether it was a common feature of all melanocytic cells. Eleven melanoma cell lines from varying stages of progression were evaluated for their ability to induce HUVEC patterning over time. Primary human melanocytes were also evaluated for their ability to induce HUVEC patterning in this co-culture system. It was found that melanoma cell lines from later stages of progression demonstrated an increased ability to promote HUVEC network assembly versus radial growth phase melanomas or primary human melanocytes suggesting that the ability of melanoma cells to communicate with endothelial cells is acquired as melanomas gain the ability to invade and metastasize (Figure 15).

### Cellular Crosstalk Between Tumor Cells and Endothelial Cells Varies with Tumor Type

Although tumor-directed endothelial cell patterning has been observed previously (reviewed in (Kopfstein and Christofori, (2006). Metastasis: cell-autonomous mechanisms versus contributions by the tumor microenvironment. Cell Mol Life Sci 63, 449-468), cellular communication within a co-culture system has not been widely investigated. The ability of various tumor cells to promote HUVEC patterning was evaluated in the described co-culture system. Interestingly, a range of HUVEC pattern induction by various tumor cell lines was found, with minimal patterning seen in co-cultures with pancreatic cancer cells, colorectal cancer cells, and prostate cancer cells, moderate patterning induced in co-cultures with ovarian cancer cells, non-small call lung cancer cells, and breast cancer cells, and the strongest patterning induced by glioblastoma cells and metastatic melanoma cells (Figure 16).

### Melanoma-Endothelial Cell Crosstalk Promotes an Invasive Tumor Phenotype

In order to define the molecular pathways governing melanoma-HUVEC crosstalk, gene expression profiling was performed on GFP-1205Lu and RFP-HUVECs either alone or following 48 hours of co-culture (Figure 17). Figure 17 shows global gene expression profiling of melanoma-endothelial cell crosstalk pathways and identifies Nrp-2 as a mediator of cellular communication. Analysis of gene signatures associated with the co-culture system demonstrated specific influences of tumor cells on endothelial cell expression profiles and vice versa. The biological classifications of genes upregulated in melanoma cells following co-culture with HUVECs includes cellular processes specifically associated with tumor progression and metastasis including alterations in cell adhesion, cell migration, extracellular matrix organization, and angiogenesis. These genes are shown in Table 9, below. In the Table, melanoma cells and HUVECs were co-cultured for 48 hours then separated by fluorescence-activated cell sorting (FACS). Gene expression profiles for single-cell cultures were compared to the same cells following co-culture conditions using high-density Affymetrix U133 oligonucleotide arrays. Gene expression profiles for the co-cultured melanoma cells demonstrated significant upregulation of the genes above which are notably associated with tumor invasion, metastatic potential, and an "angiogenic phenotype".

**Table 9**

| GO Biological Process Description | p-value | Number of Transcripts | Number Upregulated | Number Downregulated |
|---|---|---|---|---|
| Cell adhesion | 8.94e-07 | 55 | 45 | 10 |
| Regulation of cell-cell adhesion | 0.015 | 4 | 4 | 0 |
| Cell differentiation | 0.0288 | 57 | 45 | 12 |
| Negative regulation of cell differentiation | 0.0214 | 9 | 8 | 1 |
| DNA replication initiation | 0.000971 | 6 | 0 | 6 |
| Regulation of apoptosis | 0.000703 | 33 | 22 | 11 |
| Angiogenesis | 0.00111 | 21 | 19 | 2 |
| Regulation of cell migration | 0.00744 | 12 | 12 | 0 |
| Cell proliferation | 0.000397 | 52 | 26 | 26 |
| ECM organization | 0.000268 | 9 | 8 | 1 |
| Intercellular junction | 0.0436 | 10 | 10 | 0 |

One of the most interesting sets of altered genes identified were those found in tumor cells exposed to endothelial cells versus tumor cells alone. Remarkably, melanoma cells exposed to endothelial cells in a co-culture system upregulate a set of genes that promote cellular growth and invasion which have been associated with increased metastatic potentiaL Among the top 30 genes upregulated in melanoma cells under co-culture conditions are thymosin beta 4, a gene previously associated with tumor angiogenesis and melanoma metastasis (Cha et al., (2003). Role of thymosin beta4 in tumor metastasis and angiogenesis. J Natl Cancer Inst 95, 1674-1680; Clark et al., (2000). Genomic analysis of metastasis reveals an essential role for RhoC. Nature 406, 532-535; Ridley, (2000). Molecular switches in metastasis. Nature 406,466-467), multimerin 1, a gene involved in endothelial cell adhesion (Adam et al., (2005). Analyses of cellular multimerin 1 receptors: in vitro evidence of binding mediated by alphaIIbbeta3 and alphavbeta3. Thrombosis and haemostasis 94, 1004-1011), and the transmembrane cell surface co-receptor for VEGF, neuropilin-2 (NRP2). The Table shown in Figure 18A lists these genes. A complete list of all gene expression profiling data is provided in the Tables shown in 18B and 18C for melanoma cells (Panel B) and HUVECs (Panel C). Additionally, raw microarray data from these experiments has been submitted to GEO (Gene Expression Omnibus) under the series record GSE8699.

Since NRP2 has previously been shown to regulate processes essential to tumor metastasis including cell migration and angiogenesis (Ellis, (2006). The role of neuropilins in cancer. Mol Cancer Ther 5, 1099-1107) its potential significance as a mediator of melanoma progression was evaluated next. Although Nrp2 has previously been shown to be expressed in melanoma cell lines (Bielenberg et al., 2004; Chabbert-de Ponnat et al., 2006; Lacal et al., (2000). Human melanoma cells secrete and respond to placenta growth factor and vascular endothelial growth factor. J Invest Dermatol 115, 1000-1007), its precise role in melanoma development and progression has not been delineated to date.

### Neuropilin-2 is Upregulated During Melanoma-Endothelial Cell Crosstalk and is Expressed in Metastatic Melanomas

NRP2 protein expression in melanoma cells during co-culture was evaluated by Western blot in order to confirm the expression profiling data (Figure 17B). Since it was noted that the cellular patterning of HUVECs was dependent upon tumor-associated secreted factors, and NRP2 can exist in both a secreted form and as a cell surface receptor(Staton et al., 2007), the expression of NRP2 in conditioned media from either HUVEC alone, GFP-1205Lu cells alone, or conditioned media from the two-cell co-culture (Figure 17C) was examined. Significant expression of NRP2 was found in conditioned media from GFP-1205Lu cells and increased NRP2 expression was detected in conditioned media from the co-culture system versus HUVECs alone. Since the model system was expected to recapitulated critical events in tumor metastasis, it was ensured that NRP2 expression would indeed be expressed in metastatic melanomas. Examination of NRP2 expression in primary human melanoma tissues demonstrated significant expression of NRP2 within the tumor parenchyma of 5/5 metastatic melanomas evaluated (Figure 17E-G).

### Neuropilin-2 Mediates Melanoma Growth

In order to determine the functional significance of NRP-2 expression in melanomas, the effect of an NRP2 neutralizing antibody (Nasarre et al., (2003). Semaphorin SEMA3F and VEGF have opposing effects on cell attachment and spreading. Neoplasia (New York, NY 5, 83-92; Nasarre et al., (2005). Semaphorin SEMA3F has a repulsing activity on breast cancer cells and inhibits E-cadherin-mediated cell adhesion. Neoplasia (New York, NY 7, 180-189) on melanoma cell proliferation was evaluated. An NRP2-neutralizing antibody completely blocked melanoma cell proliferation in vitro (Figure 19A) suggesting that NRP2 is a significant mediator of mediated melanoma cell growth. Studies with an alternative, commercially available antibody also confirmed a significant growth inhibitory role, and BrdU incorporation assays further demonstrated significant growth inhibition at 48 hours following antibody treatment (Figure 19B). Remarkably, the growth inhibition was found to be irreversible, as removal of the antibody following 8 days of treatment did not allow for additional cellular proliferation to occur (Figure 19C). Evaluation of cellular apoptosis by TUNEL staining did not demonstrate notable increased melanoma cell apoptosis (Figure 23). In addition, tumor cell morphology was not significantly altered following treatment with neutralizing antibody (Figure 19D). Furthermore, antibody neutralization of NRP2 did not significantly alter cellular migration in a scratch assay when cellular proliferation effects were taken into account (Figure 23). Additional studies were performed using siRNA to Nrp2 to confirm the growth inhibitory effects seen in the antibody neutralization assays. Notably, no significant inhibition of melanoma cell growth was seen with targeted Nrp2 gene silencing with siRNA (Figure 19E) despite notable inhibition of NRP2 protein expression throughout the growth assay timecourse (Figure 19F) and significant inhibition of NRP2 secreted protein in conditioned media (Figure 19G).

### Neuropilin-2 Expression by Melanoma Cells Promote Cellular Crosstalk and Distinct HUVEC Patterning

The experiments described herein demonstrate that Nrp2 increases collective movement of HUVEC during early stages of co-culture. The initial melanoma-endothelial cell co-culture experiments suggested that the formation of branched patterns by HUVEC cells depended on external soluble cues secreted by melanoma cells. To investigate the role played by Nrp2 in the dynamic interactions leading to collective endothelial cell reorganization, time-lapse imaging of HUVEC cell movement was performed in a more refined co-culture model system. A type of microfabrication technology, soft lithography, was leveraged to precisely regulate the spatial relationships between melanoma and HUVEC cells, by precisely controlling the initial cell number and geometry (circular or triangular) of a two-dimensional colony of HUVEC cells. After the micropatterned HUVEC colonies were formed, melanoma cells were introduced to the surrounding area to establish the co-culture environment. In control experiments, HUVEC colony re-organization in the absence of melanomal cells was also examined.

Using this microfabricated co-culture system, it was observed that HUVEC cells collectively migrated forming directed multi-cellular streams and sprouting into branched structures. In co-cultures, these patterns started forming almost immediately after initiation of co-culture, where HUVEC cells cultured without melanoma cells displaed more organized collective movement at much later expansion stages (> 36 hrs.). Collective movement of endothelial cells was quantified using a spatial correlation function calculated from the spatial distribution of cell velocities (REF) for control and co-culture experiments and both geometries at an early timepoint (~5 hour after co-culture began) and a late timepoint (~40th hour). It was found that melanoma had a significant enhancement effect on the collective migration of HUVEC cells at the early time point but not later time points (Fig.20A and 20B). Nrp-2 neutralizing antibody completely abolished this early enhancement effect by melanoma cells (Fig. 20C), suggesting that Nrp2 plays a critical role in endothelial cell organization in the initial, critical stages of collective cell movement and pattern formation. It was further noted that this effect was independent of the initial geometry of the colony, which suggested that differences in the length and shape of the cell-cell contact boundaries did not play an immediate role in regulating collective movement of HUVEC cells. Furthermore, the effect of the antibody was most pronounced at relatively large cell-cell distances used for calculation of the degree of collective cell movement. These observations, in combination, further suggest that the effect of Nrp2 is likely to be long range, mostly likely influencing cell behavior through a diffusion based process, rather than contact based cell-cell interactions.

### Nrp2 is Expressed in Late Stage Melanomas and Binds VEGFR2 in Melanoma Cells

In order to determine the mechanism of melanoma growth inhibition by a neutralizing antibody to NRP2 and define the pathways associated with NRP2-induced HUVEC patterning, the expression of additional NRP2 ligands and co-receptors within the melanoma cells was evaluated. Previous studies of melanoma cell lines from varying stages of malignant progression have allowed the definition of the molecular signatures associated with melanoma progression (Ryu et al., 2007). Expression analysis of VEGF receptors, VEGF-A, VEGF-C, Nrp1, Nrp2, plexinA4A, plexinA3, and Sema3F in melanoma cell lines shows increased expression of Nrp2 in later stage melanomas versus radial growth phase melanomas (2/3) and primary human melanocytes and increased expression of VEGF-A in early versus late stage melanomas (Figure 21A). Of note, significant expression of Plexins, Sema3F, Nrp2, and VEGF-C is seen in the majority of melanoma cell lines evaluated. NRP2 protein expression was noted to be highest in vertical growth phase melanomas, with little detectable protein in 2/3 of early (radial) growth phase melanomas tested (Figure 21B). Since NRP2 has recently been shown to promote tumor metastasis through complex formation with VEGFR2 and VEGFR3 in lymphendothelial cells (Caunt et al., 2008) the question was asked whether NRP2 is bound to VEGFR2 or VEGFR3 in vivo. Co-immunoprecipitation assays in 1205Lu melanoma cells showed specific binding of NRP2 to VEGFR2 in vivo.

Described herein is a simple two-cell co-culture system in order to evaluate critical molecular crosstalk pathways for melanoma cells and endothelial cells in vitro. Comprehensive gene expression profiling of co-cultured melanoma and endothelial cells has allowed the identification of communication networks between these two cell types which may be important for melanoma metastasis. The upregulation of genes associated with both the angiogenic phenotype, cellular migration, remodeling of extracellular matrix, and cellular adhesion in melanoma cells following crosstalk with endothelial cells suggests that such a simple model system may allow for accurate recapitulation of these communication networks in vivo. Although previous investigators have observed evidence for such crosstalk in vitro (Boyd et al., (2002). Uveal melanomas express vascular endothelial growth factor and basic fibroblast growth factor and support endothelial cell growth. The British journal of ophthalmology 86, 440-447; Folkman and Haudenschild, (1980). Angiogenesis by capillary endothelial cells in culture. Transactions of the ophthalmological societies of the United Kingdom 100, 346-353; Khodarev et al., (2003). Tumour-endothelium interactions in co-culture: coordinated changes of gene expression profiles and phenotypic properties of endothelial cells. Journal of cell science 116, 1013-1022; Matsuo et al., (2004). Enhanced angiogenesis due to inflammatory cytokines from pancreatic cancer cell lines and relation to metastatic potential. Pancreas 28, 344-352; Tsujii et al., (1998). Cyclooxygenase regulates angiogenesis induced by colon cancer cells. Cell 93, 705-716; Ye and Yuan, 2007) and complex systems have been developed to study the nature of this network formation in vitro (Velazquez et al., (2002). Fibroblast-dependent differentiation of human microvascular endothelial cells into capillary-like 3-dimensional networks. Faseb J 16, 1316-1318), the nature of these model systems has not allowed for a comprehensive assessment of the molecular determinants of such crosstalk to date. The simple two-cell system described herein allows for the clear real-time distinction of tumor-endothelial cell activities during cellular crosstalk and an opportunity to retrieve post-communication cells for molecular evaluations of the melanoma-endothelial cell communication network. It is notable that the expression profiling of post-communication melanoma cells revealed upregulation, which can be striking, of genes associated with an angiogenic phenotype. Indeed, previous investigations have remarked on the vasculogenic phenotype of advanced melanomas (Dome et al., (2007). Alternative vascularization mechanisms in cancer: Pathology and therapeutic implications. Am J Pathol 170, 1-15; Hendrix et al., (2003). Vasculogenic mimicry and tumour-cell plasticity: lessons from melanoma. Nat Rev Cancer 3, 411-421; Hess et al., (2007). Deciphering the signaling events that promote melanoma tumor cell vasculogenic mimicry and their link to embryonic vasculogenesis: role of the Eph receptors. Dev Dyn 236, 3283-3296; Velazquez and Herlyn, (2003). The vascular phenotype of melanoma metastasis. Clinical & experimental metastasis 20, 229-235) which have been variously attributed to cell-autonomous angiogenic properties of melanoma cells themselves. The results presented herein suggest that a particular relationship exists between melanoma cells and endothelial cells that allows for enhanced cellular communication upon direct contact. Such communication networks allow for upregulated expression of cellular proteins including cell membrane receptors that enhance further melanoma-endothelial cell interactions. Although some network formation is observed between other tumor cell types and endothelial cells (Figure 16), the results suggest that the tumor cells with the greatest propensity to promote endothelial cell network formation are melanoma cells and glioblastoma cells.

The studies presented herein further evaluate neuropilin-2 as a mediator of melanoma-endothelial cell communication and investigate its role in melanoma development and progression. The data show a specific and irreversible growth inhibitory effect on melanoma cells of a neutralizing antibody to NRP2. This inhibitory effect is not reproduced through the use of targeted Nrp2 gene silencing by siRNA suggesting a specific growth inhibitory effect of the antibody that may be related to inhibitory effects on soluble NRP2 binding partners. Indeed, a recent study of Nrp2 function in colorectal tumors also found no inhibitory effect of Nrp2 silencing on tumor cell proliferation in vitro; however, significant reduction of tumor xenograft growth in vivo was noted in tumors stably expressing Nrp2 shRNA (Gray et al., 2008). Colorectal tumors with reduced expression of Nrp2 in vivo also showed significant inhibition in tumor metastatic potential and tumor invasion(Gray et al., (2008). Therapeutic targeting of neuropilin-2 on colorectal carcinoma cells implanted in the murine liver. J Natl Cancer Inst 100, 109-120). The assessments of tumor and endothelial cell migration patterns during cross-communication have demonstrated an effect of an NRP2 neutralizing antibody on HUVEC patterning induced by melanoma cells in co-culture. Inhibition of NRP2 function in this co-culture system reduces HUVEC patterning to that seen in HUVEC single-cell cultures and suggests that Nrp2 is a critical mediator of this particular metric of melanoma-endothelial cell crosstalk. Recent data on Nrp2 functions in tumor-associated angiogenesis in vivo suggest that specific blockade of the VEGF-C binding site of NRP2 in vivo leads to inhibition of tumor-associated lymphangiogenesis and tumor metastasis (Caunt et al., 2008). Moreover, these studies suggest that a VEGF receptor-independent function may be attributable to NRP2 in lymphendothelial cells. Recent structural information on NRP2 and inhibitory antibody binding has shed light on the specific effects of antibody inhibition to NRP2 ligand binding (Appleton et al., (2007). Structural studies of neuropilin/antibody complexes provide insights into semaphorin and VEGF binding. Embo J 26, 4902-4912). Notably, it was found that semaphorins and VEGF bind to distinct extracellular domains of NRP2 and that specific targeted antibodies might inhibit the binding of a specific ligand to NRP2 without influencing the binding of other ligands at other antigenic sites. The antibody evaluated in the present studies was generated against amino acids 560-858 of NRP2 and would therefore block binding of both semaphorin and VEGF ligands. Thus, one proposed model for Nrp2 function in melanomas allows for the seemingly disparate activities of NRP2 antibody neutralization and Nrp2 gene silencing by siRNA (Figure 22). In this model, one possible suggestion is that an anti-NRP2 antibody may function as a molecular sponge to bind up soluble VEGF and prevent NRP2-enhanced signaling by VEGF receptors. Alternatively, soluble NRP2 may function to bind up soluble growth inhibitory semphorins which would be released in the presence of NRP2 neutralizing antibody. A third alternative mechanism would include a direct functional activity of NRP2 neutralizing antibody on melanoma cell proliferation with antibody binding to cell surface NRP2 leading to a direct inhibitory intracellular signaling cascade. Indeed, the presence of such large quantities of soluble NRP2 in conditioned media from melanoma cells and the specific soluble effects of NRP2 on HUVEC patterning may ascribe particular functional significance to these NRP2 fragments not previously recognized.

The studies presented herein suggest that melanoma cells and other neuronal tumors promote specific cellular crosstalk with endothelial cells that allows for endothelial cell patterning to occur. In the case of melanoma cells, this patterning is mediated by a cell surface receptor that mediates neuronal cell migration and endothelial cell proliferation, Neuropilin-2. It is striking that Neuropilin-2 functions at an interface of neural cell and endothelial cell fates, and that melanoma cells elicit such a strong response in their communication with endothelial cells through this receptor. Given that the cell of origin for melanoma is the neural crest derived melanocyte, it is not surprising that a strong communication network exists for these tumor cells with its associated vasculature. Indeed, it may be that the striking ability for melanomas to metastasize at such early stages of development relates to genetic memory of developmental cues associated with neural crest migration to the periphery. Neuropilins, cellular receptors that function at this neural-endothelial cell interface, are likely to be critical mediators of this important communication pathway which serves a critical developmental role in early life but appears to recapitulate developmental cues in the malignant state to promote metastasis. Thus, neuropilin-2, as a mediator of melanoma cell proliferation and melanoma-endothelial cell communication, may be a critical preventive and therapeutic target in this disease.

### Methods

The above-described examples were carried out using, but not limited to, the following materials and methods:

### Cell Culture

Ten melanoma cell lines (WM35, SBcl12, WM1552C, WM1341D, WM902B, WM278, WM983A, WM793, WM852, WM983B, and 1205Lu) were provided by Meenhard Herlyn (Wistar Institute, Philadelphia, PA) and cultured in DMEM with 10% FBS. Human Umbilical Vein Endothelial Cells (HUVEC) were purchased from Cambrex Bio Science Walkersville, Inc and cultured in EGM-2 (Lonza). HUVEC were discarded following passage 10.

### GFP and RFP labeling

EF-CMV-GFP and EF-CMV-RFP plasmids were a generous gift from Curt Civin (Johns Hopkins University, Baltimore, MD). Lentivirus was produced in HEK293T cells using previously described protocols (Dunlap et al 2004). Viral transduction of cells was performed as previously described with some modifications. Briefly, 1.5 x 10⁵ 1205Lu cells or 1 x 10⁵ HUVEC were plated in one well of a 6-well plate 24 hours before infection. Cells were infected with lentivirus in the presence of 4µg/ml polybrene (Sigma) for 48 hours.

### Co-culture

GFP-1205Lu and RFP-HUVEC cells were plated at a 1:1 ratio at 95% confluence in EGM-2. Cells were cultured for 48 hours before sorting. Control cultures of individual cell types were grown under identical conditions. Co-cultured and control cells were washed 2X with PBS and collected by trypsinization. Collected cells were resuspended in EGM-2 and sorted by FACS into pure populations.

### Microarray analysis

Total RNA was isolated using the RNeasy Mini Kit (Qiagen) according to the manufacturer's instructions. The JHMI Microarray Core carried out the microarray hybridization and initial analysis. Briefly, RNA from control and experimental samples was processed using the RNA amplification protocol described by Affymetrix. 10µg of total fragmented cRNA was hybridized to the Affymetrix GeneChip human U133Plus 2.0 arrays. The chips were then washed, stained, and scanned on an Affymetrix G3000 GeneArray Scanner and image analysis of each GeneChip was done through the GeneChip Operating System 1.1.1 (GCOS) software from Affymetrix, using the standard default settings. For comparison between different chips, all probesets were globally scaled to a user-defined target intensity (TGT) of 150.

### Data Analysis

A spreadsheet containing the expression levels from the four GeneChips (GFP-1205Lu alone, GFP-1205Lu co-culture, RFP-HUVEC alone, RFP-HUVEC co-culture) was generated in Microsoft Excel. The signals from the chips were normalized using RMAExpress *(*Bolstad, B.M., Irizarry R A., Astrand, M., and Speed, T.P. (2003), A Comparison of Normalization Methods for High Density Oligonucleotide Array Data Based on Bias and Variance. Bioinformatics 19(2): 185-193*).* Two new spreadsheets were produced to generate a ratio of the expression between the co-cultured and control samples for each cell type. Only the genes tagged as present ("P") by the Affymetrix software were considered for evaluation. A cutoff signal ratio 0f 2 and above was considered upregulated in co-cultured cells, and a signal of 0.5 and below was considered downregulated.

### MIAME compliance

All samples were run in commercial arrays from Affymetrix, using Affymetrix GeneChip human U133Plus 2.0 arrays as described in the Affymetrix web site (http://www.affymetrix.com). The JHMI Microarray Core Facility abides in all its procedures by current MIAME guidelines. Microarray data has been submitted to the Gene Expression Omnibus (GEO) repository (http://www.ncbi.nlm.nih.gov/geo/) under the series record GSE8699.

### Immunoprecipitation

Protein extract was prepared using RIPA buffer with protease inhibitor cocktail (Sigma) and PMSF (Sigma). The extract was precleared with normal rabbit IgG for 60 min at 4°C. For immunoprecipitation, precleared samples were mixed with either NRP2 (sc-5542, Santa Cruz) or VEGFR2 (sc-504, Santa Cruz) antibody for 2 hours at 4°C, then rotated overnight at 4°C in the presence of ProteinA/G-Sepharose beads (Santa Cruz). The beads were collected by centrifugation and washed three times with PBS, then resuspended in 2X sample buffer. Western blotting was performed as below.

### Western blotting

Western blotting was performed using standard techniques and proteins were visualized using ECL reagents (Amersham). Antibodies used included: NRP2 (sc-5542, Santa Cruz), VEGFR2 (sc-504, Santa Cruz) and actin (sc-1616-R, Santa Cruz).

### Immunohistochemistry

Standard immunohistochemical techniques were performed using a rabbit polyclonal NRP2 antibody (Santa Cruz) and the EnVision Plus detection system (Dako). Briefly, tissue sections were deparaffinized and rehydrated, followed by antigen retrieval in citrate buffer. Primary NRP2 antibody was applied overnight followed by application of anti-rabbit HRP conjugated secondary antibody for 30 minutes. AEC chromogen was used rather than DAB to distinguish positive NRP2 staining from melanin present in the tissues.

### Antibodies

A rabbit polyclonal NRP2 antibody (sc-5542, Santa Cruz) and normal rabbit IgG (sc-2027, Santa Cruz), or a mouse monoclonal NRP2 antibody (sc-13117, Santa Cruz) and normal mouse IgG (sc-2025, Santa Cruz) were used at a final concentration of 10 µg/ml for functional studies.

### siRNA Transfection

1205Lu cells were transfected with siGENOME duplex human NRP2 siRNA or siCONTROL non-targeting siRNA ,,1 (Dharmacon) using Oligofectamine (Invitrogen) according to the manufacturer's instructions. Briefly, 1.5 x 10⁵ cells were plated per well of a 6-well plate in antibiotic free medium and incubated for 6 hours. Transfection with siRNA was performed using 15 µl Oligofectamine and 300 pmol siRNA in OPTI-MEM I (Invitrogen).

### Cell Proliferation

Cell population changes were quantified using a colorimetric-based XTT proliferation kit (Cell Proliferation Kit II, Roche Applied Science). Cells were plated at 3,000 cells/well in a flat bottom 96-well plate. XTT reagents were added to a subset of wells every 24 hours for 8 days and color change was monitored by spectrophotometer. Cell numbers were extrapolated from standard curves. Cells were plated in the presence of antibody or following overnight transfection with siRNA, and medium was changed and antibody refreshed on days 2, 4, and 6. All experiments were performed in triplicate. BrdU incorporation was measured using the BrdU Labeling and Detection Kit I (Roche Applied Science), following the manufacturer's instructions. BrdU labeling was carried out for 2 hours following 48 hours of antibody treatment or 48 hours after transfection with siRNA. DAPI staining was used to visualize nuclei. Photographs were taken on a Nikon Eclipse E800 microscope with MetaMorph software (Molecular Devices).

### Apoptosis

TUNEL staining was performed using the *In situ* Cell Death Detection Kit, TMR Red (Roche Applied Science). Cells were plated in the presence of antibody or following transfection with siRNA, and TUNEL staining was performed following 48 hours of antibody treatment or 48 hours post-transfection. DAPI staining was used to visualize nuclei. Photographs were taken on a Nikon Eclipse E800 microscope with MetaMorph software (Molecular Devices).

### Scratch Assay

GFP-1205Lu were plated at 100% confluence in a 24 well plate. A 200 µl pipet tip was used to scratch a line in the cell monolayer, and the cells were washed 3 times in PBS. Mitomycin C was added at a final concentration of 0 µM or 3 µM in DMEM with 10% FBS. Photographs of the scratched area were taken after the medium was added (time=0) and 24 hours later on a Nikon Eclipse TS100.

### Example 4. Neuropilin-2 expression in primary and metastatic melanoma: use as a biomarker and therapeutic target.

Determining the prognosis for a particular melanoma patient using current clinical criteria is often fraught with error. The most useful prognostic indicators of primary cutaneous melanomas are Breslow depth and presence or absence of ulceration (Fecher LA, et al. Toward a molecular classification of melanoma. J Clin Oncol 2007 Apr 20; 25(12): 1606-20; Balch CM, Soong SJ, Atkins MB, et al. An evidence-based staging system for cutaneous melanoma. CA Cancer J Clin 2004 May-Jun; 54(3): 131-49). However, many patients with thick melanomas are free of metastasis, while others with thin tumors die from their disease (Torabian S, Kashani-Sabet M. Biomarkers for melanoma. Curr Opin Oncol 2005 Mar; 17(2): 167-71). The vertical growth phase is felt to delineate the ability of a melanoma to metastasize. During vertical growth, prognosis can be predicted by depth measurements, mitotic counts, host response, anatomic site, presence or absence of regression, angioinvasion and ulceration (Crowson AN, et al. Prognosticators of melanoma, the melanoma report, and the sentinel lymph node. Mod Pathol 2006 Feb; 19 Suppl 2: S71-87.). Despite numerous investigations to date, there are currently no adequate methods to identify which melanomas will progress to vertical growth and metastasis (Rhodes AR. Cutaneous melanoma and intervention strategies to reduce tumor-related mortality: what we know, what we don't know, and what we think we know that isn't so. Dermatol Ther 2006 Jan-Feb; 19(1): 50-69).

Increased tumor vascularity and lymphatic invasion have been shown to contribute to melanoma migration and metastasis (Medic S, et al. Molecular markers of circulating melanoma cells. Pigment Cell Res 2007 Apr; 20(2): 80-91). In the transition to vertical growth phase, melanoma progression is heralded by the expression and release of vascular endothelial growth factor (VEGF), which facilitates the growth of both new blood vessels and the tumor itself (Mahabeleshwar GH, Byzova TV. Angiogenesis in melanoma. Semin Oncol 2007 Dec; 34(6): 555-65). In addition, increased lymphangiogenesis has been shown to occur with the transition to melanoma invasion and may precede the development of metastases in sentinel lymph nodes (Massi D, et al. Tumour lymphangiogenesis is a possible predictor of sentinel lymph node status in cutaneous melanoma: a case-control study. Journal of clinical pathology 2006 Feb; 59(2): 166-73; Dadras SS, et al. Tumor lymphangiogenesis predicts melanoma metastasis to sentinel lymph nodes. Mod Pathol 2005 Sep; 18(9): 1232-42; Giorgadze TA, et al. Lymphatic vessel density is significantly increased in melanoma. J Cutan Pathol 2004 Nov; 31(10): 672-7). Two subtypes of VEGF (VEGF-A and VEGF-C) production by melanoma are critical for the reorganization and proliferation of endothelial cells, leading to the development of both blood and lymphatic vasculature which generates a route for metastatic dissemination (Graells J, et al. Overproduction of VEGF concomitantly expressed with its receptors promotes growth and survival of melanoma cells through MAPK and PI3K signaling. J Invest Dermatol 2004 Dec; 123(6): 1151-61; Dadras SS, et al. Tumor lymphangiogenesis: a novel prognostic indicator for cutaneous melanoma metastasis and survival. Am J Pathol 2003 Jun; 162(6): 1951-60.)

Blocking Nrp2 function has recently been shown to inhibit tumor metastasis through effects on lymphendothelial cell migration and tumor-associated lymphangiogenesis (Caunt M, et al. Blocking neuropilin-2 function inhibits tumor cell metastasis. Cancer Cell 2008 Apr; 13(4): 331-42.). Neuropilins are expressed in a variety of cancers (Bielenberg DR, Klagsbrun M. Targeting endothelial and tumor cells with semaphorins. Cancer Metastasis Rev 2007 Dec; 26(3-4): 421-31; Chen C, et al. Roles of neuropilins in neuronal development, angiogenesis, and cancers. World journal of surgery 2005 Mar; 29(3): 271-5; Ellis LM. The role of neuropilins in cancer. Mol Cancer Ther 2006 May; 5(5): 1099-107; Guttmann-Raviv N, et al. The neuropilins and their role in tumorigenesis and tumor progression. Cancer Lett 2006 Jan 8; 231(1): 1-11; Klagsbrun M, Takashima S, Mamluk R. The role of neuropilin in vascular and tumor biology. Advances in experimental medicine and biology 2002; 515: 33-48). While Nrp1 is generally expressed strongly in epithelial tumors, NRP2 is more highly expressed in tumor cells of neural origin including glioblastomas, melanomas, neuroblastomas, in addition to osteosarcomas, bladder, pancreatic, and lung tumors (Bielenberg DR, et al. Neuropilins in neoplasms: expression, regulation, and function. Exp Cell Res 2006 Mar 10; 312(5): 584-93, 25; Sanchez-Carbayo M, et al. Gene discovery in bladder cancer progression using cDNA microarrays. Am J Pathol 2003 Aug; 163(2): 505-16) based on studies in tumor cell lines. In melanoma, exogenous expression of the NRP2 ligand Semaphorin 3F (Sema 3F) in tumor xenografts has been shown to inhibit tumor cell migration and metastasis to lymph nodes and lung without significant effects on tumor cell growth, leading to poorly vascularized tumors (Bielenberg DR, et al. Semaphorin 3F, a chemorepulsant for endothelial cells, induces a poorly vascularized, encapsulated, nonmetastatic tumor phenotype. J Clin Invest 2004 Nov; 114(9): 1260-71). In contrast, others have demonstrated inhibition of melanoma cell growth by Sema 3 F (Chabbert-de Ponnat I, et al. Antiproliferative effect of semaphorin 3F on human melanoma cell lines. J Invest Dermatol 2006 Oct; 126(10): 2343-5.).

Studies of NRP2 expression in primary human tumors have been limited. NRP2 expression patterns in bladder cancer were shown to be significantly associated with stage and grade of the tumor (Sanchez-Carbayo M, Socci ND, Lozano JJ, et al. Gene discovery in bladder cancer progression using cDNA microarrays. Am J Pathol 2003 Aug; 163(2): 505-16) and NRP1 and NRP2 co-expression is significantly correlated with increased vascularity, tumor progression, and poorer prognosis in lung cancers (Kawakami T, et al. Neuropilin 1 and neuropilin 2 co-expression is significantly correlated with increased vascularity and poor prognosis in nonsmall cell lung carcinoma. Cancer 2002 Nov 15; 95(10): 2196-201; Lantuejoul S, et al. Expression of VEGF, semaphorin SEMA3F, and their common receptors neuropilins NP1 and NP2 in preinvasive bronchial lesions, lung tumours, and cell lines. The Journal of pathology 2003 Jul; 200(3): 336-47). Pancreatic endocrine tumors strongly express NRP-2 but it is absent on carcinoid tumors of the colon, rectum and appendix (Cohen T, et al. Neuropilin-2 is a novel marker expressed in pancreatic islet cells and endocrine pancreatic tumours. The Journal of pathology 2002 Sep; 198(1): 77-82; Cohen T, et al. Neuroendocrine cells along the digestive tract express neuropilin-2. Biochem Biophys Res Commun 2001 Jun 8; 284(2): 395-403).

Given the critical importance of NRP2 to tumor-associated lymphangiogenesis and tumor metastasis (Caunt M, Mak J, Liang WC, et al. Blocking neuropilin-2 function inhibits tumor cell metastasis. Cancer Cell 2008 Apr; 13(4): 331-42), and the critical role of lymphangiogenesis to melanoma development and progression, the studies described herein focus on whether NRP2 expression might also be associated with advanced melanomas. The experiments describe the immunostaining of NRP2 in normal tissues, a variety of tumors, and primary and metatsatic melanomas of varying histologic subtypes including desmoplastic, spindle cell nodular, amelanotic, pigmented, and metastatic. The data presented herein demonstrate that NRP2 is highly expressed in the majority of melanomas excluding desmoplastic variants which may be useful in the diagnosis of disseminated disease or as a prognostic indicator.

### NRP-2 expression in normal tissue

The qualitative immunohistochemical analysis of NRP2 staining for normal tissues is found in Table 10, below, which shows the tissue microarry immunohistochemical analysis of normal tissues with Neuropilin-2.

**Table 10**

| Normal Tissues | Average NRP2 percent staining | Intensity |
|---|---|---|
| | | |
| Esophagus | - | |
| Stomach | - | |
| Small Bowel | - | |
| Appendix | - | |
| Colon | - | |
| Gallbladder | - | |
| Lung | - | |
| Parotid | - | |
| Omentum | - | |
| Thymus | - | |
| Adrenal | - | |
| Lymph node | - | |
| Bladder | - | |
| Vaginal tissue | - | |
| Thyroid | - | |
| Amnion | - | |
| Tonsil | - | |
| Endometrial | + | low |
| Pancreas | + | low |
| Prostate | + | moderate |
| Spleen | + | moderate |
| Breast | + | moderate |
| Muscle | ++ | moderate |
| Fallopian tube | ++ | moderate |
| Liver | ++ | moderate |
| Skin | ++ | high |
| Placenta | ++ | high |
| Kidney | ++ | high |
| Testes | +++ | high |

| | | |
|---|---|---|
| - Negative, +, <20% of tissue positive, ++, 20 to 60% of tissue positive, +++, >60% of tissue positive by pathologist review. | | |

As shown in the Table and in Figure 24, NRP2 staining was notable in liver, kidney, fallopian tubes, pancreas, placental tissue, testes, prostate, striated muscle cells, specimen specific breast ductal tissue, skin epidermis, spleen, and endometrial tissue (Figure 24). All samples of normal liver were mildly NRP-2 positive with scattered hepatocyte staining. The majority of normal kidney tissue samples showed strong NRP2 staining of the glomerular endothelial cells, collecting tubules and collecting ducts. The mucosal lining cells of fallopian tubes stained intermittently positive in all specimens. Placental specimens showed intense, intermittent NRP2 staining of the syncytiotrophoblast cells of the placental villi. These same specimens also showed intermittent staining of the fetal capillaries within the villous cores. Breast tissue showed selective NRP2 breast duct epithelial cell staining, based on the core sample. Striated muscle cells showed strong scattered NRP-2 staining in all available specimens. The skin specimens stained strongly positive for NRP2 only within the epidermal layer and some specimens possessed minimal staining of the basal cell layer of the epidermis. Endometrial tissue stroma cells and glandular cells stained intermittently positive for NRP2 within their nuclei with minimal staining of the cytoplasm. The testis stained strongly positive for NRP2 within the epithelium of the seminiferous tubules. Prostate specimens stained mildly positive for NRP2 in the prostatic glandular epithelial cells, predominantly as a light staining hue to the foamy cytoplasm. All other tissue types were negative for NRP2.

### NRP-2 expression in tumors

Immunohistochemical staining for NRP2 was evaluated for a variety of tumors, as shown in Table 11, below, and in Figure 25). Table 4 shows tissue microarray immunohistochemical analysis for neuropilin-2 staining of various non-melanocytic tumors.

**Table 11**

| Tumor Type | Average NRP2 Positive Computer | Number of NRP2 Positive Per Total Number of Cases | |
|---|---|---|---|
| | Mean + (%) | | |
| Breast carcinoma, lobular | + | 2/5 | 2.8 |
| Breast carcinoma, ductal | + | 3/5 | 5.1 |
| Leiomyosarcoma | + | 2/3 | 9.9 |
| Ovarian Mucinous | - | 0/1 | - |
| Ovarian Serous | - | 0/4 | - |
| Colon adenocarcinoma | + | 2/4 | 3.7 |
| Transitional Cell Carcinoma | + | 2/3 | 9.7 |
| Lung adenocarcinoma | - | 0/1 | - |
| Liposarcoma | - | 0/4 | - |
| Spindle cell sarcoma | - | 0/1 | - |
| Malignant Fibrous Histiocytoma | - | 0/4 | - |
| Non-small cell lung ca.(squamous) | - | 0/1 | - |
| Renal Cell Carcinoma (clear cell) | +++ | 4/5 | |
| | 50.0 | | |

| | | | |
|---|---|---|---|
| - Negative, +, <20% of tissue positive, ++, 20 to 60% of tissue positive, +++, >60% of tissue positive by pathologist review. | | | |

Tumors of the breast stained specifically for NRP2 with 2/5 lobular breast carcinoma cell cases staining mildly positive, and 3/5 ductal breast carcinoma cases staining NRP2 positive, but not across all sections (Figure 25). Leiomyosarcoma specimens also stained in a case-specific manner for NRP2 with one specimen staining negatively, and two others staining positively. Four of the five Renal Cell carcinoma (clear cell) cases stained positively for NRP2, and most sections stained strongly positive. Colon adenocarcinoma stained case specifically positive for NRP2, with 2/4 cases staining mildly positive for NRP2. The colon adenocarcinomas that stained positively had scattered intranuclear and crypt cell cytoplasmic staining. Transitional cell carcinoma of the bladder staining for NRP2 was also case-specific, with 2/3 cases staining mildly positive. All ovarian mucinous, ovarian serous, lung adenocarcinoma, liposarcomas, spindle cell sarcomas, non-small cell lung cancer (squamous cell carcinoma), and malignant fibrous histiocytoma cases were negative for NRP2.

The FRIDA computer analysis of the variety of tumors indicated the mean percentage of all stained tumor tissues was 10.4%. Renal Cell Carcinoma had the highest mean percent stained with 49.9%. The computer analysis of the remaining positive neuropilin-2 tumors calculated the average percentage stained as follows: breast carcinoma ductal 5.1%, breast carcinoma lobular 2.9%, colon adenocarcinoma 3.7%, leiomyosarcoma 9.9%, transitional cell carcinoma 9.7% (Figure 26B-D).

The immunohistochemical staining assessments for Neuropilin-2 in various primary malignant melanomas and metastatic melanomas are shown in Table 12, below. Table 5 shows tissue microarray immunohistochemical analysis of malignant melanomas and metastatic melanomas with NRP2.

**Table 12**

| Tumor Type | Average Computer NRP2 Positive | Average Intensity | Positive cases/ Cases examined | |
|---|---|---|---|---|
| | Mean+ (%) | | | |
| Pigmented Epithelial Melanoma | +++ | high | 8/8 | 42.6 |
| Amelanotic Epithelial Melanoma | +++ | high | 6/6 | 40.1 |
| Spindle Cell Nodular Melanoma | +++ | moderate | 3/3 | 15.5 |
| Desmoplastic Malignant Melanoma | + | low | 5/5 | 8.5 |
| Malignant Melanoma | +++ | high | 17/18 | 46.4 |
| | | | | |

| **Metastatic Melanomas** | | | | |
|---|---|---|---|---|
| Met. Malignant Melanoma | +++ | moderate | 5/5 | 50.6 |
| Met. amelanotic small cell malign. melanoma | ++ | low | 8/9 | 22.2 |
| Met. amelanotic epitheliod malign.melanoma | +++ | high | 8/8 | 63.5 |

| | | | | |
|---|---|---|---|---|
| - Negative, +, <20% of tissue positive, ++, 20 to 60% of tissue positive, +++, >60% of tissue positive by pathologist review. | | | | |

Pigmented epithelial melanomas demonstrated the most positive NRP-2 staining with all cases staining positive (8/8) for NRP2 and most specimens staining greater than 60% by pathologist review with moderate to high intensity (Table 12 and Figure 26). Amelanotic epithelial melanoma cases all stained positive for NRP2 (6/6) with all staining at least 20% by pathologist review and all staining moderate to high intensity. All of the spindle cell nodular melanoma cases also stained positive for NRP2 (3/3), with most having mild to moderate intensity and greater than 20% staining by pathologist review. Of all the melanoma cases, desmoplastic malignant melanoma had the mildest staining. All of the desmoplastic malignant melanoma cases were positive (5/5), and all stained less than 20% by pathologist review. The other malignant melanoma cases stained NRP2 positive in 17/18 specimens. A large majority of these stained greater than 20% of the field and staining intensity varied from mild to intense (Figure 26).

The FRIDA analysis for the variety of melanomas stained for NRP-2 showed a mean for all the tissues analyzed of 46.9%, a marked increase from the other tumors analyzed (Figure 26B-D). Desmoplastic malignant melanoma had the least amount of percentage stained with an average of 8.5%. The computer analysis of the spindle cell nodular melanoma indicated a mean of 13.9% positive for NRP-2. The epithelial type melanomas had the greatest staining for NRP-2 with pigmented epithelial melanoma expression having an average of 42.6% and amelanotic epithelial melanoma having a mean percentage NRP-2 positivity of 48.9%. Other melanomas had a mean NRP-2 of 46.4% by computer analysis.

Metastatic melanomas were also analyzed for neuropilin-2. The metastatic cases of malignant melanoma stained NRP2 positive in all five cases analyzed. All cases stained greater than 60% by pathologist review and all with moderate to intense staining. Metastatic amelanotic small cell melanomas stained NRP2 positive in 8/9 specimens and staining intensity varied from mild to intense. For cases of metastatic amelanotic epithelial malignant melanoma, all cases stained positive (8/8), with all sections staining greater than 20% by pathologist review. The vast majority of cases stained moderate to intense for NRP2.

The FRIDA analysis of metastatic melanomas was similar to that for non-metastatic melanomas. Metastatic amelanotic small cell melanomas had an average percent NRP-2 staining of 22.2% by computer analysis; whereas metastatic amelanotic epithelial cell melanoma and other metastatic malignant melanomas had higher percentages of 63.4% and 50.6% respectively (Figure 26B-D).

Neuropilin-2 has been demonstrated to play a major role in the development of the normal lymphatic vasculature (32) and recent studies suggest that blocking of NRP2 binding to VEGF-C inhibits tumor cell metastasis (19). Since melanoma progression and metastasis are intimately linked to the process of lymphangiogenesis (33), the experiments described herein evaluated the expression of the lymphangiogenesis-associated receptor, Neuropilin-2 in a variety of primary human melanomas. These studies demonstrated elevated NRP2 expression in all melanomas evaluated with the exception of desmoplastic melanomas. These results are interesting given the notable favorable prognosis for these tumors versus those of other histologic subtypes of melanoma (34). Previous studies have suggested an associated between NRP2 expression and tumor prognosis in bladder and lung cancers tumor with elevated expression of NRP2 associated with a worse prognosis (25, 28, 29). These data suggest that NRP2 may also serve as a prognostic indicator in patients with melanoma given the notable low expression in the desmoplastic melanomas evaluated. The tissue microarray panel of normal tissue confirmed previous studies that showed that NRP2 receptors are found in glomeruli, islet cells of the pancreas, and skin (30, 35, 36). The data also showed unexpected staining patterns in the primary human tissues evaluated in these studies including a notably high expression in testis and well as staining in striated muscle, liver, placenta, fallopian tubes, endometrium, breast, spleen and prostate. Neuropilin-2 staining of a variety of tumors suggests specific elevated expression in renal cell carcinoma versus a range of epithelial malignancies and leiomyosarcoma. Expression patterns in these tumors supported the few previous studies to date and the study data were consistent for both the pathologist and computer analyses. Ductal and lobularbreast carcinomas were positive as were colon adenocarcinoma, transitional cell carcinoma and renal cell carcinoma (clear cell). The results also indicated that leiomyosarcomas are mildly positive for neuropilin 2. Of all the tumors studied, only renal cell carcinoma (clear cell) stained strongly positive for NRP-2 with greater than 20% of the tissue staining, which is not surprising considering that normal renal tissue stains strongly positive for NRP2 in renal glomeruli and tubules (35). All other tumors that stained NRP2 positive were of low intensity, with <20% stained, which is significantly lower than the majority of melanomas stained evaluated (Figure 26B-D).

The study results for neuropilin-2 expression in melanomas demonstrated increased expression in epithelioid melanomas versus other subtypes. All of the epithelial melanomas stained >60% by pathologist review and >40% with computer analysis, with high intensity for NRP-2. The study results also indicate that the same high intensity and high percentage staining were also true for the amelanotic epithelial melanomas, which may be very helpful in diagnosis, since immunohistochemical reagents are most commonly used in the evaluation of amelanotic metastatic melanomas (37). Spindle cell nodular melanomas also stained with high intensity and high percentage but less so with the computer analysis. This discrepancy may be due to the limitations of the computer readings, which cause the computer positive results to be less than those of the pathologist. Desmoplastic melanomas had the lowest intensity and lowest percentage stained, but all cases 5/5 were positive for NRP-2 which may be useful in diagnosing these lesions since most melanoma biomarkers are absent in these tumors (37). With 17/18 other malignant melanomas staining NRP2 positive on average greater than 60% by pathologist review, (46.4% by computer analysis), and with high intensity, these results would suggest that NRP2 may have the potential to be a valuable marker for this disease and aid in the differentiation of melanocytic tumors from those of other tissue origins.

Since the intense tissue staining for NRP2 was also consistent throughout metastatic melanomas, it is difficult to determine whether NRP2 staining will be of use as a prognostic marker in melanoma; however, given that NRP2 can be expressed in a secreted form(38), detection of this secreted protein may be useful in the identification of patients with occult metastatic disease, or as a prognostic indicator. Additionally, evaluation of circulating cells for NRP2 surface markers may also assist in the identification of occult circulating melanoma cells.

Thus, the results described herein indicate that melanomas express high levels of Neuropilin-2 within the tissue parenchyma which is significantly elevated relative to all other malignancies evaluated in this study with the exception of renal cell carcinomas. Since the diagnosis of tissue origin in metastatic melanomas can be difficult to discern, NRP2 may be useful in determining tumor origin for diagnostic purposes. Further, of all melanoma subtypes evaluated that express NRP2, a significant outlyer in terms of extent and intensity of is desmoplastic melanoma. Since this melanoma subtype is known to have a significantly better prognosis versus other melanomas of equal stage, these early studies suggest that NRP2 may also serve as a prognostic marker in melanoma. Finally, as NRP2 is synthesized as both a cell surface receptor and in a secreted form, one suggestion is that that assessments of NRP2 protein levels in the peripheral blood may assist in the diagnosis of occult disease in patients with a history of melanoma is a manner analogous to the prostate-sepcific antigen in prostate cancer.

### Methods

The above-described examples were carried out using, but not limited to, the following materials and methods:

### Immunohistochemistry

The tissues for immunohistochemical analysis were paraffin- embedded tissue microarrays provided from the archives of the Department of Pathology of Memorial Sloan-Kettering Cancer Center and collected under appropriate protocols. Immunohistochemistry was performed using the EnVision System HRP (DakoCytomation). The slides were deparaffinized and rehydrated using a graded alcohol series. A citrate buffer was used for antigen retrieval. Using the capillary gap method, the sections were incubated overnight with rabbit polyclonal antibodies against NRP-2 (SC-5542, Santa Cruz Biotechnology). 3-amino-9-ethyl carbazole (AEC) was the final chromogen and the sections were counterstained with hematoxylin. A dilution of 1:50 was found to provide the optimum staining results.

### Quantification of Staining and Statistical Analysis

The tissues used for analysis were the following: normal tissues, various types of tumors, and numerous malignant melanomas. The TMA slides were scanned and digitized using the Bacus Labs Inc. Slide Scanner (BLISS, Bacus laboratories, Lombard, IL.) The images were uploaded into the TMAJ database for evaluation. Tissues that were not considered representative samples of the tissue being studied were removed from the analysis. The slides were examined qualitatively and tissue staining was estimated and graded as follows: less than 20%, 20-60%, or greater than 60% of the tissue present. The intensity of neuropilin-2 staining was also scored from 0 to 3 with 0 having no NRP-2 staining and 3 having the highest intensity. The extent and intensity of staining was documented and compared to control samples that were strongly positive for neuropilin-2.

Further tissue evaluation was performed by the FRIDA (FRamework for Image Dataset Analysis) software program designed by Toby Cornish MD, Angelo DeMarzo, PhD, Bora Gurel PhD, and James Morgan BS. FRIDA is a custom open source image analysis software package used for the analysis of color image datasets, including those generated from automated scanning of tissue microarrays. The program is used to define an image field "mask" using select pixels of specific colors. Numerical values are given by the software program for hue, saturation and brightness which are then used consistently across all tissue samples. The FRIDA software generates quantitative continuous scale parameters including the number of pixels in the image matching the values defined, the sum of the intensity values for each pixel for each color in the image, and the mean intensity of pixels in the mask. Using the specific color pixel definition for total tissue, positive neuropilin-2 staining tissue, stained nuclei, and cytoplasm, the Java software program analyzed images with the selected color pixels to quantify positive staining. In the studies described herein, the tissue areas were defined as "tissue area", stained nuclei were defined labeled "nuclei", and the specific neuropilin-2 staining color positive mask was defined as "NRP-2 area". Since nuclei were not expected to be stained according to the preliminary testing studies, and nuclei can be very large in tumor cells, the area of cytoplasm that was expected to stain for NRP-2 was redefined as tissue that is in the "tissue area" but not in the "nuclei" and subsequently labeled "cytoplasm". By redefining the total tissue area without nuclei as cytoplasm, a more accurate calculation based on total possible staining area for neuroplin-2 was established. The percentage of staining was calculated by the FRIDA program as the "NRP-2 area" / "cytoplasm" (total tissue area without nuclei). The results of the FRIDA computer analysis along with the pathologist evaluations were analyzed using the R version 2.6 statistical software program (The R Working Group, 2008, Vienna, Austria, available on the world wide web at CRAN.R-project.org/doc/FAQ/.

### Example 5. Mediators of Melanoma development and progression: identification of downstream biomarkers and therapeutic targets

Further studies were aimed at determining mediators of melanoma development and progression, in order to identify novel biomarkers and therapeutic targets. In particular, the studies aimed to identify a proliferative target gene signature for BRAF kinase in melanoma cells.

BRAF kinase has been identified as a critical mediator of melanoma development and progression. As BRAF kinase has been found to be mutationally activated in up to 70% of benign nevi and melanomas, it has been implicated as a critical mediator of melanocyte growth and melanoma development, suggesting that this event is important for melanocyte proliferation and melanoma initiation in vivo. The V600E activating mutation represents the most commonly mutated form of BRAF in nevi and melanomas (Davies et al. 2002. Mutations of the BRAF gene in human cancer. Nature 417:949-954; Pollock et al. 2003. High frequency of BRAF mutations in nevi. Nat Genet 33:19-20; Tuveson et al. 2003. BRAF as a potential therapeutic target in melanoma and other malignancies. Cancer Cell 4:95-98). To date, however, the precise mechanism of BRAF kinase action in these lesions remains to be elucidated. Expression of activated BRAF kinase has previously been shown to lead to transformation of murine melanocytes in vitro (Wellbrock et al. 2004. V599EB-RAF is an oncogene in melanocytes. Cancer Res 64:2338-2342) while suppression of BRAF kinase activity abrogates cellular transformation of melanoma cells (Hingorani et al. 2003. Suppression of BRAF(V599E) in human melanoma abrogates transformation. Cancer Res 63:5198-5202) suggesting BRAF kinase or its downstream effectors may be useful therapeutic targets in melanoma.

The experiments described herein use comprehensive gene expression profiling to evaluate the downstream effectors of activated BRAFV600E kinase in primary human melanocytes and result in the identification of a dominant proliferative target gene signature for BRAF kinase in these cells. Among the most highly upregulated BRAF kinase target genes that was identified in these studies was matrix metalloproteinase-1 (MMP-1). Further evaluation of the functional significance of MMP-1 expression in primary human melanocytes and melanomas demonstrates that MMP-1 is increased in both expression and function in melanocytes expressing BRAF^{V600E}. Furthermore, silencing of MMP-1 in melanoma cell lines significantly decreased growth of melanoma cells possessing the BRAF^{V600E} mutation versus melanomas possessing wildtype BRAF.

Several studies that have sought to identify a BRAF gene signature in melanomas (Edwards RH et al., 2004. Absence of BRAF mutations in UV-protected mucosal melanomas. J Med Genet 41:270-272; Hoek et al. 2006. Metastatic potential of melanomas defined by specific gene expression profiles with no BRAF signature. Pigment Cell Res 19:290-302; Tsavachidou D. et al. 2004. SPRY2 is an inhibitor of the ras/extracellular signal-regulated kinase pathway in melanocytes and melanoma cells with wild-type BRAF but not with the V599E mutant. Cancer Res 64:5556-5559; Johansson, P. et al. Confirmation of a BRAF mutation-associated gene expression signature in melanoma. Pigment Cell Res 20:216-221; Pavey, S., et al. 2004. Microarray expression profiling in melanoma reveals a BRAF mutation signature. Oncogene 23:4060-4067; Shields, J.M., et al. 2007. Lack of extracellular signal-regulated kinase mitogen-activated protein kinase signaling shows a new type of melanoma. Cancer Res 67:1502-1512; Ryu, B., et al. 2007. Comprehensive expression profiling of tumor cell lines identifies molecular signatures of melanoma progression. PLoS ONE 2:e594; Haqq, C., et al. 2005. The gene expression signatures of melanoma progression. Proc Natl Acad Sci U S A 102:6092-6097; Lewis, T.B., et al. 2005. Molecular classification of melanoma using real-time quantitative reverse transcriptase-polymerase chain reaction. Cancer 104:1678-1686) have reported conflicting data suggesting that the molecular heterogeneity of melanomas may mask any underlying BRAF-associated defects across BRAF-mutant tumors (Fecher et al. 2008. The MAPK pathway in melanoma. Curr Opin Oncol 20:183-189). Recent studies have allowed for some clarification of the BRAF kinase signature in melanoma cell lines through the identification of gene signatures associated with ERK activation and MEK inhibition (Shields, 2007; Bloethner, et al. 2005. Effect of common B-RAF and N-RAS mutations on global gene expression in melanoma cell lines. Carcinogenesis 26:1224-1232); however, these studies were unable to discem molecular diifferences between BRAF and NRAS mutant melanomas, nor did they allow for identification of melanocyte-specific BRAF signature genes that might be activated in benign BRAF-associated lesions including nevi. Additional studies have allowed for the identification of genes associated with BRAF-induced senescence in fibroblasts and their assessment in primary human melanocytes (Wajapeyee et al. 2008. Oncogenic BRAF induces senescence and apoptosis through pathways mediated by the secreted protein IGFBP7. Cell 132:353-374); however, these studies did not specifically identify the downstream effectors of BRAF kinase in the melanoma cell of origin, the melanocyte. Thus, despite strong evidence implicating BRAF kinase as a bona-fide oncogene in melanoma, its precise downstream targets in melanocytes have not been defined to date, and a BRAF-specific gene signature in melanomas remains uncertain (Hoek, 2006; Pavey, 2004; Ryu, 2007).

Over the past few years, melanoma investigators and clinicians have been invigorated by the identification of high-frequency activating genetic mutation of BRAF kinase in nevi and melanomas (Haluska et al. 2007. The RTK/RAS/BRAF/PI3K Pathways in Melanoma: Biology, Small Molecule Inhibitors, and Potential Applications. Semin Oncol 34:546-554 However, despite the initial enthusiasm surrounding this discovery, translation to meaningful clinical endpoints with significant therapeutic benefits for melanoma patients has been slow (Kalinsky et al., Novel inhibitors in the treatment of metastatic melanoma. Expert Rev Anticancer Ther 7:715-724.]. In order to identify additional therapeutic targets in melanomas possessing activating BRAF kinase mutations, the studies described herein clarify the functional significance of BRAF^{V600E} in primary human melanocytes and melanomas.

The experiments described herein evaluate the global gene expression signature for BRAF kinase in primary human melanocytes in order to clarify the mechanism of action of BRAF kinase in the development of benign nevi and melanomas. The gene expression profiles define a predominant proliferative phenotype induced by BRAF kinase in these cells.

One finding from these experiments is that BRAF kinase promotes melanoma growth through activation of MMP-1, which is a critical mediator of cell growth in melanomas possessing the BRAFV600E mutation, and suggests that MMP-1 maybe a useful therapeutic target in melanomas possessing activating BRAF mutations.

### BRAF^{V600E} induces growth of primary human melanocytes

In a first set of experiments, activated BRAF^{V600E} was introduced into primary human melanocytes (PHMs) in order to define its specific function in vivo. PHMs were infected with bicistronic lentiviral vectors expressing green fluorescent protein (GFP), alone or in combination with either an active BRAF^{V600E} mutant or an inactive kinase, BRAF^{DEAD} (Karbowniczek et al. 2004. Regulation of B-Raf kinase activity by tuberin and Rheb is mammalian target of rapamycin (mTOR)-independent. J Biol Chem 279:29930-29937). BRAF expression levels in infected cells were found to be similar to those observed in melanomas possessing a mutated BRAF^{V600E} kinase (Figure 27A). Cells were evaluated for BRAF kinase activity by assessing the phosphorylation state of the BRAF kinase substrate, MEK. PhosphoMEK (p-MEK1/2) and total MEK protein levels were evaluated at various times following infection. Throughout these studies all cells possessed the expected levels of BRAF kinase activity (Figure 27A, B). A significant increase in cellular proliferation (P<001) was observed in BRAF^{V600E} expressing PHMs for up to 21 days following infection (Figure 27C); however, proliferation of these cells abated at later timepoints (Figure 27D). While previous studies noted growth arrest and senescence in PHMs expressing exogenous BRAF^{V600E} by day 21 (Michaloglou, et al. 2005. BRAFE600-associated senescence-like cell -cycle arrest-of human naevi. Nature et al., 2006. Cellular senescence in naevi and immortalisation in melanoma: a role for p16? Br J Cancer 95:496-505), no evidence was found for BRAF^{V600E}-associated cell cycle arrest or premature senescence in PHMs in either growth assays (Figure 27C, D) or senescence-associated β-galactosidase assays. In addition, there was no upregulation of p16/INK4a in BRAF^{V600E}-expressing cells at later passage (Figure 27E), and telomere lengths in late passage cells showed no substantial differences by quantitative fluorescence in-situ hybridization (Figure 27F). This conflicting result may attribute to the different primary melanocyte culture conditions and different levels of oncogenic BRAF level induction in primary cells. Because a growth promoting effect of BRAF^{V600E} kinase in PHMs was found, downstream effectors of this kinase activity which could mediate BRAF-induced proliferation using gene expression profiling of a genome-wide DNA microarray were next evaluated.

### BRAF^{V600E} promotes a proliferative gene signature in primary human melanocytes

The gene expression signature of PHMs expressing activated BRAF^{V600E} was assessed in comparison to PHMs expressing GFP alone and normalized using RMAExpress software (Irizarry et al 2003. Summaries of Affymetrix GeneChip probe level data. Nucleic Acids Res 31:e15). 38 genes were found to be upregulated 5-fold or more in PHMs expressing BRAF^{V600E} including the known BRAF kinase target genes VEGF-A (Mukhopadhyay et al. 1995. Hypoxic induction of human vascular endothelial growth factor expression through c-Src activation. Nature 375:577-581) and IL-8 (Bruder and Kovesdi, I. 1997. Adenovirus infection stimulates the Raf/MAPK signaling pathway and induces interlenkin-8 expression. J Virol 71:398-404). These genes are shown in the Table in Figure 31. Interestingly, only 3 genes were found to be downregulated 5-fold or greater following BRAF activation. Overall, the BRAF^{V600E} signature of PHMs was characterized by upregulation of several growth promoting genes and genes involved in cellular motility and inflammation (Table 13 and the Table shown in Figure 33) with a common network activation of cellular growth/proliferation and apoptosis (Figure 28). Table 6 is shown below.

**Table 13**

| Gene ontology analysis of BRAF downstream effectors in primary human melanocytes^{a}. | | | | |
|---|---|---|---|---|
| Number of Gene ontology downregulated | | Number of genes | p-value^{b} | Number of upregulated |
| Molecular Function | | | | |
| 1 | Receptor binding | 14 | 8.44e-7 | 13 |
| 0 | Protease inhibitor activity | 5 | 0.00185 | 5 |
| 0 | ECM structural constituent | 4 | 0.000305 | 4 |
| 0 | Glycosaminoglycan binding | 2 | 0.00182 | 2 |
| 0 | Collagen Binding | **1** | **1**.35e-5 | 1 |

| Biological Process | | | | |
|---|---|---|---|---|
| 7 | Biological regulation | 28 | 0.000128 | 21 |
| 4 | Cell cycle progression | 11 | 4.33e-5 | 7 |
| 0 | Cell proliferation | 9 | 8.83e-5 | 9 |
| 1 | Homeostatic process | 7 | 4.19e-5 | 6 |
| 0 | Chemotaxis | 2 | 0.000713 | 2 |
| 3 | Reproductive process | 6 | 0.000906 | 3 |
| 5 | Response to stimulus | 19 | 1.39e-5 | 14 |
| 1 | Locomotory behavior | 5 | 0.00212 | 4 |
| 2 | Inflammatory response | 7 | 6.37e-5 | 5 |
| 0 | Acute inflammatory | 3 | 6.89e-6 | 3 |

| | | | | |
|---|---|---|---|---|
| a: 82 annotated genes from 137 probesets which are identified as greater than 3-fold differentially expressed genes were analyzed. b: p-value < 0.005 was used for identification of the Molecular functions and biological processes that may regulated by BRAF downstream effectors. | | | | |

Among the most highly upregulated genes were several genes that were previously implicated in promoting tumor cell growth including matrix metalloproteinase-1 (MMP-1) (Hofmann et al. 2000. Matrix metalloproteinases in human melanoma. J Invest Dermatol 115:337-344), SerpinB2 (Montgomery et al. 1993. Melanoma-mediated dissolution of extracellular matrix: contribution of urokinase-dependent and metalloproteinase-dependent proteolytic pathways. Cancer Res 53:693-700), amphiregulin, (Berasain et al. 2007. Amphiregulin: A new growth factor in hepatocarcinogenesis. Cancer Lett.), CXCL5 (Yang, 2001. Constitutive IkappaB kinase activity correlates with nuclear factor-kappaB activation in human melanoma cells. Cancer Res 61:4901-4909), IL-8 (Bar-Eli, 1999. Role of interleukin-8 in tumor growth and metastasis of huyman melanoma. Pathobiology 67:12-18), RAP1a (Ehrhardt, 2002. Ras and relatives--job sharing and networking keep an old family together. Exp Hematol 30:1089-1106), and epiregulin (Normanno et al., 2001. The role of EGF-related peptides in tumor growth. Front Biosci 6:D685-707). Given that MMP-1 showed the highest level of upregulation (315.9-fold increase), its role was further examined as a BRAF kinase effector.

### BRAF^{V600E} promotes melanoma growth through activation of MMP-1

Since melanomas possess a high frequency of BRAF kinase activating mutations, initially the expression of MMP-1 in PHMs and melanoma cell lines containing either wildtype or mutant BRAF kinase was evaluated in order to determine whether MMP-1 expression correlated with BRAF^{V600E} expression in melanomas. Melanoma cell lines and PHMs were examined for MMP-1 expression using gene expression profiling as previously described (Ryu, 2007). A 25-fold increased expression of MMP-1 in melanoma cells possessing an activated BRAF^{V600E} kinase versus melanoma cells expressing wildtype BRAF, while PHMs expressed lower MMP-1 levels than either melanoma cell line evaluated (Figure 29A), suggesting that increased BRAF kinase activity may be associated with elevated MMP-1 expression in melanomas. Since the expression profiling of PHM expressing BRAF^{V600E} only accounted for activation of MMP-1 transcript levels by BRAF, MMP-1 was next confirmed as an effector of BRAF kinase at the protein level as well as through functional assays. MMP-1 is a secreted collagenase; therefore, conditioned media from PHMs expressing either control lentivirus (GFP) or BRAF^{V600E} were assessed for MMP-1 protein levels and collagenase activity. Melanocytes expressing BRAF^{V600E} possessed 168-fold increased levels of secreted MMP-1 protein versus PHM controls (Figure 29B). Furthermore, BRAF^{V600E} expressing melanocytes possessed 63.6-fold increased MMP-1 collagenase activity versus PHM controls suggesting that activated BRAF kinase can induce both MMP-1 protein expression and activity (Figure 29C). In order to determine the functional significance of BRAF kinase induction of MMP-1 in human melanomas, the effect of MMP-1 gene silencing on the proliferative functions of BRAF kinase was next assessed. MMP-1 transcript levels were efficiently downregulated in melanomas possessing either wildtype BRAF kinase (WM852) or mutant BRAFV600E kinase (WM793) using targeted MMP-1 siRNA (Figure 29D), as were associated secreted protein levels obtained from conditioned media (Figure 29E). Cellular proliferation was assessed in both BRAF wildtype and BRAF^{V600E} mutant melanomas following MMP-1 silencing by siRNA (Figure 29F) and a neutralizing MMP-1 antibody (data not shown). Significant inhibition of cellular proliferation was seen in both BRAF wildtype and BRAF mutant melanoma cells following silencing of MMP-1 gene expression; however, while cell growth was inhibited by 17% with MMP-1 siRNA versus control siRNA in BRAF wildtype melanomas, growth inhibition by MMP-1 siRNA in the BRAF mutant melanoma cells was significantly more effective at 80% inhibition. It can be concluded then that BRAF^{V600E} promotes cellular growth in primary human melanocytes and melanomas through activated expression of MMP-1.

The data presented herein supports a role for BRAF kinase in promoting the growth of primary human melanocytes and has allowed definition of a dominant proliferative gene signature associated with BRAF activation in these cells. While many growth-associated genes were identified as being upregulated in primary human melanocytes expressing activated BRAF kinase, the most highly upregulated gene was the matrix-associated collagenase, MMP-1. MMP-1 was also found to be specifically upregulated in expression and function in melanoma cells expressing mutant BRAF^{V600E} kinase. Although previous studies suggested that BRAF kinase activity promotes expression of MMP-1 in melanoma (Huntington, et al. 2004. Overexpression of collagenase 1 (MMP-1) is mediated by the ERK pathway in invasive melanoma cells: role of BRAF mutation and fibroblast growth factor signaling. J Biol Chem 279:33168-33176), and that MMP-1 promotes melanoma progression (Blackburnet al. 2007. RNA interference inhibition of matrix metalloproteinase-1 prevents melanoma metastasis by reducing tumor collagenase activity and angiogenesis. Cancer Res 67:10849-10858), it can be concluded that induction of MMP-1 in melanoma is specifically important for melanoma progression and metastasis through degradative functions on interstitial collagens. Additionally, MMP-1 serum levels have been associated with worse prognosis in melanoma patients (Nikkola et al. 2005. High serum levels of matrix metalloproteinase-9 and matrix metalloproteinase-1 are associated with rapid progression in patients with metastatic melanoma. Clin Cancer Res 11:51.58-5166); however, much of this association has been attributed to MMP-1 effects on degradation of the ECM and tumor cell migration (Egeblad et al. 2002. New functions for the matrix metalloproteinases in cancer progression. Nat Rev Cancer 2:161-174). Here the data show that MMP-1 is a critical mediator of the growth promoting functions of BRAF kinase in both primary human melanocytes and melanoma cells which is consistent with a proliferative role for BRAF kinase in the development of benign melanocytic lesions, or nevi in addition to melanomas. Indeed, although MMP-1 is a collagenase involved in the degradation of extracellular matrix (ECM), recent studies have suggested an additional important role for MMPs in activating latent growth factors which may be critical to the effects of MMP-1 as shown in the results presented herein. Notably, MMP-1 has been implicated in activating breast cancer cell growth through proteolytic activation of the cell surface receptor PAR1 (Boire et al. 2005. PAR1 is a matrix metalloprotease-1 receptor that promotes invasion and tumorigenesis of breast cancer cells. Cell 120:303-313).

As BRAF kinase effectors may differ depending on their cellular milieu, it is interesting to note the specific BRAF effectors identified in primary human melanocytes which are likely to be cell-type specific. Of note, amphiregulin, an epidermal growth factor receptor (EGFR) ligand, was found to be highly induced by BRAF^{V600E} in PHMs. Because amphiregulin is synthesized as a precursor protein that is released from the plasma membrane by metalloproteinases, the results presented herein suggest that BRAF^{V600E} may activate paracrine growth of keratinocytes through MMP-1 cleavage and activation of amphiregulin, with resultant keratinocyte-associated EGFR activation of BRAF kinase and MNP-1 expression (Westermarck et al. 1999. Regulation of matrix metalloproteinase expression in tumor invasion. Faseb J 13:781-792). Since the tumor microenvironment and melanoma-keratinocyte interactions have been shown to be critical to tumor progression (reviewed in(Lee, 2007. Microenvironmental influences in melanoma progression. J Cell Biochem 101:862-872)) such a paracrine growth-promoting function for BRAF kinase would not be wholly unexpected. Other downstream effectors of BRAF kinase in PHMs included IL-8 and serpinB2 which can also promote cellular growth and have been implicated in promoting tumorigenesis. SKP-2 is a particularly noteworthy BRAF target gene (as shown in the Table in Figure 33) as it is able to activate the cyclin D/cdk4 pathway through proteosomal degradation of the cyclin-dependent kinase inhibitor, p27. This cell cycle pathway is of particular importance in melanoma as evidenced by frequent activation through either amplification of cyclin D or inactivation of p16/INK4a (Curtin, et al. 2005. Distinct sets of genetic alterations in melanoma. N Engl J Med 353:2135-2147). Interestingly, one of the most highly induced BRAF kinase effectors was IL-24 which has been associated with differentiation of melanoma cells, induction of apoptosis, and cellular growth arrest (Jiang, et al. 1996. The melanoma differentiation associated gene mda-7 suppresses cancer cell growth. Proc Natl Acad Sci U S A 93:9160-9165). As BRAF kinase is activated in benign nevi which exhibit both a proliferative phase and a growth arrest/terminal differentiation/senescence phase, it can be suggested that both of these functions may be attributed to activated BRAF kinase based on the BRAF effector signature genes in PHMs. Thus, a model can be proposed for BRAF functions in primary human melanocytes (Figure 30) where BRAF kinase can exhibit either proliferative functions or growth arrest functions, depending on the cellular context. With early activation of BRAF in melanocytes, as seen in the majority of benign nevi, kinase activity promotes cellular proliferation via MMP-1, autocrine/paracrine growth signals, and activated cdk4/cyclin D complexes; however, as proliferative signals continue, a tumor suppressor function must ensue to prevent the accumulation of mutational events that may lead to malignant conversion. Growth arrest signals may include pathways such as IL-24 associated "differentiation" mechanisms (Jiang, et al. 1996), oncogene stress-associated responses, or senescence-associated pathways (Michaloglou, 2005; Gray-Schopfer et al, 2006).

Taken together, the results presented herein demonstrate a BRAF kinase signature in primary human melanocytes, and define MMP-1 as a mediator of proliferation in melanomas possessing activating BRAF^{V600E} mutations. Since current targeted therapeutic strategies directed against BRAF kinase. alone in patients with advanced melanoma have been somewhat disappozrrting (Fecher, et al. 2007. Toward a molecular classification of melanoma. J Clin Oncol 25:1606-1620), a potentially promising new therapeutic intervention is combination therapies that are directed against BRAF kinase and its downstream effectors, including MMP-1, that may provide additional therapeutic benefits in patients with advanced melanoma.

### Methods

The above-described examples were carried out using, but not limited to, the following materials and methods:

### Cell Culture

Primary human melanocytes were prepared from neonatal foreskins as previously outlined (Dunlap et al. 2004. High-efficiency stable gene transduction in primary human melanocytes using a lentiviral expression system. Journal of Investigative Dermatology 122:549-551). In order to minimize genetic variability, melanocytes from 4-5 individuals were pooled for each culture. Once epidermal cells were dispersed with trypsin and neutralized in serum-containing media, cells were plated in melanocyte growth medium (Cell Applications, Inc., San Diego, California). Cells were used at passage 2 for viral infection. Melanoma cell lines 1250Lu and WM938B were obtained from Meenhard Herlyn and cultured in DMEM with 10% FCS.

### Lentivirus preparation and primary cell transduction

BRAF^{V600E} and dead kinase mutant *BRAF*^{*T595A*/*S602A*} constructs have been described previously (Karbowniczek et al., 2004) and were obtained from Gavin Robertson (Hershey, PA). BRAF mutants were amplified by PCR Using the following primers: 5'-GGATCCCAGTGTGGTGGTA-3' and 5'-CCACTGTGCTGGCGAATTC-3'. PCR amplified products were blunt ended and inserted into Eco RV site of a bicistronic lentiviral vector(Yu et al. 2003. Lentiviral vectors with two independent internal promoters transfer high-level expression of multiple transgenes to human hematopoietic stem-progenitor cells. Mol Ther 7:827-838). Virus was produced in HEK293T cells using previously described protocols (Yu et al, 2003). Viral transduction of primary human melanocytes was performed as described previously(Dunlap, et al. 2004) with slight modification. Briefly, 1 x 10⁶ melanocytes were plated in a well of a 6-well cell culture plate with melanocyte growth medium for 24 hours prior to viral transduction. Cells were infected with a multiplicity of infection (MOI) of 2 and 10 in the presence of 6 ug/ml of polybrene (Sigma, St Louis, Missouri) for 4 hours. Primary melanocytes transduced with MOI of 10 were used for the comparative analysis of expression profiles to detect BRAF downstream effectors. Primary melanocytes transduced with MOI of 2 used for the rest of experiments. Transduced cells were maintained for 5 days in culture prior to the initiation of studies (experimental day 0).

### Proliferation assay

The XTT cell growth assay was carried out using the manufacturer's protocol (Sigma, St. Louis, MO). Melanocytes (0 to 1.6 x 104 cells per well) were seeded in a flat 96-well plate and cultured for 18 hours prior to the measurement of absorbance at 492 nm. Standard curves were generated with the values of absorbance and corresponding cell numbers. For cell proliferation assays, melanocytes (2.0 x 103 per well) were seeded uniformly in a 96 well plate and evaluated by XTT assay. Cell numbers were extrapolated from the standard curves. All experiments were performed in triplicate.

### Antibodies for western blotting.

Cell lysates were prepared from melanocytes on day 4 (early), day 30 (late). Western blotting was performed using standard techniques and reactive proteins were visualized using ECL reagents. Antibodies used included: Raf-B (sc-5284; Santa Cruz), Phospho-MEK1/2 (,,9121, Cell Signaling), MELK1/2 antibody (,,9122, Cell Signaling), p16^{INK4a} (16P04, NeoMarkers), PCNA (sc-56 Santa Crutz), actin (N350, Amersham).

### Telomere fluorescence in-situ hybridization

Cells were grown on chamber slides and fixed on day 30 for 4 hours in 10% neutral buffered formalin at room temperature. The protocol for telomeric DNA FISH performed without protease digestion, as previously described(Meeker et al., 2002. Telomere length assessment in human archival tissues: combined telomere fluorescence in situ hybridization and immunostaining. Am J Pathol 160:1259-1268). Briefly, slides underwent heat-induced antigen retrieval in citrate buffer followed by hybridization with a Cy3-labeled, telomere specific peptide nucleic acid (PNA) probe having the sequence (N-terminus to C-terminus) CCCTAACCCTAACCCTAA with an N-ternninal covalently linked Cy3 fluorescent dye (Applied Biosystems, Framingham, MA). Slides were counterstained with DAPI (4'-6-diamidino-2-phenylindole, Sigma Chemical Co., St. Louis, MO) and imaged with a Zeiss Axioskop epifluorescence microscope equipped with appropriate fluorescence filter sets (Carl Zeiss Inc, Thornwood, NY; Omega Optical, Brattleboro, VT). Telomeric staining produced a speckled pattern of widely distributed nuclear signals in all cells examined. Telomere lengths were evaluated by visual assessment of the fluorescent intensities of the telomeric signals, which are proportional to the length of telomeric TTAGGG DNA repeats (Meeker et al., 2002)

### Expression Profiling of BkAFV600E melanocytes.

Primary human melanocytes (1.0 x 10⁶ per one well of 6-well plate) were transduced by BRAF^{V600E} expressing lentivirus and control virus with MOI of 10 respectively in duplicate (total 4 samples, two samples of primary human melanocytes witbBRAF^{V600E} and two samples of melanocytes with control GFP). Cells were harvested 5 days following infection (experimental day 0) and total RNA was extracted and purified as described previously (Ryu et al. 2007) RNA quality check, double strand complementary DNA synthesis, hybridization with Human Genome U133 Plus 2.0 Array Chips (Affymetrix Inc. Santa Clara, CA), initial data extraction, normalization using RMAExpress software (Irizarry, et al. 2003) statistical analysis for the identification of differentially expressed genes were performed at the Johns Hopkins Medical Institute Micro array Core Facility (on the world wide web at microarray.jhzni.edu). Expression data from one sample of melanocyte with BRAF^{V600E} were discarded because of irregular hybridization. Comparative analysis of gene expression profiles for the identification differently expressed genes and statistical analysis for p-value calculation, therefore, was performed with the expression data from one sample of melanocytes with BRAF^{V600E} and two samples of melanocytes with GFP control. Gene Ontology analysis and network target mapping to detect cellular processes that may be regulated by the BRAF downstream effectors were performed using SPOTFIRE (Spotfire Inc., Cambridge, MA, USA) and Pathway Architect software (Stratagege, La Jolla, CA) respectively.

The RNA samples were analyzed with Affymetrix GeneChip human U133 Plus 2.0 Arrays. Quality of the microarray experiment was assessed with affyPLM and Affy, two Bioconductor packages for statistical analysis of microarray data. To estimate the gene expression signals, data analysis was conducted on the chips* CEL file probe signal values at the Affymetrix probe pair (perfect match (PM) probe and mismatch (MM) probe) level, using the statistical algorithm Robust Multiarray Analysis (RMA) expression measure (Irizarry et al. (2003) Exploration, Normalization, and Summaries of High Density Oligonucleotide Array Probe Level Data. Biostatistics. Vol. 4, Number 2: 249-264) with Affy. This probe level data processing includes a normalization procedure utilizing quantile normalization method (Bolstad et al., 2003 A Comparison of Normalization Methods for High Density Oligonucleotide Array Data Based on Bias and Variance Bioinformatics. 19(2):185-193) to reduce the obscuring variation between microarrays, which might be introduced during the processes of sample preparation, manufacture, fluorescence labeling, hybridization and/or scanning.

Exploratory data analysis (EDA) was performed with the preprocessed data above. Between-treatment and between-replicate variations were examined with the pair-wise MvA plots, in which the base 2 log ratios

(M) between two samples are plotted against their averaged base 2 log signals (A). With the signal estimates, Multidimensional Scaling (MDS) analysis was also performed to assess sample variability. The quality assessment and MDS analyses identified and disqualified a discordant sample chip, Braf-1a.

Upon excluding the discordant chip, the signal data were obtained with the remaining chips using the RMA algorithm above. With the signal intensities estimated above, an empirical Bayes method implemented in the bioconductor package EBarrays, was attempted with both the Gamma-Gamma and lognormal-normal modeling methods to estimate the posterior probabilities of the differential expression of genes between the sample conditions (Newton et al. 2001 On differential variability of expression ratios: Improving statistical inference about gene expression changes from microarray data. Journal of Computational Biology 8 37-52; Kendziorski et al. 2003. On parametric empirical Bayes methods for comparing multiple groups using replicated gene expression profiles. Statistics in Medicine 22 3899-3914; Newton, 2003).

Though the Gamma-Gamma modeling fits better than the lognormal-normal modeling, both parametric modeling methods fit poorly. It has been shown in a simulation study on a yeast example that in spite of poor fit, the resulting inference methods from the parametric modeling have good operating characteristics. (Newton et al., 2004 Detecting differential gene expression with a semiparametric hierarchical mixture method. Biostatistics 5,155-176) Thus, the posterior probability from the Gamma-Gamma modeling might provide a way to make inferences on differential gene expression in this study in spite of the poor fit. Specifically, the criterion of the posterior probability > 0.5, which means the posterior odds favoring change, was used to produce the differentially expressed gene list.

All Bioconductor packages are available on the world wide web at www.bioconductor.org and all computation was performed under R environment (on the world wide web at www.r-project.org; Ihaka and Gentleman: A language for data analysis and graphics. Journal of Computational and Graphical Statistics, 5(3): 299-314, 1996).

### MMP-1 siRNA

The MMP-1 siRNA (5'-gagcaagatgtggacttag-3') and scrambled siRNA (5'-gattcaggtgtagaacgag-3') sequences were designed using Dharmacon siDESIGN Center. Transfection of the siRNAs was performed using 2 µL of Dharma*FECT* 1 transfection reagent (Dharmacon) and 100 nM of MMP-1 siRNA and scrambled siRNA for the WM852 cell line and 250 nM for the WM793 cell line. Cells were collected and pelleted 24 hours after transfection for RNA extraction using the RNeasy Mini Kit (Invitrogen), cDNA production by using SUPERSCRIPT First-Strand Synthesis System for RT-PCR according to manufacturer's instructions (Invitrogen), and semi-quantitative duplex PCR to confirm MMP-1 knockdown.

### Semi-quantitative duplex PCR

Semi-quantitative duplex RT-PCR was performed by a MJ Research Programmable Thermal Controller (PTC-100, Inc.) and the amplified products were separated on an agarose gel. The duplex PCR utilized 20 bp oligonucleotides to amplify regions of~350 bp from MMP-1. Optimization experiments were conducted to determine a favorable primer concentration for the GAPDH internal control. The following prime-oligonucleotides were used: MMP-1 forward {5'-tgcttgaccctcagacagct-3'), MMP-1 reverse (5'-gatgggaggcaagttgaaaa-3'), GAPDH forward (5'-gatcatcagcaatgcctcct-3'), and GAPDH reverse (5'-ttcagctcagggatgacctt-3'). The PCR was carried out in a total volume of 25 uL, containing 2.5 uL of 10X PCR Buffer (containing 15 mM MgCl2), 0.2 mM dNTPs, and 0.3 ul AmpliTaq Gold DNA Polymerase (Applied Biosystems). Forty amplification cycles were performed by an MJ Research Programmable Thermal Controller (PTC-100, Inc.), using a denaturing temperature of 95°C for 30 seconds, an annealing temperature of 55°C for 30 seconds, and primer extension at 72°C for 25 seconds. Following amplification, 25 uL of the samples were separated via electrophoresis on a 2% agarose gel.

### ³H-thymidine Cell Proliferation Assay

WM852 and WM793 cell lines were seeded at 15,000 cells per well into a 96-well tissue culture plate (Becton Dickinson) 24 hours after siRNA transfection. Twenty-two hours later, 10 µL of 0.1 µCi/µL ³H-thymidine (Perkin Elmer) was added to each well. After 4 hours, the cells were washed with PBS and 50 µL of trypsin was added into each well for 10 minutes. The trypsinized cells were harvested to a filtermat using the Perkin Elmer FilterMate cell harvester (Perkin Elmer). The dried filtermat was sealed in a sample bag with 4 mL of scintillation fluid and then read on a Perkin Elmer 1450 Microbeta Liquid Scintillator (Perkin Elmer).

### Total MMP-1 concentration and Activity Assay

Cell culture media with no serum was added to the cell lines. After 72 hours, the media was collected and the protein concentration was determined using the Bio-Rad Protein Assay reagent (Bio-Rad Laboratories). The amount of human MMP-1 pro and active forms was determined using the RAYBIO Human MMP-1 ELISA kit (RayBiotech, Inc.) following the manufacturer's instructions. MMP-1 activity in the collected media was measured using the SensoLyte Plus™ 520 MMP-1 Assay Kit (AnaSpec) following the manufacturer's protocol including the step to add 4-aminophenylmecuric acetate (APMA) to the samples in order to activate all the pro-MMP-1.

### Other Embodiments

From the foregoing description, it will be apparent that variations and modifications may be made to the invantion described herein to adopt it to various usages and conditions. Such embodiments are also within the scope of the following claims.

The recitation of a listing of elements in any definition of a variable herein includes definitions of that variable as any single element or combination (or subcombination) of listed elements. The recitation of an embodiment herein includes that embodiment as any single embodiment or in combination with any other embodiments or portions thereof.

All patents and publications mentioned in this specification are herein incorporated by reference to the same extent as if each independent patent and publication was specifically and individually indicated to be incorporated by reference.

## Claims

1. A panel of biomarkers comprising matrix metallopeptidase 1 (MMP-1).

2. The panel of biomarkers according to claim 1, further comprising
i) CXCL1, IL-8, IGFBP3 and CXCL2, or
ii) CXCL1, CXCL2, IL-8 and IL-6, or
iii) serpinB2, amphiregulin, CXCL5, IL-8, RAP1a and epiregulin, or
iv) CDH3, KIT, DPP4 and SYK.

3. Use of a panel of biomarkers comprising matrix metallopeptidase 1 (MMP-1) in an in vitro method of diagnosing melanoma status wherein the panel of biomarkers is correlated with a stage of melanoma progression in a subject.

4. The use of claim 3, further comprising
i) CXCL I, IL-8, IGFBP3 and CXCL2, or
ii) CXCL1, CXCL2, IL-8 and IL-6, or
iii) serpinB2, amphiregulin, CXCL5, IL-8, RAP1a and epiregulin, or
iv) CDH3, KIT, DPP4 and SYK.

5. The use of claim 3, wherein the melanoma is one or more of in situ, radial growth phase, vertical growth phase, metastatic melanoma.

6. The use of claim 3, wherein melanoma progression is further defined as a stage selected from the group consisting of: stage I, stage II, stage III and stage IV.

7. The use of claim 3, wherein the biomarker is further used to determine the malignant potential of a melanocytic tumor of undetermined malignant potential, or a tumor of undetermined classification.

8. The use of one of claims 1 to 7, wherein the subject has melanoma with a BRAF mutation.

9. An in vitro method for predicting the invasiveness of a melanoma by screening melanoma-endothelial cell communication of a melanoma in a subject comprising:
(a) performing an in vitro two-dimensional co-culture system of melanoma-cells and endothelial cells, wherein the different cells are separated by a physical barrier;
(b) measuring expression of biomarkers comprising matrix metallopeptidase 1 (MMP-1) in a sample of the melanoma cells after co-culture; and
(c) correlating the biomarker expression with melanoma invasiveness, thereby predicting the invasiveness of a melanoma in a subject.

10. The method of claim 9, wherein the subject is a subject that has previously been diagnosed with melanoma or a subject that has previously been treated for melanoma.

11. The method of claim 9, wherein the subject diagnosed with melanoma carries a BRAF mutation.

12. An in vitro method for detecting melanoma recurrence comprising determining the biomarker according to claim 1 in the serum of a subject.

13. A kit for aiding the diagnosis of melanoma, comprising:
a panel of capture reagents that bind biomarkers comprising matrix metallopeptidase 1 (MMP-1), wherein each capture reagent within the panel selectively binds a single biomarker, and written instructions for use of the kit for detection of melanoma.

14. The kit of claim 13, further comprising a panel of capture reagents that bind
i) CXCL1, IL-8, IGFBP3 and CXCL2, or
ii) CXCL1, CXCL2, IL-8 and IL-6, or
iii) serpinB2, amphiregulin, CXCL5, IL-8, RAP1a and epiregulin, or
iv) CDH3, KIT, DPP4 and SYK.

15. The kit of claim 13, wherein the capture reagent comprises an antibody or an antigen-binding fragment thereof, a single stranded oligonucleotide, a double stranded oligonucleotide, a protein, or a peptide.
